# EUROPEAN PATENT SPECIFICATION

(11) **EP 2 831 111 B1**
(45) Date of publication and mention of the grant of the patent: **20.03.2019**
(21) Application number: 13718796.9
(22) Date of filing: 27.03.2013
(51) Int. Cl.: C07K 16/22, A61K 39/395, A61P 35/00

(54) **ANG2-BINDING MOLECULES**
ANG2 BINDENDE MOLEKÜLE
MOLÉCULES LIANT ANG2

(30) Priority: 30.03.2012 EP 12162615
(43) Date of publication of application: 04.02.2015
(73) Proprietor: Boehringer Ingelheim International GmbH, 55216 Ingelheim (DE)
(72) Inventor: BORGES, Eric, 55216 Ingelheim Am Rhein (DE); GSCHWIND, Andreas, 55216 Ingelheim Am Rhein (DE); OTT, Rene Georg, 55216 Ingelheim Am Rhein (DE); BUYSE, Marie-Ange, 9820 Merelbeke (BE); BOUCNEAU, Joachim, 9840 De Pinte (BE); MERCHIERS, Pascal, 2460 Kasterlee (BE); DEPLA, Erik, 9070 Destelbergen (BE); STEVENAERT, Frederik, 9680 Maarkedal (BE)
(74) Representative: Weik, Steffen
(86) International application number: PCT/EP2013/056635
(87) International publication number: WO 2013/144266

(56) References cited:
- WO-A2-2009/147248
- WO-A2-2010/066836
- US-A1- 2011 027 286
- WARK K L ET AL: "Latest technologies for the enhancement of antibody affinity", ADVANCED DRUG DELIVERY REVIEWS, ELSEVIER BV, AMSTERDAM, NL, vol. 58, no. 5-6, 7 August 2006 (2006-08-07), pages 657-670, XP024892147, ISSN: 0169-409X, DOI: 10.1016/J.ADDR.2006.01.025 [retrieved on 2006-08-07]
- CARTER P J: "POTENT ANTIBODY THERAPEUTICS BY DESIGN", NATURE REVIEWS. IMMUNOLOGY, NATURE PUBLISHING GROUP, GB, vol. 6, 7 April 2006 (2006-04-07), pages 343-357, XP007901440, ISSN: 1474-1733, DOI: 10.1038/NRI1837
- REVETS H ET AL: "NANOBODIES AS NOVEL AGENTS FOR CANCER THERAPY", EXPERT OPINION ON BIOLOGICAL THERAPY, ASHLEY, LONDON, GB, vol. 5, no. 1, 1 January 2005 (2005-01-01) , pages 111-124, XP009076361, ISSN: 1471-2598, DOI: 10.1517/14712598.5.1.111

## Description

### FIELD OF THE INVENTION

The invention relates to the field of human therapy, in particular cancer therapy and agents and compositions useful in such therapy.

### BACKGROUND OF THE INVENTION

As described in e.g. US 2008/0014196, WO 2008101985 and US 2011/0027286, angiogenesis is the biological process whereby new blood vessels are formed and being implicated in the pathogenesis of a number of disorders, including solid tumors and metastasis as well as eye diseases. When tumors reach a critical size of approximately 1 mm³ they become dependent on angiogenesis for maintaining blood supply with oxygen and nutritients to allow for further growth. Anti-angiogenesis therapies have become an important treatment option for several types of tumors.

One of the most important pro-angiogenic factors is Angiopoietin2 (Ang2), a ligand of the Tie2 receptor (Tie2), which controls vascular remodeling by enabling the functions of other angiogenic factors, such as VEGF. Ang2 is also referred to in the art as Tie2 ligand (US. Pat. No. 5,643,755, Yancopoulos et al. 2000, Nature 407: 242-248).

Ang2 is primarily expressed by endothelial cells, strongly induced by hypoxia and other angiogenic factors and has been demonstrated to regulate tumor vessel plasticity, allowing vessels to respond to VEGF and FGF2 (Augustin et al., Nat Rev Mol Cell Biol. 2009 Mar;10(3):165-77). Consistent with this role, the deletion or inhibition of Ang2 results in reduced angiogenesis (Falcón et al., Am J Pathol. 2009 Nov;175(5):2159-70). Elevated Ang2 serum concentrations have been reported for patients with colorectal cancer, NSCLC and melanoma (Goede et al., Br J Cancer. 2010 Oct 26;103(9):1407-14),(Park et al., Chest. 2007 Jul;132(1): 200-6),(Helfrich et al., Clin Cancer Res. 2009 Feb 15;15(4):1384-92). In CRC cancer Ang2 serum levels correlate with therapeutic response to anti-VEGF therapy.

The Ang-Tie system consists of 2 receptors (Tie1 and Tie2) and 3 ligands (Ang1, Ang2 and Ang4) (Augustin et al., Nat Rev Mol Cell Biol. 2009 Mar;10(3):165-77). Tie2, Ang1 and Ang2 are the best studied members of this family, Tie1 is an orphan receptor and the role of Ang4 for vascular remodelling still needs to be defined. Ang2 and Ang1 mediate opposing functions upon Tie2 binding and activation. Ang2-mediated Tie2 activation results in endothelial cell activation, pericyte dissociation, vessel leakage and induction of vessel sprouting. In contrast to Ang2, Ang1 signaling maintains vessel integrity by recruitment of pericytes, thereby maintaining endothelial cell quiescence.

Angiopoietin 2 (Ang2) is a secreted, 66 kDa ligand for the Tie2 receptor tyrosine kinase (Augustin et al., Nat Rev Mol Cell Biol. 2009 Mar;10(3):165-77). Ang2 consists of an N-terminal coiled-coil domain and a C-terminal fibrinogen-like domain, the latter is required for Tie2 interaction. Ang2 is primarily expressed by endothelial cells and strongly induced by hypoxia and other angiogenic factors, including VEGF. Tie2 is found on endothelial cells, haematopoietic stem cells and tumor cells. Ang2-Tie2 has been demonstrated to regulate tumor vessel plasticity, allowing vessels to respond to VEGF and FGF2.

*In vitro* Ang2 has been shown to act as a modest mitogen, chemoattractant and inducer of tube formation in human umbilical vein endothelial cells (HUVEC). Ang2 induces tyrosine phosphorylation of ectopically expressed Tie2 in fibroblasts and promotes downstream signaling events, such as phosphorylation of ERK-MAPK, AKT and FAK in HUVEC. An antagonistic role of Ang2 in Ang1-induced endothelial cell responses has been described.

Ang2 -deficiency has been shown to result in a profound lymphatic patterning defect in mice. Although the loss of Ang2 is dispensable for embryonic vascular development, Ang2 -deficient mice have persistent vascular defects in the retina and kidney. Together with the dynamic pattern of Ang2 expression at sites of angiogenesis (for example ovary), these findings indicate that Ang2 controls vascular remodeling by enabling the functions of other angiogenic factors, such as VEGF.

The Ang2-Tie2 system exerts crucial roles during the angiogenic switch and later stages of tumor angiogenesis. Ang2 expression is strongly up-regulated in the tumor-associated endothelium. Reduced growth of tumors has been observed when implanted into Ang2 -deficient mice, especially during early stages of tumor growth. Therapeutic blocking of Ang2 with Ang2 mAbs has shown broad efficacy in a variety of tumor xenograft models.

As summarized in the above mentioned US 2008/0014196, angiogenesis is implicated in the pathogenesis of a number of disorders, including solid tumors and metastasis.

In the case of tumor growth, angiogenesis appears to be crucial for the transition from hyperplasia to neoplasia, and for providing nourishment for the growth and metastasis of the tumor. Folkman et al., Nature 339 -58 (1989), which allows the tumor cells to acquire a growth advantage compared to the normal cells. Therefore, anti-angiogenesis therapies have become an important treatment option for several types of tumors. These therapies have focused on blocking the VEGF pathway (Ferrara et al., Nat Rev Drug Discov. 2004 May;3(5):391-400)).

Antibodies and other peptide inhibitors that bind to Ang2 and Ang1 are mentioned in e.g. U.S. Patent Nos 6,166,185; 7,521,053; 7,205,275; and US Patent Application Nos.: 2006/0018909 and 2006/0246071. Furthermore, US 2011/0027286 discloses specific monoclonal Ang2 antibodies which do not antagonize the related molecule Ang1.

However, the state-of-the art monoclonal antibodies (MAbs) and fusion proteins have several shortcomings in view of their therapeutic application: To prevent their degradation, they must be stored at near freezing temperatures. Also, since they are quickly digested in the gut, they are not suited for oral administration. Another major restriction of MAbs for cancer therapy is poor transport, which results in low concentrations and a lack of targeting of all cells in a tumor.

It has been an object of the present invention to provide novel improved Ang2-binding molecules, i.e. nanobodies that block binding of Ang2 to Tie2 but not the binding of Ang1 to Tie2.

More in particular, it has been object of the invention to provide novel Ang2- binding molecules, and, specifically, Ang2-binding molecules that bind to mammalian Ang2 but not to mammalian Ang1 and, especially, to human Ang2 but not to human Ang1, wherein such molecules or polypeptides are suitable for the therapeutic and diagnostic purposes as described herein. It has been a further object of the invention to provide immunoglobulin single variable domains that specifically bind to Ang2 but not to Ang1.

Such Ang2- binding molecules, or Ang2 antagonists, are useful as pharmacologically active agents in compositions in the prevention, treatment, alleviation and/or diagnosis of diseases or conditions associated with Ang2-mediated effects on angiogenesis.

Examples for such diseases are cancer or cancerous diseases such as breast cancer, renal cell carcinoma, ovarian cancer and pancreatic cancer, eye diseases such as age-related macular degeneration and diabetic retinopathy, and/or chronic kidney diseases such as diabetic nephropathy, postrenal failure, prerenal azotemia and intrinsic renal failure.

It has been a further object of the invention to provide methods for the prevention, treatment, alleviation and/or diagnosis of such diseases, disorders or conditions, involving the use and/or administration of such Ang2-binding molecules and compositions comprising them. In particular, it is has been an object of the invention to provide such pharmacologically active Ang2-binding molecules, compositions and/or methods that provide advantages compared to the agents, compositions and/or methods currently used and/or known in the art. These advantages include improved therapeutic and/or pharmacological properties and/or other advantageous properties, e.g. for manufacturing purposes, especially as compared to conventional antibodies as those described above, or fragments thereof.

### BRIEF SUMMARY OF THE INVENTION

According to a first aspect, there is provided an Ang2-binding molecule, comprising an immunoglobulin single variable domain, wherein said immunoglobulin single variable domain comprises three complementarity determining regions CDR1, CDR2 and CDR3, wherein CDR1 has an amino acid sequence shown in SEQ ID NO: 168, CDR2 has an amino acid sequence shown in SEQ ID No: 171 and CDR3 has an amino acid sequence shown in SEQ ID NO: 175.

The invention further relates to an Ang2-binding molecule consisting of said immunoglobulin single variable domain.

Furthermore, the invention relates to an Ang2-binding molecule having a sequence according to SEQ ID No: 166.

According to another aspect, there is provided a nucleic acid encoding said Ang2-binding molecule as well as an expression vector comprising said nucleci acid.

The invention further relates to a host cell carrying one or more expression vectors comprising said nucleic acids.

The invention further relates to a method for producing or generating an Ang2-binding molecule according to the invention, comprising the steps of:
(a) transfecting a host cell with one or more said vectors comprising said nucleic acid molecule,
(b) culturing said host cell, and
(c) recovering and purifying said Ang2-binding molecule.

Further aspect of the invention relates to a pharmaceutical composition comprising, as the active ingredient, one or more said Ang2-binding molecules as defined above and at least a physiologically acceptable carrier

The invention further relates to applications and uses of the Ang2-binding molecules, nucleic acids, host cells, products and compositions described above, as well as to methods for the prevention and/or treatment for diseases associated with Ang2-mediated effects on angiogenesis, preferably cancer, cancerous diseases and eye diseases using the Ang2-binding molecules as defined above.

These and other aspects, embodiments, advantages and applications of the invention will become clear from the further description hereinbelow.

### DEFINITIONS

Unless indicated or defined otherwise, all terms used have their usual meaning in the art, which will be clear to the skilled person. Reference is for example made to the standard handbooks, such as Sambrook et al, "Molecular Cloning: A Laboratory Manual" (2nd Ed.), Vols. 1-3, Cold Spring Harbor Laboratory Press (1989); Lewin, "Genes IV", Oxford University Press, New York, (1990), and Roitt et al., "Immunology" (2nd Ed.), Gower Medical Publishing, London, New York (1989), as well as to the general background art cited herein. Furthermore, unless indicated otherwise, all methods, steps, techniques and manipulations that are not specifically described in detail can be performed and have been performed in a manner known *per se,* as will be clear to the skilled person. Reference is for example again made to the standard handbooks, to the general background art referred to above and to the further references cited therein.

The term *"angiopoetin-2"* or *"Ang2",* unless specified as being from non-human species (e.g. "mouse Ang2", etc) refers to human Ang2 or a biologically active fragment thereof (e.g. a fragment of the Ang2 protein which is capable of inducing angiogenesis *in vitro* or *in vivo*), i.e. to human Ang2 (variant 1) with accession no. NM 001147.2, to human Ang2 (variant 2) having accession no. NM_001118887.1 or to human Ang2 (variant 3) having accession no. NM_001118888.1, and/or their biologically active fragments thereof. The amino acid sequences of Ang2 non human species, such as mouse Ang2 and cyno Ang2 are avialable from protein sequence database under Accession No: NM_007426.3 (SEQ ID NO: (SEQ ID 188) and AB172643.1 (SEQ ID NO: 187), respectively.

The term *"angiopoietin-1"* or *"Ang1",* unless specified as being from non-human species (e.g. "mouse Ang2", etc) refers to human Ang1 or a biologically active fragment thereof (e.g. a fragment of the Ang1 protein which is capable of inducing angiogenesis *in vitro* or *in vivo*), i.e. to human Ang1 with accession no. NM_001146.3, or a biologically active fragment thereof.

Unless indicated otherwise, the terms *"immunoglobulin"* and *"immunoglobulin sequence"* - whether used herein to refer to a heavy chain antibody or to a conventional 4-chain antibody - are used as general terms to include both the full-size antibody, the individual chains thereof, as well as all parts, domains or fragments thereof (including but not limited to antigen-binding domains or fragments such as VHH domains or VH/VL domains, respectively). In addition, the term "sequence" as used herein (for example in terms like "immunoglobulin sequence", "antibody sequence", "(single) variable domain sequence", "VHH sequence" or "protein sequence"), should generally be understood to include both the relevant amino acid sequence as well as nucleic acid sequences or nucleotide sequences encoding the same, unless the context requires a more limited interpretation.

The term *"domain"* (of a polypeptide or protein) as used herein refers to a folded protein structure which has the ability to retain its tertiary structure independently of the rest of the protein. Generally, domains are responsible for discrete functional properties of proteins and in many cases may be added, removed or transferred to other proteins without loss of function of the remainder of the protein and/or of the domain.

The term *"immunoglobulin domain"* as used herein refers to a globular region of an antibody chain (such as e.g. a chain of a conventional 4-chain antibody or of a heavy chain antibody), or to a polypeptide that essentially consists of such a globular region. Immunoglobulin domains are characterized in that they retain the immunoglobulin fold characteristic of antibody molecules, which consists of a 2-layer sandwich of about 7 antiparallel beta-strands arranged in two beta-sheets, optionally stabilized by a conserved disulphide bond.

The term *"immunoglobulin variable domain"* as used herein means an immunoglobulin domain essentially consisting of four "framework regions" which are referred to in the art and hereinbelow as "framework region 1" or "FR1"; as "framework region 2" or"FR2"; as "framework region 3" or "FR3"; and as "framework region 4" or "FR4", respectively; which framework regions are interrupted by three "complementarity determining regions" or "CDRs", which are referred to in the art and hereinbelow as "complementarity determining region 1"or "CDR1"; as "complementarity determining region 2" or "CDR2"; and as "complementarity determining region 3" or "CDR3", respectively. Thus, the general structure or sequence of an immunoglobulin variable domain can be indicated as follows: FR1 - CDR1 - FR2 - CDR2 - FR3 - CDR3 - FR4. It is the immunoglobulin variable domain(s) that confer specificity to an antibody for the antigen by carrying the antigen-binding site.

The term *"immunoglobulin single variable domain"* as used herein means an immunoglobulin variable domain which is capable of specifically binding to an epitope of the antigen without pairing with an additional variable immunoglobulin domain. One example of immunoglobulin single variable domains in the meaning of the present invention are "domain antibodies", such as the immunoglobulin single variable domains VH and VL (VH domains and VL domains). Another example of immunoglobulin single variable domains are "VHH domains" (or simply "VHHs") from camelids, as defined hereinafter.

In view of the above definition, the antigen-binding domain of a conventional 4-chain antibody (such as an IgG, IgM, IgA, IgD or IgE molecule; known in the art) or of a Fab fragment, a F(ab')2 fragment, an Fv fragment such as a disulphide linked Fv or a scFv fragment, or a diabody (all known in the art) derived from such conventional 4-chain antibody, would normally not be regarded as an immunoglobulin single variable domain, as, in these cases, binding to the respective epitope of an antigen would normally not occur by one (single) immunoglobulin domain but by a pair of (associating) immunoglobulin domains such as light and heavy chain variable domains, i.e. by a VH-VL pair of immunoglobulin domains, which jointly bind to an epitope of the respective antigen.

*"VHH domains",* also known as VHHs, V_{H}H domains, VHH antibody fragments, and VHH antibodies, have originally been described as the antigen binding immunoglobulin (variable) domain of "heavy chain antibodies" (i.e. of "antibodies devoid of light chains"; Hamers-Casterman C, Atarhouch T, Muyldermans S, Robinson G, Hamers C, Songa EB, Bendahman N, Hamers R.: "Naturally occurring antibodies devoid of light chains"; Nature 363, 446-448 (1993)). The term "VHH domain" has been chosen in order to distinguish these variable domains from the heavy chain variable domains that are present in conventional 4-chain antibodies (which are referred to herein as "V_{H} domains" or "VH domains") and from the light chain variable domains that are present in conventional 4-chain antibodies (which are referred to herein as "V_{L} domains" or "VL domains"). VHH domains can specifically bind to an epitope without an additional antigen binding domain (as opposed to VH or VL domains in a conventional 4-chain antibody, in which case the epitope is recognized by a VL domain together with a VH domain). VHH domains are small, robust and efficient antigen recognition units formed by a single immunoglobulin domain.

In the context of the present invention, the terms VHH domain, VHH, V_{H}H domain, VHH antibody fragment, VHH antibody, as well as "Nanobody®" and "Nanobody® domain" ("Nanobody" being a trademark of the company Ablynx N.V.; Ghent; Belgium) are used interchangeably and are representatives of immunoglobulin single variable domains (having the structure FR1-CDR1-FR2-CDR2-FR3-CDR3-FR4 and specifically binding to an epitope without requiring the presence of a second immunoglobulin variable domain), and which are distinguished from VH domains by the so-called "hallmark residues", as defined in e.g. WO2009/109635, Fig. 1.

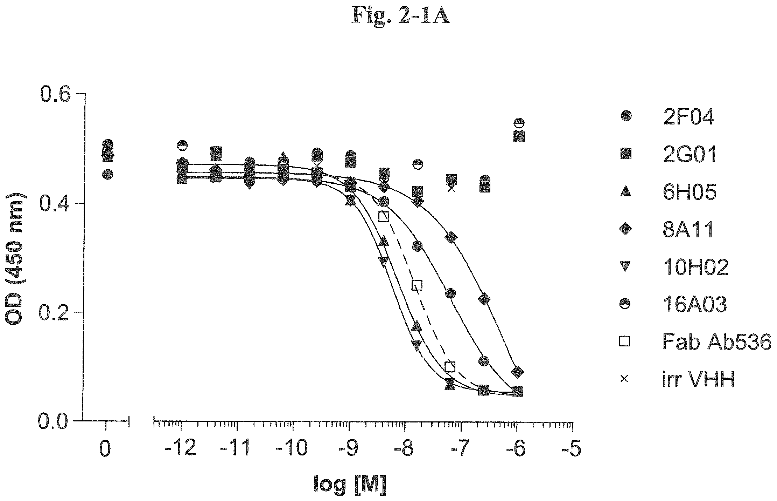

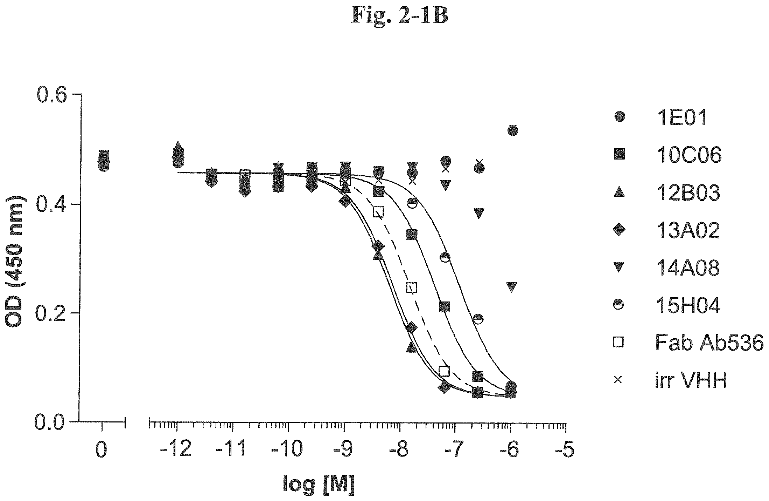

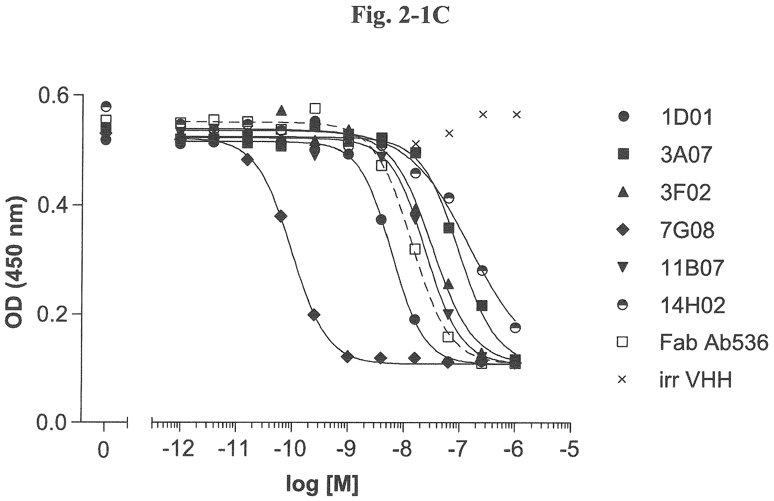

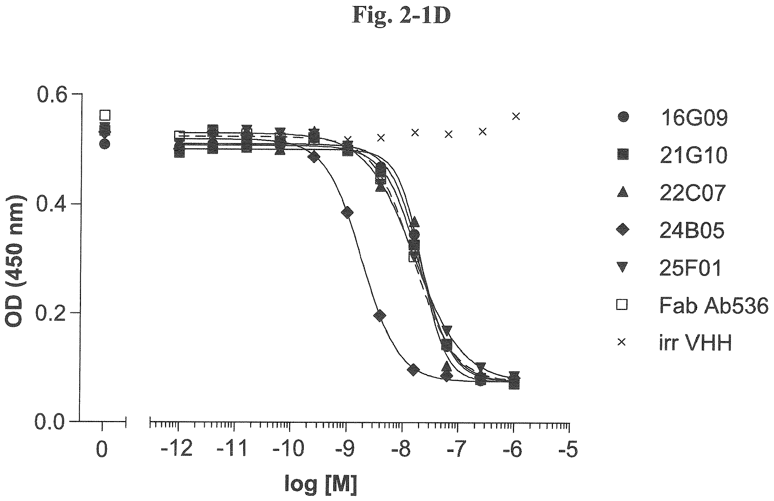

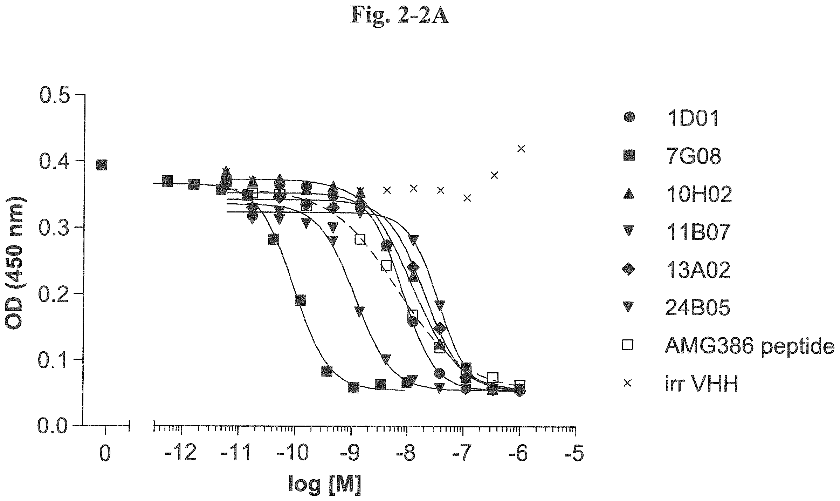

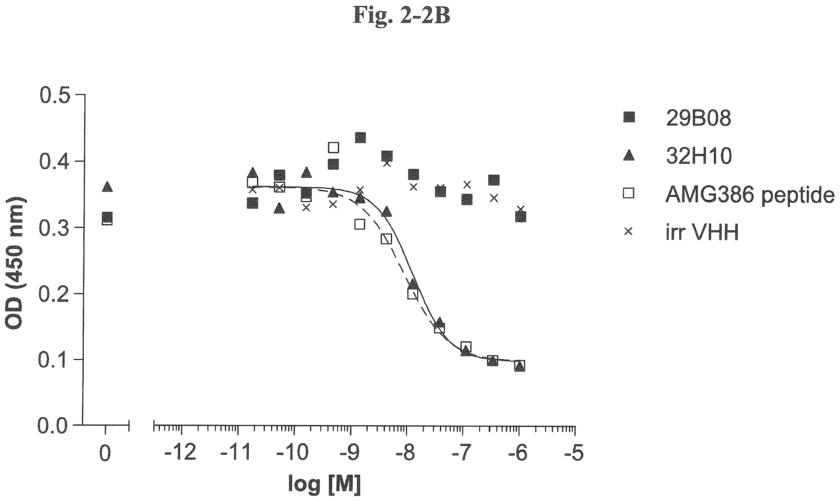

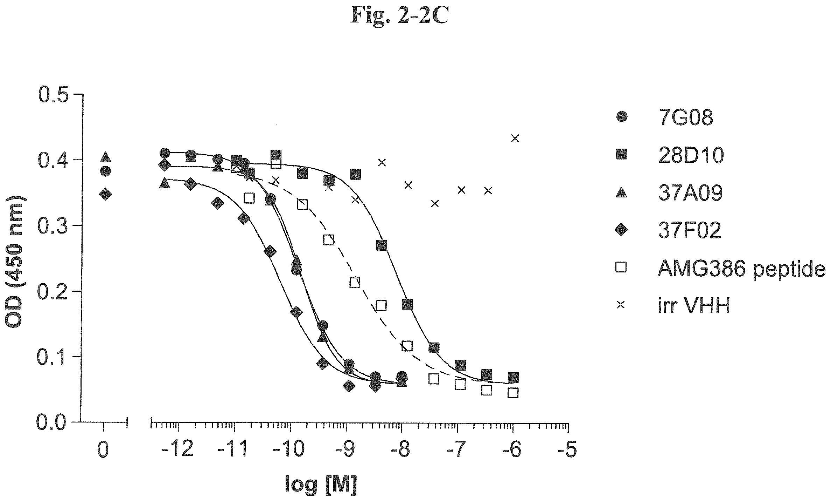

The amino acid residues of a immunoglobulin single variable domain, e.g. a VHH, are numbered according to the general numbering for V_{H} domains given by Kabat et al. ("Sequence of proteins of immunological interest", US Public Health Services, NIH Bethesda, MD, Publication No. 91), as applied to VHH domains from Camelids, as shown e.g. in Figure 2 of Riechmann and Muyldermans, J. Immunol. Methods 231, 25-38 (1999).

According to this numbering,
- FR1 comprises the amino acid residues at positions 1-30,
- CDR1 comprises the amino acid residues at positions 31-35,
- FR2 comprises the amino acids at positions 36-49,
- CDR2 comprises the amino acid residues at positions 50-65,
- FR3 comprises the amino acid residues at positions 66-94,
- CDR3 comprises the amino acid residues at positions 95-102, and
- FR4 comprises the amino acid residues at positions 103-113.

However, it should be noted that - as is well known in the art for V_{H} domains and for VHH domains - the total number of amino acid residues in each of the CDRs may vary and may not correspond to the total number of amino acid residues indicated by the Kabat numbering (that is, one or more positions according to the Kabat numbering may not be occupied in the actual sequence, or the actual sequence may contain more amino acid residues than the number allowed for by the Kabat numbering). This means that, generally, the numbering according to Kabat may or may not correspond to the actual numbering of the amino acid residues in the actual sequence.

Alternative methods for numbering the amino acid residues of V_{H} domains, which methods can also be applied in an analogous manner to VHH domains, are known in the art. However, in the present description, claims and figures, the numbering according to Kabat and applied to VHH domains as described above will be followed, unless indicated otherwise.

The total number of amino acid residues in a VHH domain will usually be in the range of from 110 to 120, often between 112 and 115. It should however be noted that smaller and longer sequences may also be suitable for the purposes described herein.

Immunoglobulin single variable domains, e.g. VHHs and domain antibodies, according to the preferred embodiments of the invention as defined above, have a number of unique structural characteristics and functional properties which makes them highly advantageous for use in therapy as functional antigen-binding molecules. In particular, and without being limited thereto, VHH domains (which have been "designed" by nature to functionally bind to an antigen without pairing with a light chain variable domain) can function as single, relatively small, functional antigen-binding structural units.

Due to their unique properties, immunoglobulin single variable domains, as defined herein, like VHHs or VHs (or VLs) - either alone or as part of a larger polypeptide, e.g. a biparatopic molecule - offer a number of significant advantages:
- only a single domain is required to bind an antigen with high affinity and with high selectivity, so that there is no need to have two separate domains present, nor to assure that these two domains are present in the right spacial conformation and configuration (i.e. through the use of especially designed linkers, as with scFv's);
- immunoglobulin single variable domains can be expressed from a single nucleic acid molecule and do not require any post-translational modification (like glycosylation);
- immunoglobulin single variable domains can easily be engineered into multivalent and multispecific formats (as further discussed herein);
- immunoglobulin single variable domains have high specificity and affinity for their target, low inherent toxicity and can be administered via alternative routes than infusion or injection;
- immunoglobulin single variable domains are highly stable to heat, pH, proteases and other denaturing agents or conditions and, thus, may be prepared, stored or transported without the use of refrigeration equipments;
- immunoglobulin single variable domains are easy and relatively inexpensive to prepare, both on small scale and on a manufacturing scale. For example, immunoglobulin single variable domains can be produced using microbial fermentation (e.g. as further described below) and do not require the use of mammalian expression systems, as with for example conventional antibodies;
- immunoglobulin single variable domains are relatively small (approximately 15 kDa, or 10 times smaller than a conventional IgG) compared to conventional 4-chain antibodies and antigen-binding fragments thereof, and therefore show high(er) penetration into tissues (including but not limited to solid tumors and other dense tissues) and can be administered in higher doses than such conventional 4-chain antibodies and antigen-binding fragments thereof;
- VHHs have specific so-called "cavity-binding properties" (inter alia due to their extended CDR3 loop, compared to VH domains from 4-chain antibodies) and can therefore also access targets and epitopes not accessible to conventional 4-chain antibodies and antigen-binding fragments thereof;
- VHHs have the particular advantage that they are highly soluble and very stable and do not have a tendency to aggregate (as with the mouse-derived antigen-binding domains described by Ward et al., Nature 341: 544-546 (1989)).

The immunoglobulin single variable domains of the invention are not limited with respect to a specific biological source from which they have been obtained or to a specific method of preparation. For example, obtaining VHHs may include the following steps:
(1) isolating the VHH domain of a naturally occurring heavy chain antibody; or screening a library comprising heavy chain antibodies or VHHs and isolating VHHs therefrom;
(2) expressing a nucleic acid molecule encoding a VHH with the naturally occurring sequence;
(3) "humanizing" (as described herein) a VHH, optionally after affinity maturation, with a naturally occurring sequence or expressing a nucleic acid encoding such humanized VHH;
(4) "camelizing" (as described below) a immunoglobulin single variable heavy domain from a naturally occurring antibody from an animal species, in particular a species of mammal, such as from a human being, or expressing a nucleic acid molecule encoding such camelized domain;
(5) "camelizing" a VH, or expressing a nucleic acid molecule encoding such a camelized VH;
(6) using techniques for preparing synthetically or semi-synthetically proteins, polypeptides or other amino acid sequences;
(7) preparing a nucleic acid molecule encoding a VHH domain using techniques for nucleic acid synthesis, followed by expression of the nucleic acid thus obtained;
(8) subjecting heavy chain antibodies or VHHs to affinity maturation, to mutagenesis (e.g. random mutagenesis or site-directed mutagenesis) and/or any other technique(s) in order to increase the affinity and/or specificity of the VHH; and/or
(9) combinations or selections of the foregoing steps.

Suitable methods and techniques for performing the above-described steps are known in the art and will be clear to the skilled person. By way of example, methods of obtaining VHH domains binding to a specific antigen or epitope have been described in WO2006/040153 and WO2006/122786.

According to specific embodiments, the immunoglobulin single variable domains of the invention or present in the polypeptides of the invention are VHH domains with an amino acid sequence that essentially corresponds to the amino acid sequence of a naturally occurring VHH domain, but that has been "humanized" or "sequence-optimized" (optionally after affinity-maturation), i.e. by replacing one or more amino acid residues in the amino acid sequence of said naturally occurring VHH sequence by one or more of the amino acid residues that occur at the corresponding position(s) in a variable heavy domain of a conventional 4-chain antibody from a human being. This can be performed using methods known in the art, which can by routinely used by the skilled person.

A humanized VHH domain may contain one or more fully human framework region sequences, and, in an even more specific embodiment, may contain human framework region sequences derived from the human germline Vh3 sequences DP-29, DP-47, DP-51, or parts thereof, or be highly homologous thereto, optionally combined with JH sequences, such as JH5. Thus, a humanization protocol may comprise the replacement of any of the VHH residues with the corresponding framework 1, 2 and 3 (FRI, FR2 and FR3) residues of germline VH genes such as DP 47, DP 29 and DP 51) either alone or in combination. Suitable framework regions (FR) of the immunoglobulin single variable domains of the invention can be selected from those as set out e.g. in WO 2006/004678 and specifically, include the so-called "KERE" and "GLEW" classes. Examples are immunoglobulin single variable domains having the amino acid sequence G-L-E-W at about positions 44 to 47, and their respective humanized counterparts. A humanized VHH domain may contain one or more fully human framework region sequences.

By way of example, a humanizing substitution for VHHs belonging to the 103 P,R,S-group and/or the GLEW-group (as defined below) is 108Q to 108L. Methods for humanizing immunoglobulin single variable domains are known in the art.

Binding immunoglobulin single variable domains with improved properties in view of therapeutic application, e.g. enhanced affinity or decreased immunogenicity, may be obtained from individual binding molecules by techniques known in the art, such as affinity maturation (for example, starting from synthetic, random or naturally occurring immunoglobulin sequences), CDR grafting, humanizing, combining fragments derived from different immunoglobulin sequences, PCR assembly using overlapping primers, and similar techniques for engineering immunoglobulin sequences well known to the skilled person; or any suitable combination of any of the foregoing, also termed "sequence optimization", as described herein. Reference is, for example, made to standard handbooks, as well as to the further description and Examples.

If appropriate, a binding molecule with increased affinity may be obtained by affinity-maturation of another binding molecule, the latter representing, with respect to the affinity-matured molecule, the "parent" binding molecule.

Methods of obtaining VHHs that bind to a specific antigen or epitope have been described earlier, e.g. in WO2006/040153 and WO2006/122786. As also described therein in detail, VHH domains derived from camelids can be "humanized" (also termed "sequence-optimized" herein, "sequence-optimizing" may, in addition to humanization, encompass an additional modification of the sequence by one or more mutations that furnish the VHH with improved properties, such as the removal of potential post translational modification sites) by replacing one or more amino acid residues in the amino acid sequence of the original VHH sequence by one or more of the amino acid residues that occur at the corresponding position(s) in a VH domain from a conventional 4-chain antibody from a human being. A humanized VHH domain can contain one or more fully human framework region sequences, and, in an even more specific embodiment, can contain human framework region sequences derived from DP-29, DP-47, DP-51, or parts thereof, optionally combined with JH sequences, such as JH5.

*"Domain antibodies",* also known as "Dab"s and "dAbs" (the terms "Domain Antibodies" and "dAbs" being used as trademarks by the GlaxoSmithKline group of companies) have been described in e.g. Ward, E.S., et al.: "Binding activities of a repertoire of single immunoglobulin variable domains secreted from Escherichia coli"; Nature 341: 544-546 (1989); Holt, L.J. et al.: "Domain antibodies: proteins for therapy"; TRENDS in Biotechnology 21(11): 484-490 (2003); and WO2003/002609.

Domain antibodies essentially correspond to the VH or VL domains of antibodies from non-camelid mammals, in particular human 4-chain antibodies. In order to bind an epitope as a single antigen binding domain, i.e. without being paired with a VL or VH domain, respectively, specific selection for such antigen binding properties is required, e.g. by using libraries of human single VH or VL domain sequences.

Domain antibodies have, like VHHs, a molecular weight of approximately 13 to approximately 16 kDa and, if derived from fully human sequences, do not require humanization for e.g. therapeutical use in humans. As in the case of VHH domains, they are well expressed also in prokaryotic expression systems, providing a significant reduction in overall manufacturing cost.

Furthermore, it will also be clear to the skilled person that it is possible to "graft" one or more of the CDR's mentioned above onto other "scaffolds", including but not limited to human scaffolds or non-immunoglobulin scaffolds. Suitable scaffolds and techniques for such CDR grafting are known in the art.

The terms *"epitope"* and *"antigenic determinant",* which can be used interchangeably, refer to the part of a macromolecule, such as a polypeptide that is recognized by antigen-binding molecules, such as conventional antibodies or the polypeptides of the invention, and more particularly by the antigen-binding site of said molecules. Epitopes define the minimum binding site for an immunoglobulin, and thus represent the target of specificity of an immunoglobulin.

A polypeptide (such as an immunoglobulin, an antibody, an immunoglobulin single variable domain of the invention, or generally an antigen-binding molecule or a fragment thereof) that can *"bind to"* or *"specifically bind to",* that *"has affinity for"* and/or that *"has specificity for"* a certain epitope, antigen or protein (or for at least one part, fragment or epitope thereof) is said to be *"against"* or *"directed against"* said epitope, antigen or protein or is a *"binding"* molecule with respect to such epitope, antigen or protein. In this context, an Ang2-binding molecule may also be referred to as *"Ang2-neutralizing".*

Generally, the term *"specificity"* refers to the number of different types of antigens or epitopes to which a particular antigen-binding molecule or antigen-binding protein (such as an immunoglobulin single variable domain of the invention) molecule can bind. The specificity of an antigen-binding molecule can be determined based on its affinity and/or avidity. The affinity, represented by the equilibrium constant for the dissociation of an antigen with an antigen-binding protein (KD), is a measure for the binding strength between an epitope and an antigen-binding site on the antigen-binding protein: the lesser the value of the KD, the stronger the binding strength between an epitope and the antigen-binding molecule (alternatively, the affinity can also be expressed as the affinity constant (KA), which is 1/KD). As will be clear to the skilled person (for example on the basis of the further disclosure herein), affinity can be determined in a manner known per se, depending on the specific antigen of interest. Avidity is the measure of the strength of binding between an antigen-binding molecule (such as an immunoglobulin, an antibody, an immunoglobulin single variable domain or a polypeptide containing it and the pertinent antigen. Avidity is related to both the affinity between an epitope and its antigen binding site on the antigen-binding molecule and the number of pertinent binding sites present on the antigen-binding molecule.

The part of an antigen-binding molecule (such as an antibody or an immunoglobulin single variable domain of the invention) that recognizes the epitope is called a *"paratope".*

Unless indicated otherwise, the term *"Ang2-binding molecule"* includes anti- Ang2 antibodies, anti- Ang2 antibody fragments, *"anti- Ang2 antibody-like molecules"* and conjugates with any of these. Antibodies include, but are not limited to, monoclonal and chimerized monoclonal antibodies. The term *"antibody"* encompasses complete immunoglobulins, like monoclonal antibodies produced by recombinant expression in host cells, as well as Ang2-binding antibody fragments or *"antibody-like molecules",* including single-chain antibodies and linear antibodies, so-called *"SMIPs"* ("Small Modular Immunopharmaceuticals"), as e.g described in WO02/056910. Anti- Ang2 antibody-like molecules include immunoglobulin single variable domains, as defined herein. Other examples for antibody-like molecules are immunoglobulin super family antibodies (IgSF), or CDR-grafted molecules.

*"Ang2-binding molecule"* refers to monovalent Ang2-binding molecules (i.e. molecules that bind to one epitope of Ang2) as well as to Ang2-binding molecules containing more than one Ang2-binding immunoglobulin single variable domain, also termed "*formatted*" Ang2-binding molecules. The formatted Ang-2binding molecules may, in addition to the Ang2-binding immunoglobulin single variable domains, comprise linkers and/or moieties with effector functions, e.g. half-life-extending moieties like albumin-binding immunoglobulin single variable domains, and/or a fusion partner like serum albumin and/or an attached polymer like PEG.

A formatted Ang2-binding molecule may, albeit less preferred, also comprise two identical Ang2-binding immunoglobulin single variable domains or two different immunoglobulin single variable domains that recognize the same or overlapping epitopes. In this case, the two immunoglobulin single variable domains may bind to the same or an overlapping epitope in each of the two monomers that form the Ang2 dimer. Exeprimental data including competitive ELISA assay discloses a significant improvement in the potency of the formatted Ang2 dimers when compared to the individual building blocks of mono Ang2-binding molecules (data not shown).

Specific binding of an antigen-binding protein to an antigen or epitope can be determined in any suitable manner known per se, including, for example, the assays described herein, Scatchard analysis and/or competitive binding assays, such as radioimmunoassays (RIA), enzyme immunoassays (EIA) and sandwich competition assays, and the different variants thereof known per se in the art.

Amino acid residues will be indicated according to the standard three-letter or one-letter amino acid code, as generally known and agreed upon in the art. When comparing two amino acid sequences, the term *"amino acid difference"* refers to insertions, deletions or substitutions of the indicated number of amino acid residues at a position of the reference sequence, compared to a second sequence. In case of substitution(s), such substitution(s) will preferably be conservative amino acid substitution(s), which means that an amino acid residue is replaced with another amino acid residue of similar chemical structure and which has little or essentially no influence on the function, activity or other biological properties of the polypeptide. Such conservative amino acid substitutions are well known in the art, for example from WO98/49185, wherein conservative amino acid substitutions preferably are substitutions in which one amino acid within the following groups (i) - (v) is substituted by another amino acid residue within the same group: (i) small aliphatic, nonpolar or slightly polar residues: Ala, Ser, Thr, Pro and Gly; (ii) polar, negatively charged residues and their (uncharged) amides: Asp, Asn, Glu and Gln; (iii) polar, positively charged residues: His, Arg and Lys; (iv) large aliphatic, nonpolar residues: Met, Leu, Ile, Val and Cys; and (v) aromatic residues: Phe, Tyr and Trp. Particularly preferred conservative amino acid substitutions are as follows:
Ala into Gly or into Ser; Arg into Lys; Asn into Gln or into His; Asp into Glu; Cys into Ser; Gln into Asn; Glu into Asp; Gly into Ala or into Pro; His into Asn or into Gln; Ile into Leu or into Val; Leu into Ile or into Val; Lys into Arg, into Gln or into Glu; Met into Leu, into Tyr or into Ile; Phe into Met, into Leu or into Tyr; Ser into Thr; Thr into Ser;Trp into Tyr; Tyr into Trp or into Phe; Val into Ile or into Leu.

A polypeptide or nucleic acid molecule is considered to be *"(in) essentially isolated (form)"* - for example, when compared to its native biological source and/or the reaction medium or cultivation medium from which it has been obtained - when it has been separated from at least one other component with which it is usually associated in said source or medium, such as another protein/polypeptide, another nucleic acid, another biological component or macromolecule or at least one contaminant, impurity or minor component. In particular, a polypeptide or nucleic acid molecule is considered *"essentially isolated*" when it has been purified at least 2-fold, in particular at least 10- fold, more in particular at least 100-fold, and up to 1000-fold or more. A polypeptide or nucleic acid molecule that is *"in essentially isolated form"* is preferably essentially homogeneous, as determined using a suitable technique, such as a suitable chromatographical technique, such as polyacrylamide gel electrophoresis.

The term *"N-terminus"* (also known as the amino-terminus, NH₂-terminus, N-terminal end or amine-terminus) refers to the start of a protein/polypeptide (i.e. Ang2-binding molecule) terminated by an amino acid with a free amine group (-NH₂). The convention for writing peptide sequences is to put the N-terminus on the left and write the sequence from N- to C-terminus. When the protein is translated from messenger RNA, it is created from N-terminus to C-terminus.

*"Sequence identity"* between two Ang2-binding molecule sequences indicates the percentage of amino acids that are identical between the sequences. It may be calculated or determined as described in paragraph f) on pages 49 and 50 of WO08/020079. *"Sequence similarity"* indicates the percentage of amino acids that either is identical or that represent conservative amino acid substitutions.

Alternative methods for numbering the amino acid residues of V_{H} domains, which methods can also be applied in an analogous manner to VHH domains, are known in the art. However, in the present description, claims and figures, the numbering according to Kabat and applied to VHH domains as described above will be followed, unless indicated otherwise.

An *"affinity-matured"* Ang2-binding molecule, in particular a VHH or a domain antibody, has one or more alterations in one or more CDRs which result in an improved affinity for Ang2, as compared to the respective parent Ang2-binding molecule. Afffinity-matured Ang2-binding molecules of the invention may be prepared by methods known in the art, for example, as described by Marks et al., 1992, Biotechnology 10:779-783, or Barbas, et al., 1994, Proc. Nat. Acad. Sci, USA 91: 3809-3813.; Shier et al., 1995, Gene 169:147-155; Yelton et al., 1995, Immunol. 155: 1994-2004; Jackson et al., 1995, J. Immunol. 154(7):3310-9; and Hawkins et al., 1992, J. Mol. Biol. 226(3): 889 896; KS Johnson and RE Hawkins, "Affinity maturation of antibodies using phage display", Oxford University Press 1996.

An "amino acid sequences of SEQ ID NO: x" means an amino acid sequence that is 100% identical with the sequence shown in the respective SEQ ID NO: x.

The terms *"cancer"* and *"cancerous"* refer to or describe the physiological condition in mammals that is typically characterized by unregulated cell growth/proliferation. Examples of cancer to be treated with an Ang2-binding molecule of the invention include but are not limited to carcinoma, lymphoma, blastoma, sarcoma, and leukemia. More particular examples of such cancers, include squamous cell cancer, small-cell lung cancer, non-small cell lung cancer, adenocarcinoma of the lung, squamous carcinoma of the lung, cancer of the peritoneum, hepatocellular cancer, gastrointestinal cancer, pancreatic cancer, glioblastoma, cervical cancer, ovarian cancer, liver cancer, bladder cancer, hepatoma, breast cancer, colon cancer, colorectal cancer, renal cell carcinoma, endometrial or uterine carcinoma, salivary gland carcinoma, kidney cancer, liver cancer, prostate cancer, vulval cancer, thyroid cancer, hepatic carcinoma, gastric cancer, melanoma, and various types of head and neck cancer. Dysregulation of angiogenesis can lead to many disorders that can be treated by compositions and methods of the invention. These disorders include both non-neoplastic and neoplastic conditions. Neoplasties include but are not limited those described above.

Non-neoplastic disorders include, but are not limited to undesired or aberrant hypertrophy, arthritis, rheumatoid arthritis (RA), psoriasis, psoriatic plaques, sarcoidosis, atherosclerosis, atherosclerotic plaques, diabetic and other proliferative retinopathies including retinopathy of prematurity, retrolental fibroplasia, neovascular glaucoma, age-related macular degeneration, diabetic macular edema, corneal neovascularization, corneal graft neovascularization, corneal graft rejection, retinal/choroidal neovascularization, neovascularization of the angle (rubeosis), ocular neovascular disease, vascular restenosis, arteriovenous malformations (AVM), meningioma, hemangioma, angiofibroma, thyroid hyperplasias (including Grave's disease), corneal and other tissue transplantation, chronic inflammation, lung inflammation, acute lung injury/ ARDS, sepsis, primary pulmonary hypertension, malignant pulmonary effusions, cerebral edema (e.g., associated with acute stroke/ closed head injury/ trauma), synovial inflammation, pannus formation in RA, myositis ossificans, hypertropic bone formation, osteoarthritis (OA), refractory ascites, polycystic ovarian disease, endometriosis, 3^{rd} spacing of fluid diseases (pancreatitis, compartment syndrome, burns, bowel disease), uterine fibroids, premature labor, chronic inflammation such as IBD (Crohn's disease and ulcerative colitis), renal allograft rejection, inflammatory bowel disease, nephrotic syndrome, undesired or aberrant tissue mass growth (non-cancer), hemophilic joints, hypertrophic scars, inhibition of hair growth, Osier-Weber syndrome, pyogenic granuloma retrolental fibroplasias, scleroderma, trachoma, vascular adhesions, synovitis, dermatitis, preeclampsia, ascites, pericardial effusion (such as that associated with pericarditis), and pleural effusion.

The term *"eye diseases"* refers to proliferative retinopathies including retinopathy of prematurity, retrolental fibroplasia, neovascular glaucoma, age-related macular degeneration, diabetic macular edema, corneal neovascularization, corneal graft neovascularization, corneal graft rejection, retinal/choroidal neovascularization.

The term *"chronic kidney diseases"* refers to diabetic nephropathy, postrenal failure, prerenal azotemia and intrinsic renal failure.

### DETAILED DESCRIPTION OF THE INVENTION

In a first aspect, the present invention relates to an Ang2-binding molecule comprising an immunoglobulin single variable domain, wherein said immunoglobulin single variable domain comprises three complementarity determining regions CDR1, CDR2 and CDR3, wherein CDR1 has an amino acid sequence shown in SEQ ID NO: 168, CDR2 has an amino acid sequence shown in SEQ ID NO: 171 and CDR3 has an amino acid sequence shown in SEQ ID NO: 175.
SEQ ID NO: 168 DYAIG
SEQ ID NO: 171 AIRSSGGSTYYADSVKG
SEQ ID NO: 175 VPAGRLRYGEQWYPIYEYDA

According to preferred embodiments, the Ang2-binding molecule comprises an immunoglobulin single variable domain, wherein said immunoglobulin single variable domain is a VHH or a domain antibody.

According to a preferred embodiment, the Ang2-binding molecule comprises an immunoglobulin single variable domain, wherein said immunoglobulin single variable domain is a VHH.

According to specific embodiments, said VHH consists of an immunoglobulin single variable domain having a sequence of SEQ ID NO: 166.

In another aspect, the present invention relates to an Ang2-binding molecule consisting of said immunoglobulin single variable domain.

The sequence of the Ang2 binders according to the invention can be modified at their N-terminus (i.e. deletion or exchange of the first amino acid) without significant reduction of their binding activity. This modification enhances the co-/post-translational cleavage of N-terminal methionine during intracellular/cytoplasmic expression in bacterial hosts (e.g. but not limited to *Escherichia coli).*

In one aspect said VHH consisting of an immunoglobulin single variable domain has a modification or exchange on N terminus, wherein said modification is a deletion of a first amino acid and said exchange is a replacement of the first amino acid by another amino acid.

In one of preferred embodiments the first amino acid on N terminus is Valine (V) or Aspartic acid (D) replaced by e.g. by Alanine (A).

Ang2-binding components with improved properties in view of therapeutic application, e.g. enhanced affinity or decreased immunogenicity, may be obtained from individual Ang2-binding components of the invention by techniques such as affinity maturation (for example, starting from synthetic, random or naturally occurring immunoglobulin sequences), CDR grafting, humanizing, combining fragments derived from different immunoglobulin sequences, PCR assembly using overlapping primers, and similar techniques for engineering immunoglobulin sequences well known to the skilled person; or any suitable combination of any of the foregoing. Reference is, for example, made to standard handbooks, as well as to the further description and Examples.

In yet another embodiment, the representatives of the class of Ang2-binding immunoglobulin single variable domains of the invention which include the CDRs as set out above have amino acid sequences that correspond to the amino acid sequence of a naturally occurring VH domain that has been "camelized", i.e. by replacing one or more amino acid residues in the amino acid sequence of a naturally occurring variable heavy chain from a conventional 4-chain antibody by one or more amino acid residues that occur at the corresponding position(s) in a VHH domain of a heavy chain antibody. This can be performed in a manner known per se, which will be clear to the skilled person, and reference is additionally be made to WO 1994/04678. Such camelization may preferentially occur at amino acid positions which are present at the VH-VL interface and at the so-called Camelidae Hallmark residues (see for example also WO 1994/04678). A detailled description of such "humanization" and "camelization" techniques and preferred framework region sequences consistent therewith can additionally be taken from e.g. pp. 46 and pp. 98 of WO 2006/040153 and pp. 107 of WO 2006/122786.

The Ang2-binding components of the invention, e.g. immunoglobulin single variable domains and or polypeptides containing them, have specificity for Ang2 in that they comprise one or more immunoglobulin single variable domains specifically binding to one or more epitopes within the Ang2 molecule.

Specific binding of an Ang2-binding component to its antigen Ang2 can be determined in any suitable manner known per se, including, for example, the assays described herein, Scatchard analysis and/or competitive binding assays, such as radioimmunoassays (RIA), enzyme immunoassays (EIA and ELISA) and sandwich competition assays, and the different variants thereof known per se in the art.

According to another embodiment, the immunoglobulin single variable domains are domain antibodies, as defined herein.

In such case, immunoglobulin single variable domains as defined above present in the monospecific binding molecules of the invention have sequences that correspond to the amino acid sequence of a naturally occurring VH domain that has been "camelized", i.e. by replacing one or more amino acid residues in the amino acid sequence of a naturally occurring variable heavy chain from a conventional 4-chain antibody by one or more amino acid residues that occur at the corresponding position(s) in a VHH domain of a heavy chain antibody. This can be performed in a manner known per se, which will be clear to the skilled person, and reference is additionally be made to WO 94/04678. Such camelization may preferentially occur at amino acid positions which are present at the VH-VL interface and at the so-called Camelidae Hallmark residues (see for example also WO 94/04678). A detailled description of such "humanization" and "camelization" techniques and preferred framework region sequences consistent therewith can additionally be taken from e.g. pp. 46 and pp. 98 of WO 2006/040153 and pp. 107 of WO 2006/122786.

The binding components have specificity for Ang2, in that they comprise one immunoglobulin single variable domain specifically binding to the Ang2 molecule.

Specific binding of a binding component to its antigen Ang2 can be determined in any suitable manner known *per se,* including, for example, the assays described herein, Scatchard analysis and/or competitive binding assays, such as radioimmunoassays (RIA), enzyme immunoassays (EIA and ELISA) and sandwich competition assays, and the different variants thereof known per se in the art.

The present invention also relates to a nucleic acid encoding the Ang2-binding molecule according to the invention.

In a preferred embodiment an Ang2-binding molecule according to the invention when expressed in *Escherichia coli* is encoded by the nucelotide seuquence:

### SEQ ID NO: 179

The invention relates to nucleic acid molecules that encode Ang2 binding monospecific binding molecules of the invention. Such nucleic acid molecules will also be referred to herein as "nucleic acids of the invention" and may also be in the form of a genetic construct, as defined herein. A nucleic acid of the invention may be genomic DNA, cDNA or synthetic DNA (such as DNA with a codon usage that has been specifically adapted for expression in the intended host cell or host organism). According to one embodiment of the invention, the nucleic acid of the invention is in essentially isolated form, as defined hereabove.

Further aspect of the invention relates to an expression vector comprising the nucleic acid molecule encoding said Ang2- binding molecule according to invention.

In preferred embodiments, the nucleic acid of the invention may also be in the form of, may be present in and/or may be part of a vector, such as for example a plasmid, cosmid or YAC.

The vector may especially be an expression vector, i.e. a vector that can provide for expression of the monospecific binding molecule *in vitro* and/or *in vivo* (i.e. in a suitable host cell, host organism and/or expression system). Such expression vector generally comprises at least one nucleic acid of the invention that is operably linked to one or more suitable regulatory elements, such as promoter(s), enhancer(s), terminator(s), and the like. Such elements and their selection in view of expression of a specific sequence in a specific host are common knowledge of the skilled person. Specific examples of regulatory elements and other elements useful or necessary for expressing molecules of the invention, such as promoters, enhancers, terminators, integration factors, selection markers, leader sequences, reporter genes, and the like, are disclosed e.g. on pp. 131 to 133 of WO 2006/040153.

Such vectors express or are capable of expressing one or more monospecific binding molecules of the invention; and/or contain a nucleic acid of the invention.

The nucleic acids of the invention may be prepared or obtained in a manner known *per se* (e.g. by automated DNA synthesis and/or recombinant DNA technology), based on the information on the amino acid sequences for the polypeptides of the invention given herein, and/or can be isolated from a suitable natural source.

In another aspect, the invention relates to a host cell carrying one or more expression vectors comprising a nucleic acid molecule encoding the Ang2-binding molecule according to the invention.

According to a particularly preferred embodiment, said host cells are bacterial cells; other useful cells are yeast cells, fungal cells or mammalian cells.

Suitable bacterial cells include cells from gram-negative bacterial strains such as strains of *Escherichia coli, Proteus,* and *Pseudomonas,* and gram-positive bacterial strains such as strains of *Bacillus, Streptomyces, Staphylococcus,* and *Lactococcus.* Suitable fungal cell include cells from species of *Trichoderma, Neurospora,* and *Aspergillus.* Suitable yeast cells include cells from species of *Saccharomyces (*for example *Saccharomyces cerevisiae), Schizosaccharomyces* (for example *Schizosaccharomyces pombe*), *Pichia* (for example *Pichia pastoris* and *Pichia methanolica*), and *Hansenula.*

Suitable mammalian cells include for example CHO cells, BHK cells, HeLa cells, COS cells, and the like. However, amphibian cells, insect cells, plant cells, and any other cells used in the art for the expression of heterologous proteins can be used as well.

The invention further provides methods of manufacturing a monospecific binding molecule of the invention, such methods generally comprising the steps of:
- culturing host cells comprising a nucleic acid capable of encoding a monospecific binding molecule under conditions that allow expression of the monospecific binding molecule of the invention; and
- recovering or isolating the polypeptide expressed by the host cells from the culture; and
- optionally further purifying and/or modifying and/or formulating the monospecific binding molecule of the invention.

The preferred embodiment represents a method for producing an Ang2 binding molecule having sequence SEQ ID NO: 166 comprising the steps of:
(a) transfecting a host cell with one or more said vectors
(b) culturing said host cell, and
(c) recovering and purifying said Ang2 binding molecule.

For production on an industrial scale, preferred host organisms include strains of *E. coli, Pichia pastoris,* and *S. cerevisiae* that are suitable for large scale expression, production and fermentation, and in particular for large scale pharmaceutical expression, production and fermentation.

The choice of the specific expression system depends in part on the requirement for certain post-translational modifications, more specifically glycosylation. The production of a monospecific binding molecule of the invention for which glycosylation is desired or required would necessitate the use of mammalian expression hosts that have the ability to glycosylate the expressed protein. In this respect, it will be clear to the skilled person that the glycosylation pattern obtained (i.e. the kind, number and position of residues attached) will depend on the cell or cell line that is used for the expression.

Monospecific binding molecules of the invention may be produced either in a cell as set out above intracellullarly (e.g. in the cytosol, in the periplasma or in inclusion bodies) and then isolated from the host cells and optionally further purified; or they can be produced extracellularly (e.g. in the medium in which the host cells are cultured) and then isolated from the culture medium and optionally further purified.

Methods and reagents used for the recombinant production of polypeptides, such as specific suitable expression vectors, transformation or transfection methods, selection markers, methods of induction of protein expression, culture conditions, and the like, are known in the art. Similarly, protein isolation and purification techniques useful in a method of manufacture of a polypeptide of the invention are well known to the skilled person.

The invention further relates to a product or composition containing or comprising at least one Ang2 binding monospecific binding molecule of the invention and optionally one or more further components of such compositions known *perse,* i.e. depending on the intended use of the composition.

In further aspect, the invention relates to use of an Ang2 binding monospecific binding molecule of the invention as a medicament.

In still another aspect, the invention relates to use of an Ang2 binding monospecific binding molecule of the invention for method of treating of cancer, cancerous or eye diseases.

For pharmaceutical use, an Ang2 binding monospecific binding molecule of the invention or a polypeptide containing same may be formulated as a pharmaceutical preparation or composition comprising at least one such monospecific binding molecule of the invention and at least one physiologically acceptable carrier, diluent or excipient and/or adjuvant, and optionally one or more further pharmaceutically active polypeptides and/or compounds. By means of non-limiting examples, such a formulation may be in a form suitable for oral administration, for parenteral administration (such as by intravenous, intramuscular or subcutaneous injection or intravenous infusion), for topical administration, for administration by inhalation, by a skin patch, by an implant, by a suppository, etc. Such suitable administration forms - which may be solid, semi-solid or liquid, depending on the manner of administration - as well as methods and carriers for use in the preparation thereof, will be clear to the skilled person, and are further described herein.

Thus, in a further aspect, the invention relates to a pharmaceutical composition that contains at least one Ang2 binding monospecific binding molecule, in particular one immunoglobulin single variable domain of the invention as defined above or a polypeptide containing same and at least one suitable carrier, diluent or excipient (i.e. suitable for pharmaceutical use), and optionally one or more further active substances.

The Ang2 binding monospecific binding molecules of the invention may be formulated and administered in any suitable manner known per se: Reference, in particular for the immunoglobulin single variable domains, is for example made to WO 2004/041862, WO 2004/041863, WO 2004/041865, WO 2004/041867 and WO 2008/020079, as well as to the standard handbooks, such as Remington's Pharmaceutical Sciences, 18th Ed., Mack Publishing Company, USA (1990), Remington, the Science and Practice of Pharmacy, 21th Edition, Lippincott Williams and Wilkins (2005); or the Handbook of Therapeutic Antibodies (S. Dubel, Ed.), Wiley, Weinheim, 2007 (see for example pages 252-255).

For example, an immunoglobulin single variable domain of the invention as defined above may be formulated and administered in any manner known per se for conventional antibodies and antibody fragments (including ScFv's and diabodies) and other pharmaceutically active proteins. Such formulations and methods for preparing the same will be clear to the skilled person, and for example include preparations suitable for parenteral administration (for example intravenous, intraperitoneal, subcutaneous, intramuscular, intraluminal, intra-arterial or intrathecal administration) or for topical (i.e. transdermal or intradermal) administration.

Preparations for parenteral administration may for example be sterile solutions, suspensions, dispersions or emulsions that are suitable for infusion or injection. Suitable carriers or diluents for such preparations for example include, without limitation, sterile water and pharmaceutically acceptable aqueous buffers and solutions such as physiological phosphate-buffered saline, Ringer's solutions, dextrose solution, and Hank's solution; water oils; glycerol; ethanol; glycols such as propylene glycol or as well as mineral oils, animal oils and vegetable oils, for example peanut oil, soybean oil, as well as suitable mixtures thereof. Usually, aqueous solutions or suspensions will be preferred.

Thus, the Ang2 binding monospecific binding molecule of the invention may be systemically administered, e.g., orally, in combination with a pharmaceutically acceptable vehicle such as an inert diluent or an assimilable edible carrier. For oral therapeutic administration, the molecule of the invention may be combined with one or more excipients and used in the form of ingestible tablets, buccal tablets, troches, capsules, elixirs, suspensions, syrups, wafers, and the like. Such compositions and preparations should contain at least 0.1% of the Ang2-binding molecule of the invention. Their percentage in the compositions and preparations may, of course, be varied and may conveniently be between about 2 to about 60% of the weight of a given unit dosage form. The amount of the molecule of the invention in such therapeutically useful compositions is such that an effective dosage level will be obtained.

The tablets, pills, capsules, and the like may also contain binders, excipients, disintegrating agents, lubricants and sweetening or flavouring agents, for example those mentioned on pages 143-144 of WO 08/020079. When the unit dosage form is a capsule, it may contain, in addition to materials of the above type, a liquid carrier, such as a vegetable oil or a polyethylene glycol. Various other materials may be present as coatings or to otherwise modify the physical form of the solid unit dosage form. For instance, tablets, pills, or capsules may be coated with gelatin, wax, shellac or sugar and the like. A syrup or elixir may contain the molecules of the invention, sucrose or fructose as a sweetening agent, methyl and propylparabens as preservatives, a dye and flavoring such as cherry or orange flavor. Of course, any material used in preparing any unit dosage form should be pharmaceutically acceptable and substantially non-toxic in the amounts employed. In addition, the molecules of the invention may be incorporated into sustained-release preparations and devices.

Preparations and formulations for oral administration may also be provided with an enteric coating that will allow the constructs of the invention to resist the gastric environment and pass into the intestines. More generally, preparations and formulations for oral administration may be suitably formulated for delivery into any desired part of the gastrointestinal tract. In addition, suitable suppositories may be used for delivery into the gastrointestinal tract.

The Ang2 binding monospecific binding molecules of the invention may also be administered intravenously or intraperitoneally by infusion or injection, as further described on pages 144 and 145 of WO 2008/020079.

For topical administration of the Ang2 binding monospecific binding molecules of the invention, it will generally be desirable to administer them to the skin as compositions or formulations, in combination with a dermatologically acceptable carrier, which may be a solid or a liquid, as further described on page 145 of WO 2008/020079.

Generally, the concentration of the Ang2 binding monospecific binding molecules of the invention in a liquid composition, such as a lotion, will be from about 0.1-25 wt-%, preferably from about 0.5-10 wt-%. The concentration in a semi-solid or solid composition such as a gel or a powder will be about 0.1-5 wt-%, preferably about 0.5-2.5 wt-%.

The amount of the Ang2 binding monospecific binding molecules of the invention required for use in treatment will vary not only with the particular monospecific binding molecule selected, but also with the route of administration, the nature of the condition being treated and the age and condition of the patient and will be ultimately at the discretion of the attendant physician or clinician. Also, the dosage of the monospecific binding molecules of the invention varies depending on the target cell, tumor, tissue, graft, or organ. The desired dose may conveniently be presented in a single dose or as divided doses administered at appropriate intervals, for example, as two, three, four or more sub-doses per day. The sub-dose itself may be further divided, *e.g.,* into a number of discrete loosely spaced administrations; such as multiple inhalations from an insufflator or by application of a plurality of drops into the eye.

An administration regimen may include long-term, daily treatment. By "long-term" is meant at least two weeks and preferably, several weeks, months, or years of duration. Necessary modifications in this dosage range may be determined by one of ordinary skill in the art using only routine experimentation given the teachings herein. See Remington's Pharmaceutical Sciences (Martin, E.W., ed. 4), Mack Publishing Co., Easton, PA. The dosage can also be adjusted by the individual physician in the event of any complication.

According to a further embodiment, the invention relates to the use of Ang2 binding monospecific binding molecules of the invention, i.e. immunoglobulin single variable domains or polypeptides containing them, for therapeutic purposes, such as
- for the prevention, treatment and/or alleviation of a disorder, disease or condition, especially in a human being, that is associated with Ang2-mediated and/or Ang2-related effects on angiogenesis or that can be prevented, treated or alleviated by modulating the Notch signaling pathway and/or the Tie2 signalling pathway with a monospecific binding molecule according to the invention;
- in a method of treatment of a patient in need of such therapy, such method comprising administering, to a subject in need thereof, a pharmaceutically active amount of at least one monospecific binding molecule of the invention, e.g. an immunoglobulin single variable domain, or a pharmaceutical composition containing same;
- for the preparation of a medicament for the prevention, treatment or alleviation of disorders, diseases or conditions associated with Ang2-mediated and/or Ang2-mediated effects on angiogenesis;
- as an active ingredient in a pharmaceutical composition or medicament used for the above purposes.

According to a specific aspect, said disorder disorder, disease or condition is a cancer or cancerous disease, as defined herein.

In preferred embodiments, the invention relates to said pharmaceutical composition for treatment of cancer and cancerous diseases, such as breast, renal cell carcinoma, ovarian cancer and pancreatic cancer.

According to another aspect, the disease is an eye disease associated with Ang2-mediated and/or Ang2-mediated effects on angiogenesis or which can be treated or alleviated by modulating the Notch signaling pathway and/or the Tie2 signalling pathway with an Ang2 binding monospecific binding molecule of the invention.

In another preferred embodiments, the invention relates to said pharmaceutical composition for treatment of eye diseases, such as age-related macular degeneration and diabetic retinopathy.

In still another preferred embodiments, the invention relates to said pharmaceutical composition for treatment of chronic kidney diseases.

Depending on cancer/ cancerous diseases, eye diseases and/or chronic kidney diseases to be treated, an Ang2 binding monospecific binding molecule of the invention may be used on its own or in combination with one or more additional therapeutic agents.

In preferred embodiments, the invention relates to the pharmaceutical composition comprising, as the active ingredient one or more said Ang-2 binding molecules of the invention, further comprising one or more additional therapeutic agents, such as chemotherapeutic agents like DNA damaging agents and/or anti-mitotic drugs in cancer cells (e.g. taxol) or therapeutically active compounds that inhibit angiogenesis (an anti- angiogenic drug such as anti VEGF/VEGF receptor inhibitor, e.g. avastin,nitedanib and sunitinib), or signal transduction pathway inhibitors such as mTOR inhibitors (e.g. temsirolimus) or hormonal therapy agents (e.g. tamoxifen).

The additional therapeutic agent may be administered simultaneously with, optionally as a component of the same pharmaceutical preparation, or before or after administration of the monospecific binding molecule.

In certain embodiments, the additional therapeutic agent may be, without limitation (and in the case of the receptors, including the respective ligands), one or more inhibitors selected from the group of inhibitors of EGFR, VEGF, VEGFR, HER2-neu, Her3, AuroraA, AuroraB, PLK and PI3 kinase, FGFR, PDGFR, Raf, KSP, PDK1, PTK2, IGF-R or IR.

Further examples of additional therapeutic agents are inhibitors of CDK, Akt, src/bcr abl, cKit, cMet/HGF, c-Myc, Flt3, HSP90, hedgehog antagonists, inhibitors of JAK/STAT, Mek, mTor, NFkappaB, the proteasome, Rho, an inhibitor of wnt signaling or an inhibitor of the ubiquitination pathway or another inhibitor of the Notch signaling pathway.

Examples for Aurora inhibitors are, without limitation, PHA-739358, AZD-1152, AT 9283, CYC-116, R-763, VX-680, VX-667, MLN-8045, PF-3814735.

An example for a PLK inhibitor is GSK-461364.

Examples for VEGF inhibitor are avastin (Roche), aflibercept (Regeneron,)

Examples for raf inhibitors are BAY-73-4506 (also a VEGFR inhibitor), PLX 4032, RAF-265 (also in addition a VEGFR inhibitor), sorafenib (also in addition a VEGFR inhibitor), and XL 281.

Examples for KSP inhibitors are ispinesib, ARRY-520, AZD-4877, CK-1122697, GSK 246053A, GSK-923295, MK-0731, and SB-743921.

Examples for a src and/or bcr-abl inhibitors are dasatinib, AZD-0530, bosutinib, XL 228 (also an IGF-1R inhibitor), nilotinib (also a PDGFR and cKit inhibitor), imatinib (also a cKit inhibitor), and NS-187.

An example for a PDK1 inhibitor is BX-517.

An example for a Rho inhibitor is BA-210.

Examples for PI3 kinase inhibitors are PX-866, BEZ-235 (also an mTor inhibitor), XL 418 (also an Akt inhibitor), XL-147, and XL 765 (also an mTor inhibitor).

Examples for inhibitors of cMet or HGF are XL-184 (also an inhibitor of VEGFR, cKit, Flt3), PF-2341066, MK-2461, XL-880 (also an inhibitor of VEGFR), MGCD-265 (also an inhibitor of VEGFR, Ron, Tie2), SU-11274, PHA-665752, AMG-102, and AV-299.

An example for a c-Myc inhibitor is CX-3543.

Examples for Flt3 inhibitors are AC-220 (also an inhibitor of cKit and PDGFR), KW 2449, lestaurtinib (also an inhibitor of VEGFR, PDGFR, PKC), TG-101348 (also an inhibitor of JAK2), XL-999 (also an inhibitor of cKit, FGFR, PDGFR and VEGFR), sunitinib (also an inhibitor of PDGFR, VEGFR and cKit), and tandutinib (also an inhibitor of PDGFR, and cKit).

Examples for HSP90 inhibitors are tanespimycin, alvespimycin, IPI-504 and CNF 2024.

Examples for JAK/STAT inhibitors are CYT-997 (also interacting with tubulin), TG 101348 (also an inhibitor of Flt3), and XL-019.

Examples for Mek inhibitors are ARRY-142886, PD-325901, AZD-8330, and XL 518.

Examples for mTor inhibitors are temsirolimus, AP-23573 (which also acts as a VEGF inhibitor), everolimus (a VEGF inhibitor in addition). XL-765 (also a PI3 kinase inhibitor), and BEZ-235 (also a PI3 kinase inhibitor).

Examples for Akt inhibitors are perifosine, GSK-690693, RX-0201, and triciribine.

Examples for cKit inhibitors are AB-1010, OSI-930 (also acts as a VEGFR inhibitor), AC-220 (also an inhibitor of Flt3 and PDGFR), tandutinib (also an inhibitor of Flt3 and PDGFR), axitinib (also an inhibitor of VEGFR and PDGFR), XL-999 (also an inhibitor of Flt3, PDGFR, VEGFR, FGFR), sunitinib (also an inhibitor of Flt3, PDGFR, VEGFR), and XL-820 (also acts as a VEGFR- and PDGFR inhibitor), imatinib (also a bcr-abl inhibitor), nilotinib (also an inhibitor of bcr-abl and PDGFR).

Examples for hedgehog antagonists are IPI-609 and CUR-61414.

Examples for CDK inhibitors are seliciclib, AT-7519, P-276, ZK-CDK (also inhibiting VEGFR2 and PDGFR), PD-332991, R-547, SNS-032, PHA-690509, and AG 024322.

Examples for proteasome inhibitors are bortezomib, carfilzomib, and NPI-0052 (also an inhibitor of NFkappaB).

An example for an NFkappaB pathway inhibitor is NPI-0052.

An example for an ubiquitination pathway inhibitor is HBX-41108.

In preferred embodiments, the additional therapeutic agent is an anti-angiogenic agent.

Examples for anti-angiogenic agents are inhibitors of the FGFR, PDGFR and VEGFR or the respective ligands (e.g VEGF inhibitors like pegaptanib or the anti-VEGF antibody bevacizumab), and thalidomides, such agents being selected from, without limitation, bevacizumab, motesanib, CDP-791, SU-14813, telatinib, KRN-951, ZK-CDK (also an inhibitor of CDK), ABT-869, BMS-690514, RAF-265, IMC-KDR, IMC-18F1, IMiDs (immunomodulatory drugs), thalidomide derivative CC-4047, lenalidomide, ENMD 0995, IMC-D11, Ki 23057, brivanib, cediranib, XL-999 (also an inhibitor of cKit and Flt3), 1B3, CP 868596, IMC 3G3, R-1530 (also an inhibitor of Flt3), sunitinib (also an inhibitor of cKit and Flt3), axitinib (also an inhibitor of cKit), vemurafenib (also known as PLX4032, RG7204 or RO5185426, marketed as zelboraf) a B-Raf enzyme inhibitor, crizotinib known as an ALK (anaplastic lymphoma kinase) and ROS1 (c-ros oncogene1, receptor tyrosine kinase) inhibitor, lestaurtinib (also an inhibitor of Flt3 and PKC), vatalanib, tandutinib (also an inhibitor of Flt3 and cKit), pazopanib, GW 786034, PF-337210, IMC-1121B, AVE-0005, AG-13736, E-7080, CHIR 258, sorafenib tosylate (also an inhibitor of Raf), RAF-265 (also an inhibitor of Raf), vandetanib, CP-547632, OSI-930, AEE-788 (also an inhibitor of EGFR and Her2), BAY-57-9352 (also an inhibitor of Raf), BAY-73-4506 (also an inhibitor of Raf), XL 880 (also an inhibitor of cMet), XL-647 (also an inhibitor of EGFR and EphB4), XL 820 (also an inhibitor of cKit), and nilotinib (also an inhibitor of cKit and brc-abl) and nitedanib.

The additional therapeutic agent may also be selected from EGFR inhibitors; it may be a small molecule EGFR inhibitor or an anti-EGFR antibody. Examples for anti-EGFR antibodies, without limitation, are cetuximab, panitumumab, matuzumab; an example for a small molecule EGFR inhibitor is gefitinib. Another example for an EGFR modulator is the EGF fusion toxin.

Among the EGFR and Her2 inhibitors useful for combination with the molecule of the invention are lapatinib, gefitinib, erlotinib, cetuximab, trastuzumab, nimotuzumab, zalutumumab, vandetanib (also an inhibitor of VEGFR), pertuzumab, XL-647, HKI-272, BMS-599626 ARRY-334543, AV 412, mAB-806, BMS-690514, JNJ-26483327, AEE-788 (also an inhibitor of VEGFR), ARRY-333786, IMC-11F8, Zemab.

Other agents that may be advantageously combined in a therapy with the Ang2 binding monospecific binding molecule of the invention are tositumumab and ibritumomab tiuxetan (two radiolabelled anti-CD20 antibodies), alemtuzumab (an anti-CD52 antibody), denosumab, (an osteoclast differentiation factor ligand inhibitor), galiximab (a CD80 antagonist), ofatumumab (a CD20 inhibitor), zanolimumab (a CD4 antagonist), SGN40 (a CD40 ligand receptor modulator), rituximab (a CD20 inhibitor) or mapatumumab (a TRAIL-1 receptor agonist) or OMP-21M18 (DII4 inhibitors).

Other chemotherapeutic drugs that may be used in combination with the molecules of the present invention are selected from, but not limited to hormones, hormonal analogues and antihormonals (e.g. tamoxifen, toremifene, raloxifene, fulvestrant, megestrol acetate, flutamide, nilutamide, bicalutamide, cyproterone acetate, finasteride, buserelin acetate, fludrocortisone, fluoxymesterone, medroxyprogesterone, octreotide, arzoxifene, pasireotide, vapreotide), aromatase inhibitors (e.g. anastrozole, letrozole, liarozole, exemestane, atamestane, formestane), LHRH agonists and antagonists (e.g. goserelin acetate, leuprolide, abarelix, cetrorelix, deslorelin, histrelin, triptorelin), antimetabolites (e.g. antifolates like methotrexate, pemetrexed, pyrimidine analogues like 5 fluorouracil, capecitabine, decitabine, nelarabine, and gemcitabine, purine and adenosine analogues such as mercaptopurine thioguanine, cladribine and pentostatin, cytarabine, fludarabine); antitumor antibiotics (e.g. anthracyclines like doxorubicin, daunorubicin, epirubicin and idarubicin, mitomycin-C, bleomycin dactinomycin, plicamycin, mitoxantrone, pixantrone, streptozocin); platinum derivatives (e.g. cisplatin, oxaliplatin, carboplatin, lobaplatin, satraplatin); alkylating agents (e.g. estramustine, meclorethamine, melphalan, chlorambucil, busulphan, dacarbazine, cyclophosphamide, ifosfamide, hydroxyurea, temozolomide, nitrosoureas such as carmustine and lomustine, thiotepa); antimitotic agents (e.g. vinca alkaloids like vinblastine, vindesine, vinorelbine, vinflunine and vincristine; and taxanes like paclitaxel, docetaxel and their formulations, larotaxel; simotaxel, and epothilones like ixabepilone, patupilone, ZK-EPO); topoisomerase inhibitors (e.g. epipodophyllotoxins like etoposide and etopophos, teniposide, amsacrine, topotecan, irinotecan) and miscellaneous chemotherapeutics such as amifostine, anagrelide, interferone alpha, procarbazine, mitotane, and porfimer, bexarotene, celecoxib.

Particularly preferred combination partners of the molecules of the present invention are VEGF antagonists, like bevacizumab (Avastin®), nitedanib, Sorafenib and Sunitinib.

According to another embodiment of the invention, there is provided a method of diagnosing a disease by
a) contacting a sample with an Ang2 binding molecule of the invention as defined above, and
b) detecting binding of said binding molecule to said sample, and
c) comparing the binding detected in step (b) with a standard, wherein a difference in binding relative to said sample is diagnostic of a disease or disorder associated with VEGF- and/or Ang2-mediated effects on angiogenesis.

For this and other uses, it may be useful to further modify an Ang2 binding monospecific binding molecule of the invention, such as by introduction of a functional group that is one part of a specific binding pair, such as the biotin-(strept)avidin binding pair. Such a functional group may be used to link the binding molecule of the invention to another protein, polypeptide or chemical compound that is bound to the other half of the binding pair, i.e. through formation of the binding pair. For example, such monospecific binding molecule of the invention may be conjugated to biotin, and linked to another protein, polypeptide, compound or carrier conjugated to avidin or streptavidin. For example, such a conjugated monospecific binding molecule of the invention may be used as a reporter, for example in a diagnostic system where a detectable signal-producing agent is conjugated to avidin or streptavidin.

The efficacy of Ang2 binding monospecific binding molecule of the invention or polypeptides, and of compositions comprising the same, can be tested using any suitable *in vitro* assay, cell-based assay, *in vivo* assay and/or animal model known per se, or any combination thereof, depending on the specific disease or disorder of interest. Suitable assays and animal models will be clear to the skilled person, and for example include the assays described herein and used in the Examples below, e.g. a proliferation assay.

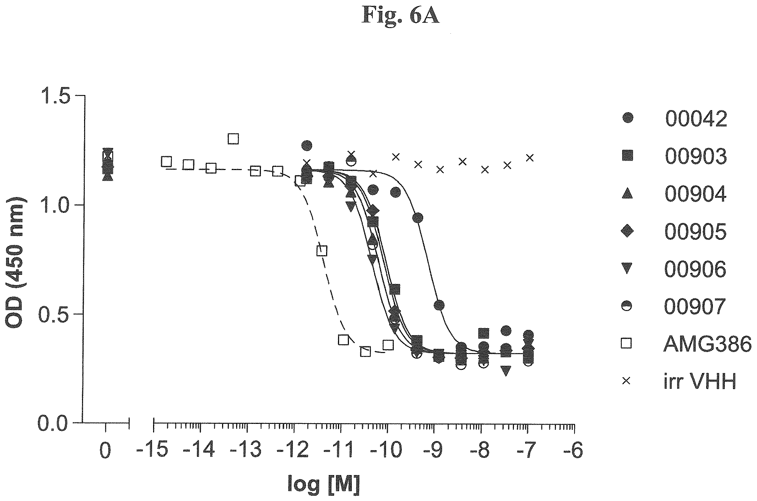

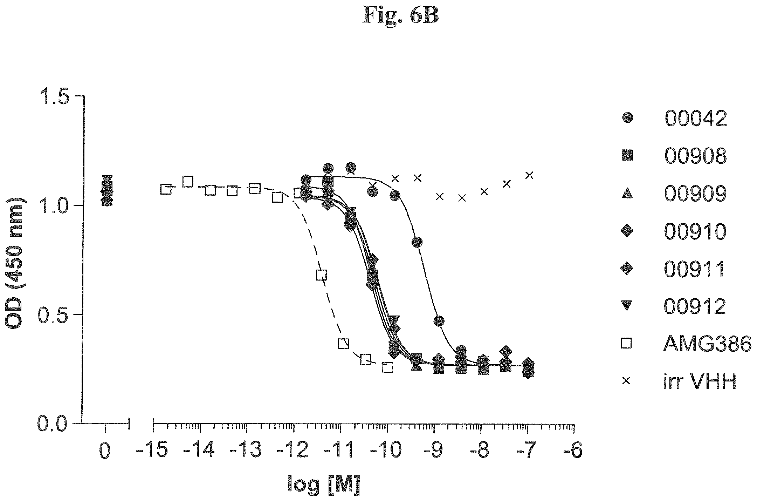

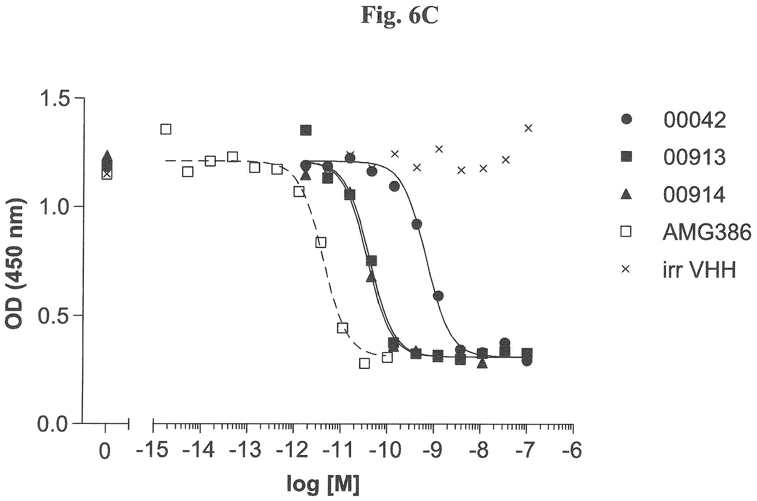

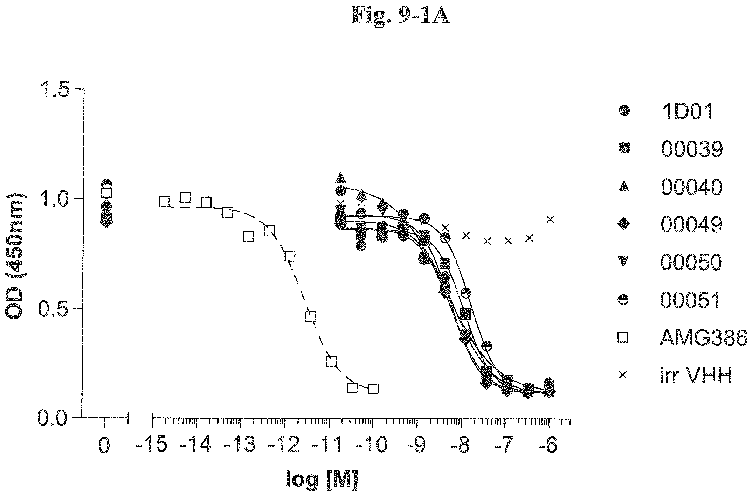

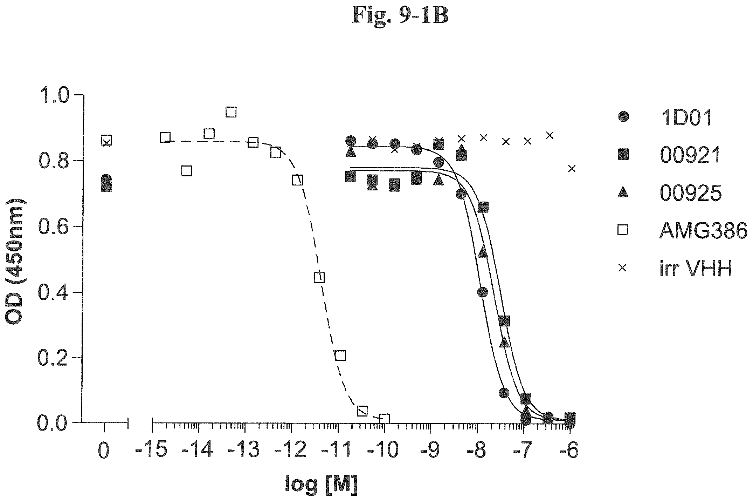

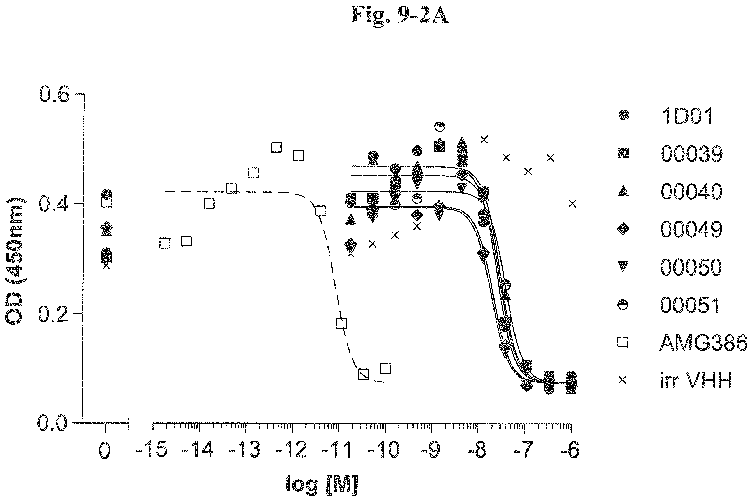

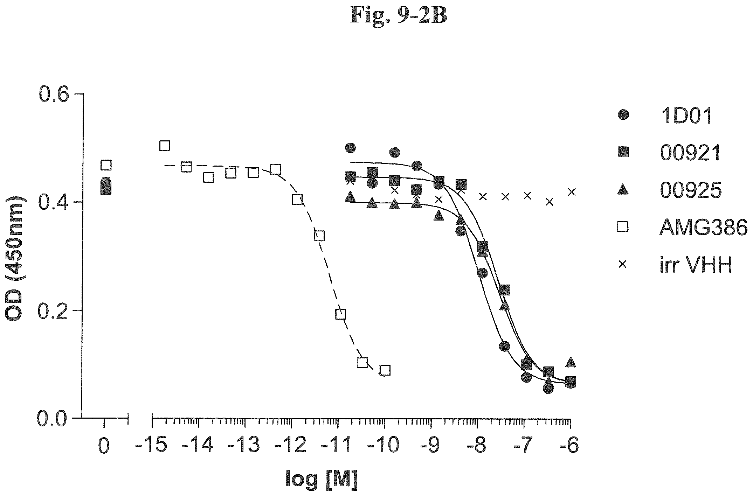

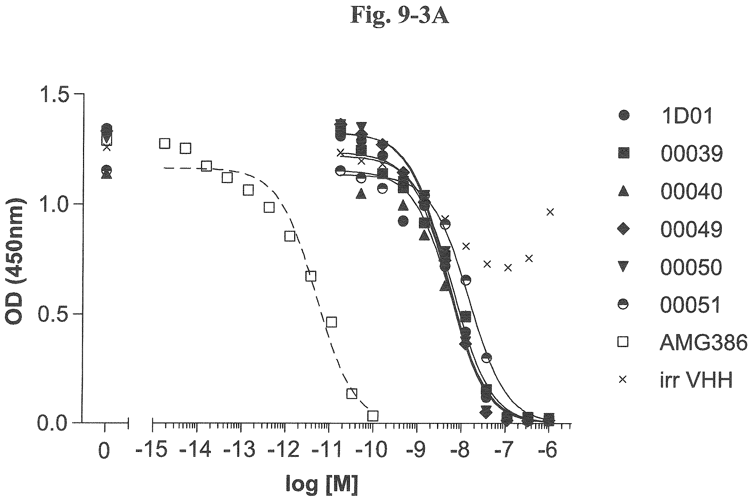

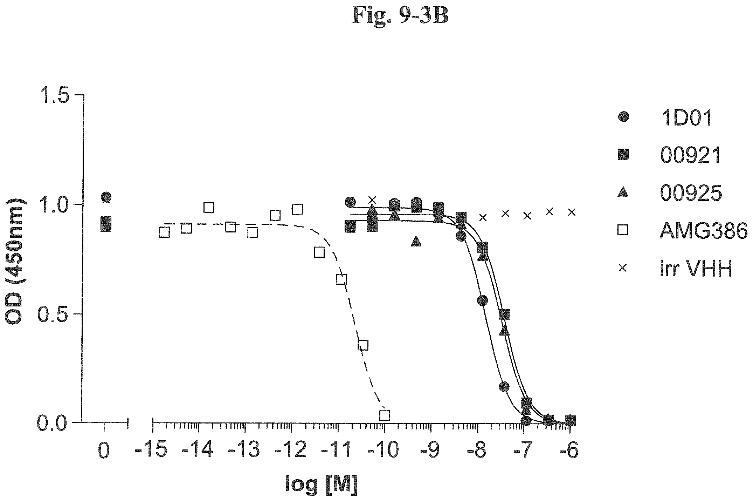

Ang2 binding monospecific binding molecules of the invention have undergone an extensive sequence optimization process involving affinity maturation, humanization and removal of potential posttranslational modification sites to ensure low immunogenicity potential in man and improved biophysical stability. Unexpectedly, the data show that such monospecific binding molecules of the invention have properties that are superior to those of binding molecules of the prior art. Among such properties are high selectivity for Ang2 neutralization as compared to Ang1 neutralization, as can e.g. be taken from the data of Figures 9 to 10, 13 to 14, 16 to 19; complete inhibition of the Ang2-Tie2 interaction with high potency, as can e.g. be taken from the ELISA data of Figures 6, 9, 13, 16, 18 and 20 and Tables 12 to 13, 16 to 17, 20 to 22, as well as the IC50 (nM) values for VHHs in the AlphaScreen assay as shown e.g. in Table 7 (Example 7); and the affinity KD (nM) of purified VHHs on recombinant human Ang2, cyno Ang2, mouse Ang2 in Tables 8, 14, 18 and 23.

This indicates that Ang2 binding monospecific binding molecules of the invention are promising candidates to have therapeutic efficacy in diseases and disorders associated with Ang2-mediated effects on angiogenesis, such as cancer, cancerous diseases, eye diseases and/or chronic kidney diseases.

### Brief description of the Figures:

The axes annotation in Figures 1 to 4, 6 to 7, 9 to 10, 12 to 14 and 16 to 20: X axes stand for OD 450 (nm) and Y axes stand for log competitor (M).
**Figure 1** **(****Figure 1-1A to 1-2C****):** Purified VHHs blocking hAng2-hTie2 interaction (ELISA).
**Figure 2** **(****Figure 2-1A to 2-2C****):** Purified VHHs blocking mAng2-mTie2 interaction (ELISA).
**Figure 3 (Figure 3A to 3B****):** Purified VHHs blocking cAng2-cTie2 interaction (ELISA).
**Figure 4** **(****Figure 4A to 4I****):** Purified VHHs blocking hAng1-hTie2 interaction (ELISA).
**Figure 5****:** Sequence alignment of affinity matured variants of VHH 28D10. The amino acid sequence is aligned to the human germline VH3/JH consensus sequence. Residues are numbered according to Kabat, CDRs are shown in bold according to AbM definition. Residues that have been substituted are underlined.
**Figure 6 (Figure 6A to 6C****):** Purified affinity matured variants of VHH 28D10 blocking hAng2-hTie2 interaction (ELISA).
**Figure 7 (Figure 7A to 7C****):** Purified affinity matured variants of VHH 28D10 blocking hAng1-hTie2 interaction (ELISA).
**Figure 8 (Figure 8A to 8B****):** Sequence alignment of VHH 1D01 with hVH3-JH consensus (A) and of sequence optimized variants of VHH 1D01 (B). The amino acid sequence is aligned to the human germline VH3/JH consensus sequence. Residues are numbered according to Kabat, CDRs are shown in bold according to AbM definition. Residues to be mutated to their human counterpart are underlined. Potential post-translational modification sites to be tackled are boxed.
**Figure 9** **(****Figure 9-1A to 9-3B****):** Purified sequence optimized variants of VHH 1D01 blocking hAng2-hTie2 (10-1), mAng2-mTie2 (10-2) and cAng2-cTie2 (11-3) interaction (ELISA).
**Figure 10****:** Purified sequence optimized variants of VHH 1D01 blocking hAng1-hTie2 interaction (ELISA).
**Figure 11 (Figure 11A to 11C****):** Sequence alignment of VHH 37F02 with hVH3-JH consensus (A), of cycle 1 (B) and of cycle 2 (C) sequence optimized variants of VHH 37F02. The amino acid sequence is aligned to the human germline VH3/JH consensus sequence. Residues are numbered according to Kabat, CDRs are shown in bold according to AbM definition. Residues to be mutated to their human counterpart are underlined. Potential post-translational modification sites to be tackled are boxed.
**Figure 12** **(****Figure 12-1 to 12-3****):** Purified cycle 1 sequence optimized variants of VHH 37F02 blocking hAng2-hTie2 (14-1), mAng2-mTie2 (14-2) and cAng2-cTie2 (14-3) interaction (ELISA).
**Figure 13** **(****Figure 13-1A to 13-3****):** Purified cycle 2 sequence optimized variants of VHH 37F02 blocking hAng2-hTie2 (15-1), mAng2-mTie2 (15-2) and cAng2-cTie2 (15-3) interaction (ELISA).
**Figure 14****:** Purified cycle 2 sequence optimized variants of VHH 37F02 blocking hAng1-hTie2 interaction (ELISA).
**Figure 15 (Figure 15A to 15D****):** Sequence alignment of VHH 28D10 with hVH3-JH consensus (A), of cycle 1 sequence optimized variants (B), of cycle 2 variants (C) and of cycle 3 (D) sequence optimized variants of VHH 28D10. The amino acid sequence is aligned to the human germline VH3/JH consensus sequence. Residues are numbered according to Kabat, CDRs are shown in bold according to AbM definition. Residues to be mutated to their human counterpart are underlined. Potential post-translational modification sites to be tackled are boxed.
**Figure 16** **(****Figure 16-1A to 16-3C****):** Purified cycle 1 sequence optimized variants of VHH 28D10 blocking hAng2-hTie2 (18-1), mAng2-mTie2 (18-2) and cAng2-cTie2 (18-3) interaction (ELISA).
**Figure 17 (Figure 17A to 17B****):** Purified cycle 1 sequence optimized variants of VHH 28D10 blocking hAng1-hTie2 interaction (ELISA).
**Figure 18 (Figure 18** **-1A to 18 -3):** Purified sequence optimized C₅₀X-S₅₃X variants of VHH 28D10 blocking hAng2-hTie2 (20-1), mAng2-mTie2 (20-2) and cAng2-cTie2 (20-3) interaction (ELISA).
**Figure 19****:** Purified sequence optimized C₅₀X-S₅₃X variant of VHH 28D10 blocking hAng1-hTie2 interaction (ELISA).
**Figure 20** **(****Figure 20-1A to 20-3C****):** Purified cycle 2 sequence optimized variants of VHH 28D10 blocking hAng2-hTie2 (22-1), mAng2-mTie2 (22-2) and cAng2-cTie2 (22-3) interaction (ELISA).

### Examples

### Materials and methods

### a) Generation of HEK293H stable cell lines overexpressing human or mouse Tie2 receptor

The cDNAs encoding human Tie2 (NM_000459.3; SEQ ID NO:182;), mouse Tie2 (NM_013690.2; SEQ ID NO:183) and cyno Tie2 (SEQ ID NO:184); are cloned in pcDNA3.1-neo expression vector (Invitrogen, Carlsbad, CA, USA). To establish Human Embryonic Kidney (HEK) cells overexpressing human Tie2 or mouse Tie2, parental HEK293H cells undergo lipid mediated transfection with Fugene (Roche) with pcDNA3.1-neo-hTie2 or pcDNA3.1-neo-mTie2, respectively. For all conditions, transfectants are selected 2 days post-transfection by adding 1 mg/mL geneticin (Invitrogen, Carlsbad, CA, USA). For human, mouse and cyno Tie2, final high expressing clones are selected by single cell sorting clones binding to PE labeled anti-human Tie2 (R&D Systems, Minneapolis, MN, US), PE labeled anti-mouse Tie2 (eBioscience, San Diego, CA, USA) and a 2-step goat-anti-human Tie2 (R&D Systems, Minneapolis, MN, US) followed by PE labeled donkey-anti-goat (Jackson ImmunoResearch, West Grove, PA, USA), respectively, using the FACSAria Cell Sorter (BD Biosciences, San Jose, CA, USA).

### b) Generation of HEK293T cell lines overexpressing mouse or cynomolgus Ang2 and production of recombinant mouse and cynomolgus Ang2 conditioned medium

The cDNAs encoding N-terminally FLAG-tagged mouse Ang2 (NM_007426.3; SEQ ID SEQ ID NO: 185) and cynomolgus Ang2 (AB172643.1; SEQ ID NO: 186) are cloned in a pSecTag2B expression vector (Invitrogen, Carlsbad, CA, USA). Human Embryonic Kidney (HEK) cells transiently overexpressing mouse Ang2 or cynomolgus Ang2 are generated by lipid-mediated transfection (Fugene; Roche) of pSecTag2B-mAng2 or pSecTag2B-cAng2, respectively, in the HEK293T parental cell line. Productions are performed in 1.5 liter CF10 Bag, and 1.5L conditioned medium (CM) is collected 5 days post-transfection.

### c) Production of recombinant cynomolgus Tie2/Fc chimera in HEK293-F Cells

The cDNA encoding for the extracellular domain of Tie-2 is subcloned into the expression plasmid pSecTag2b using appropriate restriction sites to generate an Fc-fusion protein. Transfection into HEK293-F cells (Invitrogen) is performed as described by the manufacturer using Megaprep (Qiagen) preparations of plasmids, Optimem-Medium (Invitrogen), 293-fectin (Invitrogen) at an initial cell density of 1 x 10⁶ viable cells/mL with 1µg plasmid DNA/10⁶ cells. Transfected cells are cultivated in shaker flasks for 7 days at 37 °C. Conditioned Medium (CM) is harvested by centrifugation at 4000g for 10 min and filtered through a sterile filter (0.45µm membrane).

Fc-fusion proteins are purified using affinity chromatography by loading the CM at 5 ml/min onto a 5 ml MabSelect SuRe Protein A column equilibrated with DPBS. After a washing step with DPBS, bound Fc-protein is eluted with 10 mM sodium citrate buffer pH 3.0 and subsequently neutralized to pH 7.0 by adding 1M Tris/HCl pH 8.0. The purified protein is concentrated and buffer exchanged to DPBS with a Millipore Amicon Ultra (10 kDa molecular weight cuttoff) centrifugal concentrator. Presence of the protein is confirmed with standard analytical methods (electrophoresis with Experion Pro 260 kit- BioRad; mass spectrometry). The protein is further analysed by size-exclusion chromatography and the endotoxin-content is determined (Endosafe PTS kit - Charles River).

### d) Production of recombinant human, cynomolgus, mouse and rat Ang2-FLD in HEK293-F Cells

Molecular cloning and cell culture is performed as described for Tie2-Fc-fusion protein. For purification of His-tagged proteins the CM is loaded at 5 ml/min on a 2 ml Ni²⁺ chelating sepharose fast flow column (His-Trap - GE Healthcare Life Sciences) equilibrated with DPBS. After loading in the presence of 4% elution buffer (DPBS + 0.5% imidazol) to prevent unspecific binding, the column is washed with DPBS. Ang2-FLD-proteins are eluted from the column with DPBS containing 0.5% imidazol. Subsequently an ultrafiltration step was done for concentration and buffer exchange (10 kDa molecular withgt cut off). An aliquot of the protein is retained for analytical characterization as described for Tie2-Fc.

### Example 1

### Immunization with recombinant human Ang2 induces a humoral immune response in llama

### 1.1. Immunizations

After approval of the Ethical Committee of the faculty of Veterinary Medicine (University Ghent, Belgium), 4 llamas (designated No. 406, 408, 454, 455) are immunized with 4 intramuscular injections (day 0: 50 µg, day 14: 20 µg, day 28: 17.5 µg and day 42: 17.5 µg dose) of recombinant human Ang2 (R&D Systems, Minneapolis, MN, US). The antigen is formulated in Complete Freund's Adjuvant for the prime injection at day 0 (Difco, Detroit, MI, USA) and in Incomplete Freund's Adjuvant for the booster injections (Difco, Detroit, MI, USA).

### 1.2. Evaluation of induced immune responses in llama

To evaluate the induction of an immune response in the animals against human Ang2 by ELISA, sera are collected at day 0 (pre-immune), day 35 and day 46 (time of peripheral blood lymphocyte [PBL] collection). In short, 1 µg/mL of recombinant human Ang2 (R&D Systems, Minneapolis, MN, USA) is immobilized overnight at 4°C in a 96-well MaxiSorp plate (Nunc, Wiesbaden, Germany). Wells are blocked with a casein solution (PBS + 1% casein). After addition of serial serum dilutions, specifically bound immunoglobulins are detected using a horseradish peroxidase (HRP)-conjugated goat anti-llama immunoglobulin (Bethyl Laboratories Inc., Montgomery, TX, USA) and a subsequent enzymatic reaction in the presence of the substrate TMB (3,3',5,5'-tetramentylbenzidine) (Pierce, Rockford, IL, USA), showing that a significant antibody dependent immune response against human Ang2 is induced. The antibody response is mounted both by conventional and heavy-chain only antibody expressing B-cell repertoires, since bound immunoglobulins can be detected with antibodies specifically recognizing the conventional llama IgG1 antibodies or the heavy chain only llama IgG2 or IgG3 antibodies. In all llamas injected with human Ang2, an antibody response is mounted by conventional and heavy chain only antibody expressing B-cells specifically against human Ang2. The Ang2 serum titer responses for each llama are depicted in Table 1.

**Table 1: Antibody mediated specific serum response against recombinant human Ang2.**

| | | **IgG reponse** | | |
|---|---|---|---|---|
| **Llama** | **Immunogen** | **IgG1** | **IgG2** | **IgG3** |
| 406 | rec. human Ang2 | +++ | ++ | ++ |
| 408 | rec. human Ang2 | +++ | ++ | ++ |
| 454 | rec. human Ang2 | +++ | ++ | ++ |
| 455 | rec. human Ang2 | +++ | ++ | +++ |

| | | | | |
|---|---|---|---|---|
| Legend: (*) Low (or +/-): 1,000 ≥ HSD_{S/N≥2} < 1,500 Moderate (or +): 1,500 ≥ HSD_{S/N≥2} < 13,500 Good (or ++): 13,500 ≥ HSD_{S/N≥2} < 365,000 Excellent (or +++): HSD_{S/N≥2} ≥ 365,000 (*) HSD, Highest Serum Dilution; S/N ≥ 2, signal-to-noise ratio ≥ 2 | | | | |

### Example 2

### Cloning of the heavy-chain only antibody fragment repertoires and preparation of phage

Following the final immunogen injection, immune tissues as the source of B-cells that produce the heavy-chain only antibodies are collected from the immunized llamas. Typically, two 150 mL blood samples collected 4 and 10 days after the last antigen injection, and one lymph node biopsy, collected 4 days after the last antigen injection are collected per animal. From the blood samples, peripheral blood mononuclear cells (PBMCs) are prepared using Ficoll-Hypaque according to the manufacturer's instructions (Amersham Biosciences, Piscataway, NJ, USA). From the PBMCs and the lymph node biopsy (not prelevated from animal No. 406), total RNA is extracted, which is used as starting material for RT-PCR to amplify the VHH encoding DNA segments, as described in Example 3 (page 46) of WO 05/044858. For each immunized llama, a library is constructed by pooling the total RNA isolated from all collected immune tissues of that animal. In short, the PCR-amplified VHH repertoire is cloned via specific restriction sites into a vector designed to facilitate phage display of the VHH library. The vector is derived from pUC119 and contains the LacZ promoter, a M13 phage gill protein coding sequence, a resistance gene for ampicillin or carbenicillin, a multiple cloning site and a hybrid gIII-peIB leader sequence. In frame with the VHH coding sequence, the vector encodes a C-terminal c-myc tag and a His6 tag. Phage are prepared according to standard protocols and stored after filter sterilization at 4°C for further use.

### Example 3

### Selection of Ang2 specific VHHs via phage display

VHH repertoires obtained from all llamas and cloned as phage library are used in different selection strategies, applying a multiplicity of selection conditions. Variables include i) the Ang2 protein format: biotinylated C-terminally His-tagged full length recombinant human Ang2 (R&D Systems, Minneapolis, MN, USA) and C-terminally His-tagged full length mouse Ang2 (produced at GeneArt, now Invitrogen, Carlsbad, CA, USA), ii) the Ang2 presentation method: plates directly coated with mouse Ang2 or incubation in solution with biotinylated human Ang2 followed by capturing on neutravidin-coated plates, and iii) the antigen concentration. All selections are done in 96 well MaxiSorp plates (Nunc, Wiesbaden, Germany).

Multi-round selections are performed as follows: Ang2 preparations for solid and solution phase selection formats are presented as described above at multiple concentrations (biotinylated human Ang2: 50, 5, 0.5, 0.05 and 0.005 nM; mouse Ang2: 10, 1, 0.1 and 0.01 µg/mL). After 2h incubation with the phage libraries, followed by extensive washing, bound phages are eluted with trypsin (1 mg/mL) for 15-30 minutes at room temperature. Trypsin activity is then immediately neutralized by applying 0.8 mM protease inhibitor ABSF. As background control, selections w/o antigen are performed in parallel. Phage outputs that show enrichment over background are used to infect E. *coli.* Infected E. *coli* cells are either used to prepare phage for the next selection round (phage rescue) or plated on LB agar plates (ampicillin + glucose^{2%}) for analysis of individual VHH clones. In order to screen a selection output for specific binders or blockers, single colonies are picked from the agar plates and grown in 1 mL 96-deep-well plates. LacZ-controlled VHH expression is induced by adding IPTG (0.1-1mM final) in the absence of glucose. Periplasmic extracts (in a volume of -80 uL) are prepared according to standard protocols (as disclosed in for example WO 2006/040153 cited herein). Briefly, cultures were centrifuged for 15 minutes at 4,500 rpm. The pellet was frozen overnight or for 1 hour at -20°C. Next, the pellet was thawed at room temperature for 40 minutes, re-suspended in 15 ml peri buffer (50 mM NaHPO4, 300mM NaCl) and shaken for 1 hour. Periplasmic fraction was isolated by centrifugation for 20 minutes at 14000 rpm.

### Example 4

### Screening of periplasmic extracts in Ang2-Tie2 and Ang1-Tie2 ELISA and AlphaScreen competition assays

Periplasmic extracts containing expressed VHHs are screened in a human Ang2-human Tie2 AlphaScreen competition assay to assess their blocking capacity. In brief, human Tie2/Fc chimera (R&D Systems, Minneapolis, MN, USA) is biotinylated using *N-*hydroxysulfosuccinimide ester of biotin (Thermo Fisher Scientific, Rockford, IL, USA). FLAG tagged human Ang2 (Alexis Biochemicals, San Diego, CA, USA) is captured using Acceptor beads (Perkin Elmer, Waltham, MA, US) coated with anti-FLAG M2 antibody (Sigma, St Louis, MO, USA). To evaluate the capacity of the VHHs to inhibit binding of human Ang2 to its receptor human Tie2, 1:25 dilutions of the periplasmic extracts containing expressed VHHs are incubated with 0.1 nM FLAG tagged human Ang2. To this mixture, the Acceptor beads and 0.3 nM biotinylated human Tie2/Fc chimeras are added and further incubated for 2 hours at room temperature. Finally, streptavidin conjugated Donor beads (Perkin Elmer, Waltham, MA, US) are added and the mixture is incubated for an additional 2 hours at room temperature. Assay buffer is PBS + 0.03% Tween-20 + 0.1% BSA. Fluorescence is measured using the Envision Multilabel Plate reader (Perkin Elmer, Waltham, MA, USA) using an excitation wavelength of 680 nm and an emission wavelength of 520 nm. Decrease in fluorescence signal indicates that the binding of human Ang2 to human Tie2 is blocked by the VHH expressed in the periplasmic extract. VHHs able to block the human Ang2-human Tie2 interaction for at least 50% are screened in a confirmatory ELISA based competition assay. Additionally, cross-reactivity for binding to mouse Ang2 and selectivity over human Ang1 is also assessed in a competition ELISA. In brief, human or mouse Tie2/Fc chimera (R&D Systems, Minneapolis, MN, USA) are immobilized at 2 µg/mL overnight at 4°C in a 96-well MaxiSorp plate (Nunc, Wiesbaden, Germany). Wells are blocked with a 1% casein solution. A 1:5 dilution of periplasmic extract containing expressed VHHs is incubated with the following Ang species according to the type of assay: 0.02 nM FLAG tagged human Ang2, a 1:3,000 dilution of HEK293 conditioned medium containing FLAG tagged mouse Ang2 or 0.02 nM FLAG-tagged human Ang1 (Alexis Biochemicals, San Diego, CA, USA). This mixture is added to the Tie2/Fc coated well and incubated for 2 hours at room temperature.

Residual binding of Ang is detected using HRP-conjugated anti-FLAG M2 antibody (Sigma, St Louis, MO, USA).

In a second screening cycle, periplasmic extracts containing expressed VHHs of selection outputs that yielded a high diversity of mouse Ang2 cross-reactive blocking VHHs are screened at a 1:300 dilution. VHHs inhibiting the binding of human Ang2 to human Tie2, mouse Ang2 to mouse Tie2 and showing no inhibition of human Ang1 binding to human Tie2 are selected. Sequence analysis revealed 86 unique VHHs belonging to 38 different B-cell lineages. The total number of unique sequence variants found for each B-cell lineage, a representative VHH and the selection condition used, is depicted in Table 2. An overview of AlphaScreen and ELISA based screening data is given in Table 3. The amino acid sequences of all unique VHHs are shown in the Sequence Listing (SEQ ID NOs: 1 to 86) and in Table 4.

**Table 2: Selection parameters used for the identification of Ang2 specific VHH B-cell lineages.**

| **B-cell Lineage no.** | **Representative VHH ID** | **# Unique variants** | **Library** | **Selection format** | **Selection rounds** |
|---|---|---|---|---|---|
| 1 | 2F04 | 3 | 408 | biot-hAng2 | 1 or 2 |
| | | | | biot-hAng2 > biot-hAng2 | |
| | | | | rmAng2 > rmAng2 | |
| 2 | 1D01 | 2 | 406 | biot-hAng2 | 1 or 2 |
| | | | | biot-hAng2 > rmAng2 | |
| 3 | 10H02 | 7 | 408 | biot-hAng2 > biot-hAng2 | 1 or 2 |
| | | | | rmAng2 > rmAng2 | |
| 4 | 3A07 | 3 | 454 | biot-hAng2 | 1 or 2 |
| | | | | biot-hAng2 > rmAng2 | |
| 5 | 7G08 | 3 | 454 | biot-hAng2 > biot-hAng2 | 1 or 2 |
| 6 | 2G01 | 1 | 408 | biot-hAng2 | 1 |
| 7 | 8A11 | 1 | 455 | biot-hAng2 > biot-hAng2 | 2 |
| 8 | 16A03 | 4 | 455 | biot-hAng2 | 1 or 2 |
| | | | | biot-hAng2 > rmAng2 | |
| 9 | 14A09 | 2 | 408 | biot-hAng2 | 1 or 2 |
| | | | | biot-hAng2 > rmAng2 | |
| 10 | 11B07 | 18 | 454 | biot-hAng2 | 1 or 2 |
| | | | | biot-hAng2 > biot-hAng2 | |
| | | | | biot-hAng2 > rmAng2 | |
| | | | | rmAng2 > rmAng2 | |
| 11 | 1E01 | 1 | 406 | biot-hAng2 | 1 |
| 12 | 13A03 | 2 | 406 | biot-hAng2 > rmAng2 | 2 |
| 13 | 15A06 | 1 | 454 | biot-hAng2 > rmAng2 | 2 |
| 14 | 11A03 | 2 | 454 | rmAng2 > rmAng2 | 2 |
| 15 | 14H02 | 3 | 408 | biot-hAng2 > rmAng2 | 2 |
| | | | | rmAng2 > rmAng2 | |
| 16 | 14A08 | 1 | 408 | biot-hAng2 > rmAng2 | 2 |
| 17 | 15H04 | 1 | 454 | biot-hAng2 > rmAng2 | 2 |
| 18 | 16G09 | 3 | 455 | biot-hAng2 > rmAng2 | 2 |
| 19 | 13A02 | 1 | 406 | biot-hAng2 > rmAng2 | 2 |
| 20 | 10C06 | 4 | 408 | rmAng2 > rmAng2 | 2 |
| 21 | 12A08 | 1 | 455 | rmAng2 > rmAng2 | 2 |
| 22 | 12B03 | 2 | 455 | biot-hAng2 > rmAng2 | 2 |
| | | | | rmAng2 > rmAng2 | |
| 23 | 10A03 | 1 | 408 | rmAng2 > rmAng2 | 2 |
| 24 | 16A02 | 2 | 455 | biot-hAng2 > rmAng2 | 2 |
| | | | | rmAng2 > rmAng2 | |
| 25 | 10A09 | 3 | 408 | biot-hAng2 > rmAng2 | 2 |
| | | | | rmAng2 > rmAng2 | |
| 26 | 22C07 | 1 | 408 | rmAng2 > rmAng2 | 2 |
| 27 | 21G10 | 2 | 408 | biot-hAng2 > rmAng2 | 2 |
| | | | | rmAng2 > rmAng2 | |
| 28 | 19A03 | 1 | 406 | biot-hAng2 > rmAng2 | 2 |
| 29 | 23C10 | 1 | 454 | biot-hAng2 > rmAng2 | 2 |
| 30 | 25B01 | 1 | 455 | biot-hAng2 > rmAng2 | 2 |
| 31 | 25F01 | 1 | 455 | biot-hAng2 > rmAng2 | 2 |
| 32 | 25D08 | 1 | 455 | biot-hAng2 > rmAng2 | 2 |
| 33 | 24B05 | 1 | 454 | rmAng2 > rmAng2 | 2 |
| 34 | 22G11 | 1 | 408 | rmAng2 > rmAng2 | 2 |
| 35 | 25G04 | 1 | 455 | biot-hAng2 > rmAng2 | 2 |
| 36 | 28D10 | 1 | 408 | biot-hAng2 > rmAng2 | 2 |
| 37 | 32H10 | 1 | 408 | rmAng2 > rmAng2 | 2 |
| 38 | 29B08 | 1 | 408 | biot-hAng2 > rmAng2 | 2 |

**Table 3: Screening of periplasmic extracts containing expressed anti-Ang2 VHH^{(a)}**

| | | | **AlphaScreen** | **ELISA** | | |
|---|---|---|---|---|---|---|
| **B-cell Lineage No.** | **Representative VHH ID** | **# Unique variants** | **hAng2 (% inh)** | **hAng2 (% inh)** | **mAng2 (% inh)** | **hAng1 (% inh)** |
| 1 | 2F04 | 3 | 79-84 | 50-86 | 13-27 | 0-4 |
| 2 | 1D01 | 2 | 77-83 | 97-101 | 85-102 | 0 |
| 3 | 10H02 | 7 | 54-94 | 65-101 | 42-98 | 0-3 |
| 4 | 3A07 | 3 | 53-80 | 0-75 | 1-27 | 0-4 |
| 5 | 7G08 | 3 | 96-96 | 101 | 101 | 0 |
| 6 | 2G01 | 1 | 55 | 39 | 2 | 0 |
| 7 | 8A11 | 1 | 57 | 67 | 13 | 0 |
| 8 | 16A03 | 4 | 54-96 | 0 | 0-4 | 0-2 |
| 9 | 14A09 | 2 | 0-69 | 6-13 | 0-2 | 0 |
| 10 | 11B07 | 18 | 46-92 | 26-107 | 9-93 | 0-2 |
| 11 | 1E01 | 1 | 0 | 2 | 3 | 4 |
| 12 | 13A03 | 2 | 50-53 | 28-40 | 0 | 0-2 |
| 13 | 15A06 | 1 | 72 | 0 | 0 | 0 |
| 14 | 11A03 | 2 | 57-74 | 0-3 | 0 | 0 |
| 15 | 14H02 | 3 | 54-63 | 54-56 | 36-48 | 0-7 |
| 16 | 14A08 | 1 | 52 | 5-5 | 4 | 0 |
| 17 | 15H04 | 1 | 57 | 82 | 38 | 0 |
| 18 | 16G09 | 3 | 55-95 | 57-99 | 21-96 | 1-7 |
| 19 | 13A02 | 1 | 91 | 96 | 97 | 1 |
| 20 | 10C06 | 4 | 64-85 | 84-90 | 43-50 | 0-2 |
| 21 | 12A08 | 1 | 98 | 0 | 2 | 3 |
| 22 | 12B03 | 2 | 83-92 | 92-94 | 11-99 | 0-7 |
| 23 | 10A03 | 1 | 67 | 0 | 0 | 0 |
| 24 | 16A02 | 2 | 67-90 | 0 | 0-1 | 0-1 |
| 25 | 10A09 | 3 | 52-65 | 0-11 | 0-1 | 0-4 |
| 26 | 22C07 | 1 | 73 | 78 | 82 | 0 |
| 27 | 21G10 | 2 | 55-85 | 45-72 | 13-30 | 0 |
| 28 | 19A03 | 1 | 74 | 74 | 22 | 0 |
| 29 | 23C10 | 1 | 57 | 37 | 24 | 0 |
| 30 | 25B01 | 1 | 61 | 5 | 24 | 4 |
| 31 | 25F01 | 1 | 67 | 85 | 42 | 14 |
| 32 | 25D08 | 1 | 78 | 94 | 6 | 10 |
| 33 | 24B05 | 1 | 97 | 102 | 98 | 3 |
| 34 | 22G11 | 1 | 96 | 100 | 98 | 0 |
| 35 | 25G04 | 1 | 90 | 87 | 9 | 0 |
| 36 | 28D10 | 1 | 89 | 118 | 93 | 4 |
| 37 | 32H10 | 1 | 55 | 112 | 58 | 17 |
| 38 | 29B08 | 1 | 60 | 112 | 63 | 0 |

| | | | | | | |
|---|---|---|---|---|---|---|
| ^{(a)}if multiple unique VHH variants within a B-cell lineage are identified, the range (min-max) of % inhibition is given. | | | | | | |

**Table 4: Amino acid sequence of unique anti-Ang2 VHHs identified during screening**

| **VHH ID/SEQ ID NOs:** | **FR1** | **CDR1** | **FR2** | **CDR2** | **FR3** | **CDR3** | **FR4** |
|---|---|---|---|---|---|---|---|
| 001D01/1 | | | | | | | |
| 001E01/2 | | | | | | GPY | |
| 002A01/3 | | | | | | | |
| 002F04/4 | | | | | | | |
| 002G01/5 | | | | | | | |
| 003A07/6 | | | | | | | |
| 003D01/7 | | | | | | | |
| 003E10/8 | | | | | | | |
| 003F02/9 | | | | | | | |
| 003F07/10 | | | | | | | |
| 004B06/11 | | | | | | | |
| 006F05/12 | | | | | | | |
| 006H05/13 | | | | | | | |
| 007B09/14 | | | | | | | |
| 007C01/15 | | | | | | | |
| 007C07/16 | | | | | | | |
| 007G08/17 | | | | | | | |
| 008A11/18 | | | | | | | |
| 010A03/19 | | | | | | | |
| 010A09/20 | | | | | | LLWSGNL | |
| 010A10/21 | | | | | | LLWSANY | |
| 010B02/22 | | | | | | | |
| 010B08/23 | | | | | | | |
| 010C06/24 | | | | | | | |
| 010C07/25 | | | | | | | |
| 010D04/26 | | | | | | | |
| 010E02/27 | | | | | | | |
| 010F10/28 | | | | | | | |
| 010G02/29 | | | | | | | |
| 010G11/30 | | | | | | | |
| 010H02/31 | | | | | | | |
| 011A02/32 | | | | | | | |
| 011A03/33 | | | | | | | |
| 011B07/34 | | | | | | | |
| 011C01/35 | | | | | | | |
| 012A02/36 | | | | | | | |
| 012A08/37 | | | | | | | |
| 012B03/38 | | | | | | | |
| 013A02/39 | | | | | | | |
| 013A03/40 | | | | | | | |
| 014A08/41 | | | | | | | |
| 014A09/42 | | | | | | | |
| 014A11/43 | | | | | | LLWSGNY | |
| 014D03/44 | | | | | | | |
| 014H02/45 | | | | | | | |
| 015A06/46 | | | | | | | |
| 015C05/47 | | | | | | | |
| 015D05/48 | | | | | | | |
| 015H04/49 | | | | | | | |
| 016A01/50 | | | | | | | |
| 016A02/51 | | | | | | | |
| 016A03/52 | | | | | | | I |
| 016A05/53 | | | | | | | |
| 016G09/54 | | | | | | AYEQHTY | |
| 019A03/55 | | | | | | | |
| 019G07/56 | | | | | | | |
| 019G08/57 | | | | | | | |
| 021G10/58 | | | | | | | |
| 022B09/59 | | | | | | | |
| 022C07/60 | | | | | | | |
| 022G03/61 | | | | | | | |
| 022G05/62 | | | | | | | |
| 022G11/63 | | | | | | | |
| 023A04/64 | | | | | | | |
| 023C10/65 | | | | | | | |
| 023D01/66 | | | | | | | |
| 023E02/67 | | | | | | | |
| 023E08/68 | | | | | | | |
| 023F10/69 | | | | | | | |
| 023F11/70 | | | | | | | |
| 024B05/71 | | | | | | | |
| 024G05/72 | | | | | | | |
| 025B01/73 | | | | | | | |
| 025C06/74 | | | | | | AYEQHTY | |
| 025D08/75 | | | | | | | |
| 025F01/76 | | | | | | | |
| 025F07/77 | | | | | | | |
| 025G04/78 | | | | | | | |
| 025G10/79 | | | | | | AYEQHTY | |
| 028D10/80 | | | | | | | |
| 029B08/81 | | | | | | | |
| 032H10/82 | | | | | | | |
| 036H10/83 | | | | | | | |
| 037A09/84 | | | | | | | |
| 037F02/85 | | | | | | | |
| 043E10/86 | | | | | | | |

### Example 5

### Characterization of purified anti-Ang2 VHHs

A subset of inhibitory anti-Ang2 VHHs selected from the screening described in Example 4 are further purified and characterized. Selected VHHs are expressed in *E. coli* TG1 as c-myc, His6-tagged proteins. Expression is induced by addition of 1 mM IPTG and allowed to continue for 4 hours at 37°C. After spinning the cell cultures, periplasmic extracts are prepared by freeze-thawing the pellets. These extracts are used as starting material and VHHs are purified via IMAC and size exclusion chromatography (SEC) resulting in ≥ 95% purity as assessed via SDS-PAGE.

### 5.1. Evaluation of hAng2 blocking VHHs in ELISA

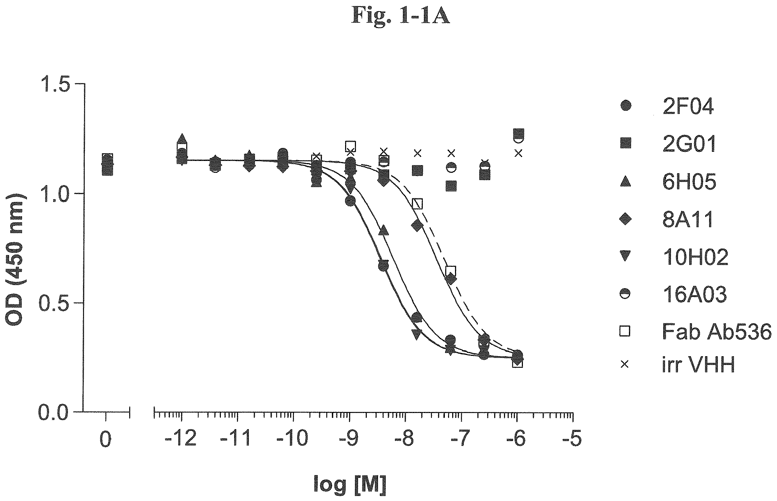

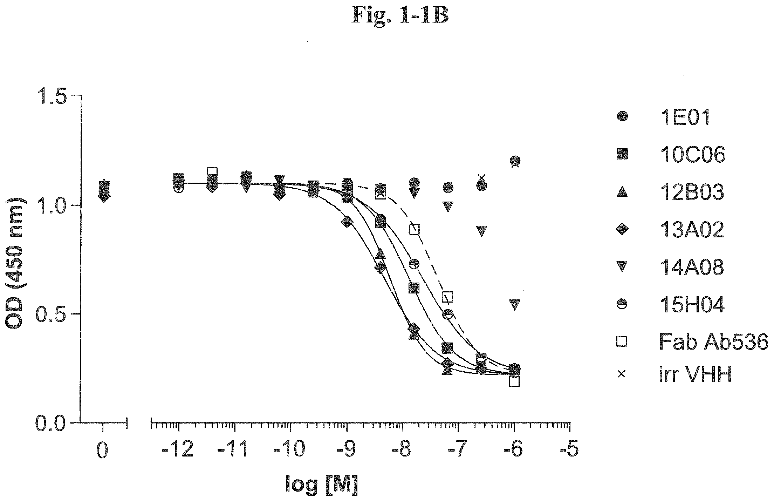

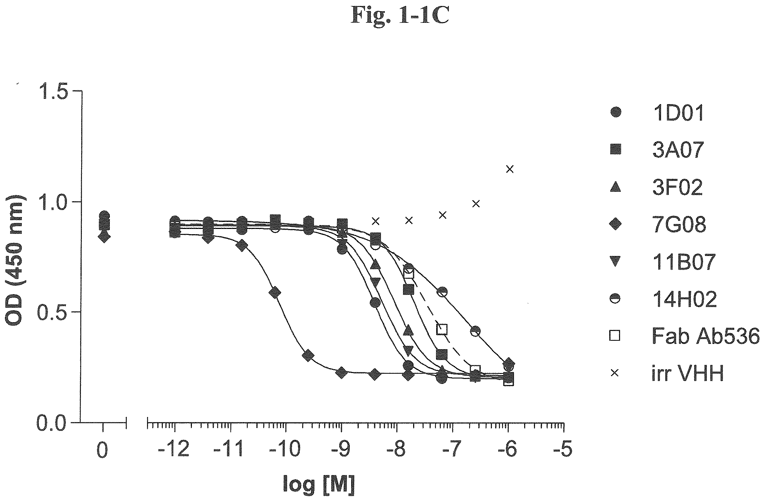

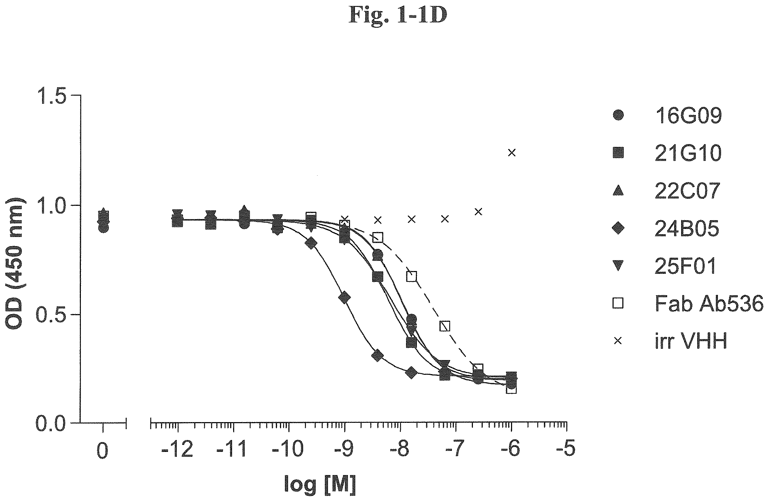

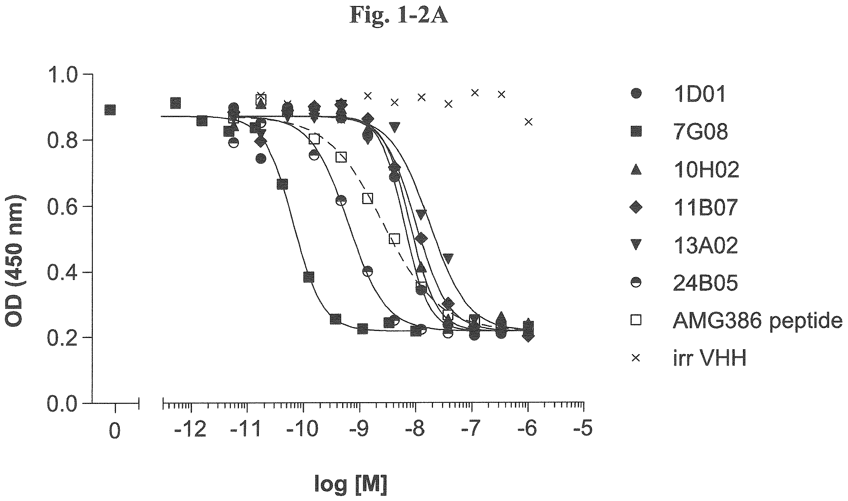

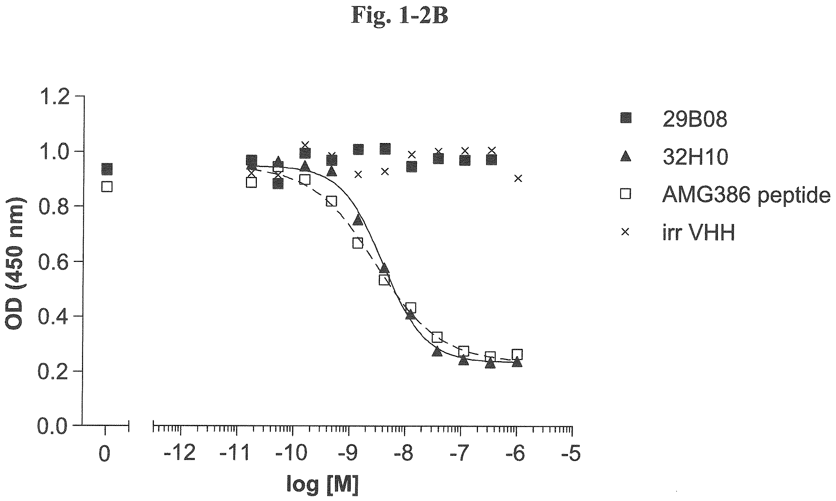

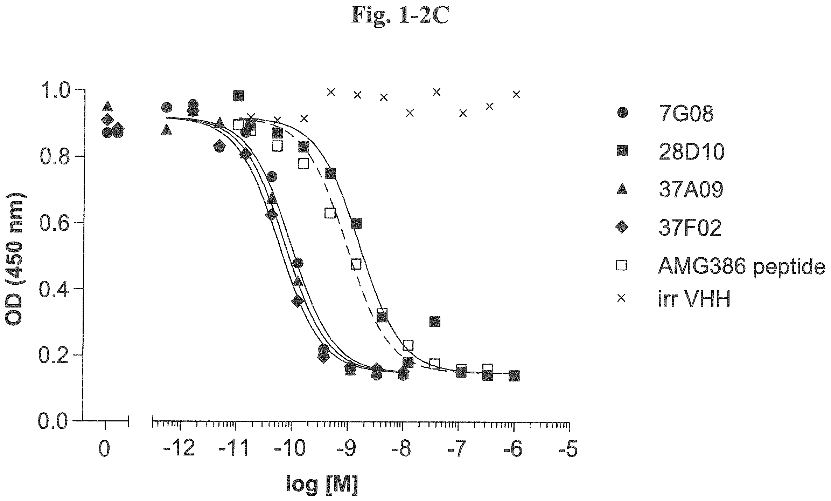

The blocking capacity of the VHHs is evaluated in a human Ang2-human Tie2 blocking ELISA. In brief, 2 µg/mL of Tie2/Fc chimera (R&D Systems, Minneapolis, MN, USA) is coated in a 96-well MaxiSorp plate (Nunc, Wiesbaden, Germany). A fixed concentration of 0.02 nM FLAG-tagged human Ang2 (Alexis Biochemicals, San Diego, CA, USA) is added to a dilution series of the purified VHH (diluted in PBS+0.1% casein+0.05% Tween-20), and incubated on the coated human Tie2 receptor for 2 hours. Residual binding of human Ang2 is detected using horseradish peroxidase (HRP) conjugated anti-FLAG M2 (Sigma, St. Louis, MO, USA) (Figure 1). Reference molecule is the Fab fragment of Ab536 (US2009/0191212) (Figure 1-1) or the peptide moiety of peptibody AMG386 (SEQ ID NO:25 in WO2004/092215) (Figure 1-2). As negative control an irrelevant VHH is used. The IC₅₀ values for VHHs blocking the human Ang2-human Tie2 interaction are depicted in Table 5-1 and Table 5-2, respectively.

**Table 5-1: IC₅₀ (nM) values of purified VHHs blocking the hAng2/hTie2 interaction (competition ELISA; VHH: n=2-3; Fab Ab536: n=6)**

| **VHH ID** | **IC₅₀ (nM)** |
|---|---|
| 1D01 | 3.4 |
| 2F04 | 2.8 |
| 3A07 | 21.0 |
| 3F02 | 9.1 |
| 6H05 | 5.3 |
| 7G08 | 0.07 |
| 8A11 | 30.2 |
| 10C06 | 7.7 |
| 10H02 | 3.0 |
| 11B07 | 5.4 |
| 12B03 | 4.6 |
| 13A02 | 4.1 |
| 14H02 | 64.4 |
| 15H04 | 18.6 |
| 16G09 | 11.3 |
| 21G10 | 6.2 |
| 22C07 | 11.0 |
| 24B05 | 1.0 |
| 25F01 | 6.5 |
| Fab Ab536 | 39.3 |

**Table 5-2: IC₅₀ (nM) values of purified VHHs blocking the hAng2-hTie2 interaction (competition ELISA; VHH: n=1-3; AMG386 peptide: n=3)**

| **VHH ID** | **IC₅₀ (nM)** |
|---|---|
| 1D01 | 6.2 |
| 7G08 | 0.04 |
| 10H02 | 8.7 |
| 11B07 | 14.0 |
| 13A02 | 23.0 |
| 24B05 | 1.1 |
| 28D10 | 1.3 |
| 32H10 | 4.0 |
| 37A09 | 0.1 |
| 37F02 | 0.08 |
| AMG386 peptide | 3.4 |

### 5.2. Evaluation of cross-reactivity towards mouse and cynomolgus Ang2 in blocking ELISA

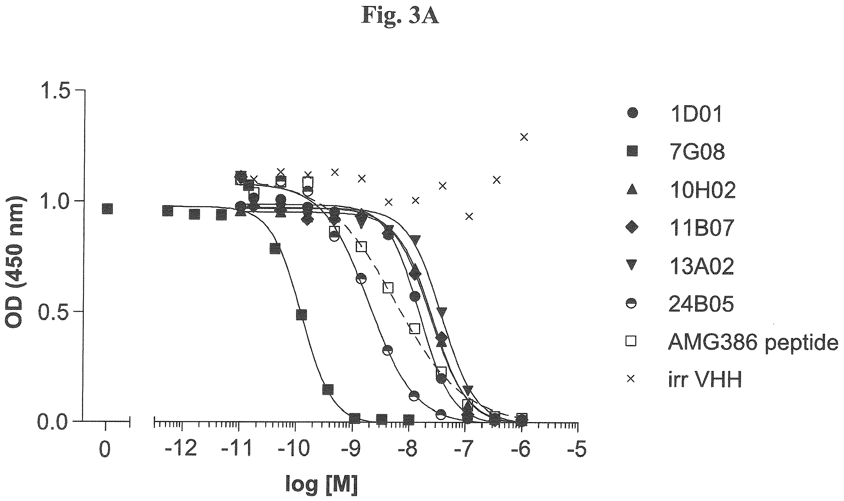

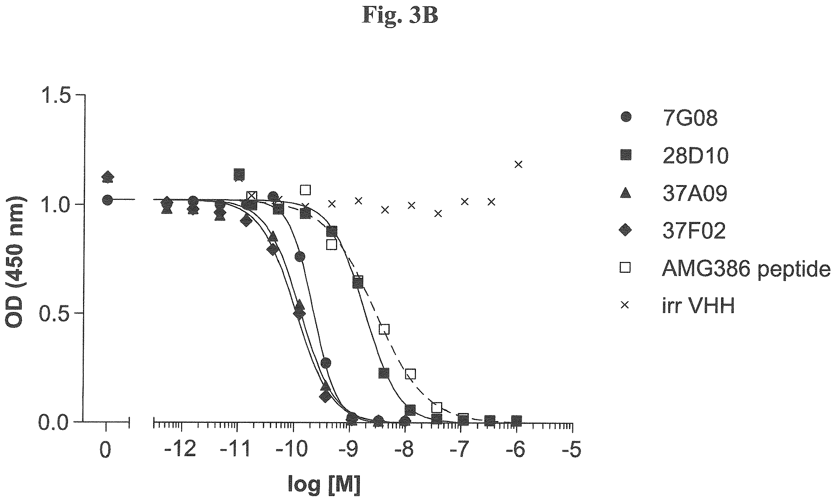

In order to determine if the VHH inhibits binding of mouse Ang2 to mouse Tie2 and cyno Ang2 to cyno Tie2, a competition ELISA is performed. In brief, 2 µg/mL of recombinant mouse Tie2-Fc or cyno Tie2-Fc is coated overnight at 4°C in a 96-well MaxiSorp plate (Nunc, Wiesbaden, Germany). Coated wells are blocked with a 1% casein solution. FLAG-tagged mouse Ang2 (1:3,000 dilution of conditioned medium from transient HEK transfection) or FLAG-tagged cyno Ang2 (1:800 dilution of conditioned medium from transient HEK transfection) and a dilution series of purified VHH (diluted in PBS+0.1% casein+0.05% Tween-20) are incubated on the coated Tie2-Fc receptor for 2 hours at room temperature to reach binding equilibrium. Residual binding of FLAG-mAng2 or FLAG-cAng2 is detected using HRP conjugated anti-FLAG M2 mAb (Sigma, St. Louis, MO, USA). Reference molecule is the Fab fragment of Ab536 (mouse: Figure 2-1) or the peptide moiety of peptibody AMG386 (mouse: Figure 2-2; cyno: Figure 3). As negative control an irrelevant VHH is used. The IC₅₀ values for VHHs blocking the mouse Ang2-mouse Tie2 interaction are depicted in Table 6-1. The IC₅₀ values for VHHs blocking the mouse and cyno Ang2 binding to mouse and cyno Tie2, respectively, is shown in Table 6-2.

**Table 6-1: IC₅₀ (nM) values of purified VHHs blocking the interaction of mAng2 to mTie2 (competition ELISA; VHH: n=2-3; Fab Ab536: n=5)**

| | **mAng2** |
|---|---|
| VHH ID | IC₅₀ (nM) |
| 1D01 | 6.3 |
| 2F04 | 57.4 |
| 3A07 | 99.3 |
| 3F02 | 32.7 |
| 6H05 | 7.7 |
| 7G08 | 0.09 |
| 8A11 | 442.1 |
| 10C06 | 45.2 |
| 10H02 | 5.2 |
| 11B07 | 21.0 |
| 12B03 | 6.7 |
| 13A02 | 6.1 |
| 14H02 | 143.2 |
| 15H04 | 124.6 |
| 16G09 | 19.4 |
| 21G10 | 16.8 |
| 22C07 | 13.6 |
| 24B05 | 1.5 |
| 25F01 | 13.3 |
| Fab Ab536 | 15.3 |

**Table 6-2: IC₅₀ (nM) values of purified VHHs blocking the interaction of mAng2 and cAng2 to mTie2 and cTie2, respectively (competition ELISA; VHH: n=1-3; AMG386 peptide: n=3; n.d., not determined)**

| | **mAng2** | **cAng2** |
|---|---|---|
| **VHH ID** | **IC₅₀ (nM)** | **IC₅₀ (nM)** |
| 1D01 | 10.0 | 16.4 |
| 7G08 | 0.07 | 0.14 |
| 10H02 | 21.4 | 23.7 |
| 11B07 | 39.7 | 24.8 |
| 13A02 | 26.6 | 33.1 |
| 24B05 | 1.1 | 2.1 |
| 28D10 | 6.1 | 2.0 |
| 32H10 | 13.0 | n.d. |
| 37A09 | 0.1 | 0.2 |
| 37F02 | 0.09 | 0.1 |
| AMG386 peptide | 5.2 | 6.6 |

### 5.3. Evaluation of selectivity of human Ang2 blocking VHHs towards human Ang1 in ELISA

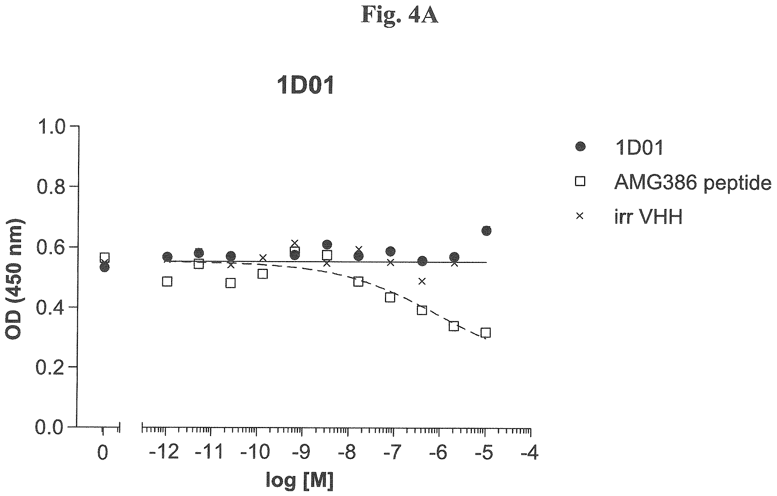

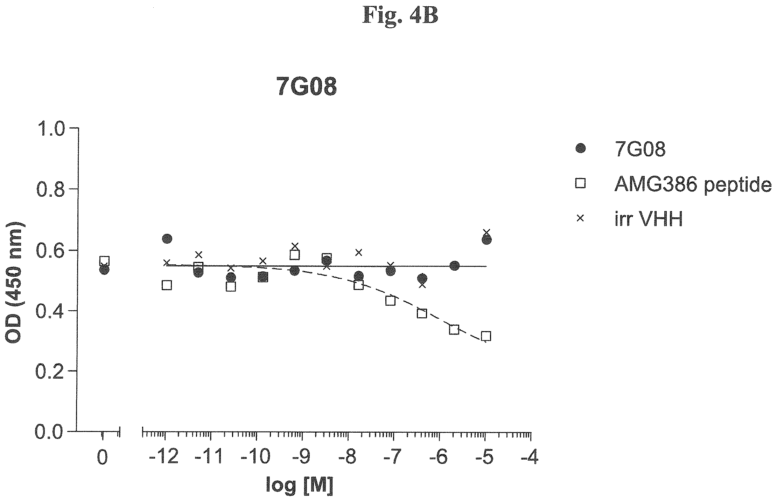

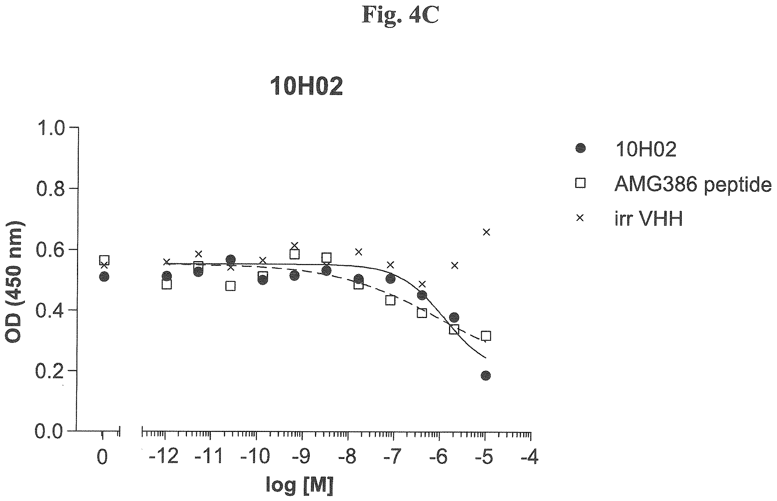

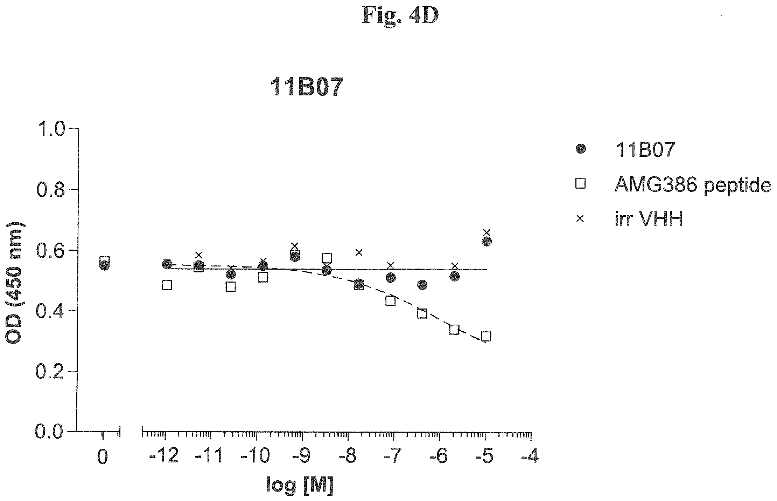

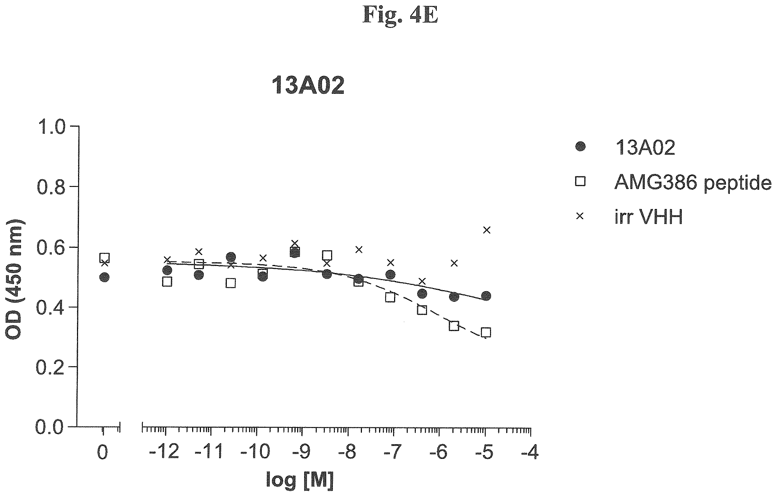

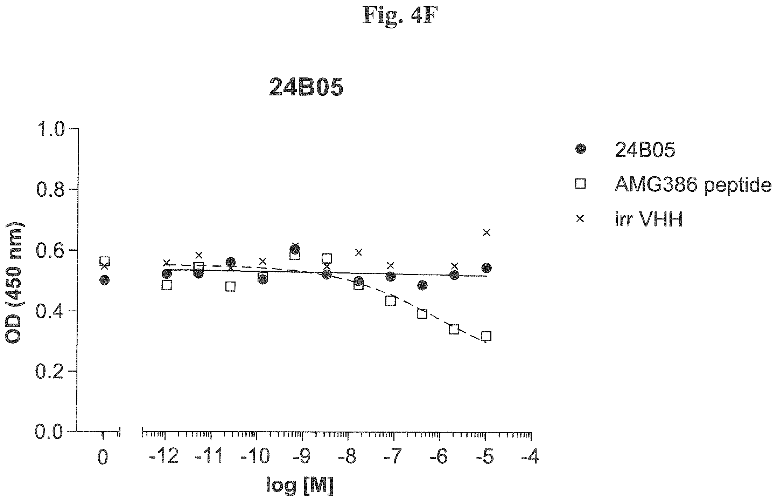

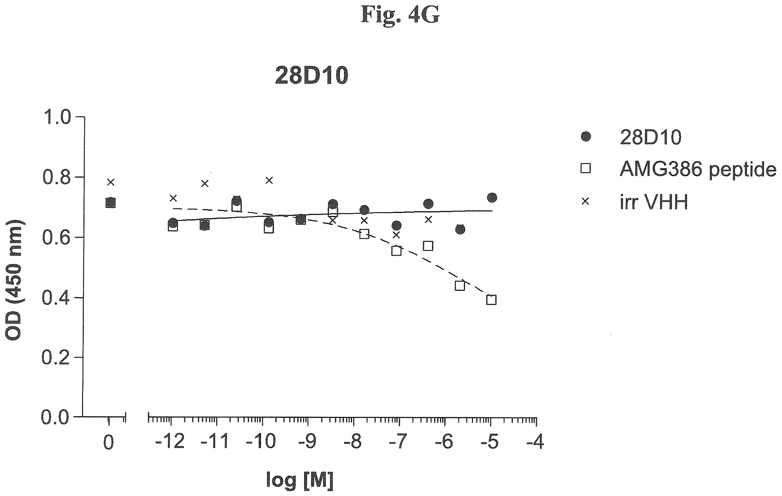

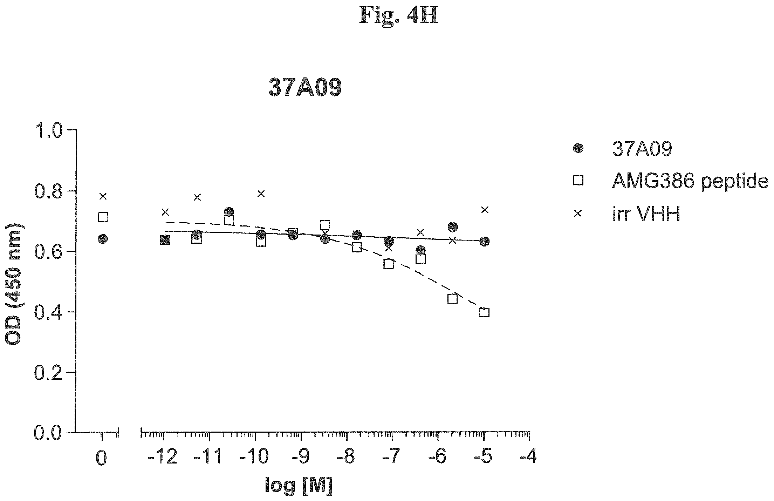

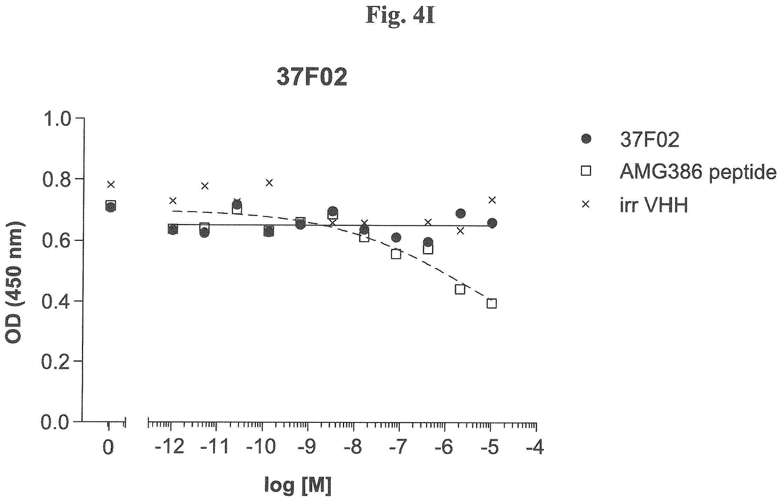

In order to determine whether the anti-Ang2 blocking VHHs are selective over human Ang1 binding to human Tie2, a competition ELISA is performed. In brief, 2 µg/mL of recombinant human Tie2-Fc (R&D Systems, Minneapolis, MN, USA) is coated overnight at 4°C in a 96-well MaxiSorp plate (Nunc, Wiesbaden, Germany). Coated wells are blocked with a 1% casein solution. A fixed concentration (0.02 nM) of FLAG-tagged recombinant human Ang1 (Alexis Biochemicals, San Diego, CA, USA) and a dilution series of VHH (diluted in PBS+0.1%casein+0.05%Tween-20) are incubated on the coated receptor Tie2-Fc for 2 hours at room temperature to reach binding equilibrium. Residual binding of FLAG-hAng1 is detected using HRP conjugated anti-FLAG M2 mAb. Reference molecule is the peptide moiety of AMG386 peptibody (Figure 4). As negative control an irrelevant VHH is used. The indicative IC₅₀ values for VHHs blocking the human Ang1 - human Tie2 interaction are depicted in Table 7.

**Table 7: IC₅₀ (nM) values of purified VHHs blocking the interaction of human Ang1 to human Tie2 (competition ELISA; VHH: n=2-3; AMG386 peptide: n=3)**

| **VHH ID** | **IC₅₀ (nM)** | **hAng1/hAng2 ratio** |
|---|---|---|
| 1D01 | >67,000 | >10,800 |
| 7G08 | >84,000 | >2,333,333 |
| 10H02 | 2,000 | 230 |
| 11B07 | 120,000 | 8,570 |
| 13A02 | 17,000 | 739 |
| 24B05 | 120,000 | 109,090 |
| 28D10 | >4,000 | > 3,076 |
| 37A09 | >10,000 | > 100,000 |
| 37F02 | >10,000 | > 100,000 |
| AMG386 peptide | 4,000 | 1,176 |

### 5.4. Determining the affinity of the human, mouse, cyno Ang2 - VHH interaction

Affinities of the VHH for binding to human, mouse and cyno Ang2 are determined using surface plasmon resonance (SPR) analysis (Biacore T100). In brief, VHH and benchmark compounds are immobilized on a CM5 chip via amine coupling. A multi-cycle kinetic approach is used: different concentrations of human, mouse and cyno Ang2-FLD (0.4-1-2.6-6.4-16-40-100 nM) are injected. Ang2-FLD species are allowed to associate for 2 min and to dissociate for 20 min at a flow rate of 45 µL/min. In between injections, the surfaces are regenerated with a 10 sec pulse of 25 mM NaOH and 60 sec stabilization period. Association/dissociation data are evaluated by fitting a 1:1 interaction model (Langmuir binding). The affinity constant K_{D} is calculated from resulting association and dissociation rate constants kₐ and k_{d} and are depicted in Table 8.

**Table 8: Affinity K_{D} (nM) of purified VHHs for human, mouse and cyno Ang2**

| **VHH ID** | **human Ang2-FLD** | | | **mouse Ang2-FLD** | | | **cyno Ang2-FLD** | | |
|---|---|---|---|---|---|---|---|---|---|
| | **kₐ (1/Ms)** | **k_{d} (1/s)** | **K_{D} (M)** | **kₐ (1/Ms)** | **k_{d} (1/s)** | **K_{D} (M)** | **kₐ (1/Ms)** | **k_{d} (1/s)** | **K_{D} (M)** |
| 1D01 | 7.7E+06 | 1.5E-02 | 2.0E-09 | 3.3E+06 | 1.5E-02 | 4.6E-09 | 9.9E+06 | 1.4E-02 | 1.4E-09 |
| 7G08 | 1.0E+06 | 1.0E-04 | 9.7E-11 | 8.1E+05 | 1.4E-04 | 1.8E-10 | 1.5E+06 | 1.1E-04 | 7.2E-11 |
| 10H02 | 5.7E+06 | 2.1E-02 | 3.6E-09 | 2.5E+06 | 2.6E-02 | 1.1E-08 | 6.9E+06 | 2.3E-02 | 3.4E-09 |
| 11B07 | 9.2E+06 | 6.2E-02 | 6.7E-09 | 4.8E+06 | 1.4E-01 | 2.9E-08 | 1.2E+07 | 7.3E-02 | 6.1E-09 |
| 13A02 | 9.2E+06 | 9.1E-02 | 9.9E-09 | 1.9E+06 | 3.4E-02 | 1.8E-08 | 1.1E+07 | 9.4E-02 | 8.7E-09 |
| 24B05 | 2.6E+06 | 2.5E-03 | 9.6E-10 | 1.7E+06 | 2.9E-03 | 1.7E-09 | 4.1 E+06 | 2.4E-03 | 5.9E-10 |
| 28D10 | 4.9E+06 | 6.2E-03 | 1.3E-09 | 1.9E+06 | 1.1E-02 | 5.6E-09 | 1.8E+07 | 2.5E-02 | 1.4E-09 |
| mAb3.19.3 | 5.0E+07 | 5.5E-02 | 1.1E-09 | 1.1E+07 | 6.1E-02 | 5.5E-09 | n.d. | n.d. | n.d. |
| FabAb536 | 3.1E+06 | 3.7E-02 | 1.2E-08 | 1.6E+06 | 1.7E-02 | 1.1E-08 | 4.1 E+06 | 4.5E-02 | 1.1E-08 |

### Example 6

### Affinity maturation of selected VHH

A variant of VHH 28D10 (00027 carrying C₅₀S/S₅₃N and Q₁₀₈L substitution - Example 7.3) is subjected to affinity maturation.

In a first cycle, amino acid substitutions are introduced randomly in both framework (FW) and complementary determining regions (CDR) using the error-prone PCR method. Mutagenesis is performed in a two-round PCR-based approach using the Genemorph II Random Mutagenesis kit (Stratagene, La Jolla, CA, USA) using 1 ng of VHH 00027 cDNA template, followed by a second error-prone PCR using 0.1 ng of product of round 1. After a polish step, PCR products are inserted via unique restriction sites into a vector designed to facilitate phage display of the VHH library. Consecutive rounds of in-solution selections are performed using decreasing concentrations of biotinylated recombinant human Ang2 (R&D Systems, Minneapolis, MN, USA) and trypsin elutions. Periplasmic extracts (in a volume of ∼ 80 uL) are prepared according to standard methods and screened for binding to recombinant human Ang2-FLD in a ProteOn (BioRad, Hercules, CA, USA) off-rate assay. In brief, a GLC ProteOn Sensor chip is coated with recombinant human Ang2-FLD on the "ligand channels" L3, L4, L5 and L6 (with L1/L2 as reference channel). Periplasmic extract of affinity matured clones is diluted 1:10 and injected across the "analyte channels" A1-A6. An average off-rate is calculated of the reference VHH 00027 which is prepared and tested in the same way as the affinity matured VHHs and serves as a reference to calculate off-rate improvements. The top 25 affinity matured variants are shown in Table **9.** VHH are sequenced (Table **10**-A) to identify amino acid mutations beneficial for improving the off-rate (Table **10**-B).

**Table 9: Off-rate and fold improvement of affinity matured variants of VHH 00027.**

| **VHH ID** | **k_{d} (1/s)** | **fold improvement** |
|---|---|---|
| 64G03 | 7.4E-05 | 15.7 |
| 64F03 | 1.1E-04 | 10.8 |
| 64D11 | 1.1E-04 | 10.3 |
| 64G11 | 1.2E-04 | 9.6 |
| 55D06 | 1.2E-04 | 9.4 |
| 64F07 | 1.2E-04 | 9.4 |
| 64G02 | 1.2E-04 | 9.4 |
| 55A06 | 1.3E-04 | 9.2 |
| 64C03 | 1.3E-04 | 9.2 |
| 64G12 | 1.3E-04 | 8.9 |
| 65F03 | 1.4E-04 | 8.9 |
| 55F02 | 1.4E-04 | 8.7 |
| 64E12 | 1.3E-04 | 8.7 |
| 60C09 | 1.6E-04 | 8.5 |
| 64B02 | 1.4E-04 | 8.5 |
| 64A03 | 1.4E-04 | 8.3 |
| 64C07 | 1.4E-04 | 8.3 |
| 60A06 | 1.6E-04 | 8.3 |
| 64B01 | 1.4E-04 | 8.2 |
| 64G01 | 1.4E-04 | 8.2 |
| 56A07 | 1.5E-04 | 8.1 |
| 58D10 | 1.3E-04 | 8.1 |
| 65F01 | 1.6E-04 | 8.1 |
| 53A06 | 1.6E-04 | 8.0 |
| 55G03 | 1.5E-04 | 8.0 |

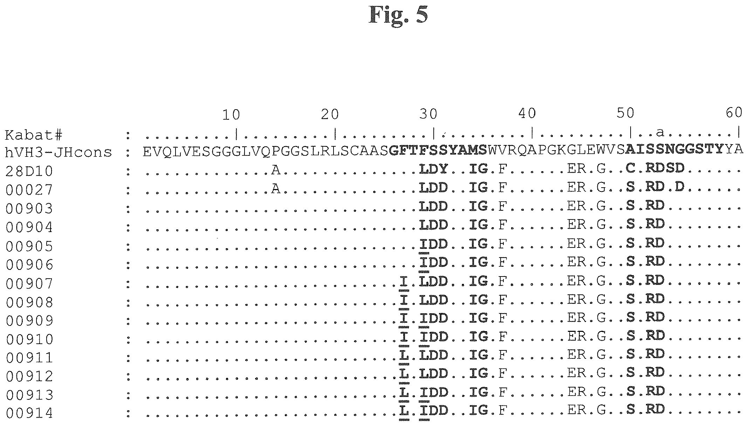

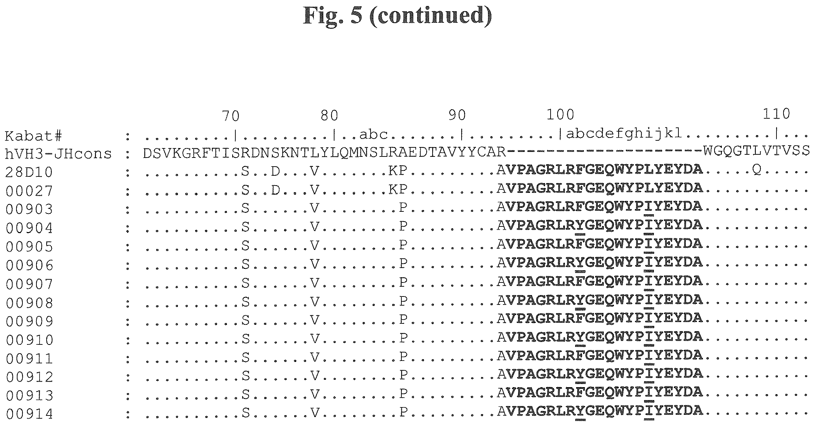

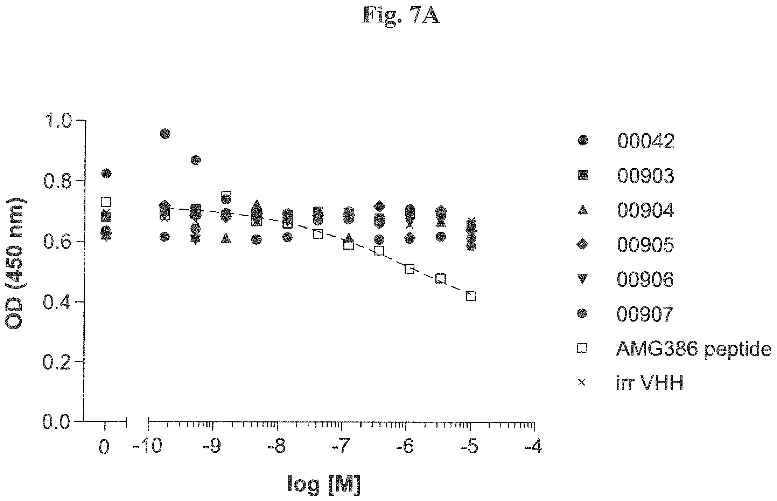

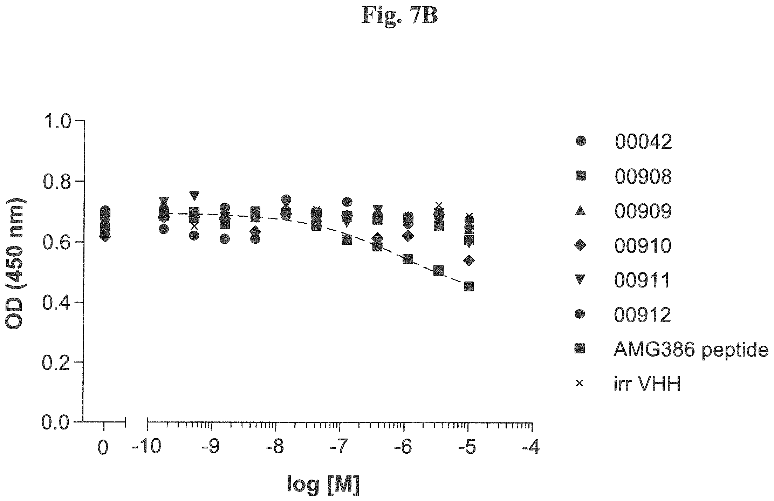

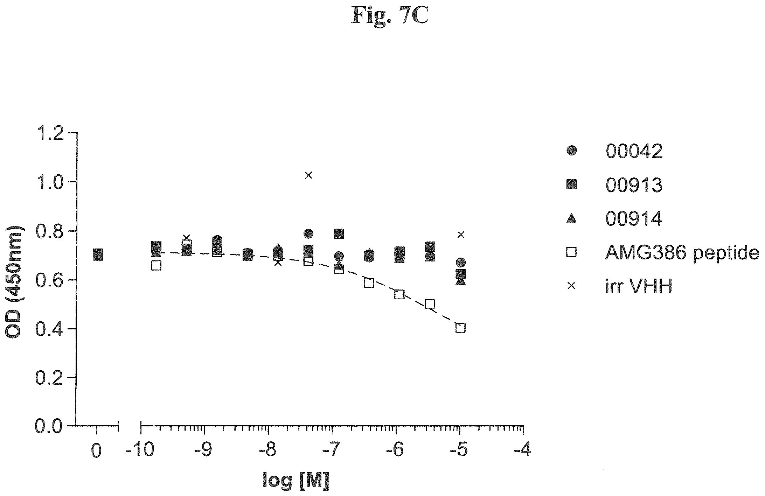

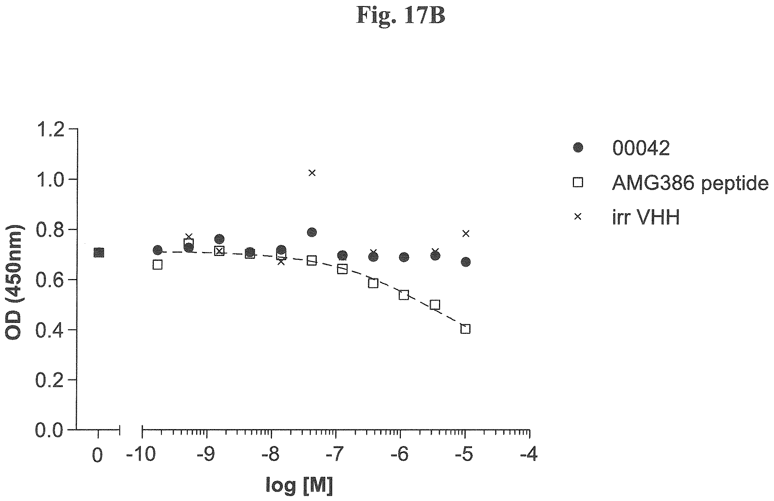

Initially 12 VHH variants containing combinations of mutations on Kabat position 27, 29, 100b and 100i (Table 11; Figure 5) are constructed. The different combinations of these 4 mutations are grafted on the sequence optimized VHH 00042 backbone (Figure 17-B) containing an additional D₅₄G substitution (Example 6.3). The amino acid sequence is aligned to the human germline VH3/JH consensus sequence. Residues are numbered according to Kabat, CDRs are shown in grey according to AbM definition (Oxford Molecular's AbM antibody modeling software). Constructs are cloned into the expression vector pAX100 in frame with a C-terminal c-myc tag and a (His)6 tag. VHH variants are produced in *E. coli* and purified by IMAC and SEC. Sequences are represented in Table 11. All these VHH are analysed in the hAng2/hTie2 (Example 5.1; results shown in Figure 6 and Table 12), and hAng1/hTie2 competition ELISA (Example 5.3; results shown in Figure 7 and Table 12). Additionally, the melting temperature (Tₘ) of each variant at pH7 is determined in a thermal shift assay, which is based on the temperature dependent change in fluorescence signal upon incorporation of Sypro Orange (Invitrogen, Carlsbad, CA, USA) (Ericsson et al, Anal. Biochem. 357 (2006), pp289-298) (Table 12).

**Table 10-A: Amino acid sequence of affinity matured anti-Ang2 VHHs**

| **VHH ID/SEQ ID NOs:** | **FR1** | **CDR1** | **FR2** | **CDR2** | **FR3** | **CDR3** | **FR4** |
|---|---|---|---|---|---|---|---|
| 64G03/87 | | | | | | | |
| 64F03/88 | | | | | | | |
| 64D11/89 | | | | | | | |
| 64G11/90 | | | | | | | |
| 55D06/91 | | | | | | | |
| 64F07/92 | | | | | | | |
| 64G02/93 | | | | | | | |
| 55A06/94 | | | | | | | |
| 64C03/95 | | | | | | | |
| 64G12/96 | | | | | | | |
| 65F03/97 | | | | | | | |
| 55F02/98 | | | | | | | |
| 64E12/99 | | | | | | | |
| 60C09/100 | | | | | | | |
| 64B02/101 | | | | | | | |
| 64A03/102 | | | | | | | |
| 64C07/103 | | | | | | | |
| 60A06/104 | | | | | | | |
| 64B01/105 | | | | | | | |
| 64G01/106 | | | | | | | |
| 56A07/107 | | | | | | | |
| 58D10/108 | | | | | | | |
| 65F01/109 | | | | | | | |
| 53A06/110 | | | | | | | |
| 55G03/111 | | | | | | | |

**Table 10-B: Single mutations or combination thereof yielding improvements in off-rate**

| **Mutation(s)** | **Fold improvement off-rate** |
|---|---|
| A24V | 2 |
| F27I | 1.9 - 2.2 |
| F27L ; L100il | 9.4 |
| L29I ; L100il | 5.6 - 6.0 |
| F100bY ; L100il | 5.1 - 9.2 |
| L100il | 3.1 - 7.0 |

**Table 11: Amino acid sequence of affinity matured anti-Ang2 VHHs**

| **VHH ID/SEQ ID NO:** | **FR1** | **CDR1** | **FR2** | **CDR2** | **FR3** | **CDR3** | **FR4** |
|---|---|---|---|---|---|---|---|
| 00903/112 | | | | | | | |
| 00904/113 | | | | | | | |
| 00905/114 | | | | | | | |
| 00906/115 | | | | | | | |
| 00907/116 | | | | | | | |
| 00908/117 | | | | | | | |
| 00909/118 | | | | | | | |
| 00910/119 | | | | | | | |
| 00911/120 | | | | | | | |
| 00912/121 | | | | | | | |
| 00913/122 | | | | | | | |
| 00914/123 | | | | | | | |

**Table 12: Overview of Tₘ, IC₅₀ (pM) in human, mouse and cyno Ang2 competition ELISA and hAng1/hAng2 IC₅₀ ratios of affinity matured variants of VHH 00027**

| | **TSA** | **IC₅₀ in Ang2/Tie2 ELISA** | | | |
|---|---|---|---|---|---|
| **VHH ID** | **Tm @ pH 7.0 (°C)** | **hAng2 (pM)** | **mAng2 (pM)** | **cAng2 (pM)** | **hAng1/hAng2 IC₅₀ ratio** |
| 00027 | 61.1 | 672 | 1975 | 728 | > 14,878 |
| 00042 | 61.9 | 646 | 1910 | 753 | > 15,476 |
| 00903 | 64.0 | 89 | n.d. | n.d. | > 112,202 |
| 00904 | 64.4 | 62 | n.d. | n.d. | > 162,181 |
| 00905 | 64.0 | 79 | n.d. | n.d. | > 125,893 |
| 00906 | 64.4 | 45 | n.d. | n.d. | > 223,872 |
| 00907 | 67.3 | 62 | n.d. | n.d. | > 162,181 |
| 00908 | 67.3 | 45 | 85 | 79 | > 192,014 |
| 00909 | 66.1 | 53 | n.d. | n.d. | > 190,546 |
| 00910 | 66.1 | 42 | n.d. | n.d. | > 239,883 |
| 00911 | 66.1 | 59 | n.d. | n.d. | > 169,824 |
| 00912 | 66.1 | 62 | n.d. | n.d. | > 162,181 |
| 00913 | 64.0 | 42 | n.d. | n.d. | > 239,883 |
| 00914 | 64.4 | 37 | n.d. | n.d. | > 269,153 |

From potency, Tₘ and sequence perspective VHH 00908 is taken forward into a second cycle of combined affinity maturation and sequence optimization (Example 7.3).

### Example 7

### Sequence optimization of selected VHH 1D01, 28D10 and 37F02

### 7.1 VHH 1D01

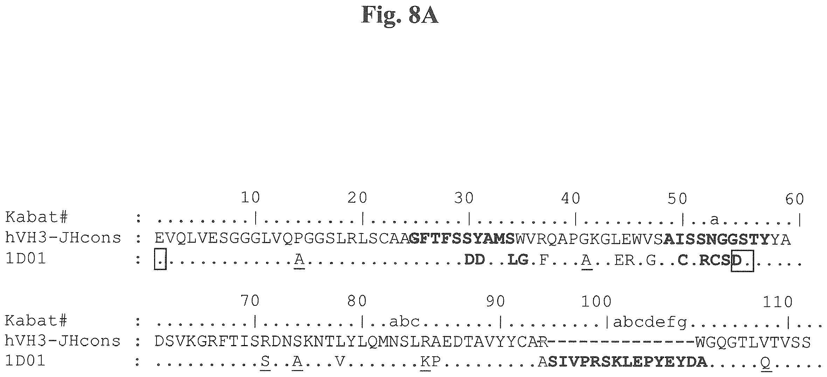

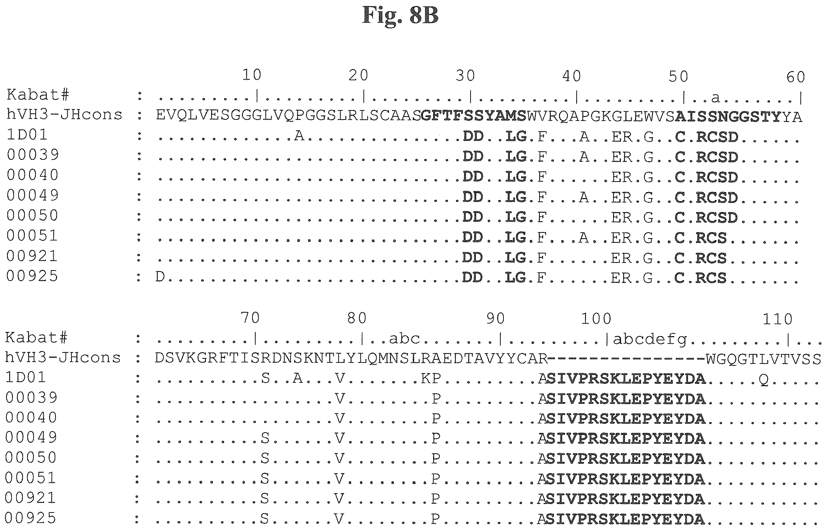

The amino acid sequence of anti-Ang2 VHH 1D01 (see Figure 8-A) is aligned to the human germline VH3/JH consensus sequence. Residues are numbered according to Kabat, CDRs are shown in grey according to AbM definition (Oxford Molecular's AbM antibody modeling software). Residues to be mutated to their human counterpart are underlined. Potential post-translational modification sites to be tackled are boxed. The alignment shows that 1D01 contains 6 framework mutations relative to the reference germline sequence. Non-human residues at positions 14, 41, 71, 74, 83 and 108 are selected for substitution with their human germline counterpart. A set of seven 1D01 variants carrying different combinations of human residues on these positions (Figure 8-B) is constructed and produced (Example 5). In parallel, in 3 of these 7 variants a potential Asp isomerization site at position D₅₄G₅₅ is removed by introducing a D₅₄G substitution, and in 1 of these 7 variants a potential pyroGlu formation site at position E₁ is removed by an E₁D substitution (AA sequences are listed in Table 15).

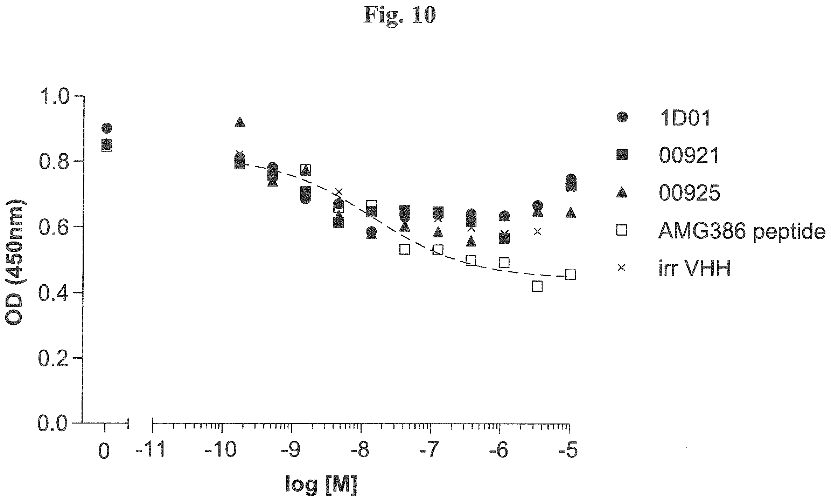

These variants are characterized as purified protein in the human (Figure 9-1), mouse (Figure 9-2) and cyno (Figure 9-3) Ang2/Tie2 competition ELISA (Example 5.1; Example 5.2), the hAng1/hTie2 competition ELISA (Example 5.3; Figure 10). Additionally, melting temperature (Tₘ) of each variant is determined in thermal shift assay (Example 6). An overview of the data can be found in Table 13. Additionally, % FR identity to the human germline is calculated according to AbM definition (Oxford Molecular's AbM antibody modeling software). Affinity of VHH 00921 for human, cyno, mouse and rat Ang2 is shown in Table 14 (Example 5.4).

**Table 13: Overview of Tₘ, IC₅₀ (nM) in human, mouse and cyno Ang2 competition ELISA and hAng1/hAng2 IC₅₀ ratios of sequence optimized variants of VHH 1D01**

| | **TSA** | **IC₅₀ in Ang2-Tie2 ELISA** | | | | **% FR identity** |
|---|---|---|---|---|---|---|
| **VHH ID** | **Tₘ (°C) @ pH7** | **hAng2 (nM)** | **mAng2 (nM)** | **cAng2 (nM)** | **hAng1/hAng2 IC₅₀ ratio** | **AbM** |
| 1D01 | 65.0 | 6.6 | 12.4 | 9.1 | > 1,511 | 85.4 |
| 00039 | 61.5 | 11.9 | 28.1 | 6.4 | n.d | 91.0 |
| 00040 | 64.0 | 6.4 | 29.0 | 5.8 | n.d | 92.1 |
| 00049 | 66.1 | 6.9 | 20.3 | 4.5 | n.d | 89.9 |
| 00050 | 67.7 | 6.0 | 18.6 | 4.8 | n.d | 91.0 |
| 00051 | 64.8 | 16.1 | 37.9 | 15 | n.d | 89.9 |
| 00921 | 67.3 | 30.4 | 27.0 | 37 | > 329 | 91.0 |
| 00925 | 66.5 | 22.1 | 28.4 | 33 | > 453 | 89.9 |

| | | | | | | |
|---|---|---|---|---|---|---|
| n.d., not determined | | | | | | |

**Table 14: Affinity K_{D} of purified VHH 00921 for recombinant human, cyno, mouse and rat Ang2**

| | **human Ang2-FLD** | | | **cyno Ang2-FLD** | | |
|---|---|---|---|---|---|---|
| | **kₐ (1/Ms)** | **k_{d} (1/s)** | **K_{D} (M)** | **kₐ (1/Ms)** | **k_{d} (1/s)** | **K_{D} (M)** |
| 00921 | 4.0E+06 | 2.7E-02 | 6.6E-09 | 1.1E+06 | 2.5E-02 | 2.3E-09 |
| | | | | | | |

| | **mouse Ang2-FLD** | | | **rat Ang2-FLD** | | |
|---|---|---|---|---|---|---|
| | **kₐ (1/Ms)** | **k_{d} (1/s)** | **K_{D} (M)** | **kₐ (1/Ms)** | **k_{d} (1/s)** | **K_{D} (M)** |
| 00921 | 3.3E+06 | 2.5E-02 | 7.5E-09 | 1.2E+06 | 4.9E-02 | 4.2E-08 |

**Table 15: Amino acid sequence of sequence optimized variants of anti-Ang2 VHH 1D01**

| **VHH ID/SEQ ID NO:** | **FR1** | **CDR1** | **FR2** | **CDR2** | **FR3** | **CDR3** | **FR4** |
|---|---|---|---|---|---|---|---|
| 00039/124 | | | | | | | |
| 00040/125 | | | | | | | |
| 00049/126 | | | | | | | |
| 00050/127 | | | | | | | |
| 00051/128 | | | | | | | |
| 00921/129 | | | | | | | |
| 00925/130 | | | | | | | |

### 7.2 VHH 37F02

The amino acid sequence of anti-Ang2 VHH 37F02 (see Figure 12-A) is aligned to the human germline VH3/JH consensus sequence. Residues are numbered according to Kabat, CDRs are shown in grey according to AbM definition (Oxford Molecular's AbM antibody modeling software). Residues to be mutated to their human counterpart are underlined. Potential post-translational modification sites to be tackled are boxed. The alignment shows that 37F02 contains 4 framework mutations relative to the reference germline sequence. Non-human residues at positions 60, 74, 83 and 108 are selected for substitution with their human germline counterpart. In parallel, a potential Asp isomerization site at position D₅₄G₅₅ is removed by introducing a D₅₄G substitution. A set of three cycle 1 37F02 variants carrying different combinations of human residues on these positions (Figure 12-B) is constructed and produced (Example 5; AA sequences are listed in Table 21-1).

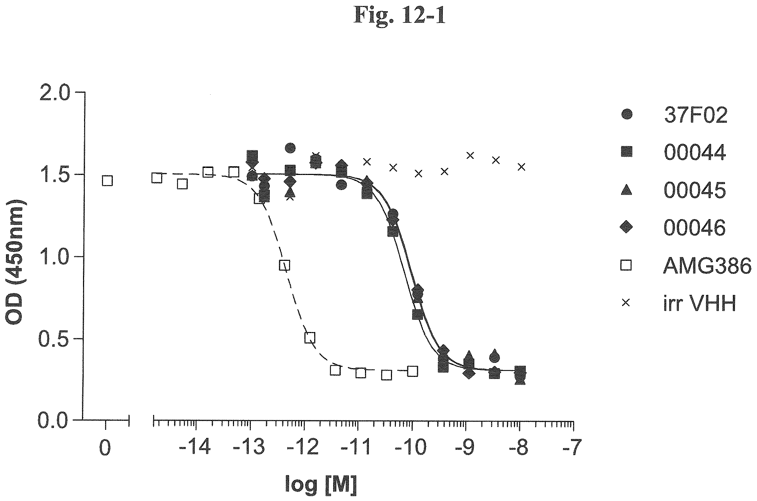

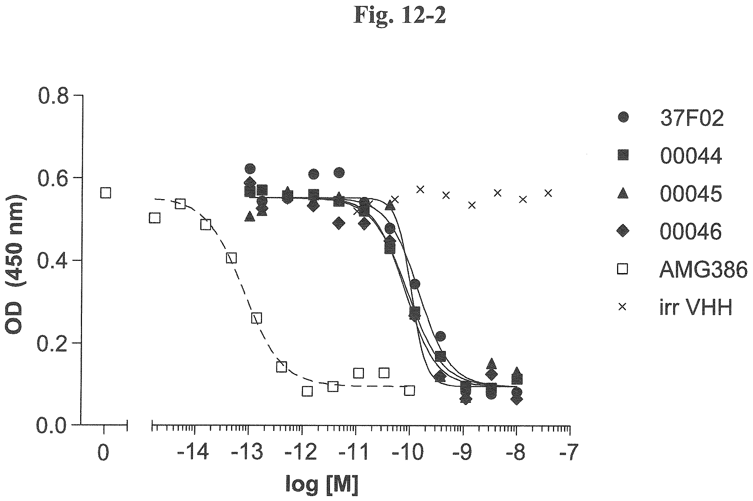

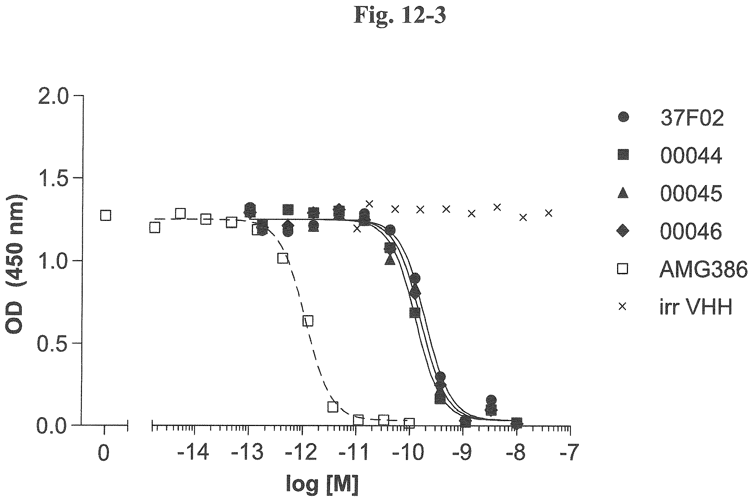

These variants are characterized as purified protein in the human (Figure 12-1), mouse (Figure 12-2) and cyno (Figure 12-3) Ang2/Tie2 competition ELISA (Example 5.1; Example 5.2). Additionally, melting temperature (Tₘ) of each variant is determined in thermal shift assay (Example 6). An overview of the data can be found in Table 16. Additionally, % FR identity to the human germline is calculated according to AbM definition (Oxford Molecular's AbM antibody modeling software).

**Table 16: Overview of Tₘ, IC₅₀ (pM) in human, mouse and cyno Ang2 competition ELISA and hAng1/hAng2 IC₅₀ ratios of cycle 1 sequence optimized variants of VHH 37F02**

| | **TSA** | **IC₅₀ in Ang2-Tie2 ELISA** | | | | **% FR identity** |
|---|---|---|---|---|---|---|
| **VHH ID** | **Tm (°C) @ pH7** | **hAng2 (pM)** | **mAng2 (pM)** | **cAng2 (pM)** | **hAng1/hAng2 IC₅₀ ratio** | **AbM** |
| 37F02 | 66.1 | 77 | 110 | 150 | > 130,317 | 87.6 |
| 00044 | 66.9 | 69 | 91 | 130 | n.d. | 91.0 |
| 00045 | 71.1 | 120 | 110 | 160 | n.d. | 92.1 |
| 00046 | 69.8 | 95 | 83 | 160 | n.d. | 91.0 |

A NNK library approach is used to knock out two potential post-translational modifications sites in CDR3: i) oxidation sensitive Met on position 100e and ii) Asp isomerization site on position D₉₅S₉₆. Since D₅₄G is tolerated (VHH 00046 and 00920; Table 16 and Table 17) no NNK approach was used to knock out this potential Asp isomerization site.

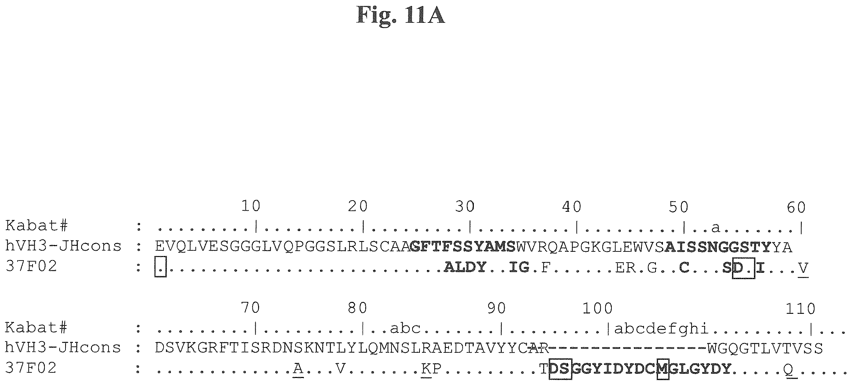

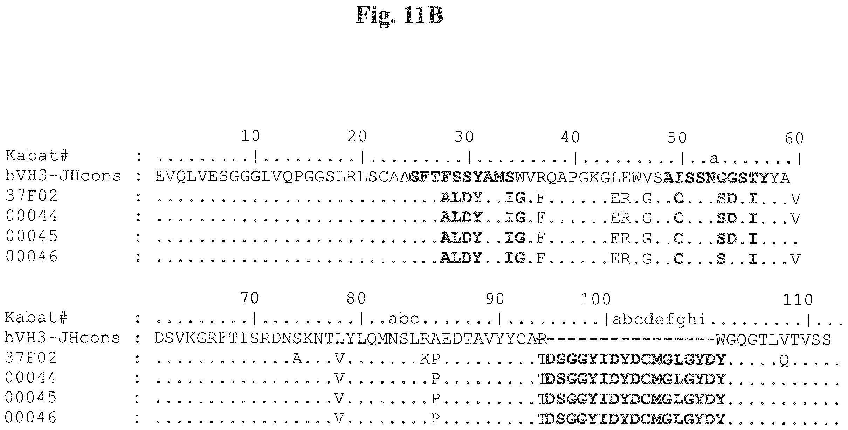

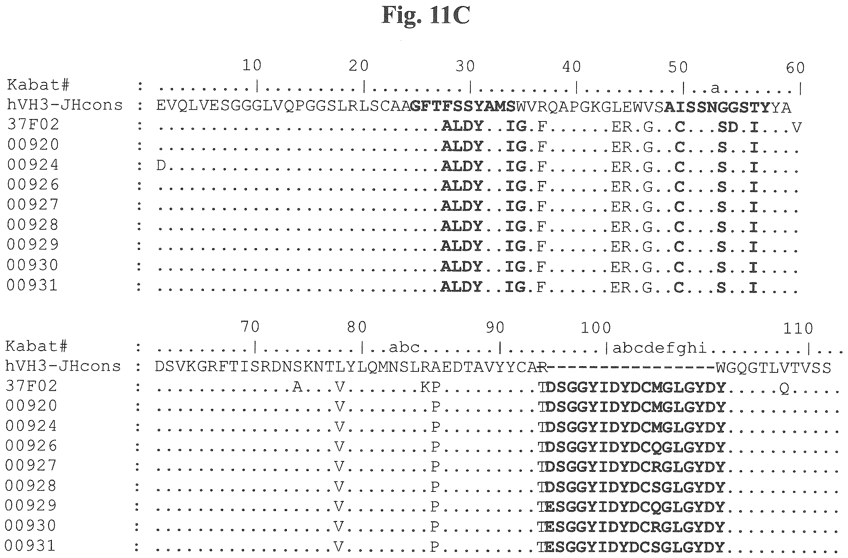

In the end, 3 NNK libraries containing VHH clones carrying substitutions at positions D₉₅, S₉₆ and M₁₀₀ₑ to all other amino acids are screened in a hAng2/hTie2 competition AlphaScreen assay (Example 2). Briefly, periplasmic extracts containing expressed VHH are screened at 3 different dilutions (corresponding roughly to EC₂₀, EC₅₀ and EC₈₀ of the parental VHH 37F02) and changes in % inhibition at the different dilution points are compared to parental 37F02. Based on the screening results and the data shown in Table 17, 8 additional cycle 2 VHH variants are constructed (based on VHH 00920 backbone) carrying different knock-out combinations of D₉₅S₉₆ and M₁₀₀ₑ (Figure 11-C; AA sequences are listed in Table 19-2).

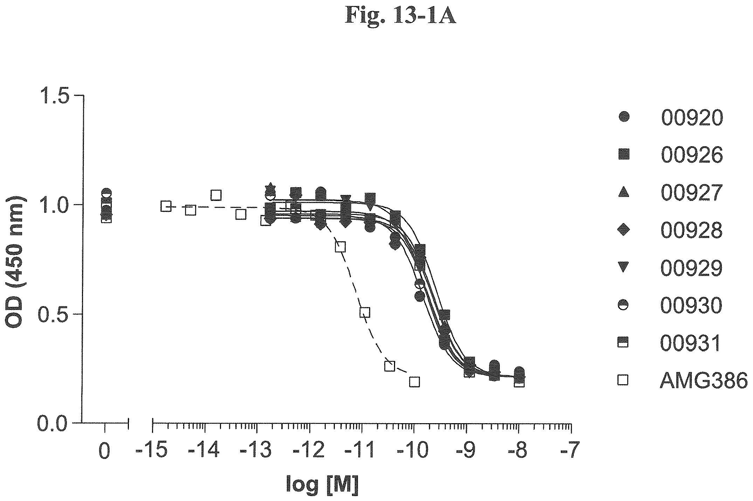

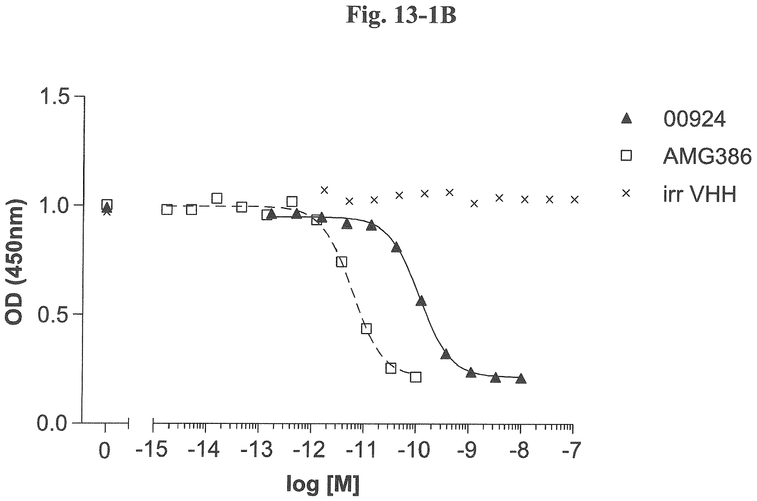

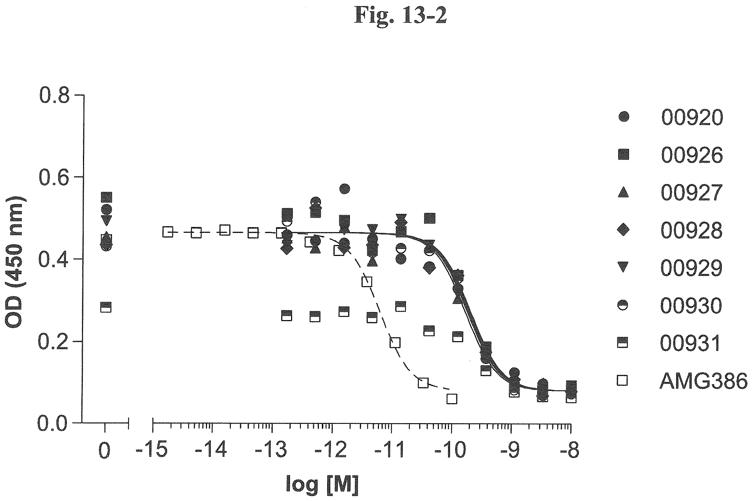

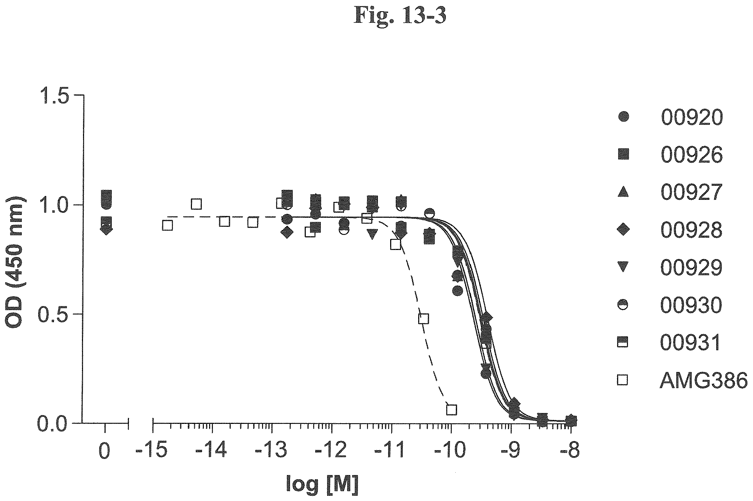

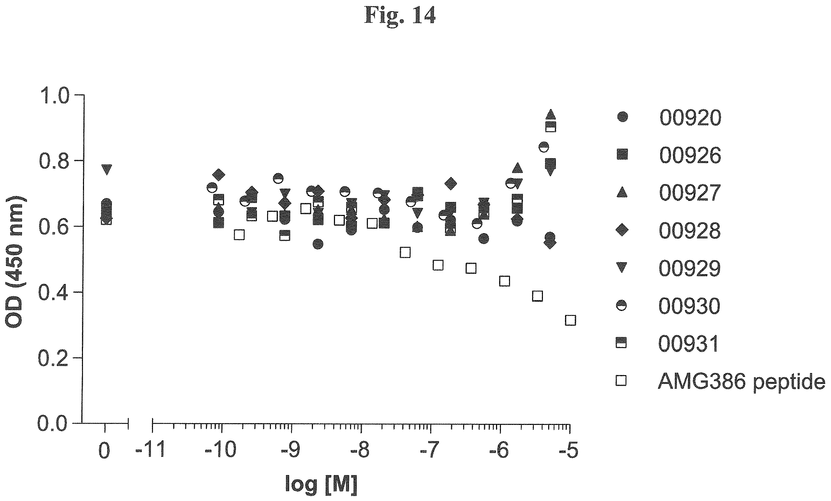

All these variants are characterized as purified protein in the human (Figure 13-1), mouse (Figure 12-2) and cyno (Figure 12-3) Ang2/Tie2 competition ELISA (Example 5.1; Example 5.2), the hAng1/hTie2 competition ELISA (Example 5.3; Figure 14). Additionally, melting temperature (Tₘ) of each variant is determined in thermal shift assay (Example 6). An overview of the data can be found in Table 17. Additionally, % FR identity to the human germline is calculated. Affinity of VHH 00928 for human, mouse, cyno and rat Ang2 is shown in Table 18.

**Table 17: Overview of Tₘ, IC₅₀ (pM) in human, mouse and cyno Ang2 competition ELISA and hAng1/hAng2 IC₅₀ ratios of cycle 2 sequence optimized variants of VHH 37F02**

| | **TSA** | **IC50 in Ang2-Tie2 ELISA** | | | | **% FR identity** |
|---|---|---|---|---|---|---|
| **VHH ID** | **Tm (°C) @ pH7** | **hAng2 (pM)** | **mAng2 (pM)** | **cAng2 (pM)** | **hAng1/hAng2 ratio** | **AbM** |
| 37F02 | 66.1 | 77 | 110 | 150 | > 130,317 | 87.6 |
| 00920 | 73.6 | 130 | 150 | 230 | > 79,159 | 92.1 |
| 00924 | 73.1 | 110 | n.d. | n.d. | n.d. | 91.0 |
| 00926 | 71.5 | 250 | 200 | 290 | > 39,446 | 92.1 |
| 00927 | 73.6 | 220 | 190 | 330 | > 44,668 | 92.1 |
| 00928 | 72.7 | 220 | 200 | 390 | > 45,604 | 92.1 |
| 00929 | 69.0 | 150 | 150 | 250 | > 65,013 | 92.1 |
| 00930 | 69.8 | 190 | 170 | 310 | > 53,580 | 92.1 |
| 00931 | 69.4 | 170 | 150 | 290 | > 57,677 | 92.1 |

| | | | | | | |
|---|---|---|---|---|---|---|
| n.d., not determined | | | | | | |

**Table 18: Affinity K_{D} of purified VHH 00928 for recombinant human, cyno, mouse and rat Ang2**

| | **human Ang2-FLD** | | | **cyno Ang2-FLD** | | |
|---|---|---|---|---|---|---|
| | **kₐ (1/Ms)** | **k_{d} (1/s)** | **K_{D} (M)** | **kₐ (1/Ms)** | **k_{d} (1/s)** | **K_{D} (M)** |
| 00928 | 6.2+05 | 4.0E-05 | 6.4E-11 | 9.7E+05 | 4.9E-05 | 5.0E-11 |
| | | | | | | |

| | **mouse Ang2-FLD** | | | **rat Ang2-FLD** | | |
|---|---|---|---|---|---|---|
| | **kₐ (1/Ms)** | **k_{d} (1/s)** | **K_{D} (M)** | **kₐ (1/Ms)** | **k_{d} (1/s)** | **K_{D} (M)** |
| 00928 | 4.2E+05 | 7.3E-05 | 1.7E-10 | 1.8E+05 | 5.4E-05 | 2.9E-10 |

**Table 19-1**

| **VHH ID/SEQ ID NOs:** | **FR1** | **CDR1** | **FR2** | **CDR2** | **FR3** | **CDR3** | **FR4** |
|---|---|---|---|---|---|---|---|
| 00044/131 | | | | | | | |
| 00045/132 | | | | | | | |
| 00046/133 | | | | | | | |

**Table 19-2**

| **VHH ID/SEQ ID NO:** | **FR1** | **CDR1** | **FR2** | **CDR2** | **FR3** | **CDR3** | **FR4** |
|---|---|---|---|---|---|---|---|
| 00920/134 | | | | | | | |
| 00924/135 | | | | | | | |
| 00926/136 | | | | | | | |
| 00927/137 | | | | | | | |
| 00928/138 | | | | | | | |
| 00929/139 | | | | | | | |
| 00930/140 | | | | | | | |
| 00931/141 | | | | | | | |

### 7.3 VHH 28010

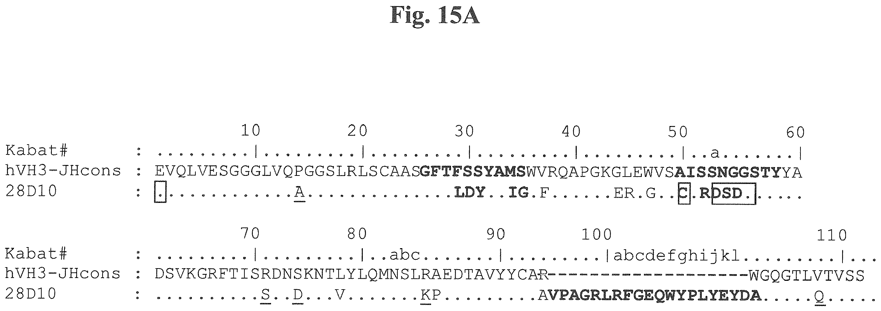

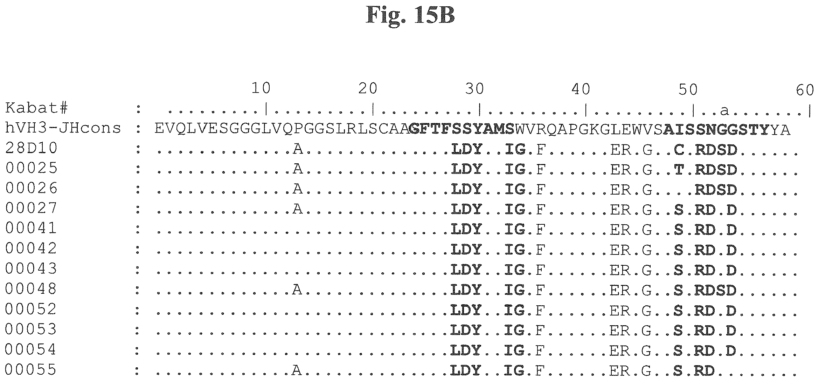

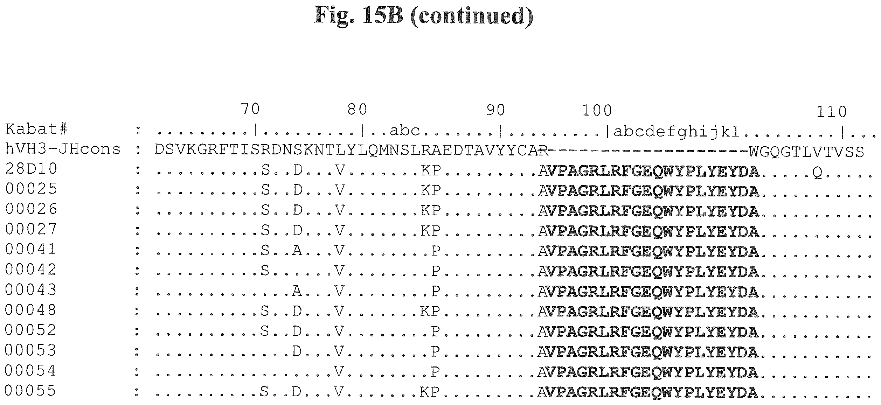

The amino acid sequence of anti-Ang2 VHH 28D10 (see Figure 15-A) is aligned to the human germline VH3/JH consensus sequence. Residues are numbered according to Kabat, CDRs are shown in grey according to AbM definition (Oxford Molecular's AbM antibody modeling software). Residues to be mutated to their human counterpart are underlined. Potential post-translational modification sites to be tackled are boxed. The alignment shows that 28D10 contains 5 framework mutations relative to the reference germline sequence. Non-human residues at positions 14, 71, 74, 83 and 108 are selected for substitution with their human germline counterpart. In parallel, a potential Asp isomerization site at position D₅₄G₅₅ is removed by introducing a D₅₄G substitution. The free cystein at position 50 was removed by substitution with Ala, Thr or Ser. In the end, a set of eleven cycle 1 28D10 variants carrying different combinations of human residues on these positions is constructed and produced (see Figure 15-B; AA sequences are listed in Table 24-1).

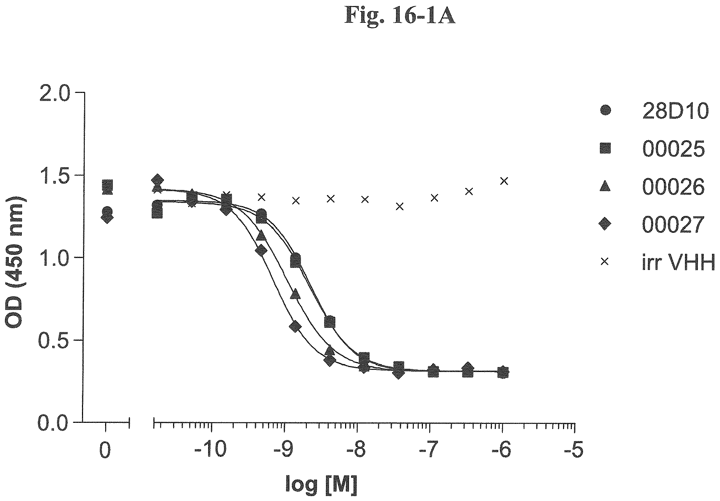

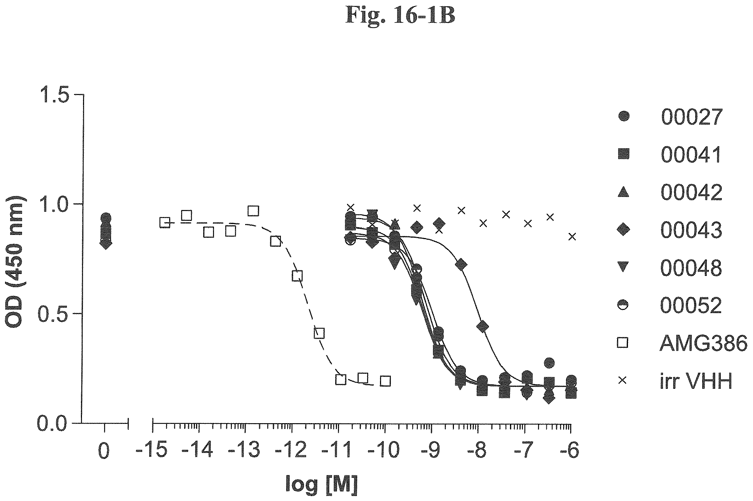

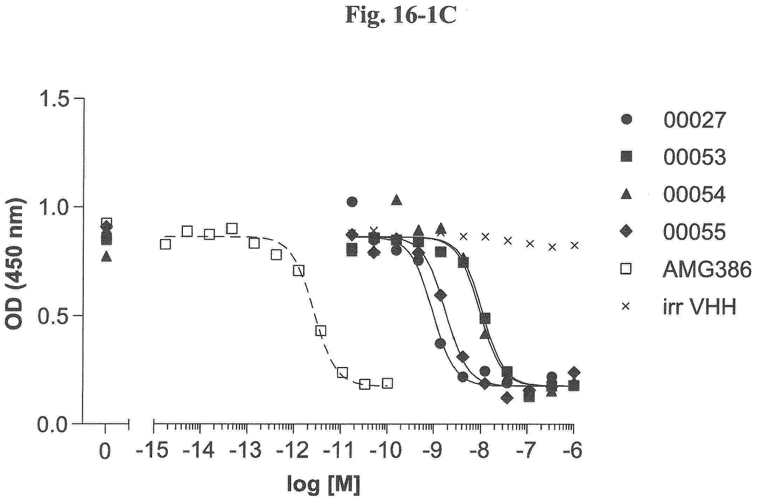

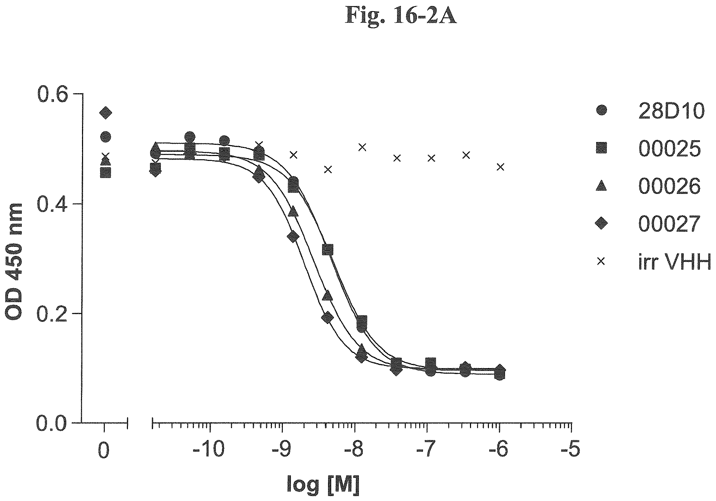

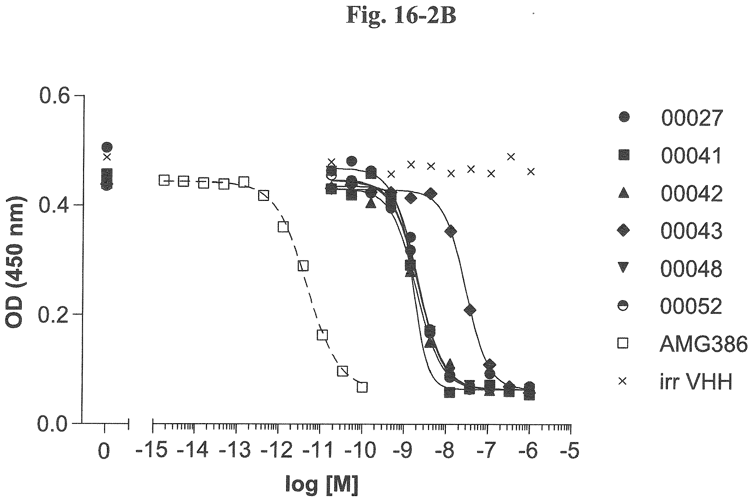

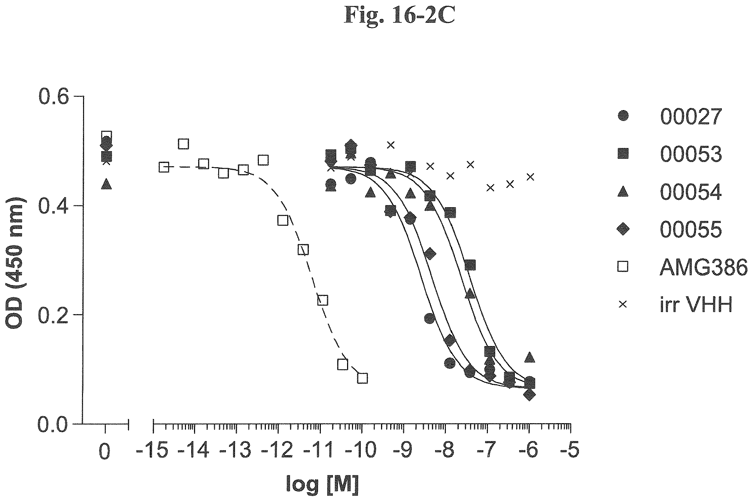

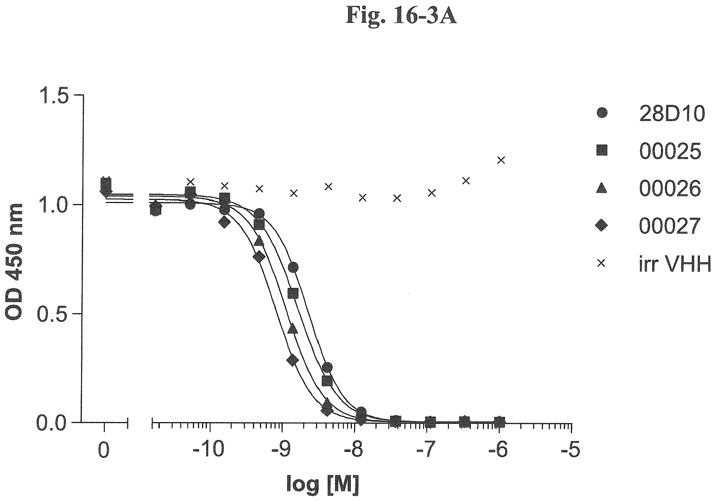

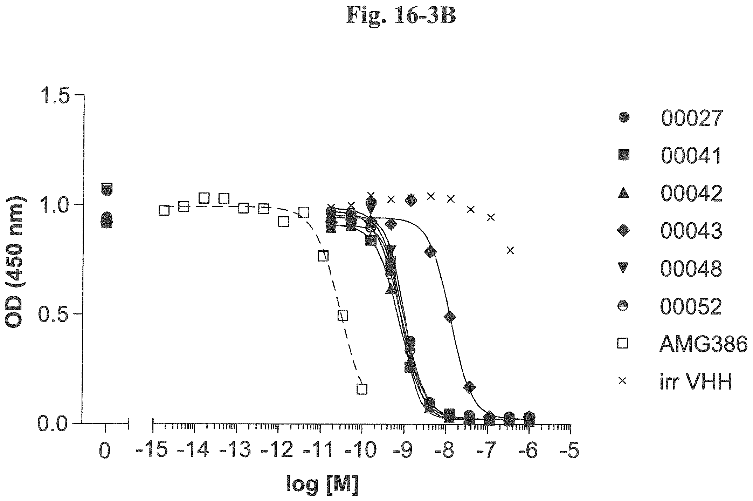

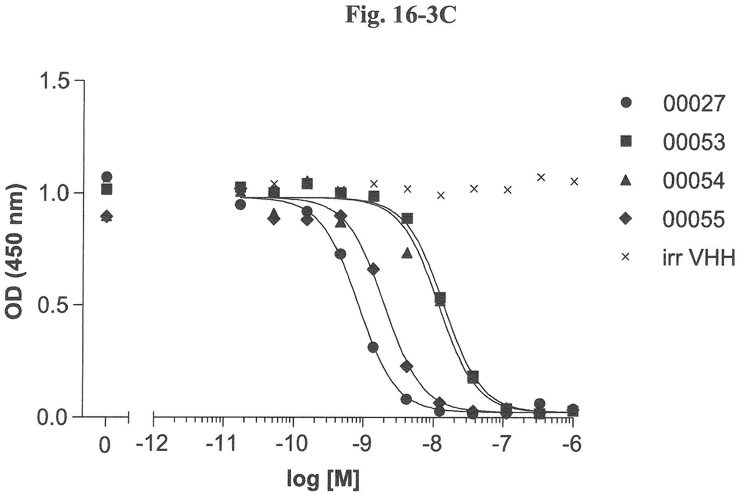

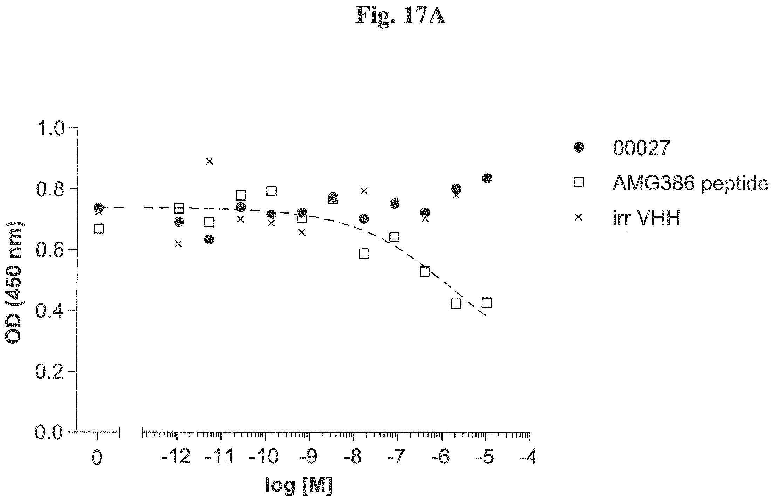

These variants are characterized as purified protein in the human (Figure 16-1), mouse (Figure 16-2) and cyno (Figure 16-3) Ang2/Tie2 competition ELISA (Example 5.1; Example 5.2), the hAng1/hTie2 competition ELISA (Example 5.3; Figure 17). Additionally, melting temperature (Tₘ) of each variant is determined in thermal shift assay (Example 6). An overview of the data can be found in Table 20. Additionally, % FR identity to the human germline is calculated.

**Table 20: Overview of Tₘ, IC₅₀ (nM) in human, mouse and cyno Ang2 competition ELISA and hAng1/hAng2 IC₅₀ ratios of cycle 1 sequence optimized variants of VHH 28D10**

| | **TSA** | **IC50 in Ang2-Tie2 ELISA** | | | | **% FR identity** |
|---|---|---|---|---|---|---|
| **VHH ID** | **Tm (°C) @ pH7** | **hAng2 (nM)** | **mAng2 (nM)** | **cAng2 (nM)** | **hAng1/hAng2 ratio** | **AbM** |
| 28D10 | 66.1 | 1.8 | 5.7 | 2.1 | > 5,675 | 86.5 |
| 00025 | 63.2 | 2.1 | 4.9 | 1.7 | n.d. | 87.6 |
| 00026 | 68.2 | 1.0 | 2.6 | 1.1 | n.d. | 87.6 |
| 00027 | 61.1 | 0.7 | 2.0 | 0.7 | > 14,878 | 87.6 |
| 00041 | 62.3 | 0.6 | 1.7 | 0.9 | n.d. | 89.9 |
| 00042 | 61.9 | 0.7 | 1.9 | 0.8 | > 15,476 | 91.0 |
| 00043 | 65.7 | 9.5 | 29.2 | 12.6 | n.d. | 91.0 |
| 00048 | 63.2 | 0.6 | 2.1 | 1.0 | n.d. | 87.6 |
| 00052 | 61.1 | 1.0 | 2.1 | 0.8 | n.d. | 89.9 |
| 00053 | 64.8 | 10.7 | 36.7 | 13.8 | n.d. | 91.0 |
| 00054 | 65.7 | 9.5 | 22.9 | 11.7 | n.d. | 92.1 |
| 00055 | 63.6 | 1.8 | 4.6 | 2.0 | n.d. | 87.6 |

| | | | | | | |
|---|---|---|---|---|---|---|
| n.d., not determined | | | | | | |

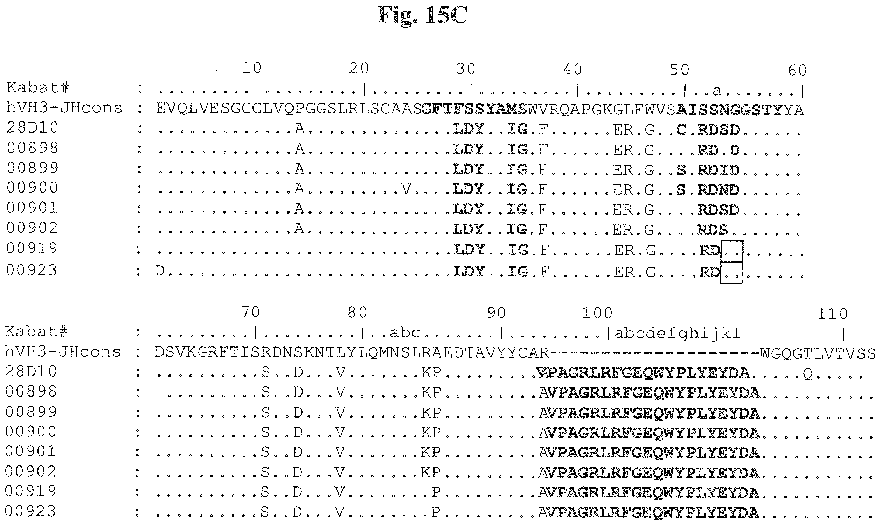

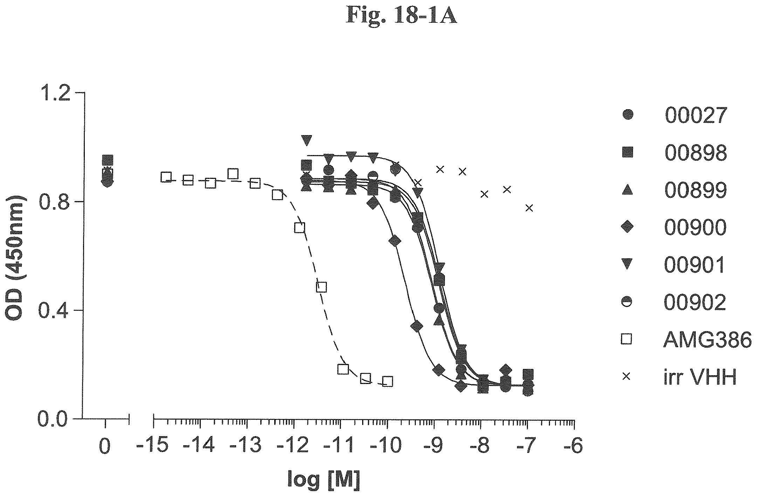

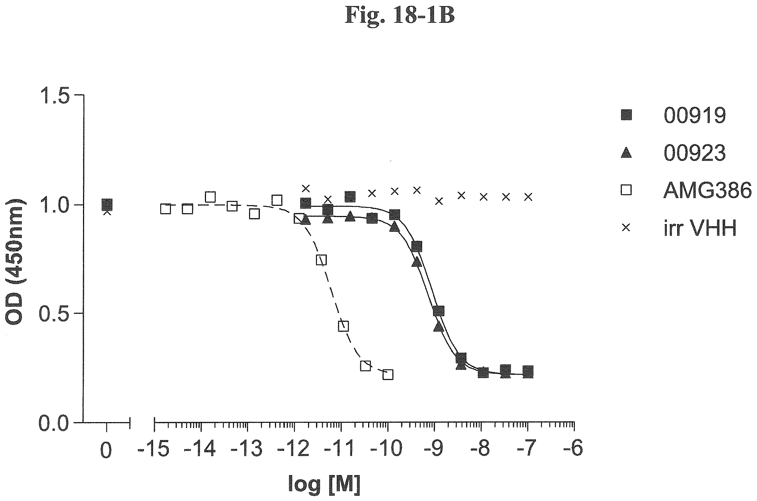

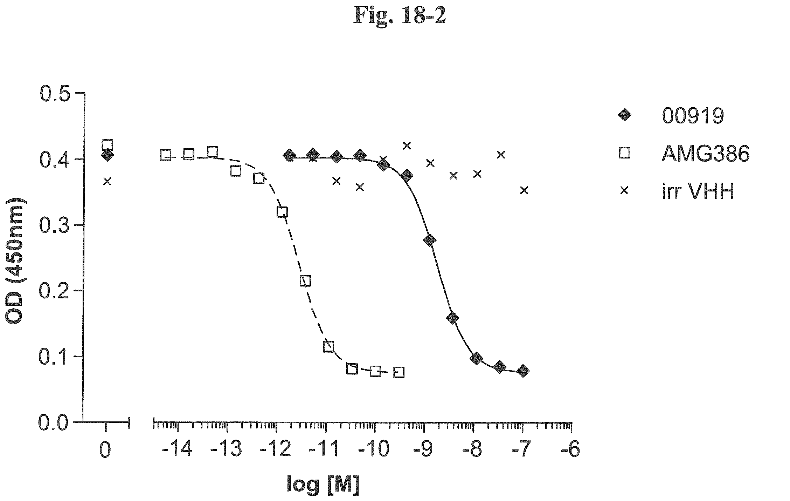

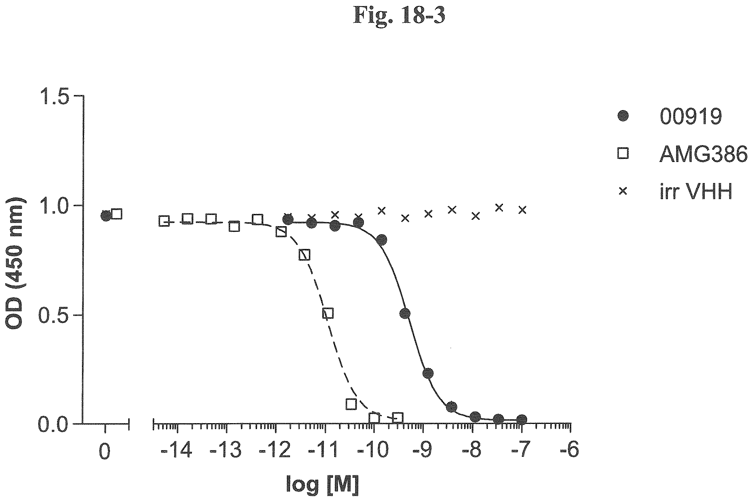

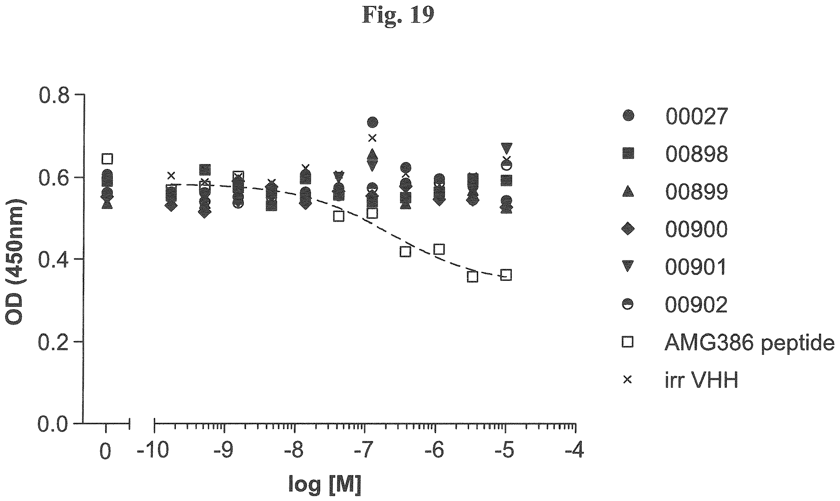

An additional set of variants (cycle 2) is created to explore the affinity maturation substitution on position A₂₄V and to further explore C₅₀X-S₅₃X variants (Table 21; Figure 15-C; AA sequences are listed in Table 23-4-2). These variants are characterized as purified protein in the human (Figure 18-1), mouse (Figure 18-2) and cyno (Figure 18-3) Ang2/Tie2 competition ELISA (Example 5.1; Example 5.2), the hAng1/hTie2 competition ELISA (Example 5.3; Figure 19). Additionally, melting temperature (Tₘ) of each variant is determined in thermal shift assay (Example 6).

**Table 21: Overview of Tₘ, IC₅₀ (nM) in human, mouse and cyno Ang2 competition ELISA and hAng1/hAng2 IC₅₀ ratios of cycle 2 sequence optimized variants of VHH 28D10**

| | **TSA** | **IC50 in Ang2-Tie2 ELISA** | | | | **% FR identity** |
|---|---|---|---|---|---|---|
| **VHH ID** | **Tm (°C) @ pH7** | **hAng2 (nM)** | **mAng2 (nM)** | **cAng2 (nM)** | **hAng1/hAng2 ratio** | **AbM** |
| 28D10 | 66.1 | 1.8 | 5.7 | 2.1 | > 5,675 | 86.5 |
| 00898 | 66.1 | 1.2 | n.d. | n.d. | > 8,279 | 87.6 |
| 00899 | 61.9 | 0.9 | n.d. | n.d. | > 11,749 | 87.6 |
| 00900 | 64.3 | 0.2 | n.d. | n.d. | > 42,855 | 86.5 |
| 00901 | 67.3 | 1.2 | n.d. | n.d. | > 8,054 | 87.6 |
| 00902 | 66.1 | 1.3 | n.d. | n.d. | > 7,907 | 87.6 |
| 00919 | 67.3 | 0.9 | 1.8 | 0.7 | > 11,212 | 91.0 |
| 00923 | 67.7 | 0.7 | n.d. | n.d. | n.d. | 89.9 |

| | | | | | | |
|---|---|---|---|---|---|---|
| n.d., not determined | | | | | | |

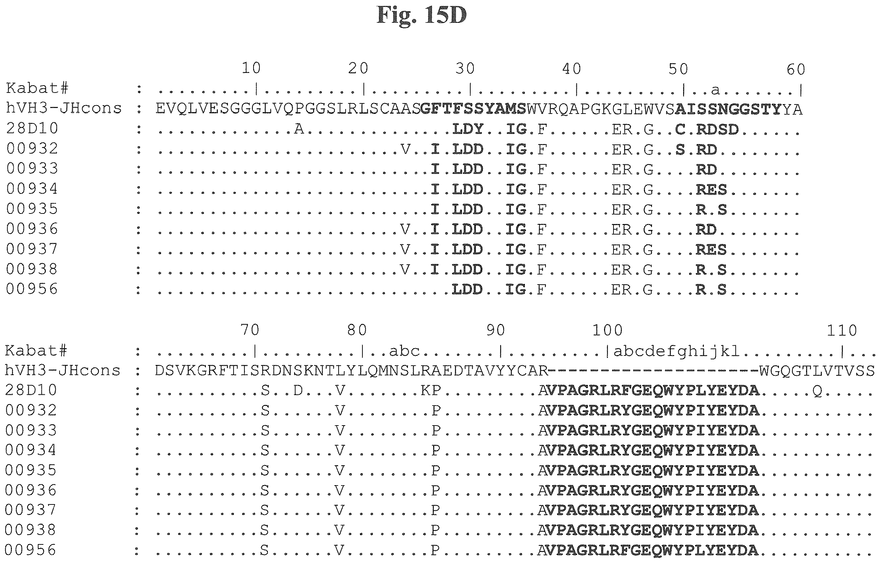

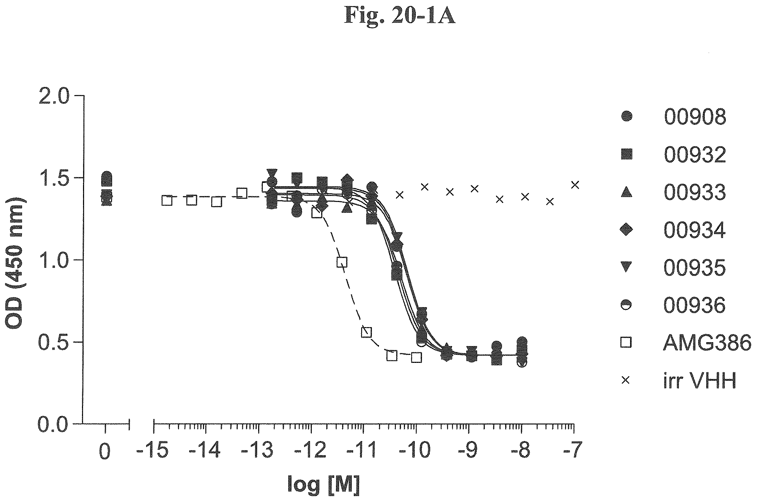

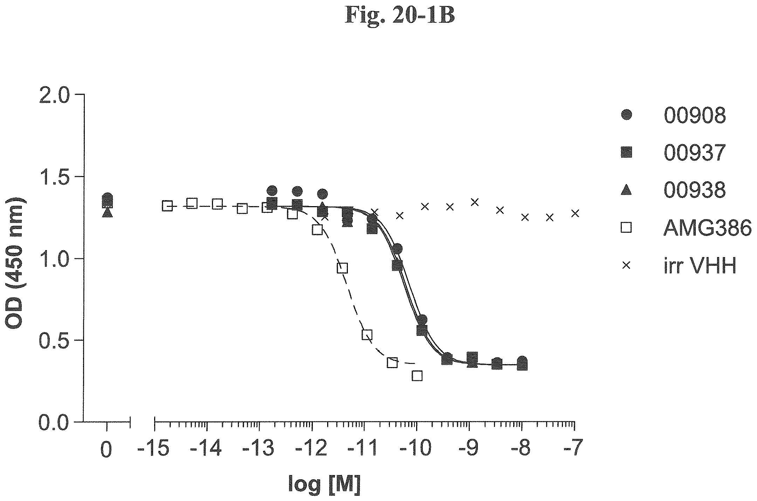

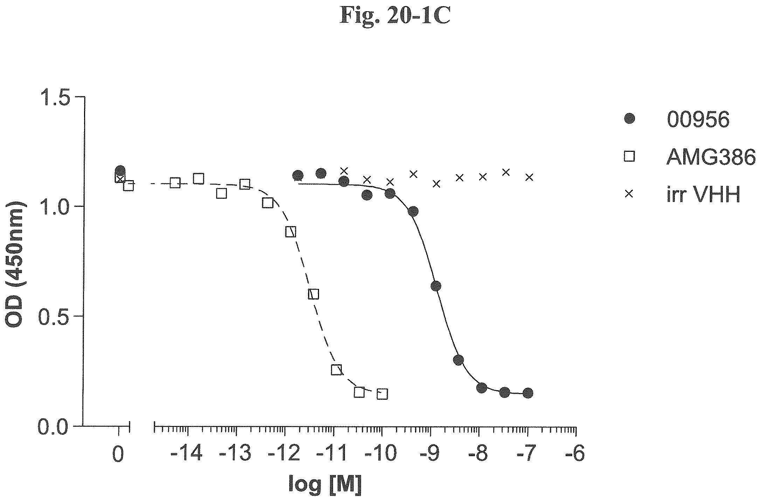

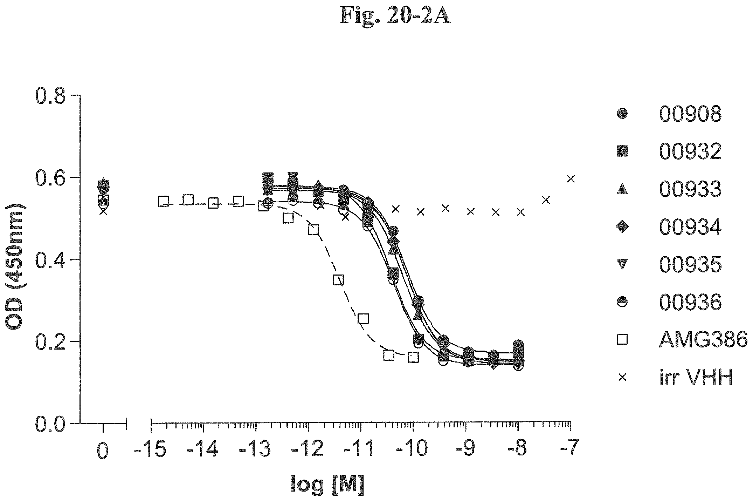

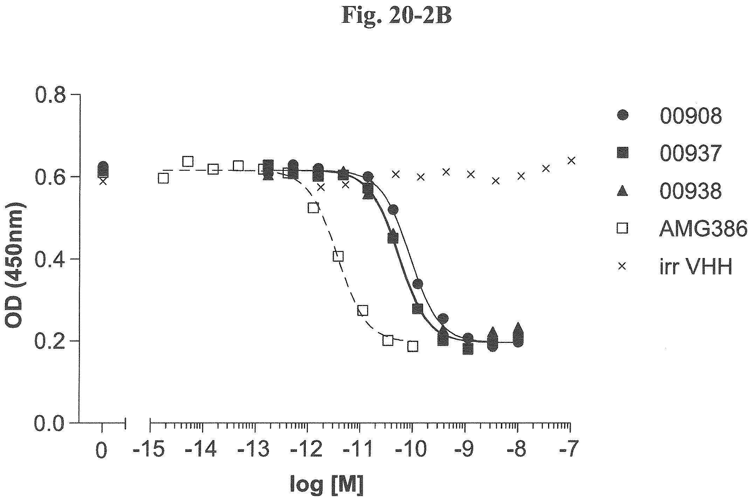

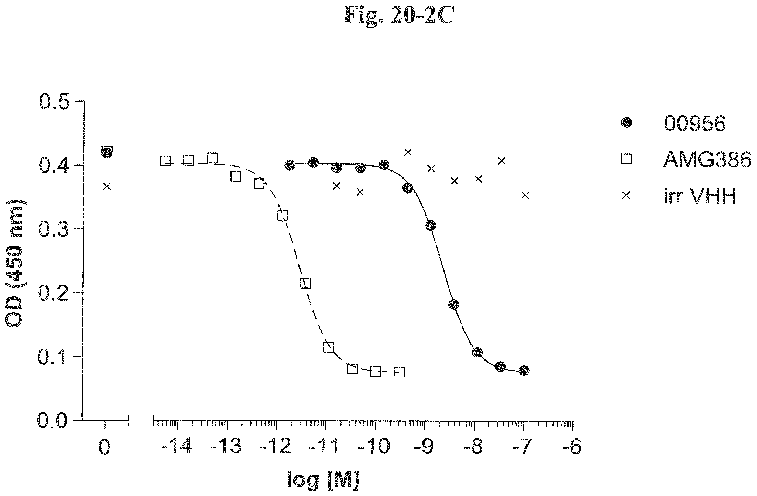

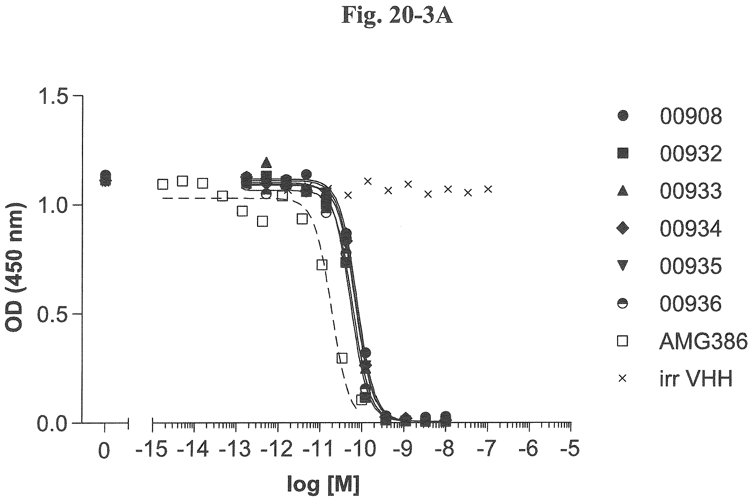

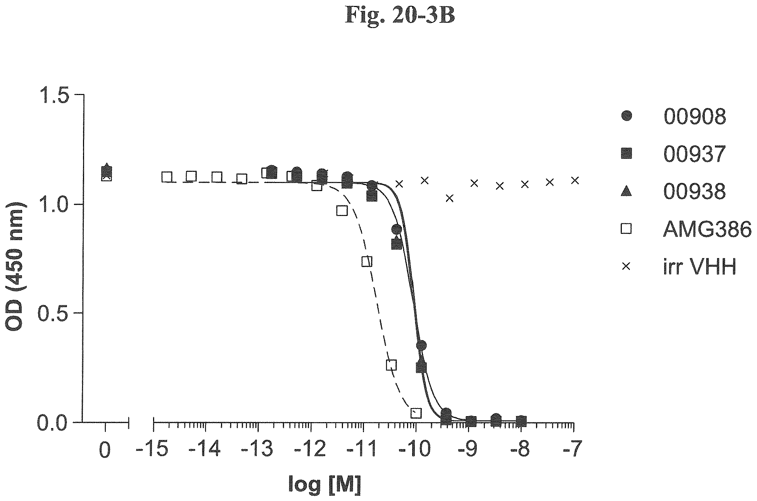

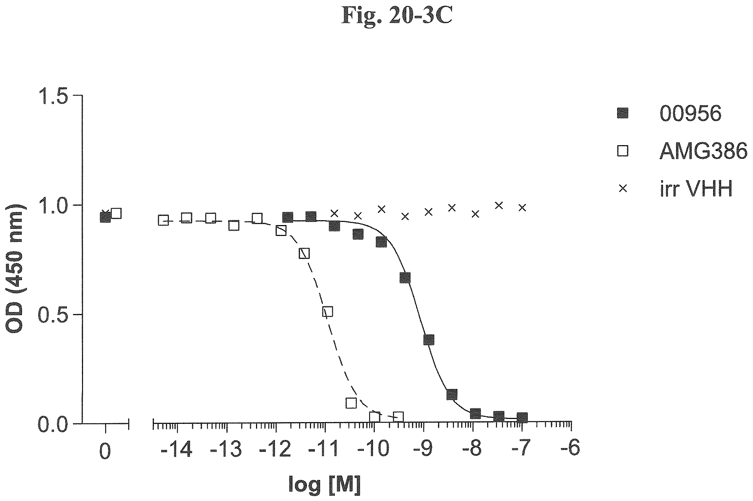

In parallel, a NNK library approach is used to knock out two post-translational modifications sites in CDR2: two sequential Asp isomerization sites on position D₅₂ₐS₅₃ and D₅₄G₅₅. Since D₅₄G is tolerated (Example 6; Table 20) no NNK approach was used to knock out this potential Asp isomerization site. In the end, 2 NNK libraries containing VHH clones carrying substitutions at positions D₅₂ₐ and S₅₃ to all other amino acids are screened in a hAng2/hTie2 competition AlphaScreen assay (Example 2). Briefly, periplasmic extracts containing expressed VHH are screened at 3 different dilutions (corresponding roughly to EC₂₀, EC₅₀ and EC₈₀ of the reference 00902) and changes in % inhibition at the different dilution points are compared to reference 00902. Based on the screening results and the data shown in Table 21, 7 additional VHH cycle 3 variants are constructed (based on 00908 backbone) (see Figure 15-D). The final aim is to construct VHH variants that retain or show increased potency, increased thermostability and have relevant PTM sites knocked out compared to VHH 28D10. (AA sequences are listed in Table 24-3). These variants are characterized as purified protein in the human (Figure 20-1), mouse (Figure 20-2) and cyno (Figure 20-3) Ang2/Tie2 competition ELISA (Example 5.1; Example 5.2), the hAng1/hTie2 competition ELISA (Example 5.3). Additionally, melting temperature (Tₘ) of each variant is determined in thermal shift assay (Example 6). An overview of the data can be found in Table 22. Additionally, % FR identity to the human germline is calculated. The most optimal sequence changes were finally applied to a non-affinity matured variant VHH 00956 (Figure 15-D). Affinity of VHH 00919, 00938 and 00956 for human, mouse, cyno and rat Ang2 is shown in Table 23.

**Table 22: Overview of Tₘ, IC₅₀ (nM) in human, mouse and cyno Ang2 competition ELISA and hAng1/hAng2 IC₅₀ ratios of cycle 3 sequence optimized variants of VHH 28D10**

| | **TSA** | **IC50 in Ang2/Tie2 ELISA** | | | | **HUVEC survival** | **% FR identity** |
|---|---|---|---|---|---|---|---|
| **VHH ID** | **Tm @ pH 7.0 (°C)** | **hAng2 (pM)** | **mAng2 (pM)** | **cAng2 (pM)** | **hAng1/hAng2 IC₅₀ ratio** | **IC₅₀ (nM)** | **AbM** |
| 00027 | 61.1 | 672 | 1,975 | 728 | > 14,878 | n.d. | 87.6 |
| 00908 | 67.3 | 45 | 85 | 79 | > 192,014 | n.d. | 91.0 |
| 00932 | 70.5 | 49 | 38 | 54 | > 205,747 | n.d. | 89.9 |
| 00933 | 70.5 | 56 | 62 | 71 | > 179,887 | n.d. | 91.0 |
| 00934 | 67.1 | 64 | 74 | 71 | > 156,675 | n.d. | 91.0 |
| 00935 | 68.6 | 68 | 78 | 69 | > 146,218 | n.d. | 91.0 |
| 00936 | 73.9 | 45 | 42 | 60 | > 223,872 | n.d. | 89.9 |
| 00937 | 72.0 | 54 | 52 | 85 | > 186,209 | n.d. | 89.9 |
| 00938 | 73.9 | 50 | 55 | 91 | > 201,064 | 4.3 | 89.9 |
| 00956 | 68.0 | 1,300 | 2,200 | 830 | > 7,727 | 6.8 | 91.0 |

| | | | | | | | |
|---|---|---|---|---|---|---|---|
| n.d. not determined | | | | | | | |

**Table 23: Affinity K_{D} of purified VHHs 00919, 00938 and 00956 for recombinant human, cyno, mouse and rat Ang2**

| | **human Ang2-FLD** | | | **cyno Ang2-FLD** | | |
|---|---|---|---|---|---|---|
| | **kₐ (1/Ms)** | **k_{d} (1/s)** | **K_{D} (M)** | **kₐ (1/Ms)** | **k_{d} (1/s)** | **K_{D} (M)** |
| 00919 | 9.5E+05 | 1.5E-03 | 1.6E-09 | 2.3E+06 | 1.2E-03 | 5.4E-10 |
| 00938 | 1.6E+06 | 2.8E-05 | 1.7E-11 | 2.6E+06 | 2.2E-05 | 8.7E-12 |
| 00956 | 1.3E+06 | 1.5E-03 | 1.2E-09 | 1.7E+06 | 1.3E-03 | 7.2E-10 |
| | | | | | | |

| | **mouse Ang2-FLD** | | | **rat Ang2-FLD** | | |
|---|---|---|---|---|---|---|
| | **kₐ (1/Ms)** | **k_{d} (1/s)** | **K_{D} (M)** | **kₐ (1/Ms)** | **k_{d} (1/s)** | **K_{D} (M)** |
| 00919 | 1.3E+06 | 3.9E-03 | 3.0E-09 | 5.0E+05 | 5.0E-03 | 1.0E-08 |
| 00938 | 1.1E+06 | 5.6E-05 | 5.0E-11 | 5.1 E+05 | 7.2E-05 | 1.4E-10 |
| 00956 | 9.8E+05 | 3.9E-03 | 4.0E-09 | 4.2E+05 | 5.2E-03 | 1.3E-08 |

**Table 24-1**

| **VHH ID/SEQ ID NO:** | **FR1** | **CDR1** | **FR2** | **CDR2** | **FR3** | **CDR3** | **FR4** |
|---|---|---|---|---|---|---|---|
| 00025/142 | | | | | | | |
| 00026/143 | | | | | | | |
| 00027/144 | | | | | | | |
| 00041/145 | | | | | | | |
| 00042/146 | | | | | | | |
| 00043/147 | | | | | | | |
| 00048/148 | | | | | | | |
| 00052/149 | | | | | | | |
| 00053/150 | | | | | | | |
| 00054/151 | | | | | | | |
| 00055/152 | | | | | | | |

**Table 24-2**

| **VHH ID/SEQ ID NO:** | **FR1** | **CDR1** | **FR2** | **CDR2** | **FR3** | **CDR3** | **FR4** |
|---|---|---|---|---|---|---|---|
| 00898/153 | | | | | | | |
| 00899/154 | | | | | | | |
| 00900/155 | | | | | | | |
| 00901/156 | | | | | | | |
| 00902/157 | | | | | | | |

**Table 24-3**

| **VHH ID/SEQ ID NO:** | **FR1** | **CDR1** | **FR2** | **CDR2** | **FR3** | **CDR3** | **FR4** |
|---|---|---|---|---|---|---|---|
| 00919/158 | | | | | | | |
| 00923/159 | | | | | | | |
| 00932/160 | | | | | | | |
| 00933/161 | | | | | | | |
| 00934/162 | | | | | | | |
| 00935/163 | | | | | | | |
| 00936/164 | | | | | | | |
| 00937/165 | | | | | | | |
| 00938/166 | | | | | | | |
| 00956/167 | | | | | | | |

VHH domain 00938 is an embodiment of the invention as claimed in the appended claims. Other VHH domains, shown above but not falling under these claims, are shown for comparative purposes or to explain how VHH domain 00938 was obtained.

## Claims

1. An Ang2-binding molecule comprising an immunoglobulin single variable domain, wherein said immunoglobulin single variable domain comprises three complementarity determining regions CDR1, CDR2 and CDR3, wherein
CDR1 has an amino acid sequence shown in SEQ ID NO: 168,
CDR2 has an amino acid sequence shown in SEQ ID NO: 171 and
CDR3 has an amino acid sequence shown in SEQ ID NO: 175.

2. The Ang2-binding molecule according to claim 1, wherein said immunoglobulin single variable domain is a VHH.

3. The Ang2-binding molecule according to claim 2, wherein said VHH consists of an immunoglobulin single variable domain having a sequence consisting of SEQ ID NO: 166.

4. The Ang2-binding molecule according to any one of claims 1 to 3, wherein said VHH or said immunoglobulin single variable domain has a modification or exchange on its N-terminus, wherein said modification is a deletion of the first amino acid and said exchange is a replacement of the first amino acid by another amino acid.

5. A nucleic acid molecule encoding the Ang2-binding molecule according to any one of claims 1 to 4.

6. An expression vector comprising said nucleic acid molecule according to claim 5.

7. A host cell carrying one or more expression vectors according to claim 6.

8. A method for producing the Ang2-binding molecule according to any one of claims 1 to 4, comprising the steps of:
transfecting a host cell with one or more of said expression vectors according to claim 6,
culturing said host cell, and
recovering and purifying said Ang2-binding molecule.

9. A pharmaceutical composition comprising, as the active ingredient, one or more said Ang2-binding molecules according to any one of claims 1 to 4, and at least a physiologically acceptable carrier.

10. The pharmaceutical composition according to claim 9, further comprising one or more additional therapeutic agents.

11. The pharmaceutical composition according to any one of claims 9 or 10 for use in the treatment of a disease that is associated with Ang2-mediated effects on angiogenesis.

12. The pharmaceutical composition according to claim 11 for use in the treatment of cancer and cancerous diseases.

13. The pharmaceutical composition according to claim 11 for use in the treatment of eye diseases.

14. The pharmaceutical composition for use of claim 13, wherein said eye disease is age-related macular degeneration or diabetic retinopathy.

15. The pharmaceutical composition according to claim 11 for use in the treatment of chronic kidney diseases.

## Patentansprüche

1. Ang2-bindendes Molekül, umfassend eine variable Immunglobulin-Einzeldomäne, wobei die variable Immunglobulin-Einzeldomäne drei komplementaritätsbestimmende Regionen CDR1, CDR2 und CDR3 umfasst, wobei
CDR1 eine in SEQ ID NO: 168 gezeigte Aminosäuresequenz aufweist,
CDR2 eine in SEQ ID NO: 171 gezeigte Aminosäuresequenz aufweist und
CDR3 eine in SEQ ID NO: 175 gezeigte Aminosäuresequenz aufweist.

2. Ang2-bindendes Molekül nach Anspruch 1, wobei die variable Immunglobulin-Einzeldomäne eine VHH ist.

3. Ang2-bindendes Molekül nach Anspruch 2, wobei die VHH aus einer variablen Immunglobulin-Einzeldomäne mit einer aus SEQ ID NO: 166 bestehenden Sequenz besteht.

4. Ang2-bindendes Molekül nach irgendeinem der Ansprüche 1 bis 3, wobei die VHH oder die variable Immunglobulin-Einzeldomäne eine Modifikation oder einen Austausch an ihrem N-Terminus aufweist, wobei die Modifikation eine Deletion der ersten Aminosäure ist und der Austausch eine Ersetzung der ersten Aminosäure durch eine andere Aminosäure ist.

5. Nucleinsäuremolekül, die das Ang2-bindende Molekül nach irgendeinem der Ansprüche 1 bis 4 kodiert.

6. Expressionsvektor, umfassend das Nucleinsäuremolekül nach Anspruch 5.

7. Wirtszelle, die einen oder mehrere Expressionsvektoren nach Anspruch 6 trägt.

8. Verfahren zur Herstellung des Ang2-bindenden Moleküls nach irgendeinem der Ansprüche 1 bis 4, umfassend die Schritte:
Transfizieren einer Wirtszelle mit einem oder mehreren der Expressionsvektoren nach Anspruch 6,
Kultivieren der Wirtszelle und
Gewinnen und Reinigen des Ang2-bindenden Moleküls.

9. Pharmazeutische Zusammensetzung, umfassend als den Wirkstoff ein oder mehrere der Ang2-bindenden Moleküle nach irgendeinem der Ansprüche 1 bis 4 und mindestens einen physiologisch verträglichen Träger.

10. Pharmazeutische Zusammensetzung nach Anspruch 9, ferner umfassend ein oder mehrere zusätzliche Therapeutika.

11. Pharmazeutische Zusammensetzung nach irgendeinem der Ansprüche 9 oder 10 zur Verwendung bei der Behandlung einer Krankheit, die mit Ang2-vermittelten Effekten auf Angiogenese assoziiert ist.

12. Pharmazeutische Zusammensetzung nach Anspruch 11 zur Verwendung bei der Behandlung von Krebs und Krebserkrankungen.

13. Pharmazeutische Zusammensetzung nach Anspruch 11 zur Verwendung bei der Behandlung von Augenerkrankungen.

14. Pharmazeutische Zusammensetzung zur Verwendung nach Anspruch 13, wobei die Augenerkrankung altersbedingte Makuladegeneration oder diabetische Retinopathie ist.

15. Pharmazeutische Zusammensetzung nach Anspruch 11 zur Verwendung bei der Behandlung von chronischen Nierenerkrankungen.

## Revendications

1. Molécule se liant à Ang2 comprenant un domaine variable unique d'immunoglobuline, dans laquelle ledit domaine variable unique d'immunoglobuline comprend trois régions de détermination de complémentarité CDR1, CDR2 et CDR3, dans laquelle
CDR1 a une séquence d'acides aminés présentée dans SEQ ID N° : 168,
CDR2 a une séquence d'acides aminés présentée dans SEQ ID N° : 171 et
CDR3 a une séquence d'acides aminés présentée dans SEQ ID N° : 175.

2. Molécule se liant à Ang2 selon la revendication 1, dans laquelle ledit domaine variable unique d'immunoglobuline est un VHH.

3. Molécule se liant à Ang2 selon la revendication 2, dans laquelle ledit VHH est constitué d'un domaine variable unique d'immunoglobuline ayant une séquence constituée de SEQ ID N° : 166.

4. Molécule se liant à Ang2 selon l'une quelconque des revendications 1 à 3, dans laquelle ledit VHH ou ledit domaine variable unique d'immunoglobuline a une modification ou un échange sur sa terminaison N, dans laquelle ladite modification est une délétion du premier acide aminé et ledit échange est un remplacement du premier acide aminé par un autre acide aminé.

5. Molécule d'acide nucléique codant la molécule se liant à Ang2 selon l'une quelconque des revendications 1 à 4.

6. Vecteur d'expression comprenant ladite molécule d'acide nucléique selon la revendication 5.

7. Cellule hôte porteuse d'un ou plusieurs vecteurs d'expression selon la revendication 6.

8. Procédé pour produire la molécule se liant à Ang2 selon l'une quelconque des revendications 1 à 4, comprenant les étapes consistant à :
transfecter une cellule hôte avec un ou plusieurs desdits vecteurs d'expression selon la revendication 6,
mettre en culture ladite cellule hôte, et
récupérer et purifier ladite molécule se liant à Ang2.

9. Composition pharmaceutique comprenant, en tant que principe actif, une ou plusieurs desdites molécules se liant à Ang2 selon l'une quelconque des revendications 1 à 4, et au moins un véhicule physiologiquement acceptable.

10. Composition pharmaceutique selon la revendication 9, comprenant en outre un ou plusieurs agents thérapeutiques supplémentaires.

11. Composition pharmaceutique selon l'une quelconque des revendications 9 ou 10 pour une utilisation dans le traitement d'une maladie qui est associée aux effets médiés par Ang2 sur l'angiogenèse.

12. Composition pharmaceutique selon la revendication 11 pour une utilisation dans le traitement du cancer et des maladies cancéreuses.

13. Composition pharmaceutique selon la revendication 11 pour une utilisation dans le traitement des maladies de l'oeil.

14. Composition pharmaceutique pour une utilisation selon la revendication 13, dans laquelle ladite maladie de l'oeil est la dégénérescence maculaire liée à l'âge ou la rétinopathie diabétique.

15. Composition pharmaceutique selon la revendication 11 pour une utilisation dans le traitement des maladies rénales chroniques.


### SEQUENCE LISTING

<110> Boehringer Ingelheim International GmbH
<120> ANG2-BINDING MOLECULES
<130> 12-0350-PCT
<160> 190
<170> PatentIn version 3.3
<210> 1
   <211> 124
   <212> PRT
   <213> Lama glama
<400> 1
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Phe Thr Phe Asp Asp Tyr
               20                  25                  30
   Ala Leu Gly Trp Phe Arg Gln Ala Ala Gly Lys Glu Arg Glu Gly Val
           35                  40                  45
   Ser Cys Ile Arg Cys Ser Asp Gly Ser Thr Tyr Tyr Ala Asp Ser Val
       50                  55                  60
   Lys Gly Arg Phe Thr Ile Ser Ser Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Ser Ile Val Pro Arg Ser Lys Leu Glu Pro Tyr Glu Tyr Asp
               100                 105                 110
   Ala Trp Gly Gln Gly Thr Gln Val Thr Val Ser Ser
           115                 120
<210> 2
   <211> 111
   <212> PRT
   <213> Lama glama
<400> 2
   Glu Val Gln Leu Val Glu Ser Gly Gly Gly Leu Val Gln Pro Gly Gly
   1               5                   10                  15
   Ser Leu Arg Leu Ser Cys Val His Ser Gly Thr Ile Ser Ser Thr His
               20                  25                  30
   Ala Met Gly Trp Tyr Arg Gln Ala Pro Gly Lys Gln Arg Glu His Val
           35                  40                  45
   Ala Thr Phe Thr Asn Arg Gly Ser Thr Tyr Tyr Ala Gly Ser Val Lys
       50                  55                  60
   Gly Arg Phe Thr Ile Ser Arg Asp Asn Ala Lys Asn Thr Met Tyr Leu
   65                  70                  75                  80
   Gln Met Asn Ser Leu Lys Pro Glu Asp Thr Ala Val Tyr Tyr Cys Asn
                   85                  90                  95
   Thr Gly Pro Tyr Trp Gly Gln Gly Thr Gln Val Thr Val Ser Ser
               100                 105                 110
<210> 3
   <211> 120
   <212> PRT
   <213> Lama glama
<400> 3
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Arg Thr Phe Ser Ser Ser
               20                  25                  30
   Val Met Gly Trp Phe Arg Gln Ala Pro Gly Lys Glu Arg Glu Phe Val
           35                  40                  45
   Ala Ala Ile Ser Gly Ser Gly Ser Ser Thr Asp Ser Ala Gln Gly Arg
       50                  55                  60
   Phe Thr Ile Ser Arg Asp Asn Ala Lys Asn Thr Val Tyr Leu Gln Met
   65                  70                  75                  80
   Asn Ser Leu Lys Pro Glu Asp Thr Ala Val Tyr Tyr Cys Ala Ala Gly
                   85                  90                  95
   Arg Ala Phe Leu Ala Arg Asp Thr Phe Tyr Tyr Asp Ile Trp Gly Gln
               100                 105                 110
   Gly Thr Gln Val Thr Val Ser Ser
           115                 120
<210> 4
   <211> 126
   <212> PRT
   <213> Lama glama
<400> 4
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Phe Ala Phe Asp Asp His
               20                  25                  30
   Leu Ile Gly Trp Phe Arg Gln Ala Pro Gly Lys Glu Arg Glu Ala Val
           35                  40                  45
   Ser Cys Ile Ser Arg Ser Ala Gly Ser Thr Tyr Tyr Ala Asp Ser Val
       50                  55                  60
   Lys Gly Arg Phe Thr Ile Ser Ser Asp Asn Ala Lys Asn Thr Val Tyr
   65                  70                  75                  80
   Leu Gln Met Asn Ser Leu Lys Ala Asp Asp Thr Ala Val Tyr Tyr Cys
                   85                  90                  95
   Ala Ala Gly Pro Ala Trp Gly Arg Pro Ala Ser Pro Leu Pro Tyr Glu
               100                 105                 110
   Tyr Asp Tyr Trp Gly Gln Gly Thr Gln Val Thr Val Ser Ser
           115                 120                 125
<210> 5
   <211> 134
   <212> PRT
   <213> Lama glama
<400> 5
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Arg Thr Ile Ser Ser Tyr
               20                  25                  30
   Ala Met Ala Trp Phe Arg Gln Ala Pro Gly Lys Glu Arg Glu Phe Val
           35                  40                  45
   Ala Ala Ile Ser Leu Ser Gly Asp Ser Thr Tyr Tyr Ala Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Glu Tyr Tyr Cys
                   85                  90                  95
   Ala Ala Thr Asp Trp Asp Phe Glu Asp Ile Pro Glu Tyr Tyr Cys Ser
               100                 105                 110
   Gly Tyr Gly Cys Asp Glu Ser Leu Phe Asp Ser Trp Gly Gln Gly Thr
           115                 120                 125
   Gln Val Thr Val Ser Ser
       130
<210> 6
   <211> 130
   <212> PRT
   <213> Lama glama
<400> 6
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Leu Asp Tyr Asp
               20                  25                  30
   Ala Ile Gly Trp Phe Arg Gln Ala Pro Gly Lys Glu Arg Glu Gly Val
           35                  40                  45
   Ser Cys Ile Thr Ser Ser Asp Gly Ile Thr Tyr Tyr Ala Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Thr Gly Asn Arg Arg Ile Tyr Tyr Ser Asp Tyr Ala Leu Ala Cys
               100                 105                 110
   Phe Pro Tyr Glu Tyr Asp Tyr Trp Gly Gln Gly Thr Gln Val Thr Val
           115                 120                 125
   Ser Ser
       130
<210> 7
   <211> 123
   <212> PRT
   <213> Lama glama
<400> 7
   Glu Val Gln Leu Val Glu Ser Gly Gly Gly Leu Val Gln Val Gly Asp
   1               5                   10                  15
   Ser Leu Arg Leu Ser Cys Ala Ala Ser Gly Arg Thr Phe Ser Thr Tyr
               20                  25                  30
   Leu Met Val Gly Trp Phe Arg Gln Ala Pro Gly Lys Glu Arg Glu Phe
           35                  40                  45
   Ala Ala Val Ile Trp Ser Ser Gly Asp Thr Ala Tyr Ala Asp Ser Val
       50                  55                  60
   Lys Gly Arg Phe Thr Ile Ser Arg Asp Ser Ala Lys Asn Thr Val Tyr
   65                  70                  75                  80
   Leu Gln Met Asn Ser Leu Lys Ala Glu Asp Thr Ala Val Tyr Tyr Cys
                   85                  90                  95
   Ala Gly Ser Tyr Asp Gly Asn Tyr Tyr Ile Pro Gly Phe Tyr Lys Asp
               100                 105                 110
   Trp Gly Gln Gly Thr Gln Val Thr Val Ser Ser
           115                 120
<210> 8
   <211> 130
   <212> PRT
   <213> Lama glama
<400> 8
   Glu Val Gln Leu Val Glu Ser Gly Gly Gly Leu Val Gln Pro Gly Gly
   1               5                   10                  15
   Ser Leu Arg Leu Ser Cys Thr Thr Ser Gly Phe Thr Leu Asp Tyr Tyr
               20                  25                  30
   Ala Val Gly Trp Phe Arg Gln Ala Pro Gly Lys Glu Arg Glu Gly Val
           35                  40                  45
   Ser Cys Ile Ser Ser Ser Asp Gly Ser Thr Tyr Tyr Ala Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Thr Gly Asn Leu Arg Ile Tyr Tyr Ser Asp Tyr Ala Leu Ala Cys
               100                 105                 110
   Phe Pro Tyr Glu Tyr Asp Tyr Trp Gly Gln Gly Thr Gln Val Thr Val
           115                 120                 125
   Ser Ser
       130
<210> 9
   <211> 123
   <212> PRT
   <213> Lama glama
<400> 9
   Glu Val Gln Leu Val Glu Ser Gly Gly Gly Leu Val Gln Val Gly Asp
   1               5                   10                  15
   Ser Leu Arg Leu Ala Cys Ala Ala Ser Gly Arg Thr Phe Ser Thr Tyr
               20                  25                  30
   Leu Met Val Gly Trp Phe Arg Gln Ala Pro Gly Lys Glu Arg Glu Phe
           35                  40                  45
   Ala Ala Gly Ile Trp Ser Ser Gly Asp Thr Ala Tyr Ala Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Thr Glu Asp Thr Ala Val Tyr Tyr Cys
                   85                  90                  95
   Ala Gly Ser Tyr Asp Gly Asn Tyr Tyr Ile Pro Gly Phe Tyr Lys Asp
               100                 105                 110
   Trp Gly Gln Gly Thr Gln Val Thr Val Ser Ser
           115                 120
<210> 10
   <211> 123
   <212> PRT
   <213> Lama glama
<400> 10
   Glu Val Gln Leu Val Glu Ser Gly Gly Gly Leu Val Gln Val Gly Asp
   1               5                   10                  15
   Ser Leu Arg Leu Ser Cys Ala Ala Ser Gly Arg Thr Phe Ser Thr Tyr
               20                  25                  30
   Leu Met Val Gly Trp Phe Arg Gln Ala Pro Gly Lys Glu Arg Glu Phe
           35                  40                  45
   Ala Ala Ala Met Trp Ser Ser Gly Val Pro Ala Tyr Ala Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Ala Glu Asp Thr Ala Val Tyr Tyr Cys
                   85                  90                  95
   Ala Gly Ser Tyr Gly Gly Asn Tyr Tyr Ile Pro Gly Phe Tyr Glu Asp
               100                 105                 110
   Trp Gly Gln Gly Thr Gln Val Thr Val Ser Ser
           115                 120
<210> 11
   <211> 124
   <212> PRT
   <213> Lama glama
<400> 11
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly His Thr Phe Ser Arg Tyr
               20                  25                  30
   Ala Met Gly Trp Phe Arg Arg Val Pro Gly Lys Glu Arg Glu Phe Val
           35                  40                  45
   Thr His Ile Thr Trp Asn Arg Gly Ser Thr Tyr Tyr Ala Asp Ser Val
       50                  55                  60
   Lys Gly Arg Phe Thr Ile Ser Arg Asp Lys Ala Ser Asn Thr Leu Tyr
   65                  70                  75                  80
   Leu Gln Met Asn Ser Leu Lys Pro Glu Asp Thr Ala Val Tyr Tyr Cys
                   85                  90                  95
   Ala Ala Gln Ile Lys Tyr Gly Ala Val Thr His Pro Glu Glu Tyr Ser
               100                 105                 110
   Tyr Trp Gly Gln Gly Thr Gln Val Thr Val Ser Ser
           115                 120
<210> 12
   <211> 126
   <212> PRT
   <213> Lama glama
<400> 12
   Lys Val Gln Leu Val Glu Ser Gly Gly Gly Leu Val Gln Ala Gly Gly
   1               5                   10                  15
   Ser Leu Arg Leu Ser Cys Ala Ala Ser Gly Phe Ala Phe Asp Asp His
               20                  25                  30
   Leu Ile Gly Trp Phe Arg Gln Ala Pro Gly Lys Glu Arg Glu Ala Val
           35                  40                  45
   Ser Cys Ile Ser Arg Ser Ala Gly Ser Thr Tyr Tyr Ala Asp Ser Val
       50                  55                  60
   Lys Gly Arg Phe Thr Ile Ser Ser Asp Asn Ala Lys Asn Thr Val Tyr
   65                  70                  75                  80
   Leu Gln Met Asn Ser Leu Lys Ala Asp Asp Thr Ala Val Tyr Tyr Cys
                   85                  90                  95
   Ala Ala Gly Pro Ala Trp Gly Arg Pro Ala Ser Pro Leu Pro Tyr Glu
               100                 105                 110
   Tyr Asp Tyr Trp Gly Gln Gly Thr Gln Val Thr Val Ser Ser
           115                 120                 125
<210> 13
   <211> 134
   <212> PRT
   <213> Lama glama
<400> 13
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Phe Thr Phe Asp Asp Tyr
               20                  25                  30
   Ala Ile Gly Trp Phe Arg Gln Ala Pro Gly Lys Glu Arg Glu Gly Val
           35                  40                  45
   Ser Cys Ile Ser Ser Ser Asp Gly Ser Thr Ala Tyr Ala Asp Ser Val
       50                  55                  60
   Lys Gly Arg Phe Thr Ile Ser Ser Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Thr Ala Val Pro Ala Thr Arg Arg Thr Pro Gln Met Val Ala Ala Asn
               100                 105                 110
   Val Cys Trp Leu Ala Glu Tyr Glu Tyr Asp Tyr Trp Gly Gln Gly Thr
           115                 120                 125
   Gln Val Thr Val Ser Ser
       130
<210> 14
   <211> 123
   <212> PRT
   <213> Lama glama
<400> 14
   Glu Val Gln Leu Val Glu Ser Gly Gly Gly Leu Val Gln Val Gly Asp
   1               5                   10                  15
   Ser Leu Arg Leu Ser Cys Ala Ala Ser Gly Arg Thr Phe Ser Thr Tyr
               20                  25                  30
   Leu Met Val Gly Trp Phe Arg Gln Ala Pro Gly Lys Glu Arg Glu Phe
           35                  40                  45
   Ala Ala Gly Ile Trp Ser Ser Gly Gly Thr Ala Tyr Ala Asp Ser Val
       50                  55                  60
   Lys Gly Arg Phe Thr Val Ser Arg Asp Asn Ala Lys Asn Thr Val Tyr
   65                  70                  75                  80
   Leu Gln Met Asn Ser Leu Lys Ala Glu Asp Thr Ala Val Tyr Tyr Cys
                   85                  90                  95
   Ala Gly Ser Tyr Asp Gly Asn Tyr Tyr Ile Pro Gly Phe Tyr Lys Asp
               100                 105                 110
   Trp Gly Gln Gly Thr Gln Val Thr Val Ser Ser
                       115                 120
<210> 15
   <211> 123
   <212> PRT
   <213> Lama glama
<400> 15
   Glu Val Gln Leu Val Glu Phe Gly Gly Gly Leu Val Gln Val Gly Asp
   1               5                   10                  15
   Ser Leu Arg Leu Ser Cys Ala Ala Ser Gly Arg Thr Phe Ser Thr Tyr
               20                  25                  30
   Leu Met Val Gly Trp Phe Arg Gln Ala Pro Gly Lys Glu Arg Glu Phe
           35                  40                  45
   Ala Ala Gly Ile Trp Ser Ser Gly Gly Thr Ala Tyr Ala Asp Ser Val
       50                  55                  60
   Lys Glu Arg Phe Thr Val Ser Arg Asp Asn Asp Lys Asn Thr Val Tyr
   65                  70                  75                  80
   Leu Gln Met Asn Ser Leu Gln Ala Glu Asp Thr Ala Val Tyr Tyr Cys
                   85                  90                  95
   Ala Gly Ser Tyr Asp Gly Asn Tyr Tyr Ile Pro Gly Phe Tyr Lys Asp
               100                 105                 110
   Trp Gly Gln Gly Thr Gln Val Thr Val Ser Ser
           115                 120
<210> 16
   <211> 123
   <212> PRT
   <213> Lama glama
<400> 16
   Glu Val Gln Leu Val Glu Ser Gly Gly Gly Leu Val Gln Val Gly Asp
   1               5                   10                  15
   Ser Leu Arg Leu Ser Cys Ala Ala Ser Gly His Thr Phe Ser Thr Tyr
               20                  25                  30
   Leu Met Val Gly Trp Phe Arg Gln Thr Pro Gly Lys Glu Arg Glu Phe
           35                  40                  45
   Ala Ala Val Ile Trp Ser Ser Gly Asp Thr Ala Tyr Ala Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Ala Glu Asp Thr Ala Val Tyr Tyr Cys
                   85                  90                  95
   Ala Gly Ser Tyr Gly Gly Asn Tyr Tyr Ile Pro Gly Phe Tyr Glu Asp
               100                 105                 110
   Trp Gly Gln Gly Thr Gln Val Thr Val Ser Ser
           115                 120
<210> 17
   <211> 126
   <212> PRT
   <213> Lama glama
<400> 17
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Ala Leu Asp Tyr Tyr
               20                  25                  30
   Ala Ile Gly Trp Phe Arg Gln Val Pro Gly Lys Glu Arg Glu Gly Val
           35                  40                  45
   Ser Cys Ile Ser Ser Ser Asp Gly Ile Thr Tyr Tyr Val Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Thr Asp Ser Gly Gly Tyr Ile Asp Tyr Asp Cys Met Gly Leu Gly
               100                 105                 110
   Tyr Asp Tyr Trp Gly Gln Gly Thr Gln Val Thr Val Ser Ser
           115                 120                 125
<210> 18
   <211> 124
   <212> PRT
   <213> Lama glama
<400> 18
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Arg Thr Phe Ser Ser Tyr
               20                  25                  30
   Ala Met Gly Trp Phe Arg Gln Ala Pro Gly Lys Glu Leu Glu Phe Val
           35                  40                  45
   Thr Ala Val Ser Trp Ser Gly Gly Ser Thr Tyr Tyr Ala Asp Ser Val
       50                  55                  60
   Lys Gly Arg Phe Thr Ile Ser Arg Asp Ser Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Gln Ser Thr Ile Val Glu Val Thr Thr Leu Glu Ala Tyr Asp
               100                 105                 110
   Tyr Trp Gly Gln Gly Thr Gln Val Thr Val Ser Ser
           115                 120
<210> 19
   <211> 122
   <212> PRT
   <213> Lama glama
<400> 19
   Glu Val Gln Leu Val Glu Ser Gly Gly Gly Ser Val Gln Ala Gly Gly
   1               5                   10                  15
   Ser Leu Arg Leu Ser Cys Ala Ala Ser Glu Arg Thr Phe Ser Pro Tyr
               20                  25                  30
   Ala Met Gly Trp Phe Arg Gln Ala Pro Gly Lys Glu Arg Glu Phe Val
           35                  40                  45
   Ala His Ile Thr Trp Ser Ala Gly Ser Thr Tyr Tyr Ala Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Lys Arg Arg Tyr Gly Ile Val Asp Arg Asp Tyr Asn Asp Trp
               100                 105                 110
   Gly Gln Gly Thr Gln Val Thr Val Ser Ser
           115                 120
<210> 20
   <211> 116
   <212> PRT
   <213> Lama glama
<400> 20
   Glu Val Gln Leu Val Glu Ser Gly Gly Gly Leu Val Gln Ala Gly Gly
   1               5                   10                  15
   Ser Leu Arg Leu Ala Cys Ile Ala Ser Gly Arg Asp Ile Phe Ser Val
               20                  25                  30
   Ser Ala Thr Gly Trp Tyr Arg Gln Ala Pro Gly Lys Gln Arg Glu Phe
           35                  40                  45
   Val Ala Gly Ile Ser Asn Ile Gly Ala Thr Lys Phe Ala Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Asn Val Leu Leu Trp Ser Gly Asn Leu Trp Gly Gln Gly Thr Gln Val
               100                 105                 110
   Thr Val Ser Ser
           115
<210> 21
   <211> 116
   <212> PRT
   <213> Lama glama
<400> 21
   Glu Val Gln Leu Val Glu Ser Gly Gly Gly Leu Val Gln Ala Gly Gly
   1               5                   10                  15
   Ser Leu Arg Leu Ser Cys Ile Ala Ser Gly Arg Asp Ile Phe Ser Ile
               20                  25                  30
   Thr Ala Ile Gly Trp Tyr Arg Gln Ala Pro Gly Lys Gln Arg Glu Phe
           35                  40                  45
   Val Ala Gly Ile Ser Asn Ile Gly Ala Thr Lys Tyr Thr Asp Ser Val
       50                  55                  60
   Lys Gly Arg Phe Thr Ile Ser Gly Asp Asn Ala Glu Asn Thr Val Tyr
   65                  70                  75                  80
   Leu Gln Met Asn Ser Leu Lys Pro Glu Asp Thr Ala Val Tyr Tyr Cys
                   85                  90                  95
   Asn Val Leu Leu Trp Ser Ala Asn Tyr Trp Gly Gln Gly Thr Gln Val
               100                 105                 110
   Thr Val Ser Ser
           115
<210> 22
   <211> 126
   <212> PRT
   <213> Lama glama
<400> 22
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Phe Ala Phe Asp Asp His
               20                  25                  30
   Leu Ile Gly Trp Phe Arg Gln Ala Pro Gly Lys Glu Arg Glu Ala Val
           35                  40                  45
   Ser Cys Ile Ser Arg Ser Ala Gly Ser Thr Tyr Tyr Ala Asp Ser Val
       50                  55                  60
   Lys Gly Arg Phe Thr Ile Ser Gly Asp Asn Ala Lys Asn Thr Val Tyr
   65                  70                  75                  80
   Leu Gln Met Asn Ser Leu Lys Ala Asp Asp Thr Ala Val Tyr Tyr Cys
                   85                  90                  95
   Ala Ala Gly Pro Ala Trp Gly Arg Pro Ala Ser Pro Leu Pro Tyr Glu
               100                 105                 110
   Tyr Asp Tyr Trp Gly Gln Gly Thr Gln Val Thr Val Ser Ser
           115                 120                 125
<210> 23
   <211> 134
   <212> PRT
   <213> Lama glama
<400> 23
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Phe Thr Phe Asp Asp Tyr
               20                  25                  30
   Ala Ile Gly Trp Phe Arg Gln Ala Pro Gly Lys Glu Arg Glu Gly Val
           35                  40                  45
   Ser Cys Ile Ser Ser Ser Asp Gly Ser Thr Ala Tyr Ala Asp Ser Val
       50                  55                  60
   Lys Gly Arg Phe Thr Ile Ser Ser Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Thr Ala Val Pro Ala Thr Arg Arg Thr Pro Gln Met Val Val Ala Asn
               100                 105                 110
   Val Cys Trp Leu Ala Glu Tyr Glu Tyr Asp Tyr Trp Gly Gln Gly Thr
           115                 120                 125
   Gln Val Thr Val Ser Ser
       130
<210> 24
   <211> 125
   <212> PRT
   <213> Lama glama
<400> 24
   Glu Val Gln Leu Val Glu Ser Gly Gly Gly Leu Val Gln Ala Gly Gly
   1               5                   10                  15
   Ser Leu Arg Leu Ser Cys Val Ala Ser Gly Phe Thr Phe Asp Asp Tyr
               20                  25                  30
   Ala Ile Gly Trp Phe Arg Gln Ala Pro Gly Lys Glu Arg Glu Gly Val
           35                  40                  45
   Ser Cys Ile Ser Ser Ser Asp Gly Ser Thr Tyr Tyr Ala Asp Ser Val
       50                  55                  60
   Lys Gly Arg Phe Thr Ile Ser Gly Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Gly Ile Thr Pro Cys Ser Asp Tyr Thr Gln Thr Tyr Glu Tyr
               100                 105                 110
   Asp Val Trp Gly Gln Gly Thr Gln Val Thr Val Ser Ser
           115                 120                 125
<210> 25
   <211> 134
   <212> PRT
   <213> Lama glama
<400> 25
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Phe Thr Phe Asp Asp Tyr
               20                  25                  30
   Ala Ile Gly Trp Phe Arg Gln Ala Pro Gly Lys Glu Arg Glu Gly Val
           35                  40                  45
   Ser Cys Ile Ser Arg Ser Asp Gly Ser Thr Ser Tyr Ala Asp Ser Val
       50                  55                  60
   Lys Gly Arg Phe Thr Ile Ser Ser Asp Asn Ala Lys Asn Thr Val Tyr
   65                  70                  75                  80
   Leu Val Met Asn Ser Leu Lys Pro Glu Asp Thr Ala Val Tyr Tyr Cys
                   85                  90                  95
   Thr Ala Val Pro Ala Thr Arg Arg Thr Pro Gln Met Val Val Ala Asn
               100                 105                 110
   Met Cys Trp Leu Ala Glu Tyr Glu Tyr Asp Tyr Trp Gly Gln Gly Thr
           115                 120                 125
   Gln Val Thr Val Ser Ser
       130
<210> 26
   <211> 125
   <212> PRT
   <213> Lama glama
<400> 26
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Phe Thr Phe Asp Asp Tyr
               20                  25                  30
   Ala Ile Gly Trp Phe Arg Gln Ala Pro Gly Lys Glu Arg Glu Gly Val
           35                  40                  45
   Ser Cys Ile Ser Ser Ser Asp Gly Ser Thr Tyr Tyr Ala Asp Ser Val
       50                  55                  60
   Lys Gly Arg Phe Thr Ile Ser Ser Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Gly Ile Thr Pro Cys Ser Asp Tyr Thr Gln Thr Tyr Glu Tyr
               100                 105                 110
   Asp Val Trp Gly Gln Gly Thr Gln Val Thr Val Ser Ser
           115                 120                 125
<210> 27
   <211> 134
   <212> PRT
   <213> Lama glama
<400> 27
   Glu Val Gln Leu Val Glu Ser Gly Gly Gly Leu Val Gln Ala Gly Gly
   1               5                   10                  15
   Ser Leu Arg Leu Ser Cys Ala Val Ser Gly Phe Thr Phe Asp Asp Tyr
               20                  25                  30
   Ala Ile Gly Trp Phe Arg Gln Ala Pro Gly Lys Glu Arg Glu Gly Val
           35                  40                  45
   Ser Cys Ile Ser Ser Ser Asp Gly Ser Thr Ala Tyr Ala Asp Ser Val
       50                  55                  60
   Lys Gly Arg Phe Thr Ile Ser Ser Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Thr Ala Val Pro Ala Thr Arg Arg Thr Pro Gln Met Val Val Ala Asn
               100                 105                 110
   Val Cys Trp Leu Ala Glu Tyr Glu Tyr Asp Tyr Trp Gly Gln Gly Thr
           115                 120                 125
   Gln Val Thr Val Ser Ser
       130
<210> 28
   <211> 132
   <212> PRT
   <213> Lama glama
<400> 28
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Phe Thr Phe Asp Asp Tyr
               20                  25                  30
   Ala Ile Gly Trp Phe Arg Gln Ala Pro Gly Lys Glu Arg Glu Gly Val
           35                  40                  45
   Ser Tyr Ile Ser Ser Ser Asp Gly Ser Thr Tyr Tyr Ala Asp Ser Val
       50                  55                  60
   Lys Gly Arg Phe Thr Ser Ser Ser Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Arg Pro Thr Leu Arg Val Arg Leu Asp Asn Asp Arg His His
               100                 105                 110
   Leu Leu Tyr Glu Tyr Glu Tyr Asp Tyr Trp Gly Gln Gly Thr Gln Val
           115                 120                 125
   Thr Val Ser Ser
       130
<210> 29
   <211> 134
   <212> PRT
   <213> Lama glama
<400> 29
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Phe Thr Phe Asp Asp Tyr
               20                  25                  30
   Thr Ile Gly Trp Phe Arg Gln Ala Pro Gly Lys Glu Arg Glu Gly Val
           35                  40                  45
   Ser Cys Ile Ser Ser Ser Asp Gly Ser Thr Ser Tyr Ala Asp Ser Val
       50                  55                  60
   Lys Gly Arg Phe Thr Ile Ser Ser Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Thr Ala Val Pro Ala Thr Arg Arg Thr Pro Gln Met Val Val Leu Asn
               100                 105                 110
   Met Cys Trp Leu Ala Glu Tyr Glu Tyr Asp Tyr Trp Gly Gln Gly Thr
           115                 120                 125
   Gln Val Thr Val Ser Ser
       130
<210> 30
   <211> 125
   <212> PRT
   <213> Lama glama
<400> 30
   Glu Val Gln Leu Val Glu Ser Gly Gly Gly Leu Val Gln Ala Gly Gly
   1               5                   10                  15
   Ser Leu Arg Leu Ser Cys Val Ala Ser Gly Phe Thr Phe Asp Asp Tyr
               20                  25                  30
   Ala Ile Gly Trp Phe Arg Gln Ala Pro Gly Lys Glu Arg Glu Gly Val
           35                  40                  45
   Ser Cys Ile Ser Ser Ser Asp Gly Ser Thr Tyr Tyr Ala Asp Ser Val
       50                  55                  60
   Lys Gly Arg Phe Thr Ile Ser Ser Asp Asn Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Ala Gly Ile Thr Pro Cys Ser Asp Tyr Thr Gln Thr Tyr Glu Tyr
   100 105 110
   Asp Val Trp Gly Gln Gly Thr Gln Val Thr Val Ser Ser
   115 120 125
<210> 31
   <211> 134
   <212> PRT
   <213> Lama glama
<400> 31
   Glu Val Gln Leu Val Glu Ser Gly Gly Gly Ser Val Gln Ala Gly Gly
   1 5 10 15
   Ser Leu Arg Leu Ser Cys Ala Ala Ser Gly Phe Thr Phe Asp Asp Tyr
   20 25 30
   Ala Ile Gly Trp Phe Arg Gln Ala Pro Gly Lys Glu Arg Glu Gly Val
   35 40 45
   Ser Cys Ile Ser Ser Ser Asp Gly Ser Thr Asn Tyr Ala Asp Ser Val
   50 55 60
   Lys Gly Arg Phe Thr Ile Ser Ser Asp Thr Ala Lys Asn Thr Val Tyr
   65 70 75 80
   Leu Gln Met Asn Ser Leu Lys Pro Glu Asp Thr Ala Val Tyr Tyr Cys
   85 90 95
   Thr Ala Val Pro Ala Thr Arg Arg Thr Pro Gln Met Val Asp Ala Asn
   100 105 110
   Met Cys Trp Leu Ala Glu Tyr Glu Tyr Asp Tyr Trp Gly Gln Gly Thr
   115 120 125
   Gln Val Thr Val Ser Ser
   130
<210> 32
   <211> 127
   <212> PRT
   <213> Lama glama
<400> 32
   Glu Val Gln Leu Val Glu Ser Gly Gly Gly Leu Ala Gln Ala Gly Gly
   1               5                   10                  15
   Ser Leu Arg Leu Ser Cys Ala Ala Ser Gly Arg Arg Phe Gly Gly His
               20                  25                  30
   Ala Met Gly Trp Phe Arg Gln Ala Pro Gly Glu Glu Arg Glu Phe Val
           35                  40                  45
   Ala Thr Ile Tyr Trp Thr Ser Gly Met Thr Arg Tyr Ala Gly Ser Val
       50                  55                  60
   Lys Gly Arg Phe Thr Ile Ser Arg Asp Asn Ala Glu Asn Thr Val Phe
   65                  70                  75                  80
   Leu Gln Met Asn Ser Leu Lys Pro Glu Asp Thr Ala Val Tyr Tyr Cys
                   85                  90                  95
   Ala Val Ile Lys Asp Phe Gln Leu Arg Val Asp Val Thr Ser Ala Ser
               100                 105                 110
   Ala Tyr Asp Tyr Trp Gly Gln Gly Thr Gln Val Thr Val Ser Ser
           115                 120                 125
<210> 33
   <211> 127
   <212> PRT
   <213> Lama glama
<400> 33
   Gly Val Gln Leu Val Glu Ser Gly Gly Gly Leu Ala Gln Ala Gly Gly
   1               5                   10                  15
   Ser Leu Arg Leu Ser Cys Ala Ala Ser Gly Arg Arg Phe Gly Gly His
               20                  25                  30
   Ala Met Gly Trp Phe Arg Gln Ala Pro Gly Lys Asp Arg Glu Phe Val
           35                  40                  45
   Ala Thr Ile Tyr Trp Thr Thr Gly Met Thr Arg Tyr Ala Asp Ser Val
       50                  55                  60
   Lys Gly Arg Phe Thr Ile Ser Arg Asp Asn Ala Glu Asn Thr Val Phe
   65                  70                  75                  80
   Leu Gln Met Asn Ser Leu Lys Pro Glu Asp Thr Ala Val Tyr Tyr Cys
   85 90 95
   Ala Leu Ile Arg Asp Phe Asn Ile Arg Leu Asp Val Thr Ser Ala Ser
   100 105 110
   Ala Tyr Gly Tyr Trp Gly Gln Gly Thr Gln Val Thr Val Ser Ser
   115 120 125
<210> 34
   <211> 123
   <212> PRT
   <213> Lama glama
<400> 34
   Glu Val Gln Leu Val Glu Ser Gly Gly Gly Leu Val Gln Val Gly Asp
   1 5 10 15
   Ser Leu Arg Leu Ser Cys Ala Ala Ser Gly Arg Thr Phe Ser Thr Tyr
   20 25 30
   Leu Met Val Gly Trp Phe Arg Gln Ala Pro Gly Lys Glu Arg Glu Phe
   35 40 45
   Ala Ala Gly Ile Trp Ser Ser Gly Asp Thr Ala Tyr Ala Asp Ser Val
   50 55 60
   Arg Gly Arg Phe Thr Ile Ser Arg Asp Asn Ala Lys Asn Thr Val Tyr
   65 70 75 80
   Leu Gln Met Asn Ser Leu Lys Thr Glu Asp Thr Ala Val Tyr Tyr Cys
   85 90 95
   Ala Gly Ser Tyr Asp Gly Asn Tyr Tyr Ile Pro Gly Phe Tyr Lys Asp
   100 105 110
   Trp Gly Gln Gly Thr Gln Val Thr Val Ser Ser
   115 120
<210> 35
   <211> 123
   <212> PRT
   <213> Lama glama
<400> 35
   Glu Val Gln Leu Val Glu Ser Gly Gly Gly Leu Val Gln Val Gly Asp
   1               5                   10                  15
   Ser Leu Arg Leu Ser Cys Ala Ala Ser Gly Arg Thr Phe Ser Thr Tyr
               20                  25                  30
   Leu Val Val Gly Trp Phe Arg Gln Ala Pro Gly Lys Glu Arg Glu Phe
           35                  40                  45
   Ala Ala Ala Ile Trp Ser Ser Gly Asp Thr Ala Tyr Ala Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Ala Glu Asp Thr Ala Val Tyr Tyr Cys
                   85                  90                  95
   Ala Gly Ser Tyr Gly Gly Asn Tyr Tyr Ile Pro Gly Phe Tyr Glu Asp
               100                 105                 110
   Trp Gly Gln Gly Thr Gln Val Thr Val Ser Ser
           115                 120
<210> 36
   <211> 121
   <212> PRT
   <213> Lama glama
<400> 36
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Arg Thr Phe Ser Arg Asn
               20                  25                  30
   Ala Met Ala Trp Phe Arg Gln Val Pro Gly Lys Val Arg Glu Phe Val
           35                  40                  45
   Ala Gly Ile Arg Trp Asn Val Gly Arg Leu Asp Tyr Ala Asp Ser Val
       50                  55                  60
   Lys Gly Arg Phe Thr Ile Ser Arg Asp Asn Ala Glu Asn Thr Val Tyr
   65                  70                  75                  80
   Leu Gln Met Asn Asp Leu Lys Thr Glu Asp Thr Ala Val Tyr Tyr Cys
                   85                  90                  95
   Ala Ala Tyr Ala Gly Leu Val Phe Ser Gly Ile Pro Asp Tyr Trp Gly
               100                 105                 110
   Gln Gly Thr Gln Val Thr Val Ser Ser
                       115                 120
<210> 37
   <211> 118
   <212> PRT
   <213> Lama glama
<400> 37
   Glu Val Gln Leu Val Glu Ser Gly Arg Gly Leu Val Gln Ala Gly Gly
   1 5 10 15
   Ser Leu Arg Leu Ser Cys Ala Ala Ser Gly Ser Ile Phe Ser Ile Asn
   20 25 30
   Ala Met Leu Trp Tyr Arg Gln Ala Pro Gly Lys Gln Arg Glu Leu Val
   35 40 45
   Ala Ala Ile Thr Ser Gly Gly Ser Thr Asn Tyr Ala Asp Ser Val Lys
   50 55 60
   Gly Arg Phe Thr Ile Ser Arg Asp Asn Ala Lys Asn Thr Val Tyr Leu
   65 70 75 80
   Gln Met Asn Ser Leu Lys Pro Glu Asp Thr Ala Val Tyr Tyr Cys Ala
   85 90 95
   Ala Asp Ser Asp Tyr Ser Ser Asp Tyr Tyr Tyr Trp Gly Gln Gly Thr
   100 105 110
   Gln Val Thr Val Ser Ser
   115
<210> 38
   <211> 133
   <212> PRT
   <213> Lama glama
<400> 38
   Glu Val Gln Leu Val Glu Ser Gly Gly Gly Leu Val Gln Ser Gly Gly
   1 5 10 15
   Ser Leu Arg Leu Ser Cys Ala Ala Ser Gly Phe Ala Leu Asp Tyr Tyr
   20 25 30
   Thr Ile Gly Trp Phe Arg Gln Ala Pro Gly Lys Glu Arg Glu Gly Val
   35 40 45
   Ser Cys Ile Ser Gly Gly Asp Thr Ser Thr Tyr Tyr Ala Asp Ser Val
   50 55 60
   Lys Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Thr Asp Ser Ala Gly Val Pro Ala Gly Pro Ala Ala Val Tyr Gly
               100                 105                 110
   Ser Thr Cys Ser Arg Leu Glu Tyr Asp Tyr Trp Gly Gln Gly Ala Gln
           115                 120                 125
   Val Thr Val Ser Ser
       130
<210> 39
   <211> 124
   <212> PRT
   <213> Lama glama
<400> 39
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Gly Thr Phe Ser Ser Tyr
               20                  25                  30
   Ser Met Gly Trp Phe Arg Gln Ala Pro Gly Lys Glu Arg Glu Phe Ile
           35                  40                  45
   Ala Ala Ile Asn Trp Asn Gly Asp Ser Thr Tyr Tyr Glu Asp Ser Val
       50                  55                  60
   Lys Gly Arg Phe Thr Val Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Thr Gly Trp Gly Arg Ala Tyr Glu Gln Ala Tyr Glu Tyr Asp
               100                 105                 110
   Val Trp Gly Gln Gly Thr Gln Val Thr Val Ser Ser
           115                 120
<210> 40
   <211> 119
   <212> PRT
   <213> Lama glama
<400> 40
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Val Asp Asp Tyr
               20                  25                  30
   Ala Met Ser Trp Val Arg Gln Ala Pro Gly Lys Gly Leu Glu Trp Val
           35                  40                  45
   Ser Thr Ile Ser Trp Asn Asp Glu Tyr Thr Tyr Tyr Ala Glu Ser Met
       50                  55                  60
   Lys Gly Arg Phe Thr Ile Ser Arg Asp Asn Ala Lys Asn Thr Leu Tyr
   65                  70                  75                  80
   Leu Gln Met Asn Ser Leu Lys Ser Glu Asp Thr Ala Val Tyr Tyr Cys
                   85                  90                  95
   Ala Lys Gly Gly Ser Arg Leu Tyr Asp Tyr His Tyr Trp Gly Gln Gly
               100                 105                 110
   Thr Gln Val Thr Val Ser Ser
           115
<210> 41
   <211> 123
   <212> PRT
   <213> Lama glama
<400> 41
   Glu Val Gln Leu Val Glu Ser Gly Gly Gly Leu Val Gln Ala Gly Gly
   1               5                   10                  15
   Ser Leu Arg Leu Ser Cys Ala Ser Ser Gly Phe Thr Phe Asp Asp Tyr
               20                  25                  30
   Ala Ile Gly Trp Phe Arg Gln Ala Pro Gly Lys Glu Arg Glu Gly Val
           35                  40                  45
   Ser Cys Ile Ser Ser Ser Asp Gly Ser Thr Tyr Tyr Ala Asp Ser Val
       50                  55                  60
   Lys Gly Arg Phe Thr Ile Ser Ser Asp Asn Ala Lys Asn Thr Val Tyr
   65                  70                  75                  80
   Leu Gln Met Asn Ser Leu Lys Pro Glu Asp Thr Ala Val Tyr Tyr Cys
   85 90 95
   Ala Ala Arg Pro Pro Phe His Ser Cys Ser Glu Tyr Glu Asn Asp Tyr
   100 105 110
   Trp Gly Gln Gly Thr Gln Val Thr Val Ser Ser
   115 120
<210> 42
   <211> 120
   <212> PRT
   <213> Lama glama
<400> 42
   Glu Val Gln Leu Val Glu Ser Gly Gly Gly Leu Val Gln Ala Gly Gly
   1 5 10 15
   Ser Leu Arg Leu Ser Cys Ala Ala Ser Gly Arg Thr Ile Ser Ser Ser
   20 25 30
   Val Leu Gly Trp Phe Arg Gln Ala Pro Gly Lys Glu Arg Glu Phe Val
   35 40 45
   Ala Ala Ile Ser Gly Ser Gly Ser Ser Thr Asp Ser Ala Lys Asp Arg
   50 55 60
   Phe Thr Ile Ser Arg Asp Asn Ala Lys Asn Thr Val Tyr Leu Gln Met
   65 70 75 80
   Asn Ser Leu Lys Pro Glu Asp Thr Ala Val Tyr Tyr Cys Ala Ala Gly
   85 90 95
   Arg Ala Phe Leu Thr Arg Asp Pro Phe Tyr Tyr Asp Ile Trp Gly Gln
   100 105 110
   Gly Thr Gln Val Thr Val Ser Ser
   115 120
<210> 43
   <211> 116
   <212> PRT
   <213> Lama glama
<400> 43
   Glu Val Gln Leu Val Glu Ser Gly Gly Gly Leu Val Gln Ala Gly Gly
   1 5 10 15
   Ser Leu Arg Leu Thr Cys Ile Ala Ser Gly Arg Asp Ile Phe Ser Val
   20 25 30
   Thr Ala Met Gly Trp Tyr Arg Gln Ala Pro Gly Lys Gln Arg Glu Phe
   35 40 45
   Val Ala Gly Leu Ser Asn Ile Gly Ala Thr Lys Tyr Ala Asp Ser Val
   50 55 60
   Lys Gly Arg Phe Thr Val Ser Gly Asp Ala Ala Lys Asn Thr Val Tyr
   65 70 75 80
   Leu Gln Met Asn Ser Leu Lys Pro Glu Asp Thr Ala Val Tyr Tyr Cys
   85 90 95
   Asn Val Leu Leu Trp Ser Gly Asn Tyr Trp Gly Gln Gly Thr Gln Val
   100 105 110
   Thr Val Ser Ser
   115
<210> 44
   <211> 132
   <212> PRT
   <213> Lama glama
<400> 44
   Glu Val Gln Leu Val Glu Ser Gly Gly Gly Leu Val Gln Ala Gly Gly
   1 5 10 15
   Ser Leu Arg Leu Ser Cys Ala Ala Ser Gly Phe Thr Phe Asp Asp Tyr
   20 25 30
   Ala Ile Gly Trp Phe Arg Gln Ala Pro Gly Lys Glu Arg Glu Gly Val
   35 40 45
   Ser Tyr Ile Ser Lys Ser Asp Gly Thr Thr Tyr Tyr Ala Asp Ser Val
   50 55 60
   Lys Gly Arg Phe Thr Ser Ser Ser Asp Asn Ala Lys Asn Thr Val Tyr
   65 70 75 80
   Leu Gln Ile Asn Ser Leu Lys Pro Glu Asp Thr Ala Val Tyr Tyr Cys
   85 90 95
   Ala Ala Arg Pro Thr Leu Arg Val Arg Leu Asp Asn Asp Arg His His
   100 105 110
   Leu Leu Tyr Glu Tyr Glu Tyr Asp Tyr Trp Gly Gln Gly Thr Gln Val
   115 120 125
   Thr Val Ser Ser
   130
<210> 45
   <211> 132
   <212> PRT
   <213> Lama glama
<400> 45
   Glu Val Gln Leu Val Glu Ser Gly Gly Gly Leu Val Gln Ala Gly Gly
   1 5 10 15
   Ser Leu Arg Leu Ser Cys Ala Ala Ser Gly Phe Thr Phe Asp Glu Tyr
   20 25 30
   Ala Ile Gly Trp Phe Arg Gln Ala Pro Gly Lys Glu Arg Glu Gly Val
   35 40 45
   Ser Tyr Ile Ser Ser Ser Asp Gly Ser Thr Tyr Tyr Ala Asp Ser Val
   50 55 60
   Lys Gly Arg Val Thr Ser Ser Ser Asp Asn Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Ala Arg Pro Thr Leu Arg Val Arg Leu Asp Asn Asp Arg His His
   100 105 110
   Leu Leu Tyr Glu Tyr Glu Tyr Asp Tyr Trp Gly Gln Gly Thr Gln Val
   115 120 125
   Thr Val Ser Ser
   130
<210> 46
   <211> 124
   <212> PRT
   <213> Lama glama
<400> 46
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Arg Thr Phe Ser Ser Tyr
               20                  25                  30
   Ala Met Gly Trp Phe Arg Gln Ala Pro Gly Lys Glu Arg Glu Phe Val
           35                  40                  45
   Ala Arg Ile Ser Trp Asn Gly Gly Ser Thr Tyr His Ala Asp Ser Val
       50                  55                  60
   Lys Gly Arg Phe Thr Ile Ser Arg Asp Asn Pro Lys Asn Thr Val Tyr
   65                  70                  75                      80
   Leu Gln Met Asp Ser Leu Lys Pro Glu Asp Ala Ala Ile Tyr Tyr Cys
                   85                  90                  95
   Ala Ala Ser Ile Ala Leu Val Gly Gly Val Thr Pro His Ser Tyr Asp
               100                 105                 110
   Tyr Trp Gly Gln Gly Thr Gln Val Thr Val Ser Ser
           115                 120
<210> 47
   <211> 123
   <212> PRT
   <213> Lama glama
<400> 47
   Glu Val Gln Leu Val Glu Ser Gly Gly Gly Leu Val Gln Val Gly Asp
   1               5                   10                  15
   Ser Leu Arg Leu Ser Cys Ala Ala Ser Gly Arg Thr Phe Ser Thr Tyr
               20                  25                  30
   Leu Met Val Gly Trp Phe Arg Gln Ala Pro Gly Lys Glu Arg Glu Phe
           35                  40                  45
   Ala Ala Val Ile Trp Ser Ser Gly Asp Thr Asp Tyr Ala Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Ala Glu Asp Thr Ala Val Tyr Tyr Cys
                   85                  90                  95
   Ala Gly Ser Tyr Gly Gly Asn Tyr Tyr Ile Pro Gly Phe Tyr Lys Asp
               100                 105                 110
   Trp Gly Gln Gly Thr Gln Val Thr Val Ser Ser
                       115                 120
<210> 48
   <211> 123
   <212> PRT
   <213> Lama glama
<400> 48
   Glu Val Gln Leu Val Glu Ser Gly Gly Gly Leu Val Gln Val Gly Asp
   1               5                   10                  15
   Ser Leu Arg Leu Ser Cys Ala Ala Ser Gly Arg Thr Phe Ser Thr Tyr
               20                  25                  30
   Leu Met Val Gly Trp Phe Arg Gln Ala Pro Gly Lys Glu Arg Glu Phe
           35                  40                  45
   Ala Ala Gly Ile Trp Ser Ser Gly Gly Thr Ala Tyr Ala Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Ala Glu Asp Thr Ala Val Tyr Tyr Cys
                   85                  90                  95
   Ala Gly Ser Tyr Asp Gly Asn Tyr Tyr Ile Pro Gly Phe Tyr Lys Asp
               100                 105                 110
   Trp Gly Gln Gly Thr Gln Val Thr Val Ser Ser
           115                 120
<210> 49
   <211> 128
   <212> PRT
   <213> Lama glama
<400> 49
   Glu Val Gln Leu Val Glu Ser Gly Gly Gly Leu Val Gln Ala Gly Gly
   1               5                   10                  15
   Ser Leu Gly Leu Ser Cys Ala Ala Ser Glu Arg Thr Leu Pro Ser Tyr
               20                  25                  30
   Val Met Gly Trp Phe Arg Gln Ala Pro Gly Lys Glu Leu Glu Phe Val
           35                  40                  45
   Ala Gly Ile Ser Trp Ser Ser Gly Arg Thr Tyr Tyr Thr Asp Ser Val
       50                  55                  60
   Lys Gly Arg Phe Thr Ile Ser Arg Asp Ala Ala Glu Asn Thr Trp Tyr
   65 70 75 80
   Leu Gln Met Asn Ser Leu Lys Pro Glu Asp Thr Ala Val Tyr Tyr Cys
   85 90 95
   Ala Ser Asn Ser Val Ser Glu Pro Thr Leu His Thr Trp Gln Tyr Glu
   100 105 110
   Ala Ser Tyr Asp Tyr Trp Gly Gln Gly Thr Gln Val Thr Val Ser Ser
   115 120 125
<210> 50
   <211> 124
   <212> PRT
   <213> Lama glama
<400> 50
   Glu Val Gln Leu Val Glu Ser Gly Gly Gly Leu Val Gln Ala Gly Gly
   1 5 10 15
   Ser Leu Arg Leu Ser Cys Ala Ala Ser Gly His Thr Phe Ser Arg Tyr
   20 25 30
   Ala Met Gly Trp Phe Arg Arg Val Pro Gly Glu Glu Arg Glu Phe Val
   35 40 45
   Thr His Ile Thr Trp Asn Arg Gly Ser Thr Tyr Tyr Ala Asp Ser Val
   50 55 60
   Lys Gly Arg Phe Thr Ile Ser Arg Asp Lys Ala Ser Asn Thr Leu Tyr
   65 70 75 80
   Leu Gln Met Asn Ser Leu Lys Pro Glu Asp Thr Ala Val Tyr Tyr Cys
   85 90 95
   Ala Ala Gln Ile Lys Tyr Gly Glu Ile Thr His Pro Glu Glu Tyr Ser
   100 105 110
   Tyr Trp Gly Gln Gly Thr Gln Val Thr Val Ser Ser
   115 120
<210> 51
   <211> 121
   <212> PRT
   <213> Lama glama
<400> 51
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Arg Thr Phe Ser Arg Asn
               20                  25                  30
   Ala Met Gly Trp Phe Arg Gln Val Pro Gly Lys Ala Arg Glu Phe Val
           35                  40                  45
   Ala Ala Ile Arg Trp Asn Val Gly Arg Leu Asp Tyr Ala Asp Ser Val
       50                  55                  60
   Lys Gly Arg Phe Ala Ile Ser Arg Asp Asn Ala Glu Asn Thr Val Tyr
   65                  70                  75                  80
   Leu Gln Met Asn Asp Leu Lys Thr Glu Asp Thr Ala Val Tyr Tyr Cys
                   85                  90                  95
   Ala Ala Tyr Ala Gly Leu Val Tyr Ser Gly Ile Pro Asp Tyr Trp Gly
               100                 105                 110
   Gln Gly Thr Gln Val Thr Val Ser Ser
           115                 120
<210> 52
   <211> 124
   <212> PRT
   <213> Lama glama
<400> 52
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly His Thr Phe Ser Arg Tyr
               20                  25                  30
   Ala Met Gly Trp Phe Arg Arg Val Pro Gly Lys Glu Arg Glu Phe Val
           35                  40                  45
   Thr His Ile Thr Trp Asn Arg Gly Ser Thr Tyr Tyr Ala Asp Ser Val
       50                  55                  60
   Lys Gly Arg Phe Thr Ile Ser Arg Asp Lys Ala Ser Asn Thr Leu Tyr
   65                  70                  75                  80
   Leu Gln Met Asn Ser Leu Lys Pro Glu Asp Thr Ala Val Tyr Tyr Cys
                   85                  90                  95
   Ala Ala Gln Ile Lys Tyr Gly Glu Ile Thr His Pro Glu Glu Tyr Ser
               100                 105                 110
   Tyr Trp Gly Gln Gly Thr Gln Val Thr Val Ser Ser
           115                 120
<210> 53
   <211> 124
   <212> PRT
   <213> Lama glama
<400> 53
   Glu Val Gln Leu Val Glu Ser Gly Gly Gly Leu Val Gln Ala Gly Asp
   1               5                   10                  15
   Ser Leu Arg Leu Ser Cys Ala Ala Ser Gly His Thr Phe Ser Arg Tyr
               20                  25                  30
   Ala Met Gly Trp Phe Arg Arg Val Pro Gly Lys Glu Arg Glu Phe Val
           35                  40                  45
   Thr His Ile Thr Trp Asn Arg Gly Ser Thr Tyr Tyr Ala Asp Ser Val
       50                  55                  60
   Lys Gly Arg Phe Thr Ile Ser Arg Asp Lys Ala Ser Asn Thr Leu Tyr
   65                  70                  75                  80
   Leu Gln Met Asn Ser Leu Lys Ser Glu Asp Thr Ala Val Tyr Tyr Cys
                   85                  90                  95
   Ala Ala Gln Thr Lys Tyr Gly Glu Ile Thr Arg Pro Glu Glu Tyr Ser
               100                 105                 110
   Tyr Trp Gly Gln Gly Thr Gln Val Thr Val Ser Ser
           115                 120
<210> 54
   <211> 115
   <212> PRT
   <213> Lama glama
<400> 54
   Glu Val Gln Leu Val Glu Ser Gly Gly Gly Leu Val Gln Ala Gly Gly
   1               5                   10                  15
   Ser Leu Arg Leu Val Cys Ser Ala Ser Gly Ile Asp Phe Ser Ile Asn
               20                  25                  30
   Ala Met Ala Trp Tyr Arg Gln Ala Pro Gly Lys Gln Arg Glu Trp Val
   35 40 45
   Ala Phe Met Ile Asn Asp Ser Ser Thr Asp Tyr Thr Asp Ser Val Lys
   50 55 60
   Gly Arg Phe Thr Ile Ser Arg Asp Ser Thr Lys Asn Ile Leu Tyr Leu
   65 70 75 80
   Gln Met Asn Asn Leu Asn Val Glu Asp Thr Ala Val Tyr Tyr Cys Asn
   85 90 95
   Thr Ala Tyr Glu Gln His Thr Tyr Trp Gly Gln Gly Thr Gln Val Thr
   100 105 110
   Val Ser Ser
   115
<210> 55
   <211> 128
   <212> PRT
   <213> Lama glama
<400> 55
   Glu Val Gln Leu Val Glu Ser Gly Gly Gly Leu Val Gln Ala Gly Gly
   1 5 10 15
   Ser Leu Arg Leu Ser Cys Ala Ala Ser Gly Phe Thr Phe Asp Asp Tyr
   20 25 30
   Ala Ile Ala Trp Phe Arg Gln Ala Pro Gly Lys Glu Arg Glu Gly Ile
   35 40 45
   Ser Cys Ile Thr Pro Ser Asp Asp Arg Thr Tyr Tyr Ala Asp Ser Val
   50 55 60
   Lys Gly Arg Phe Ile Ile Ser Ser Asp Asn Ala Lys Asn Thr Val Tyr
   65 70 75 80
   Leu Gln Met Asn Ser Leu Lys Pro Glu Asp Thr Ala Asp Tyr Tyr Cys
   85 90 95
   Ala Ala Val Pro Arg Leu Arg Gly Leu Gly Tyr Trp Pro Tyr Pro Glu
   100 105 110
   Tyr Glu Tyr Asp Tyr Trp Gly Gln Gly Thr Gln Val Thr Val Ser Ser
   115 120 125
<210> 56
   <211> 124
   <212> PRT
   <213> Lama glama
<400> 56
   Lys Val Gln Leu Val Glu Ser Gly Gly Gly Leu Val Gln Ala Gly Gly
   1               5                   10                  15
   Ser Leu Arg Leu Ser Cys Ala Ala Ser Gly Phe Thr Phe Asp Asp Tyr
               20                  25                  30
   Ala Leu Gly Trp Phe Arg Gln Ala Ala Gly Lys Glu Arg Glu Gly Val
           35                  40                  45
   Ser Cys Ile Arg Cys Ser Asp Gly Ser Thr Tyr Tyr Ala Asp Ser Val
       50                  55                  60
   Lys Gly Arg Phe Thr Ile Ser Ser Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Ser Ile Val Pro Arg Ser Lys Leu Glu Pro Tyr Glu Tyr Asp
               100                 105                 110
   Ala Trp Gly Gln Gly Thr Gln Val Thr Val Ser Ser
           115                 120
<210> 57
   <211> 119
   <212> PRT
   <213> Lama glama
<400> 57
   Glu Val Gln Leu Val Glu Ser Gly Gly Gly Leu Val Gln Pro Gly Gly
   1               5                   10                  15
   Ser Leu Arg Leu Ser Cys Ala Val Ser Gly Phe Thr Val Asp Asp Tyr
               20                  25                  30
   Ala Met Ser Trp Val Arg Gln Ala Pro Gly Lys Gly Leu Glu Trp Val
           35                  40                  45
   Ser Thr Ile Ser Trp Asn Asp Glu Tyr Thr Tyr Tyr Ala Glu Ser Met
       50                  55                  60
   Lys Gly Arg Phe Thr Ile Ser Arg Asp Asn Ala Lys Asn Thr Leu Tyr
   65                  70                  75                  80
   Leu Gln Met Asn Ser Leu Lys Ser Glu Asp Thr Ala Val Tyr Tyr Cys
                   85                  90                  95
   Ala Lys Gly Gly Ser Arg Leu Tyr Asp Tyr His Tyr Trp Gly Gln Gly
               100                 105                 110
   Thr Gln Val Thr Val Ser Ser
           115
<210> 58
   <211> 131
   <212> PRT
   <213> Lama glama
<400> 58
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Phe Thr Leu Asp Asp Tyr
               20                  25                  30
   Ala Ile Gly Trp Phe Arg Gln Ala Pro Gly Lys Glu Arg Glu Gly Val
           35                  40                  45
   Ser Cys Ile Ser Ser Ser Asp Gly Ser Thr Thr Tyr Ala Asp Ser Val
       50                  55                  60
   Lys Gly Arg Phe Thr Val Ser Ser Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Gly Leu Arg Gly Arg Tyr Tyr Arg Gly Thr Tyr Ser Leu Val
               100                 105                 110
   Cys Ala Pro Tyr Glu Tyr Asp Phe Trp Gly Gln Gly Thr Gln Val Thr
           115                 120                 125
   Val Ser Ser
       130
<210> 59
   <211> 131
   <212> PRT
   <213> Lama glama
<400> 59
   Glu Val Gln Leu Val Glu Ser Gly Gly Gly Leu Val Gln Ala Gly Gly
   1 5 10 15
   Ser Leu Arg Leu Ser Cys Ala Ala Ser Gly Phe Thr Leu Asp Asp Tyr
   20 25 30
   Ala Ile Gly Trp Phe Arg Gln Ala Pro Gly Lys Glu Arg Glu Gly Val
   35 40 45
   Ser Cys Ile Ser Ser Ser Asp Gly Ser Thr Tyr Tyr Ala Asp Ser Val
   50 55 60
   Lys Gly Arg Phe Thr Ile Ser Ala Asp Asn Ala Lys Asn Thr Val Tyr
   65 70 75 80
   Leu Gln Met His Ser Leu Lys Pro Glu Asp Thr Ala Val Tyr Tyr Cys
   85 90 95
   Ala Ala Gly Leu Arg Gly Arg Tyr Tyr Ser Gly Ser Asn Tyr Leu Val
   100 105 110
   Cys Ala Pro Tyr Glu Tyr Asp Tyr Trp Gly Gln Gly Thr Gln Val Thr
   115 120 125
   Val Ser Ser
   130
<210> 60
   <211> 125
   <212> PRT
   <213> Lama glama
<400> 60
   Glu Val Gln Leu Val Glu Ser Gly Gly Gly Leu Val Gln Ala Gly Gly
   1 5 10 15
   Ser Leu Arg Leu Ser Cys Ala Ala Ser Gly Phe Thr Phe Asp Asp Tyr
   20 25 30
   Ala Ile Gly Trp Phe Arg Gln Ala Pro Gly Lys Glu Arg Glu Gly Val
   35 40 45
   Ser Cys Ile Ser Ser Ser Asp Gly Ser Thr Tyr Tyr Ala Asp Ser Val
   50 55 60
   Lys Gly Arg Phe Thr Ile Ser Ser Asp Asn Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Ala Asp Phe Ile Ile Ser Ser Lys Arg Leu Cys Leu Asp Leu Phe
   100 105 110
   Gly Ser Arg Gly Gln Gly Thr Gln Val Thr Val Ser Ser
   115 120 125
<210> 61
   <211> 125
   <212> PRT
   <213> Lama glama
<400> 61
   Glu Val Gln Leu Val Glu Ser Gly Gly Gly Ser Val Gln Ala Gly Gly
   1 5 10 15
   Ser Leu Arg Leu Ser Cys Ala Ala Ser Gly Phe Thr Phe Asp Asp Tyr
   20 25 30
   Ala Ile Gly Trp Phe Arg Gln Ala Pro Gly Lys Glu Arg Glu Gly Val
   35 40 45
   Ser Cys Ile Ser Ser Ser Asp Gly Ser Thr Tyr Tyr Ala Asp Ser Val
   50 55 60
   Lys Gly Arg Phe Thr Ile Ser Ser Asp Asn Ala Lys Asn Thr Val Tyr
   65 70 75 80
   Leu Gln Met Asn Ser Leu Lys Pro Gly Asp Thr Ala Val Tyr Tyr Cys
   85 90 95
   Ala Ala Gly Ile Thr Pro Cys Ser Asp Tyr Thr Gln Thr Tyr Glu Tyr
   100 105 110
   Asp Val Trp Gly Gln Gly Thr Gln Val Thr Val Ser Ser
   115 120 125
<210> 62
   <211> 134
   <212> PRT
   <213> Lama glama
<400> 62
   Glu Val Gln Leu Val Glu Ser Gly Gly Gly Leu Val Gln Ala Gly Gly
   1 5 10 15
   Ser Leu Arg Leu Ser Cys Ala Ala Ser Gly Phe Thr Phe Asp Asp Tyr
   20 25 30
   Ala Ile Gly Trp Phe Arg Arg Ala Pro Gly Lys Glu Arg Glu Gly Val
   35 40 45
   Ser Cys Ile Thr Ser Ser Asp Gly Ser Thr Ser Tyr Ala Asp Ser Val
   50 55 60
   Lys Gly Arg Phe Thr Ile Ser Ser Asp Ser Ala Lys Asn Thr Val Tyr
   65 70 75 80
   Leu Gln Met Asn Ser Leu Lys Pro Glu Asp Thr Ala Val Tyr Tyr Cys
   85 90 95
   Ser Val Val Pro Ala Thr Arg Arg Asn Pro Gln Met Val Val Ala Lys
   100 105 110
   Lys Cys Trp Leu Ala Glu Tyr Glu Tyr Asp Tyr Trp Gly Gln Gly Thr
   115 120 125
   Gln Val Thr Val Ser Ser
   130
<210> 63
   <211> 130
   <212> PRT
   <213> Lama glama
<400> 63
   Glu Val Gln Leu Val Glu Ser Gly Gly Gly Leu Val Gln Ala Gly Gly
   1 5 10 15
   Ser Leu Arg Leu Ser Cys Ala Val Ser Gly Phe Thr Phe Asp Asp Tyr
   20 25 30
   Ala Ile Gly Trp Phe Arg Gln Ala Pro Gly Lys Glu Arg Glu Gly Val
   35 40 45
   Ser Cys Ile Ser Arg Ser Asp Gly Ser Pro Tyr Tyr Ala Asp Ser Val
   50 55 60
   Lys Gly Arg Phe Thr Ile Ser Ser Asp Asn Ala Lys Gly Thr Val Tyr
   65 70 75 80
   Leu Gln Met Ser Ser Leu Lys Pro Glu Asp Thr Ala Val Tyr Tyr Cys
   85 90 95
   Ala Ala Ser Trp Ser Gly Ala Tyr Tyr Ser Gly Thr Tyr Tyr Cys Asp
   100 105 110
   Arg Leu Tyr Glu Tyr Asp Ala Trp Gly Gln Gly Thr Gln Val Thr Val
   115 120 125
   Ser Ser
   130
<210> 64
   <211> 123
   <212> PRT
   <213> Lama glama
<400> 64
   Glu Val Gln Leu Val Glu Ser Gly Gly Gly Leu Val Gln Val Gly Asp
   1 5 10 15
   Ser Leu Arg Leu Ser Cys Ala Ala Ser Glu Arg Thr Phe Ser Thr Tyr
   20 25 30
   Leu Met Val Gly Trp Phe Arg Gln Ala Pro Gly Lys Glu Arg Glu Phe
   35 40 45
   Ala Ala Thr Met Trp Ser Ser Gly Asp Thr Ala Tyr Ala Asp Ser Val
   50 55 60
   Lys Gly Arg Phe Thr Ile Ser Arg Asp Asn Ala Lys Asn Thr Val Tyr
   65 70 75 80
   Leu Gln Met Asn Ser Leu Lys Ala Glu Asp Thr Ala Val Tyr Tyr Cys
   85 90 95
   Ala Gly Ser Tyr Gly Gly Asn Tyr Tyr Ile Pro Gly Phe Tyr Glu Asp
   100 105 110
   Trp Gly Gln Gly Thr Gln Val Thr Val Ser Ser
   115 120
<210> 65
   <211> 127
   <212> PRT
   <213> Lama glama
<400> 65
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Ala Leu Asp Tyr Tyr
               20                  25                  30
   Ala Ile Gly Trp Phe Arg Gln Ala Pro Gly Lys Glu Arg Glu Gly Val
           35                  40                  45
   Ser Cys Ile Ser Ser Ser Asp Gly Ser Thr Tyr Tyr Ala Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Thr Asp Ser Leu Gly Tyr Gly Ser Ser Cys Arg Met Ala Pro Tyr
               100                 105                 110
   Glu Tyr Asp Tyr Trp Gly Gln Gly Thr Gln Val Thr Val Ser Ser
           115                 120                 125
<210> 66
   <211> 123
   <212> PRT
   <213> Lama glama
<400> 66
   Glu Val Gln Leu Val Glu Ser Gly Gly Gly Leu Val Gln Val Gly Asp
   1               5                   10                  15
   Ser Leu Arg Leu Ser Cys Ala Ala Ser Gly Arg Thr Phe Ser Thr Tyr
               20                  25                  30
   Leu Met Val Gly Trp Phe Arg Gln Ala Pro Gly Lys Glu Arg Glu Phe
           35                  40                  45
   Ala Ala Val Ile Trp Ser Ser Gly Gly Thr Ala Tyr Ala Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Ala Glu Asp Thr Ala Val Tyr Tyr Cys
                   85                  90                  95
   Ala Gly Ser Tyr Asp Gly Asn Tyr Tyr Ile Pro Gly Phe Tyr Lys Asp
               100                 105                 110
   Trp Gly Gln Gly Thr Gln Val Thr Val Ser Ser
   115 120
<210> 67
   <211> 130
   <212> PRT
   <213> Lama glama
<400> 67
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Ser Asp Asp Tyr
   20 25 30
   Ala Ile Gly Trp Phe Arg Gln Ala Pro Gly Lys Glu Arg Glu Gly Val
   35 40 45
   Ser Cys Ile Ser Ser Ser Asp Gly Ile Thr Tyr Tyr Ala Asp Ser Val
   50 55 60
   Lys Gly Arg Phe Thr Ile Ser Arg Asp Asn Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Thr Gly Asn Arg Arg Ile Tyr Tyr Ser Asp Tyr Ala Leu Ala Cys
   100 105 110
   Phe Pro Tyr Glu Tyr Asp Tyr Trp Gly Gln Gly Thr Gln Val Thr Val
   115 120 125
   Ser Ser
   130
<210> 68
   <211> 123
   <212> PRT
   <213> Lama glama
<400> 68
   Glu Val Gln Leu Val Glu Ser Gly Arg Arg Leu Val Gln Val Gly Asp
   1               5                   10                  15
   Ser Leu Arg Leu Ala Cys Ala Ala Ser Gly Arg Thr Phe Ser Thr Tyr
               20                  25                  30
   Leu Met Val Gly Trp Phe Arg Gln Ala Pro Gly Lys Glu Arg Glu Phe
           35                  40                  45
   Ala Ala Gly Ile Trp Ser Ser Gly Asp Thr Ala Tyr Ala Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Thr Glu Asp Thr Ala Val Tyr Tyr Cys
                   85                  90                  95
   Ala Gly Ser Tyr Asp Gly Asn Tyr Tyr Ile Pro Gly Phe Tyr Lys Asp
               100                 105                 110
   Trp Gly Gln Gly Thr Gln Val Thr Val Ser Ser
           115                 120
<210> 69
   <211> 123
   <212> PRT
   <213> Lama glama
<400> 69
   Glu Val Gln Leu Val Glu Ser Gly Gly Gly Leu Val Gln Val Gly Asp
   1 5 10 15
   Ser Leu Arg Leu Ser Cys Ala Ala Ser Glu Arg Thr Phe Ser Thr Tyr
   20 25 30
   Leu Met Val Gly Trp Phe Arg Gln Ala Pro Gly Lys Glu Arg Glu Phe
   35 40 45
   Ala Ala Thr Met Trp Val Ser Gly Asp Thr Ala Tyr Ala Asp Ser Val
   50 55 60
   Lys Gly Arg Phe Thr Ile Ser Arg Asp Asn Pro Lys Asn Thr Val Tyr
   65 70 75 80
   Leu Gln Met Asn Ser Leu Lys Ala Glu Asp Thr Ala Val Tyr Tyr Cys
   85 90 95
   Ala Gly Ser Tyr Gly Gly Asn Tyr Tyr Ile Pro Gly Phe Tyr Lys Asp
   100 105 110
   Trp Gly Gln Gly Thr Gln Val Thr Val Ser Ser
   115 120
<210> 70
   <211> 123
   <212> PRT
   <213> Lama glama
<400> 70
   Glu Val Gln Leu Val Glu Ser Gly Gly Gly Leu Val Gln Ile Gly Asp
   1 5 10 15
   Ser Leu Arg Leu Ser Cys Ala Ala Ser Gly Arg Thr Phe Ser Thr Tyr
   20 25 30
   Leu Met Val Gly Trp Phe Arg Gln Ala Pro Gly Lys Glu Arg Glu Phe
   35 40 45
   Ala Ala Ala Ile Trp Ser Ser Gly Asp Thr Ala Val Ala Asp Ser Val
   50 55 60
   Lys Gly Arg Phe Thr Ile Ser Arg Asp Asn Ala Lys Asn Thr Val Tyr
   65 70 75 80
   Leu Gln Met Asn Ser Leu Lys Ala Glu Asp Thr Ala Val Tyr Tyr Cys
   85 90 95
   Ala Gly Ser Tyr Gly Gly Asn Tyr Tyr Ile Pro Gly Phe Tyr Glu Asp
   100 105 110
   Trp Gly Gln Gly Thr Gln Val Thr Val Ser Ser
   115 120
<210> 71
   <211> 122
   <212> PRT
   <213> Lama glama
<400> 71
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Leu Asp Tyr Tyr
   20 25 30
   Ala Ile Gly Trp Phe Arg Gln Ala Pro Gly Lys Glu Trp Glu Gly Val
   35 40 45
   Ser Cys Ile Ser Ser Ser Asp Gly Ser Thr Tyr Tyr Ala Asp Ser Val
   50 55 60
   Lys Gly Arg Phe Thr Ile Ser Arg Asp Asn Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Thr Asp Ser Ile Val Cys Gly Ser Tyr Tyr Gly Met Asp Tyr Trp
   100 105 110
   Gly Lys Gly Thr Gln Val Thr Val Ser Ser
   115 120
<210> 72
   <211> 123
   <212> PRT
   <213> Lama glama
<400> 72
   Glu Val Gln Leu Val Glu Ser Gly Gly Ala Ser Val Gln Pro Gly Gly
   1 5 10 15
   Ser Leu Arg Leu Ser Cys Ala Ala Ser Gly Arg Thr Phe Ser Thr Tyr
   20 25 30
   Leu Met Val Gly Trp Phe Arg Gln Ala Pro Gly Lys Glu Arg Glu Phe
   35 40 45
   Ala Ala Gly Ile Trp Ser Ser Gly Gly Thr Ala Tyr Ala Asp Ser Val
   50 55 60
   Lys Glu Arg Phe Thr Val Ser Arg Asp Asn Asp Lys Asn Thr Val Tyr
   65 70 75 80
   Leu Gln Met Asn Ser Leu Gln Ala Glu Asp Thr Ala Val Tyr Tyr Cys
   85 90 95
   Ala Gly Ser Tyr Asp Gly Asn Tyr Tyr Ile Pro Gly Phe Tyr Lys Asp
   100 105 110
   Trp Gly Gln Gly Thr Gln Val Thr Val Ser Ser
   115 120
<210> 73
   <211> 128
   <212> PRT
   <213> Lama glama
<400> 73
   Glu Val Gln Leu Val Glu Ser Gly Gly Gly Leu Val Gln Ala Gly Gly
   1 5 10 15
   Ser Leu Arg Leu Ser Cys Ala Ala Ser Gly Phe Thr Phe Asp Asp Tyr
   20 25 30
   Ala Ile Gly Trp Phe Arg Gln Ala Pro Gly Lys Glu Arg Glu Gly Val
   35 40 45
   Ser Cys Ile Ser Ser Ser Asp Gly Ser Thr Tyr Tyr Ala Asp Ser Val
   50 55 60
   Lys Gly Arg Phe Thr Ile Ser Ser Asp Asn Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Ala Ala Trp Gly Ala Ser Arg Leu Pro Ile Gly Thr Met Pro Pro
   100 105 110
   Tyr Glu Tyr Asp Tyr Trp Gly Gln Gly Thr Gln Val Thr Val Ser Ser
   115 120 125
<210> 74
   <211> 118
   <212> PRT
   <213> Lama glama
<400> 74
   Glu Val Gln Leu Val Glu Ser Gly Gly Gly Leu Val Gln Ala Gly Gly
   1 5 10 15
   Ser Leu Arg Leu Val Cys Ser Ala Ser Gly Ile Asp Phe Ser Ile Asn
   20 25 30
   Val Met Gly Trp Tyr Arg Gln Ala Pro Gly Lys Gln Arg Glu Trp Val
   35 40 45
   Ala Phe Ile Gly Ser Gly Gly Ser Thr Asp Tyr Ile Asp Tyr Thr Asp
   50 55 60
   Ser Val Lys Gly Arg Phe Thr Ile Ser Arg Asp Ser Thr Lys Asn Ile
   65 70 75 80
   Leu Tyr Leu Arg Met Asn Asn Leu Asn Val Glu Asp Thr Ala Val Tyr
   85 90 95
   Tyr Cys Asn Thr Ala Tyr Glu Gln His Thr Tyr Trp Gly Gln Gly Thr
   100 105 110
   Gln Val Thr Val Ser Ser
   115
<210> 75
   <211> 125
   <212> PRT
   <213> Lama glama
<400> 75
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Phe Thr Phe Asp Asp Tyr
               20                  25                  30
   Ala Ile Gly Trp Phe Arg Gln Ala Pro Gly Lys Asp Leu Glu Gly Val
           35                  40                  45
   Ser Cys Ile Ser Ser Ser Asp Gly Ser Thr Tyr Tyr Ala Asp Ser Val
       50                  55                  60
   Lys Gly Arg Phe Thr Ile Ser Ser Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Val Asn Gly Leu Gly Pro Phe Ser Val Pro Val Pro Val Tyr
               100                 105                 110
   Asp Phe Trp Gly Gln Gly Thr Gln Val Thr Val Ser Ser
           115                 120                 125
<210> 76
   <211> 130
   <212> PRT
   <213> Lama glama
<400> 76
   Glu Val Gln Leu Val Glu Ser Gly Gly Gly Leu Val Gln Ala Gly Gly
   1               5                   10                  15
   Ser Leu Arg Leu Ser Cys Ala Val Ser Gly Leu Pro Phe Asp Asp Tyr
               20                  25                  30
   Val Ile Gly Trp Phe Arg Gln Ala Pro Gly Lys Glu Arg Glu Gly Val
           35                  40                  45
   Ser Cys Ile Ser Ser Ser Asp Gly Ser Thr Tyr Tyr Ala Asp Ser Val
       50                  55                  60
   Lys Gly Arg Phe Thr Ile Ser Ser Asp Asn Ala Lys Asn Thr Val Tyr
   65                  70                  75                  80
   Leu Gln Met Asn Ser Leu Lys Pro Glu Asp Thr Ala Ala Tyr Tyr Cys
                   85                  90                  95
   Ala Ala Gly Gly Pro Arg Ile Asn Ile Ala Thr Met Thr Cys Ser His
               100                 105                 110
   Asp Glu Tyr Glu Tyr Asp Tyr Trp Gly Gln Gly Thr Gln Val Thr Val
           115                 120                 125
   Ser Ser
       130
<210> 77
   <211> 133
   <212> PRT
   <213> Lama glama
<400> 77
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Ala Leu Asp Tyr Tyr
               20                  25                  30
   Ala Ile Gly Trp Phe Arg Gln Ala Pro Gly Lys Glu Arg Glu Gly Val
           35                  40                  45
   Ser Cys Ile Glu Ser Ser Asp Gly Ser Thr Tyr Tyr Ala Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Thr Asp Ser Ala Gly Val Pro Ala Gly Pro Ala Ala Val Tyr Gly
               100                 105                 110
   Ser Ser Cys Ser Arg Leu Glu Tyr Asp Tyr Trp Gly Gln Gly Thr Gln
           115                 120                 125
   Val Thr Val Ser Ser
   130
<210> 78
   <211> 124
   <212> PRT
   <213> Lama glama
<400> 78
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Gly Ser Tyr
   20 25 30
   Asp Met Ser Trp Val Arg Gln Ala Pro Gly Lys Gly Pro Glu Trp Val
   35 40 45
   Ser Ala Ile Asn Ser Arg Gly Gly Ser Thr Tyr Tyr Ala Asp Ser Val
   50 55 60
   Lys Gly Arg Phe Thr Ile Ser Arg Asp Asn Ala Lys Asn Thr Leu Tyr
   65 70 75 80
   Leu Gln Met Asn Ser Leu Lys Pro Glu Asp Thr Ala Val Tyr Tyr Cys
   85 90 95
   Ala Thr Asp Pro Tyr Ser Leu Ser Tyr Tyr Gly Tyr Pro Leu Tyr Asp
   100 105 110
   Tyr Trp Gly Gln Gly Thr Gln Val Thr Val Ser Ser
   115 120
<210> 79
   <211> 115
   <212> PRT
   <213> Lama glama
<400> 79
   Glu Val Gln Leu Val Glu Ser Gly Gly Gly Leu Val Gln Ala Gly Gly
   1               5                   10                  15
   Ser Leu Arg Leu Val Cys Ser Ala Ser Gly Ile Asp Phe Ser Ile Asn
               20                  25                  30
   Val Met Gly Trp Tyr Arg Gln Ala Pro Gly Lys Gln Arg Glu Trp Val
           35                  40                  45
   Ala Phe Ile Gly Ser Gly Ser Ser Thr Gly Tyr Thr Asp Ser Val Lys
       50                  55                  60
   Gly Arg Phe Ser Ile Ser Arg Asp Ser Thr Lys Asn Ile Leu Tyr Leu
   65                  70                  75                  80
   Gln Met Asn Asn Leu Asn Val Glu Asp Thr Ala Val Tyr Tyr Cys Asn
                   85                  90                  95
   Thr Ala Tyr Glu Gln His Thr Tyr Trp Gly Gln Gly Thr Gln Val Thr
               100                 105                 110
   Val Ser Ser
           115
<210> 80
   <211> 129
   <212> PRT
   <213> Lama glama
<400> 80
   Glu Val Gln Leu Val Glu Ser Gly Gly Gly Leu Val Gln Ala Gly Gly
   1 5 10 15
   Ser Leu Arg Leu Ser Cys Ala Ala Ser Gly Phe Thr Leu Asp Asp Tyr
   20 25 30
   Ala Ile Gly Trp Phe Arg Gln Ala Pro Gly Lys Glu Arg Glu Gly Val
   35 40 45
   Ser Cys Ile Arg Asp Ser Asp Gly Ser Thr Tyr Tyr Ala Asp Ser Val
   50 55 60
   Lys Gly Arg Phe Thr Ile Ser Ser Asp Asn Asp Lys Asn Thr Val Tyr
   65 70 75 80
   Leu Gln Met Asn Ser Leu Lys Pro Glu Asp Thr Ala Val Tyr Tyr Cys
   85 90 95
   Ala Ala Val Pro Ala Gly Arg Leu Arg Phe Gly Glu Gln Trp Tyr Pro
   100 105 110
   Leu Tyr Glu Tyr Asp Ala Trp Gly Gln Gly Thr Gln Val Thr Val Ser
   115 120 125
   Ser
<210> 81
   <211> 124
   <212> PRT
   <213> Lama glama
<400> 81
   Glu Val Gln Leu Val Glu Ser Gly Gly Gly Val Val Gln Ala Gly Asp
   1 5 10 15
   Ser Val Arg Leu Ser Cys Ala Ala Ser Gly Pro Thr Phe Arg Ser Tyr
   20 25 30
   Thr Met Ala Trp Phe Arg Gln Thr Pro Gly Lys Glu Arg Asp Ile Val
   35 40 45
   Ala Ala Ile Ser Ser Ser Leu Gly Arg Thr Tyr Tyr Ala Asp Ser Val
   50 55 60
   Lys Gly Arg Phe Gln Ile Leu Arg Asp Asn Ala Lys Glu Thr Val Trp
   65 70 75 80
   Leu Gln Met Asn Ser Leu Lys Pro Glu Asp Thr Ala Val Tyr Ile Cys
   85 90 95
   Ala Ala Ser Arg Ser Leu Asn Leu Ala Tyr Thr Thr Lys Pro Tyr Asp
   100 105 110
   Tyr Trp Gly Gln Gly Thr Gln Val Thr Val Ser Ser
   115 120
<210> 82
   <211> 133
   <212> PRT
   <213> Lama glama
<400> 82
   Glu Val Gln Leu Val Glu Ser Gly Gly Gly Leu Val Gln Ala Gly Gly
   1 5 10 15
   Ser Leu Arg Leu Ser Cys Ala Ala Ser Gly Phe Asp Phe Glu Asp Tyr
   20 25 30
   Asp Met Gly Trp Phe Arg Gln Ala Pro Gly Lys Glu Arg Glu Gly Val
   35 40 45
   Ser Tyr Ile Ser Ser Ser Asp Gly Ser Thr Tyr Tyr Thr Asp Ser Val
   50 55 60
   Lys Gly Arg Phe Thr Ile Ser Ser Asp Asn Ala Lys Asn Thr Val Tyr
   65 70 75 80
   Leu Gln Met Asn Ser Leu Ile Pro Glu Asp Thr Ala Val Tyr Tyr Cys
                   85                  90                  95
   Ala Ala Arg Pro Trp Thr Arg Arg Val Tyr Gly Ser Ser Trp Leu Ala
               100                 105                 110
   Arg Ser Leu Asp Glu Tyr Glu Tyr Asp Tyr Trp Gly Gln Gly Thr Gln
           115                 120                 125
   Val Thr Val Ser Ser
       130
<210> 83
   <211> 123
   <212> PRT
   <213> Lama glama
<400> 83
   Glu Val Gln Leu Val Glu Ser Gly Gly Gly Leu Val Gln Val Gly Asp
   1               5                   10                  15
   Ser Leu Arg Leu Ser Cys Ala Ala Ser Gly His Thr Phe Ser Thr Tyr
               20                  25                  30
   Leu Met Val Gly Trp Phe Arg Gln Thr Pro Gly Lys Glu Arg Glu Phe
           35                  40                  45
   Ala Ala Val Ile Trp Ser Ser Gly Asp Thr Ala Tyr Ala Asp Ser Ala
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Ala Glu Asp Thr Ala Val Tyr Tyr Cys
                   85                  90                  95
   Ala Gly Ser Tyr Gly Gly Asn Tyr Tyr Ile Pro Gly Phe Tyr Glu Asp
               100                 105                 110
   Trp Gly Gln Gly Thr Gln Val Thr Val Ser Ser
           115                 120
<210> 84
   <211> 126
   <212> PRT
   <213> Lama glama
<400> 84
   Glu Val Gln Leu Met Glu Ser Gly Gly Gly Leu Val Gln Pro Gly Gly
   1 5 10 15
   Ser Leu Arg Leu Ser Cys Ala Ala Ser Gly Phe Ala Leu Asp Tyr Tyr
   20 25 30
   Ala Ile Gly Trp Phe Arg Gln Val Pro Gly Lys Glu Arg Glu Gly Val
   35 40 45
   Ser Cys Ile Ser Ser Ser Asp Gly Ile Thr Tyr Tyr Val Asp Ser Val
   50 55 60
   Lys Gly Arg Phe Thr Ile Ser Arg Asp Asn Ala Lys Asn Thr Val Tyr
   65 70 75 80
   Leu Gln Met Asn Ser Leu Lys Pro Gly Asp Thr Ala Val Tyr Tyr Cys
   85 90 95
   Ala Thr Asp Ser Gly Gly Tyr Ile Asp Tyr Asp Cys Met Gly Leu Gly
   100 105 110
   Tyr Asp Tyr Trp Gly Gln Gly Thr Gln Val Thr Val Ser Ser
   115 120 125
<210> 85
   <211> 126
   <212> PRT
   <213> Lama glama
<400> 85
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Ala Leu Asp Tyr Tyr
   20 25 30
   Ala Ile Gly Trp Phe Arg Gln Ala Pro Gly Lys Glu Arg Glu Gly Val
   35 40 45
   Ser Cys Ile Ser Ser Ser Asp Gly Ile Thr Tyr Tyr Val Asp Ser Val
   50 55 60
   Lys Gly Arg Phe Thr Ile Ser Arg Asp Asn Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Thr Asp Ser Gly Gly Tyr Ile Asp Tyr Asp Cys Met Gly Leu Gly
               100                 105                 110
   Tyr Asp Tyr Trp Gly Gln Gly Thr Gln Val Thr Val Ser Ser
           115                 120                 125
<210> 86
   <211> 123
   <212> PRT
   <213> Lama glama
<400> 86
   Glu Val Gln Leu Val Glu Ser Gly Gly Gly Leu Val Gln Val Gly Asp
   1               5                   10                  15
   Ser Leu Arg Leu Ser Cys Ala Ala Ser Gly Arg Thr Phe Ser Thr Tyr
               20                  25                  30
   Leu Met Val Gly Trp Phe Arg Gln Ala Pro Gly Lys Glu Arg Glu Phe
           35                  40                  45
   Ala Ala Val Ile Trp Ser Ser Gly Asp Thr Ala Tyr Ala Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Ala Glu Asp Thr Ala Val Tyr Tyr Cys
                   85                  90                  95
   Ala Gly Ser Tyr Asp Gly Asn Tyr Tyr Ile Pro Gly Phe Tyr Lys Asp
               100                 105                 110
   Trp Gly Gln Gly Thr Gln Val Thr Val Ser Ser
           115                 120
<210> 87
   <211> 129
   <212> PRT
   <213> Artificial
<220>
   <223> Mutated Lama sequence
<400> 87
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Phe Thr Leu Asp Asp Tyr
               20                  25                  30
   Ala Ile Gly Trp Phe Arg Gln Ala Pro Gly Lys Glu Arg Glu Gly Val
           35                  40                  45
   Ser Ser Ile Arg Asp Asn Asp Gly Ser Thr Tyr Tyr Ala Asp Ser Val
       50                  55                  60
   Lys Gly Arg Ser Thr Ile Ser Ser Asp Asn Asp Lys Asn Thr Val Tyr
   65                  70                  75                  80
   Leu Gln Met Asp Ser Leu Lys Pro Glu Asp Thr Ala Val Tyr Tyr Cys
                   85                  90                  95
   Ala Ala Val Pro Ala Gly Arg Leu Arg Tyr Gly Glu Gln Trp Tyr Pro
               100                 105                 110
   Ile Tyr Glu Tyr Asp Ala Trp Gly Gln Gly Thr Leu Val Thr Val Ser
           115                 120                 125
   Ser
<210> 88
   <211> 129
   <212> PRT
   <213> Artificial
<220>
   <223> Mutated Lama sequence
<400> 88
   Glu Val Gln Leu Val Glu Ser Gly Gly Gly Leu Val Gln Ala Gly Gly
   1               5                   10                  15
   Ser Leu Arg Leu Ser Cys Ala Val Ser Gly Ile Thr Leu Asp Asp Tyr
               20                  25                  30
   Ala Val Gly Trp Phe Arg Gln Ala Pro Gly Lys Glu Arg Glu Gly Val
           35                  40                  45
   Ser Thr Ile Arg Asp Asn Asp Gly Ser Thr Tyr Tyr Ala Asp Ser Val
       50                  55                  60
   Lys Gly Arg Phe Thr Ile Ser Ser Asp Asn Asp Lys Asn Thr Val Tyr
   65                  70                  75                  80
   Leu Gln Met Asn Ser Leu Lys Pro Lys Asp Thr Ala Val Tyr Tyr Cys
                   85                  90                  95
   Ala Ala Val Pro Ala Gly Arg Leu Arg Phe Gly Glu Gln Trp Tyr Pro
               100                 105                 110
   Ile Tyr Glu Tyr Asp Ala Trp Gly Gln Gly Thr Leu Val Thr Val Ser
           115                 120                 125
   Ser
<210> 89
   <211> 129
   <212> PRT
   <213> Artificial
<220>
   <223> Mutated Lama sequence
<400> 89
   Glu Val Gln Leu Val Glu Ser Gly Gly Gly Gln Ala Gln Ala Gly Gly
   1               5                   10                  15
   Ser Leu Arg Leu Ser Cys Ala Val Ser Gly Phe Thr Leu Asp Asp Tyr
               20                  25                  30
   Ala Ile Gly Trp Phe Arg Gln Ala Pro Gly Lys Glu Arg Glu Gly Val
           35                  40                  45
   Ser Ser Ile Arg Asp Asn Asp Gly Ser Thr Tyr Tyr Thr Asp Ser Val
       50                  55                  60
   Lys Gly Arg Phe Thr Ile Ser Ser Asp Asn Asp Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Val Pro Ala Gly Arg Leu Arg Phe Gly Glu Gln Trp Tyr Pro
               100                 105                 110
   Ile Tyr Glu Tyr Asp Ala Trp Gly Gln Gly Thr Leu Val Thr Val Ser
           115                 120                 125
   Ser
<210> 90
   <211> 129
   <212> PRT
   <213> Artificial
<220>
   <223> Mutated Lama sequence
<400> 90
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Ile Thr Leu Asp Asp Tyr
               20                  25                  30
   Ala Ile Gly Trp Phe Arg Gln Ala Pro Gly Lys Glu Arg Val Gly Val
           35                  40                  45
   Ser Ser Ile Arg Asp Asn Asp Gly Ser Thr Tyr Tyr Ala Asp Ser Val
       50                  55                  60
   Lys Gly Arg Phe Thr Ile Ser Ser Asp Asn Asp Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Val Pro Ala Gly Arg Leu Arg Phe Gly Glu Gln Trp Tyr Pro
               100                 105                 110
   Ile Tyr Glu Tyr Asp Ala Trp Gly Gln Gly Thr Leu Val Thr Val Ser
           115                 120                 125
   Ser
<210> 91
   <211> 129
   <212> PRT
   <213> Artificial
<220>
   <223> Mutated Lama sequence
<400> 91
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Leu Thr Leu Asp Asp Tyr
               20                  25                  30
   Ala Ile Gly Trp Phe Arg Gln Ala Pro Gly Lys Glu Arg Glu Gly Val
           35                  40                  45
   Ser Ser Ile Arg Asp Asn Asp Gly Ser Thr Tyr Tyr Ala Asp Ser Val
       50                  55                  60
   Lys Gly Arg Phe Thr Ile Ser Ser Asp Asn Asp Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Val Pro Ala Gly Arg Leu Arg Phe Gly Glu Gln Trp Tyr Pro
               100                 105                 110
   Ile Tyr Glu Tyr Asp Ala Trp Gly Gln Gly Thr Leu Val Thr Val Ser
           115                 120                 125
   Ser
<210> 92
   <211> 129
   <212> PRT
   <213> Artificial
<220>
   <223> Mutated Lama sequence
<400> 92
   Gln Val Gln Leu Val Glu Ser Gly Gly Gly Leu Val Gln Ala Gly Gly
   1               5                   10                  15
   Ser Leu Arg Leu Ser Cys Ala Val Ser Gly Phe Thr Leu Asp Asp Tyr
               20                  25                  30
   Ala Ile Gly Trp Phe Arg Gln Ala Pro Gly Lys Glu Arg Glu Gly Val
           35                  40                  45
   Ser Ser Ile Arg Asp Asn Asp Gly Ser Thr Tyr Tyr Ala Asp Ser Val
       50                  55                  60
   Lys Gly Arg Phe Thr Ile Ser Ser Asp Asn Asp Gln Asn Thr Val Tyr
   65                  70                  75                  80
   Leu Gln Met Asn Ser Leu Lys Ser Glu Asp Thr Ala Val Tyr Tyr Cys
                   85                  90                  95
   Ala Ala Val Pro Ala Gly Arg Leu Arg Phe Gly Glu Gln Trp Tyr Pro
               100                 105                 110
   Ile Tyr Glu Tyr Asp Ala Trp Gly Gln Gly Thr Leu Val Thr Val Ser
   115 120 125
   Ser
<210> 93
   <211> 129
   <212> PRT
   <213> Artificial
<220>
   <223> Mutated Lama sequence
<400> 93
   Glu Val Gln Leu Val Glu Ser Gly Gly Gly Leu Val Gln Ala Gly Gly
   1 5 10 15
   Ser Leu Arg Leu Ser Cys Ala Ile Ser Gly Phe Thr Leu Val Asp Tyr
   20 25 30
   Ala Ile Gly Trp Phe Arg Gln Ala Pro Gly Lys Glu Arg Glu Gly Val
   35 40 45
   Ser Ser Ile Arg Asp Asn Asp Gly Ser Thr Tyr Tyr Ala Asp Ser Val
   50 55 60
   Lys Gly Arg Phe Ile Ile Ser Ser Asp Asn Asp Lys Asn Thr Val Tyr
   65 70 75 80
   Leu Gln Met Asn Ser Leu Lys Pro Glu Asp Thr Ala Val Tyr Tyr Cys
   85 90 95
   Ala Ala Val Pro Ala Gly Arg Leu Arg Phe Gly Glu Gln Trp Tyr Pro
   100 105 110
   Ile Tyr Glu Tyr Asp Ala Trp Gly Gln Gly Thr Leu Val Thr Val Ser
   115 120 125
   Ser
<210> 94
   <211> 129
   <212> PRT
   <213> Artificial
<220>
   <223> Mutated Lama sequence
<400> 94
   Glu Val Gln Leu Met Glu Ser Gly Gly Gly Leu Val Gln Ala Gly Gly
   1 5 10 15
   Ser Leu Arg Leu Ser Cys Ala Ala Ser Gly Ile Thr Leu Asp Asp Tyr
   20 25 30
   Ala Ile Gly Trp Phe Arg Gln Ala Pro Gly Lys Glu Arg Glu Gly Val
   35 40 45
   Ser Ser Ile Arg Asp Asn Asp Gly Ser Thr Tyr Tyr Ala Asp Ser Val
   50 55 60
   Lys Gly Arg Phe Thr Ile Ser Ser Asp Asn Asp Lys Asn Thr Val Asn
   65 70 75 80
   Leu Gln Met Asn Ser Leu Lys Pro Glu Asp Thr Ala Val Tyr Tyr Cys
   85 90 95
   Ala Ala Val Pro Ala Gly Arg Leu Arg Phe Gly Glu Gln Trp Tyr Pro
   100 105 110
   Ile Tyr Glu Tyr Asp Ala Trp Gly Gln Gly Thr Leu Val Thr Val Ser
   115 120 125
   Ser
<210> 95
   <211> 129
   <212> PRT
   <213> Artificial
<220>
   <223> Mutated Lama sequence
<400> 95
   Glu Val Gln Leu Val Glu Ser Gly Gly Gly Leu Val Gln Ala Gly Gly
   1 5 10 15
   Ser Leu Arg Leu Ser Cys Ala Ala Ser Gly Phe Thr Leu Asp Asp Tyr
   20 25 30
   Ala Ile Gly Trp Phe Arg Gln Ala Pro Gly Lys Glu Arg Glu Gly Val
   35 40 45
   Ser Ser Ile Arg Asp Asn Asp Gly Ser Thr Tyr Tyr Ala Asp Ser Val
   50 55 60
   Lys Gly Arg Phe Thr Ile Ser Ser Asp Asn Asp Lys Asn Thr Val Tyr
   65 70 75 80
   Leu Gln Met Asn Ser Leu Lys Pro Glu Asp Thr Ala Val Tyr Tyr Cys
   85 90 95
   Ala Ala Val Pro Ala Gly Arg Leu Arg Tyr Gly Glu Gln Trp Tyr Pro
   100 105 110
   Ile Tyr Glu Tyr Asp Ala Trp Gly Gln Gly Thr Leu Val Thr Val Ser
   115 120 125
   Ser
<210> 96
   <211> 129
   <212> PRT
   <213> Artificial
<220>
   <223> Mutated Lama sequence
<400> 96
   Glu Val Gln Leu Val Glu Ser Gly Gly Gly Leu Val Gln Ala Gly Gly
   1 5 10 15
   Ser Leu Arg Leu Ser Cys Ala Val Ser Gly Phe Thr Leu Asp Asp Tyr
   20 25 30
   Ala Ile Gly Trp Phe Arg Gln Ala Pro Gly Lys Glu Arg Glu Gly Val
   35 40 45
   Ser Ser Ile Arg Glu Asn Asp Gly Ser Thr Tyr Tyr Ala Asp Ser Val
   50 55 60
   Gln Gly Arg Phe Thr Ile Ser Ser Asp Asn Asp Lys Asn Thr Val Tyr
   65 70 75 80
   Leu Arg Met Asn Ser Leu Lys Pro Glu Asp Thr Ala Val Tyr Tyr Cys
   85 90 95
   Ala Ala Val Pro Ala Gly Arg Leu Arg Phe Gly Glu Gln Trp Tyr Pro
   100 105 110
   Ile Tyr Glu Tyr Asp Ala Trp Gly Gln Gly Thr Leu Val Thr Val Ser
   115 120 125
   Ser
<210> 97
   <211> 129
   <212> PRT
   <213> Artificial
<220>
   <223> Mutated Lama sequence
<400> 97
   Glu Val Gln Met Val Glu Ser Gly Gly Gly Leu Val Gln Ala Gly Gly
   1               5                   10                  15
   Ser Leu Arg Leu Ser Cys Ala Ala Ser Gly Leu Thr Leu Asp Asp Tyr
               20                  25                  30
   Ala Ile Gly Trp Phe Arg Gln Ala Pro Gly Lys Glu Arg Glu Gly Val
           35                  40                  45
   Ser Ser Ile Arg Asp Asn Asp Gly Ser Thr Tyr Tyr Ala Asp Ser Val
       50                  55                  60
   Lys Gly Arg Phe Thr Ile Ser Ser Asp Asn Asp Lys Asn Thr Val Tyr
   65                  70                  75                  80
   Leu Gln Met Asn Gly Leu Lys Pro Glu Asp Thr Ala Val Tyr Tyr Cys
                   85                  90                  95
   Ala Ala Val Pro Ala Gly Arg Leu Arg Phe Gly Glu Gln Trp Tyr Pro
               100                 105                 110
   Ile Tyr Glu Tyr Asp Ala Trp Gly Gln Gly Thr Leu Val Thr Val Ser
           115                 120                 125
   Ser
<210> 98
   <211> 129
   <212> PRT
   <213> Artificial
<220>
   <223> Mutated Lama sequence
<400> 98
   Glu Val Gln Leu Val Glu Ser Gly Gly Gly Leu Val Gln Ser Gly Gly
   1               5                   10                  15
   Ser Leu Arg Leu Ser Cys Ala Ala Ser Gly Leu Thr Leu Asp Asp Tyr
   20 25 30
   Ala Ile Gly Trp Phe Arg Gln Ala Pro Gly Lys Glu Arg Glu Gly Val
   35 40 45
   Ser Ser Ile Arg Asp Asn Asp Gly Ser Thr Tyr Tyr Ala Asp Ser Val
   50 55 60
   Lys Gly Arg Phe Thr Ile Ser Ser Asp Asn Asp Lys Asn Thr Val Tyr
   65 70 75 80
   Leu Gln Met Asn Ser Leu Lys Pro Glu Asp Thr Ala Val Tyr Tyr Cys
   85 90 95
   Ala Ala Val Pro Ala Gly Arg Leu Arg Phe Gly Glu Gln Trp Tyr Pro
   100 105 110
   Ile Tyr Glu Tyr Asp Ala Trp Gly Gln Gly Thr Leu Val Thr Val Ser
   115 120 125
   Ser
<210> 99
   <211> 129
   <212> PRT
   <213> Artificial
<220>
   <223> Mutated Lama sequence
<400> 99
   Glu Val Gln Leu Val Glu Ser Gly Gly Gly Leu Val Gln Ala Gly Gly
   1 5 10 15
   Ser Leu Arg Leu Ser Cys Ala Val Ser Gly Phe Thr Leu Asp Asp Tyr
   20 25 30
   Ala Ile Gly Trp Phe Arg Gln Ala Pro Gly Lys Glu Arg Glu Gly Val
   35 40 45
   Ser Ser Ile Arg Asp Asn Asp Gly Ser Thr Tyr Tyr Ala Asp Ser Val
   50 55 60
   Lys Gly Arg Ser Thr Ile Ser Ser Asp Asn Asp Lys Asn Thr Val Tyr
   65 70 75 80
   Leu Gln Met Asn Ser Leu Lys Pro Glu Asp Thr Ala Val Tyr Tyr Cys
   85 90 95
   Ala Ala Val Pro Ala Gly Arg Leu Arg Phe Gly Glu Gln Trp Tyr Pro
   100 105 110
   Ile Tyr Glu Tyr Asp Ala Trp Gly Gln Gly Thr Leu Val Thr Val Ser
   115 120 125
   Ser
<210> 100
   <211> 129
   <212> PRT
   <213> Artificial
<220>
   <223> Mutated Lama sequence
<400> 100
   Glu Val Gln Leu Val Glu Ser Gly Gly Gly Leu Val Gln Ala Gly Gly
   1 5 10 15
   Ser Leu Arg Leu Ser Cys Ala Val Ser Gly Ile Thr Leu Asp Asp Tyr
   20 25 30
   Ala Ile Gly Trp Phe Arg Gln Ala Pro Gly Lys Glu Arg Glu Gly Val
   35 40 45
   Ser Ser Ile Arg Asp Asn Asp Gly Ser Thr Tyr Tyr Ala Asp Ser Val
   50 55 60
   Lys Trp Arg Phe Thr Ile Ser Ser Asp Asn Asp Lys Asn Thr Val Tyr
   65 70 75 80
   Leu Gln Met Asn Ser Met Lys Pro Glu Asp Thr Ala Val Tyr Tyr Cys
   85 90 95
   Ala Ala Val Pro Ala Gly Arg Leu Arg Phe Gly Glu Gln Trp Tyr Pro
   100 105 110
   Ile Tyr Glu Tyr Asp Ala Trp Gly Gln Gly Ile Leu Val Thr Val Ser
   115 120 125
   Ser
<210> 101
   <211> 129
   <212> PRT
   <213> Artificial
<220>
   <223> Mutated Lama sequence
<400> 101
   Glu Val Gln Leu Val Glu Ser Gly Gly Gly Leu Val Gln Ala Gly Gly
   1               5                   10                  15
   Phe Leu Arg Leu Thr Cys Ala Val Ser Gly Phe Thr Leu Asp Asp Tyr
               20                  25                  30
   Ala Ile Gly Trp Phe Arg Gln Ala Pro Gly Lys Glu Arg Glu Gly Val
           35                  40                  45
   Ser Ser Ile Arg Asp Asn Asp Gly Ser Thr Tyr Tyr Ala Asp Ser Val
       50                  55                  60
   Lys Gly Arg Phe Thr Ile Ser Ser Asp Asn Val Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Val Pro Ala Gly Arg Leu Arg Phe Gly Glu Gln Trp Tyr Pro
               100                 105                 110
   Ile Tyr Glu Tyr Asp Ala Trp Gly Gln Gly Thr Leu Val Thr Val Ser
           115                 120                 125
   Ser
<210> 102
   <211> 129
   <212> PRT
   <213> Artificial
<220>
   <223> Mutated Lama sequence
<400> 102
   Glu Val Gln Leu Leu Glu Ser Gly Gly Gly Leu Val Gln Ala Gly Gly
   1               5                   10                  15
   Ser Leu Arg Leu Ser Cys Ala Ala Ser Gly Phe Thr Leu Asp Asp Tyr
               20                  25                  30
   Ala Ile Gly Trp Phe Arg Gln Ala Pro Gly Lys Glu Arg Glu Gly Val
   35 40 45
   Ser Ser Ile Arg Asp Asn Asp Gly Ser Thr Tyr Tyr Ala Asp Ser Val
   50 55 60
   Lys Gly Arg Phe Thr Ile Ser Ser Asp Asn Asp Lys Asn Thr Val Tyr
   65 70 75 80
   Leu Gln Met Asn Ser Leu Lys Pro Glu Asp Thr Ala Val Tyr Tyr Cys
   85 90 95
   Ala Ala Val Pro Ala Gly Arg Leu Arg Ile Gly Glu Gln Trp Tyr Pro
   100 105 110
   Ile Tyr Glu Tyr Asp Ala Trp Gly Gln Gly Thr Leu Val Thr Val Ser
   115 120 125
   Ser
<210> 103
   <211> 129
   <212> PRT
   <213> Artificial
<220>
   <223> Mutated Lama sequence
<400> 103
   Glu Val His Leu Val Glu Ser Gly Gly Gly Leu Val Gln Ala Gly Gly
   1               5                   10                  15
   Ser Leu Arg Leu Ser Cys Ala Ala Ser Gly Leu Thr Leu Asp Asp Tyr
               20                  25                  30
   Ala Ile Gly Trp Phe Arg Gln Ala Pro Gly Val Glu Arg Glu Gly Val
           35                  40                  45
   Ser Ser Ile Arg Asp Asn Asp Gly Ser Thr Tyr Tyr Ala Asp Ser Val
       50                  55                  60
   Lys Gly Arg Phe Thr Ile Ser Ser Asp Asn Asp Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Val Pro Ala Gly Arg Leu Arg Phe Gly Glu Gln Trp Tyr Pro
               100                 105                 110
   Ile Tyr Glu Tyr Asp Ala Trp Gly Gln Gly Thr Leu Val Thr Val Ser
           115                 120                 125
   Ser
<210> 104
   <211> 129
   <212> PRT
   <213> Artificial
<220>
   <223> Mutated Lama sequence
<400> 104
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Phe Thr Leu Asp Asp Tyr
               20                  25                  30
   Ala Ile Gly Trp Phe Arg Gln Ala Pro Gly Lys Asp Arg Glu Gly Val
           35                  40                  45
   Ser Ser Ile Arg Asp Asn Asp Gly Ser Thr Tyr Tyr Ala Asp Ser Val
       50                  55                  60
   Lys Gly Arg Phe Thr Ile Ser Ser Asp Asn Asp Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Val Pro Ala Gly Arg Leu Arg Tyr Gly Glu Gln Trp Tyr Pro
               100                 105                 110
   Ile Tyr Glu Tyr Asp Ala Trp Gly Gln Gly Thr Val Val Thr Val Ser
           115                 120                 125
   Ser
<210> 105
   <211> 129
   <212> PRT
   <213> Artificial
<220>
   <223> Mutated Lama sequence
<400> 105
   Glu Val Gln Leu Val Glu Ser Gly Gly Gly Leu Val Gln Ala Gly Gly
   1 5 10 15
   Thr Leu Arg Leu Ser Cys Ala Val Ser Gly Phe Thr Leu Asp Asp Tyr
   20 25 30
   Ala Ile Gly Trp Phe Arg Gln Ala Pro Gly Lys Glu Arg Glu Gly Val
   35 40 45
   Ser Ser Ile Arg Asp Asn Asp Gly Ser Thr Tyr Tyr Ala Asp Ser Val
   50 55 60
   Lys Gly Arg Phe Thr Ile Ser Ser Asp Asn Asp Lys Asn Thr Val Tyr
   65 70 75 80
   Leu Gln Met Asn Ser Leu Lys Pro Glu Asp Thr Ala Val Tyr Tyr Cys
   85 90 95
   Ala Ala Val Pro Ala Gly Arg Leu Arg Phe Gly Glu Gln Trp Tyr Pro
   100 105 110
   Ile Tyr Glu Tyr Asp Ala Trp Gly Gln Gly Ile Leu Val Thr Val Ser
   115 120 125
   Ser
<210> 106
   <211> 129
   <212> **PRT**
   <213> Artificial
<220>
   <223> Mutated Lama sequence
<400> 106
   Glu Val Gln Leu Val Glu Ser Gly Gly Gly Leu Val Gln Ala Gly Gly
   1 5 10 15
   Ser Leu Arg Leu Ser Cys Ala Ala Ser Gly Phe Thr Leu Asp Asp Tyr
   20 25 30
   Ala Ile Gly Trp Phe Arg Gln Ala Pro Gly Lys Glu Arg Glu Gly Val
   35 40 45
   Ser Ser Ile Arg Asp Asn Asp Gly Ser Thr Tyr Tyr Ala Asp Ser Val
           50                  55                  60
   Lys Gly Arg Phe Ile Ile Ser Ser Asp Asn Asp Lys Asn Thr Val Tyr
   65                  70                  75                  80
   Leu Gln Met Asn Asn Leu Lys Pro Glu Asp Thr Ala Val Tyr Tyr Cys
                   85                  90                  95
   Ala Ala Val Pro Ala Gly Arg Leu Arg Tyr Gly Glu Gln Trp Tyr Pro
               100                 105                 110
   Ile Tyr Glu Tyr Asp Ala Trp Gly Gln Gly Thr Leu Val Thr Val Ser
           115                 120                 125
   Ser
<210> 107
   <211> 129
   <212> PRT
   <213> Artificial
<220>
   <223> Mutated Lama sequence
<400> 107
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Phe Thr Leu Asp Asp Tyr
               20                  25                  30
   Ala Ile Gly Trp Phe Arg Gln Ala Pro Gly Lys Glu Arg Glu Gly Val
           35                  40                  45
   Ser Ser Ile Arg Asp Asn Asp Gly Ser Thr Tyr Tyr Ala Asp Ser Val
       50                  55                  60
   Lys Gly Arg Ser Thr Ile Ser Ser Asp Asn Ala Arg Asn Thr Val Phe
   65                  70                  75                  80
   Leu Gln Met Asn Ser Leu Lys Pro Glu Asp Thr Ala Val Tyr Tyr Cys
                   85                  90                  95
   Ala Ala Val Pro Ala Gly Arg Leu Arg Phe Gly Glu Gln Trp Tyr Pro
               100                 105                 110
   Ile Tyr Glu Tyr Asp Ala Trp Gly Gln Gly Thr Leu Val Thr Val Ser
           115                 120                 125
   Ser
<210> 108
   <211> 129
   <212> PRT
   <213> Artificial
<220>
   <223> Mutated Lama sequence
<400> 108
   Glu Glu Leu Leu Val Glu Ser Gly Gly Gly Ser Val Gln Ala Gly Gly
   1               5                   10                  15
   Ser Leu Lys Leu Ser Cys Ala Ala Ser Gly Leu Thr Leu Asp Asp Tyr
               20                  25                  30
   Ala Ile Gly Trp Phe Arg Gln Ala Pro Gly Lys Glu Arg Glu Gly Val
           35                  40                  45
   Ser Ser Ile Arg Asp Asn Asp Gly Ser Thr Tyr Tyr Ala Asp Ser Val
       50                  55                  60
   Lys Gly Arg Phe Thr Ile Ser Ser Asp Asn Asp Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Val Pro Ala Gly Arg Leu Arg Phe Gly Glu Gln Trp Tyr Pro
               100                 105                 110
   Ile Tyr Glu Tyr Asp Ala Trp Gly Gln Gly Thr Leu Val Thr Val Ser
           115                 120                 125
   Ser
<210> 109
   <211> 129
   <212> PRT
   <213> Artificial
<220>
   <223> Mutated Lama sequence
<400> 109
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Leu Thr Leu Asp Asp Tyr
               20                  25                  30
   Ala Ile Gly Trp Phe Arg Gln Ala Pro Gly Lys Glu Arg Glu Gly Val
           35                  40                  45
   Ser Ser Ile Arg Asp Asn Asp Gly Ser Arg Tyr Tyr Ala Asp Ser Val
       50                  55                  60
   Lys Gly Arg Phe Thr Ile Ser Ser Asp Asn Asp Lys Asn Thr Val Tyr
   65                  70                  75                      80
   Leu Gln Met Asn Ser Leu Lys Pro Glu Asp Thr Ala Val Tyr Tyr Cys
                   85                  90                      95
   Ala Ala Val Pro Ala Gly Arg Leu Arg Phe Gly Glu Gln Trp Tyr Pro
               100                 105                 110
   Ile Tyr Glu Tyr Asp Ala Trp Gly Gln Gly Thr Leu Val Thr Val Ser
           115                 120                 125
   Ser
<210> 110
   <211> 129
   <212> PRT
   <213> Artificial
<220>
   <223> Mutated Lama sequence
<400> 110
   Glu Val Gln Leu Val Glu Ser Gly Gly Ser Leu Val Gln Ala Gly Gly
   1               5                   10                  15
   Ser Leu Arg Leu Ser Cys Ala Ala Ser Gly Leu Thr Leu Asp Asp Tyr
               20                  25                  30
   Ala Ile Gly Trp Phe Arg Gln Ala Pro Gly Lys Glu Arg Glu Gly Val
           35                  40                  45
   Ser Ser Ile Arg Asp Asn Asp Gly Ser Thr Tyr Tyr Ala Asp Ser Val
       50                  55                  60
   Lys Gly Arg Phe Thr Ile Ser Ser Asp Asn Asp Lys Asn Thr Val Tyr
   65                  70                  75                      80
   Leu Gln Met Asn Ser Leu Lys Pro Glu Asp Thr Ala Val Tyr Tyr Cys
   85 90 95
   Ala Ala Val Pro Ala Gly Arg Leu Arg Phe Gly Glu Gln Trp Tyr Pro
   100 105 110
   Ile Tyr Glu Tyr Asp Ala Trp Gly Gln Gly Thr Leu Val Thr Val Ser
   115 120 125
   Ser
<210> 111
   <211> 129
   <212> PRT
   <213> Artificial
<220>
   <223> Mutated Lama sequence
<400> 111
   Glu Val Gln Leu Val Glu Ser Gly Gly Ser Leu Val Gln Ala Gly Gly
   1 5 10 15
   Ser Leu Arg Leu Ser Cys Ala Ala Ser Gly Leu Thr Leu Asp Asp Tyr
   20 25 30
   Ala Ile Gly Trp Phe Arg Gln Ala Pro Gly Lys Glu Arg Glu Gly Val
   35 40 45
   Ser Ser Ile Arg Asp Asn Asp Gly Ser Thr Tyr Tyr Ala Asp Ser Val
   50 55 60
   Lys Gly Arg Phe Thr Ile Ser Ser Asp Asn Asp Lys Asn Thr Val Tyr
   65 70 75 80
   Leu Gln Met Asn Ser Leu Lys Pro Glu Asp Thr Ala Val Tyr Tyr Cys
   85 90 95
   Ala Ala Val Pro Ala Gly Arg Leu Arg Phe Gly Glu Gln Trp Tyr Pro
   100 105 110
   Ile Tyr Glu Tyr Asp Ala Trp Gly Gln Gly Thr Leu Val Thr Val Ser
   115 120 125
   Ser
<210> 112
   <211> 129
   <212> PRT
   <213> Artificial
<220>
   <223> Mutated Lama sequence
<400> 112
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Leu Asp Asp Tyr
               20                  25                  30
   Ala Ile Gly Trp Phe Arg Gln Ala Pro Gly Lys Glu Arg Glu Gly Val
           35                  40                  45
   Ser Ser Ile Arg Asp Asn Gly Gly Ser Thr Tyr Tyr Ala Asp Ser Val
       50                  55                  60
   Lys Gly Arg Phe Thr Ile Ser Ser Asp Asn Ser Lys Asn Thr Val Tyr
   65                  70                  75                  80
   Leu Gln Met Asn Ser Leu Arg Pro Glu Asp Thr Ala Val Tyr Tyr Cys
                   85                  90                  95
   Ala Ala Val Pro Ala Gly Arg Leu Arg Phe Gly Glu Gln Trp Tyr Pro
               100                 105                 110
   Ile Tyr Glu Tyr Asp Ala Trp Gly Gln Gly Thr Leu Val Thr Val Ser
           115                 120                 125
   Ser
<210> 113
   <211> 129
   <212> PRT
   <213> Artificial
<220>
   <223> Mutated Lama sequence
<400> 113
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Leu Asp Asp Tyr
               20                  25                  30
   Ala Ile Gly Trp Phe Arg Gln Ala Pro Gly Lys Glu Arg Glu Gly Val
           35                  40                  45
   Ser Ser Ile Arg Asp Asn Gly Gly Ser Thr Tyr Tyr Ala Asp Ser Val
       50                  55                  60
   Lys Gly Arg Phe Thr Ile Ser Ser Asp Asn Ser Lys Asn Thr Val Tyr
   65                  70                  75                  80
   Leu Gln Met Asn Ser Leu Arg Pro Glu Asp Thr Ala Val Tyr Tyr Cys
                   85                  90                  95
   Ala Ala Val Pro Ala Gly Arg Leu Arg Tyr Gly Glu Gln Trp Tyr Pro
               100                 105                 110
   Ile Tyr Glu Tyr Asp Ala Trp Gly Gln Gly Thr Leu Val Thr Val Ser
           115                 120                 125
   Ser
<210> 114
   <211> 129
   <212> PRT
   <213> Artificial
<220>
   <223> Mutated Lama sequence
<400> 114
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Ile Asp Asp Tyr
               20                  25                  30
   Ala Ile Gly Trp Phe Arg Gln Ala Pro Gly Lys Glu Arg Glu Gly Val
           35                  40                  45
   Ser Ser Ile Arg Asp Asn Gly Gly Ser Thr Tyr Tyr Ala Asp Ser Val
       50                  55                  60
   Lys Gly Arg Phe Thr Ile Ser Ser Asp Asn Ser Lys Asn Thr Val Tyr
   65                  70                  75                  80
   Leu Gln Met Asn Ser Leu Arg Pro Glu Asp Thr Ala Val Tyr Tyr Cys
                   85                  90                  95
   Ala Ala Val Pro Ala Gly Arg Leu Arg Phe Gly Glu Gln Trp Tyr Pro
               100                 105                 110
   Ile Tyr Glu Tyr Asp Ala Trp Gly Gln Gly Thr Leu Val Thr Val Ser
           115                 120                 125
   Ser
<210> 115
   <211> 129
   <212> PRT
   <213> Artificial
<220>
   <223> Mutated Lama sequence
<400> 115
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Ile Asp Asp Tyr
               20                  25                  30
   Ala Ile Gly Trp Phe Arg Gln Ala Pro Gly Lys Glu Arg Glu Gly Val
           35                  40                  45
   Ser Ser Ile Arg Asp Asn Gly Gly Ser Thr Tyr Tyr Ala Asp Ser Val
       50                  55                  60
   Lys Gly Arg Phe Thr Ile Ser Ser Asp Asn Ser Lys Asn Thr Val Tyr
   65                  70                  75                  80
   Leu Gln Met Asn Ser Leu Arg Pro Glu Asp Thr Ala Val Tyr Tyr Cys
                   85                  90                  95
   Ala Ala Val Pro Ala Gly Arg Leu Arg Tyr Gly Glu Gln Trp Tyr Pro
               100                 105                 110
   Ile Tyr Glu Tyr Asp Ala Trp Gly Gln Gly Thr Leu Val Thr Val Ser
           115                 120                 125
   Ser
<210> 116
   <211> 129
   <212> PRT
   <213> Artificial
<220>
   <223> Mutated Lama sequence
<400> 116
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Ile Thr Leu Asp Asp Tyr
               20                  25                  30
   Ala Ile Gly Trp Phe Arg Gln Ala Pro Gly Lys Glu Arg Glu Gly Val
           35                  40                  45
   Ser Ser Ile Arg Asp Asn Gly Gly Ser Thr Tyr Tyr Ala Asp Ser Val
       50                  55                  60
   Lys Gly Arg Phe Thr Ile Ser Ser Asp Asn Ser Lys Asn Thr Val Tyr
   65                  70                  75                  80
   Leu Gln Met Asn Ser Leu Arg Pro Glu Asp Thr Ala Val Tyr Tyr Cys
                   85                  90                  95
   Ala Ala Val Pro Ala Gly Arg Leu Arg Phe Gly Glu Gln Trp Tyr Pro
               100                 105                 110
   Ile Tyr Glu Tyr Asp Ala Trp Gly Gln Gly Thr Leu Val Thr Val Ser
           115                 120                 125
   Ser
<210> 117
   <211> 129
   <212> PRT
   <213> Artificial
<220>
   <223> Mutated Lama sequence
<400> 117
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Ile Thr Leu Asp Asp Tyr
               20                  25                  30
   Ala Ile Gly Trp Phe Arg Gln Ala Pro Gly Lys Glu Arg Glu Gly Val
           35                  40                  45
   Ser Ser Ile Arg Asp Asn Gly Gly Ser Thr Tyr Tyr Ala Asp Ser Val
       50                  55                  60
   Lys Gly Arg Phe Thr Ile Ser Ser Asp Asn Ser Lys Asn Thr Val Tyr
   65                  70                  75                  80
   Leu Gln Met Asn Ser Leu Arg Pro Glu Asp Thr Ala Val Tyr Tyr Cys
                   85                  90                  95
   Ala Ala Val Pro Ala Gly Arg Leu Arg Tyr Gly Glu Gln Trp Tyr Pro
               100                 105                 110
   Ile Tyr Glu Tyr Asp Ala Trp Gly Gln Gly Thr Leu Val Thr Val Ser
           115                 120                 125
   Ser
<210> 118
   <211> 129
   <212> PRT
   <213> Artificial
<220>
   <223> Mutated Lama sequence
<400> 118
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Ile Thr Ile Asp Asp Tyr
               20                  25                  30
   Ala Ile Gly Trp Phe Arg Gln Ala Pro Gly Lys Glu Arg Glu Gly Val
           35                  40                  45
   Ser Ser Ile Arg Asp Asn Gly Gly Ser Thr Tyr Tyr Ala Asp Ser Val
       50                  55                  60
   Lys Gly Arg Phe Thr Ile Ser Ser Asp Asn Ser Lys Asn Thr Val Tyr
   65                  70                  75                  80
   Leu Gln Met Asn Ser Leu Arg Pro Glu Asp Thr Ala Val Tyr Tyr Cys
                   85                  90                  95
   Ala Ala Val Pro Ala Gly Arg Leu Arg Phe Gly Glu Gln Trp Tyr Pro
               100                 105                 110
   Ile Tyr Glu Tyr Asp Ala Trp Gly Gln Gly Thr Leu Val Thr Val Ser
   115 120 125
   Ser
<210> 119
   <211> 129
   <212> PRT
   <213> Artificial
<220>
   <223> Mutated Lama sequence
<400> 119
   Glu Val Gln Leu Val Glu Ser Gly Gly Gly Leu Val Gln Pro Gly Gly
   1 5 10 15
   Ser Leu Arg Leu Ser Cys Ala Ala Ser Gly Ile Thr Ile Asp Asp Tyr
   20 25 30
   Ala Ile Gly Trp Phe Arg Gln Ala Pro Gly Lys Glu Arg Glu Gly Val
   35 40 45
   Ser Ser Ile Arg Asp Asn Gly Gly Ser Thr Tyr Tyr Ala Asp Ser Val
   50 55 60
   Lys Gly Arg Phe Thr Ile Ser Ser Asp Asn Ser Lys Asn Thr Val Tyr
   65 70 75 80
   Leu Gln Met Asn Ser Leu Arg Pro Glu Asp Thr Ala Val Tyr Tyr Cys
   85 90 95
   Ala Ala Val Pro Ala Gly Arg Leu Arg Tyr Gly Glu Gln Trp Tyr Pro
   100 105 110
   Ile Tyr Glu Tyr Asp Ala Trp Gly Gln Gly Thr Leu Val Thr Val Ser
   115 120 125
   Ser
<210> 120
   <211> 129
   <212> PRT
   <213> Artificial
<220>
   <223> Mutated Lama sequence
<400> 120
   Glu Val Gln Leu Val Glu Ser Gly Gly Gly Leu Val Gln Pro Gly Gly
   1 5 10 15
   Ser Leu Arg Leu Ser Cys Ala Ala Ser Gly Leu Thr Leu Asp Asp Tyr
   20 25 30
   Ala Ile Gly Trp Phe Arg Gln Ala Pro Gly Lys Glu Arg Glu Gly Val
   35 40 45
   Ser Ser Ile Arg Asp Asn Gly Gly Ser Thr Tyr Tyr Ala Asp Ser Val
   50 55 60
   Lys Gly Arg Phe Thr Ile Ser Ser Asp Asn Ser Lys Asn Thr Val Tyr
   65 70 75 80
   Leu Gln Met Asn Ser Leu Arg Pro Glu Asp Thr Ala Val Tyr Tyr Cys
   85 90 95
   Ala Ala Val Pro Ala Gly Arg Leu Arg Phe Gly Glu Gln Trp Tyr Pro
   100 105 110
   Ile Tyr Glu Tyr Asp Ala Trp Gly Gln Gly Thr Leu Val Thr Val Ser
   115 120 125
   Ser
<210> 121
   <211> 129
   <212> PRT
   <213> Artificial
<220>
   <223> Mutated Lama sequence
<400> 121
   Glu Val Gln Leu Val Glu Ser Gly Gly Gly Leu Val Gln Pro Gly Gly
   1 5 10 15
   Ser Leu Arg Leu Ser Cys Ala Ala Ser Gly Leu Thr Leu Asp Asp Tyr
   20 25 30
   Ala Ile Gly Trp Phe Arg Gln Ala Pro Gly Lys Glu Arg Glu Gly Val
   35 40 45
   Ser Ser Ile Arg Asp Asn Gly Gly Ser Thr Tyr Tyr Ala Asp Ser Val
   50 55 60
   Lys Gly Arg Phe Thr Ile Ser Ser Asp Asn Ser Lys Asn Thr Val Tyr
   65                  70                  75                  80
   Leu Gln Met Asn Ser Leu Arg Pro Glu Asp Thr Ala Val Tyr Tyr Cys
                   85                  90                  95
   Ala Ala Val Pro Ala Gly Arg Leu Arg Tyr Gly Glu Gln Trp Tyr Pro
               100                 105                 110
   Ile Tyr Glu Tyr Asp Ala Trp Gly Gln Gly Thr Leu Val Thr Val Ser
           115                 120                 125
   Ser
<210> 122
   <211> 129
   <212> **PRT**
   <213> Artificial
<220>
   <223> Mutated Lama sequence
<400> 122
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Leu Thr Ile Asp Asp Tyr
               20                  25                  30
   Ala Ile Gly Trp Phe Arg Gln Ala Pro Gly Lys Glu Arg Glu Gly Val
           35                  40                  45
   Ser Ser Ile Arg Asp Asn Gly Gly Ser Thr Tyr Tyr Ala Asp Ser Val
       50                  55                  60
   Lys Gly Arg Phe Thr Ile Ser Ser Asp Asn Ser Lys Asn Thr Val Tyr
   65                  70                  75                  80
   Leu Gln Met Asn Ser Leu Arg Pro Glu Asp Thr Ala Val Tyr Tyr Cys
                   85                  90                  95
   Ala Ala Val Pro Ala Gly Arg Leu Arg Phe Gly Glu Gln Trp Tyr Pro
               100                 105                 110
   Ile Tyr Glu Tyr Asp Ala Trp Gly Gln Gly Thr Leu Val Thr Val Ser
           115                 120                 125
   Ser
<210> 123
   <211> 129
   <212> PRT
   <213> Artificial
<220>
   <223> Mutated Lama sequence
<400> 123
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Leu Thr Ile Asp Asp Tyr
               20                  25                  30
   Ala Ile Gly Trp Phe Arg Gln Ala Pro Gly Lys Glu Arg Glu Gly Val
           35                  40                  45
   Ser Ser Ile Arg Asp Asn Gly Gly Ser Thr Tyr Tyr Ala Asp Ser Val
       50                  55                  60
   Lys Gly Arg Phe Thr Ile Ser Ser Asp Asn Ser Lys Asn Thr Val Tyr
   65                  70                  75                  80
   Leu Gln Met Asn Ser Leu Arg Pro Glu Asp Thr Ala Val Tyr Tyr Cys
                   85                  90                  95
   Ala Ala Val Pro Ala Gly Arg Leu Arg Tyr Gly Glu Gln Trp Tyr Pro
               100                 105                 110
   Ile Tyr Glu Tyr Asp Ala Trp Gly Gln Gly Thr Leu Val Thr Val Ser
           115                 120                 125
   Ser
<210> 124
   <211> 124
   <212> PRT
   <213> Artificial
<220>
   <223> Mutated Lama sequence
<400> 124
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Asp Asp Tyr
   20 25 30
   Ala Leu Gly Trp Phe Arg Gln Ala Ala Gly Lys Glu Arg Glu Gly Val
   35 40 45
   Ser Cys Ile Arg Cys Ser Asp Gly Ser Thr Tyr Tyr Ala Asp Ser Val
   50 55 60
   Lys Gly Arg Phe Thr Ile Ser Arg Asp Asn Ser Lys Asn Thr Val Tyr
   65 70 75 80
   Leu Gln Met Asn Ser Leu Arg Pro Glu Asp Thr Ala Val Tyr Tyr Cys
   85 90 95
   Ala Ala Ser Ile Val Pro Arg Ser Lys Leu Glu Pro Tyr Glu Tyr Asp
   100 105 110
   Ala Trp Gly Gln Gly Thr Leu Val Thr Val Ser Ser
   115 120
<210> 125
   <211> 124
   <212> PRT
   <213> Artificial
<220>
   <223> Mutated Lama sequence
<400> 125
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Asp Asp Tyr
   20 25 30
   Ala Leu Gly Trp Phe Arg Gln Ala Pro Gly Lys Glu Arg Glu Gly Val
   35 40 45
   Ser Cys Ile Arg Cys Ser Asp Gly Ser Thr Tyr Tyr Ala Asp Ser Val
   50 55 60
   Lys Gly Arg Phe Thr Ile Ser Arg Asp Asn Ser Lys Asn Thr Val Tyr
   65 70 75 80
   Leu Gln Met Asn Ser Leu Arg Pro Glu Asp Thr Ala Val Tyr Tyr Cys
   85 90 95
   Ala Ala Ser Ile Val Pro Arg Ser Lys Leu Glu Pro Tyr Glu Tyr Asp
   100 105 110
   Ala Trp Gly Gln Gly Thr Leu Val Thr Val Ser Ser
   115 120
<210> 126
   <211> 124
   <212> PRT
   <213> Artificial
<220>
   <223> Mutated Lama sequence
<400> 126
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Asp Asp Tyr
   20 25 30
   Ala Leu Gly Trp Phe Arg Gln Ala Ala Gly Lys Glu Arg Glu Gly Val
   35 40 45
   Ser Cys Ile Arg Cys Ser Asp Gly Ser Thr Tyr Tyr Ala Asp Ser Val
   50 55 60
   Lys Gly Arg Phe Thr Ile Ser Ser Asp Asn Ser Lys Asn Thr Val Tyr
   65 70 75 80
   Leu Gln Met Asn Ser Leu Arg Pro Glu Asp Thr Ala Val Tyr Tyr Cys
   85 90 95
   Ala Ala Ser Ile Val Pro Arg Ser Lys Leu Glu Pro Tyr Glu Tyr Asp
   100 105 110
   Ala Trp Gly Gln Gly Thr Leu Val Thr Val Ser Ser
   115 120
<210> 127
   <211> 124
   <212> PRT
   <213> Artificial
<220>
   <223> Mutated Lama sequence
<400> 127
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Asp Asp Tyr
   20 25 30
   Ala Leu Gly Trp Phe Arg Gln Ala Pro Gly Lys Glu Arg Glu Gly Val
   35 40 45
   Ser Cys Ile Arg Cys Ser Asp Gly Ser Thr Tyr Tyr Ala Asp Ser Val
   50 55 60
   Lys Gly Arg Phe Thr Ile Ser Ser Asp Asn Ser Lys Asn Thr Val Tyr
   65 70 75 80
   Leu Gln Met Asn Ser Leu Arg Pro Glu Asp Thr Ala Val Tyr Tyr Cys
   85 90 95
   Ala Ala Ser Ile Val Pro Arg Ser Lys Leu Glu Pro Tyr Glu Tyr Asp
   100 105 110
   Ala Trp Gly Gln Gly Thr Leu Val Thr Val Ser Ser
   115 120
<210> 128
   <211> 124
   <212> PRT
   <213> Artificial
<220>
   <223> Mutated Lama sequence
<400> 128
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Asp Asp Tyr
               20                  25                  30
   Ala Leu Gly Trp Phe Arg Gln Ala Ala Gly Lys Glu Arg Glu Gly Val
           35                  40                  45
   Ser Cys Ile Arg Cys Ser Gly Gly Ser Thr Tyr Tyr Ala Asp Ser Val
       50                  55                  60
   Lys Gly Arg Phe Thr Ile Ser Ser Asp Asn Ser Lys Asn Thr Val Tyr
   65                  70                  75                  80
   Leu Gln Met Asn Ser Leu Arg Pro Glu Asp Thr Ala Val Tyr Tyr Cys
                   85                  90                  95
   Ala Ala Ser Ile Val Pro Arg Ser Lys Leu Glu Pro Tyr Glu Tyr Asp
                   100                 105                 110
   Ala Trp Gly Gln Gly Thr Leu Val Thr Val Ser Ser
           115                 120
<210> 129
   <211> 124
   <212> PRT
   <213> Artificial
<220>
   <223> Mutated Lama sequence
<400> 129
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Asp Asp Tyr
               20                  25                  30
   Ala Leu Gly Trp Phe Arg Gln Ala Pro Gly Lys Glu Arg Glu Gly Val
           35                  40                  45
   Ser Cys Ile Arg Cys Ser Gly Gly Ser Thr Tyr Tyr Ala Asp Ser Val
       50                  55                  60
   Lys Gly Arg Phe Thr Ile Ser Ser Asp Asn Ser Lys Asn Thr Val Tyr
   65                  70                  75                  80
   Leu Gln Met Asn Ser Leu Arg Pro Glu Asp Thr Ala Val Tyr Tyr Cys
                   85                  90                  95
   Ala Ala Ser Ile Val Pro Arg Ser Lys Leu Glu Pro Tyr Glu Tyr Asp
               100                 105                 110
   Ala Trp Gly Gln Gly Thr Leu Val Thr Val Ser Ser
           115                 120
<210> 130
   <211> 124
   <212> PRT
   <213> Artificial
<220>
   <223> Mutated Lama sequence
<400> 130
   Asp Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Asp Asp Tyr
               20                  25                  30
   Ala Leu Gly Trp Phe Arg Gln Ala Pro Gly Lys Glu Arg Glu Gly Val
           35                  40                  45
   Ser Cys Ile Arg Cys Ser Gly Gly Ser Thr Tyr Tyr Ala Asp Ser Val
       50                  55                  60
   Lys Gly Arg Phe Thr Ile Ser Ser Asp Asn Ser Lys Asn Thr Val Tyr
   65                  70                  75                  80
   Leu Gln Met Asn Ser Leu Arg Pro Glu Asp Thr Ala Val Tyr Tyr Cys
                   85                  90                  95
   Ala Ala Ser Ile Val Pro Arg Ser Lys Leu Glu Pro Tyr Glu Tyr Asp
               100                 105                 110
   Ala Trp Gly Gln Gly Thr Leu Val Thr Val Ser Ser
           115                 120
<210> 131
   <211> 126
   <212> PRT
   <213> Artificial
<220>
   <223> Mutated Lama sequence
<400> 131
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Ala Leu Asp Tyr Tyr
   20 25 30
   Ala Ile Gly Trp Phe Arg Gln Ala Pro Gly Lys Glu Arg Glu Gly Val
   35 40 45
   Ser Cys Ile Ser Ser Ser Asp Gly Ile Thr Tyr Tyr Val Asp Ser Val
   50 55 60
   Lys Gly Arg Phe Thr Ile Ser Arg Asp Asn Ser Lys Asn Thr Val Tyr
   65 70 75 80
   Leu Gln Met Asn Ser Leu Arg Pro Glu Asp Thr Ala Val Tyr Tyr Cys
   85 90 95
   Ala Thr Asp Ser Gly Gly Tyr Ile Asp Tyr Asp Cys Met Gly Leu Gly
   100 105 110
   Tyr Asp Tyr Trp Gly Gln Gly Thr Leu Val Thr Val Ser Ser
   115 120 125
<210> 132
   <211> 126
   <212> PRT
   <213> Artificial
<220>
   <223> Mutated Lama sequence
<400> 132
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Ala Leu Asp Tyr Tyr
   20 25 30
   Ala Ile Gly Trp Phe Arg Gln Ala Pro Gly Lys Glu Arg Glu Gly Val
   35 40 45
   Ser Cys Ile Ser Ser Ser Asp Gly Ile Thr Tyr Tyr Ala Asp Ser Val
   50 55 60
   Lys Gly Arg Phe Thr Ile Ser Arg Asp Asn Ser Lys Asn Thr Val Tyr
   65 70 75 80
   Leu Gln Met Asn Ser Leu Arg Pro Glu Asp Thr Ala Val Tyr Tyr Cys
   85 90 95
   Ala Thr Asp Ser Gly Gly Tyr Ile Asp Tyr Asp Cys Met Gly Leu Gly
   100 105 110
   Tyr Asp Tyr Trp Gly Gln Gly Thr Leu Val Thr Val Ser Ser
   115 120 125
<210> 133
   <211> 126
   <212> PRT
   <213> Artificial
<220>
   <223> Mutated Lama sequence
<400> 133
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Ala Leu Asp Tyr Tyr
               20                  25                  30
   Ala Ile Gly Trp Phe Arg Gln Ala Pro Gly Lys Glu Arg Glu Gly Val
           35                  40                  45
   Ser Cys Ile Ser Ser Ser Gly Gly Ile Thr Tyr Tyr Val Asp Ser Val
       50                  55                  60
   Lys Gly Arg Phe Thr Ile Ser Arg Asp Asn Ser Lys Asn Thr Val Tyr
   65                  70                  75                  80
   Leu Gln Met Asn Ser Leu Arg Pro Glu Asp Thr Ala Val Tyr Tyr Cys
                   85                  90                  95
   Ala Thr Asp Ser Gly Gly Tyr Ile Asp Tyr Asp Cys Met Gly Leu Gly
               100                 105                 110
   Tyr Asp Tyr Trp Gly Gln Gly Thr Leu Val Thr Val Ser Ser
           115                 120                 125
<210> 134
   <211> 126
   <212> PRT
   <213> Artificial
<220>
   <223> Mutated Lama sequence
<400> 134
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Ala Leu Asp Tyr Tyr
               20                  25                  30
   Ala Ile Gly Trp Phe Arg Gln Ala Pro Gly Lys Glu Arg Glu Gly Val
           35                  40                  45
   Ser Cys Ile Ser Ser Ser Gly Gly Ile Thr Tyr Tyr Ala Asp Ser Val
       50                  55                  60
   Lys Gly Arg Phe Thr Ile Ser Arg Asp Asn Ser Lys Asn Thr Val Tyr
   65                  70                  75                  80
   Leu Gln Met Asn Ser Leu Arg Pro Glu Asp Thr Ala Val Tyr Tyr Cys
                   85                  90                  95
   Ala Thr Asp Ser Gly Gly Tyr Ile Asp Tyr Asp Cys Met Gly Leu Gly
                   100                 105                 110
   Tyr Asp Tyr Trp Gly Gln Gly Thr Leu Val Thr Val Ser Ser
           115                 120                 125
<210> 135
   <211> 126
   <212> PRT
   <213> Artificial
<220>
   <223> Mutated Lama sequence
<400> 135
   Asp Val Gln Leu Val Glu Ser Gly Gly Gly Leu Val Gln Pro Gly Gly
   1               5                   10                  15
   Ser Leu Arg Leu Ser Cys Ala Ala Ser Gly Phe Ala Leu Asp Tyr Tyr
               20                  25                  30
   Ala Ile Gly Trp Phe Arg Gln Ala Pro Gly Lys Glu Arg Glu Gly Val
           35                  40                  45
   Ser Cys Ile Ser Ser Ser Gly Gly Ile Thr Tyr Tyr Ala Asp Ser Val
       50                  55                  60
   Lys Gly Arg Phe Thr Ile Ser Arg Asp Asn Ser Lys Asn Thr Val Tyr
   65                  70                  75                  80
   Leu Gln Met Asn Ser Leu Arg Pro Glu Asp Thr Ala Val Tyr Tyr Cys
                   85                  90                  95
   Ala Thr Asp Ser Gly Gly Tyr Ile Asp Tyr Asp Cys Met Gly Leu Gly
               100                 105                 110
   Tyr Asp Tyr Trp Gly Gln Gly Thr Leu Val Thr Val Ser Ser
           115                 120                 125
<210> 136
   <211> 126
   <212> PRT
   <213> Artificial
<220>
   <223> Mutated Lama sequence
<400> 136
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Ala Leu Asp Tyr Tyr
               20                  25                  30
   Ala Ile Gly Trp Phe Arg Gln Ala Pro Gly Lys Glu Arg Glu Gly Val
           35                  40                  45
   Ser Cys Ile Ser Ser Ser Gly Gly Ile Thr Tyr Tyr Ala Asp Ser Val
       50                  55                  60
   Lys Gly Arg Phe Thr Ile Ser Arg Asp Asn Ser Lys Asn Thr Val Tyr
   65                  70                  75                  80
   Leu Gln Met Asn Ser Leu Arg Pro Glu Asp Thr Ala Val Tyr Tyr Cys
                   85                  90                  95
   Ala Thr Asp Ser Gly Gly Tyr Ile Asp Tyr Asp Cys Gln Gly Leu Gly
               100                 105                 110
   Tyr Asp Tyr Trp Gly Gln Gly Thr Leu Val Thr Val Ser Ser
           115                 120                 125
<210> 137
   <211> 126
   <212> PRT
   <213> Artificial
<220>
   <223> Mutated Lama sequence
<400> 137
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Ala Leu Asp Tyr Tyr
   20 25 30
   Ala Ile Gly Trp Phe Arg Gln Ala Pro Gly Lys Glu Arg Glu Gly Val
   35 40 45
   Ser Cys Ile Ser Ser Ser Gly Gly Ile Thr Tyr Tyr Ala Asp Ser Val
   50 55 60
   Lys Gly Arg Phe Thr Ile Ser Arg Asp Asn Ser Lys Asn Thr Val Tyr
   65 70 75 80
   Leu Gln Met Asn Ser Leu Arg Pro Glu Asp Thr Ala Val Tyr Tyr Cys
   85 90 95
   Ala Thr Asp Ser Gly Gly Tyr Ile Asp Tyr Asp Cys Arg Gly Leu Gly
   100 105 110
   Tyr Asp Tyr Trp Gly Gln Gly Thr Leu Val Thr Val Ser Ser
   115 120 125
<210> 138
   <211> 126
   <212> PRT
   <213> Artificial
<220>
   <223> Mutated Lama sequence
<400> 138
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Ala Leu Asp Tyr Tyr
               20                  25                  30
   Ala Ile Gly Trp Phe Arg Gln Ala Pro Gly Lys Glu Arg Glu Gly Val
           35                  40                  45
   Ser Cys Ile Ser Ser Ser Gly Gly Ile Thr Tyr Tyr Ala Asp Ser Val
       50                  55                  60
   Lys Gly Arg Phe Thr Ile Ser Arg Asp Asn Ser Lys Asn Thr Val Tyr
   65                  70                  75                  80
   Leu Gln Met Asn Ser Leu Arg Pro Glu Asp Thr Ala Val Tyr Tyr Cys
                   85                  90                  95
   Ala Thr Asp Ser Gly Gly Tyr Ile Asp Tyr Asp Cys Ser Gly Leu Gly
               100                 105                 110
   Tyr Asp Tyr Trp Gly Gln Gly Thr Leu Val Thr Val Ser Ser
           115                 120                 125
<210> 139
   <211> 126
   <212> PRT
   <213> Artificial
<220>
   <223> Mutated Lama sequence
<400> 139
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Ala Leu Asp Tyr Tyr
               20                  25                  30
   Ala Ile Gly Trp Phe Arg Gln Ala Pro Gly Lys Glu Arg Glu Gly Val
           35                  40                  45
   Ser Cys Ile Ser Ser Ser Gly Gly Ile Thr Tyr Tyr Ala Asp Ser Val
       50                  55                  60
   Lys Gly Arg Phe Thr Ile Ser Arg Asp Asn Ser Lys Asn Thr Val Tyr
   65                  70                  75                  80
   Leu Gln Met Asn Ser Leu Arg Pro Glu Asp Thr Ala Val Tyr Tyr Cys
                   85                  90                  95
   Ala Thr Glu Ser Gly Gly Tyr Ile Asp Tyr Asp Cys Gln Gly Leu Gly
               100                 105                 110
   Tyr Asp Tyr Trp Gly Gln Gly Thr Leu Val Thr Val Ser Ser
           115                 120                 125
<210> 140
   <211> 126
   <212> PRT
   <213> Artificial
<220>
   <223> Mutated Lama sequence
<400> 140
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Ala Leu Asp Tyr Tyr
   20 25 30
   Ala Ile Gly Trp Phe Arg Gln Ala Pro Gly Lys Glu Arg Glu Gly Val
   35 40 45
   Ser Cys Ile Ser Ser Ser Gly Gly Ile Thr Tyr Tyr Ala Asp Ser Val
   50 55 60
   Lys Gly Arg Phe Thr Ile Ser Arg Asp Asn Ser Lys Asn Thr Val Tyr
   65 70 75 80
   Leu Gln Met Asn Ser Leu Arg Pro Glu Asp Thr Ala Val Tyr Tyr Cys
   85 90 95
   Ala Thr Glu Ser Gly Gly Tyr Ile Asp Tyr Asp Cys Arg Gly Leu Gly
   100 105 110
   Tyr Asp Tyr Trp Gly Gln Gly Thr Leu Val Thr Val Ser Ser
   115 120 125
<210> 141
   <211> 126
   <212> PRT
   <213> Artificial
<220>
   <223> Mutated Lama sequence
<400> 141
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Ala Leu Asp Tyr Tyr
               20                  25                  30
   Ala Ile Gly Trp Phe Arg Gln Ala Pro Gly Lys Glu Arg Glu Gly Val
           35                  40                  45
   Ser Cys Ile Ser Ser Ser Gly Gly Ile Thr Tyr Tyr Ala Asp Ser Val
       50                  55                  60
   Lys Gly Arg Phe Thr Ile Ser Arg Asp Asn Ser Lys Asn Thr Val Tyr
   65                  70                  75                  80
   Leu Gln Met Asn Ser Leu Arg Pro Glu Asp Thr Ala Val Tyr Tyr Cys
                   85                  90                  95
   Ala Thr Glu Ser Gly Gly Tyr Ile Asp Tyr Asp Cys Ser Gly Leu Gly
               100                 105                 110
   Tyr Asp Tyr Trp Gly Gln Gly Thr Leu Val Thr Val Ser Ser
           115                 120                 125
<210> 142
   <211> 129
   <212> PRT
   <213> Artificial
<220>
   <223> Mutated Lama sequence
<400> 142
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Phe Thr Leu Asp Asp Tyr
               20                  25                  30
   Ala Ile Gly Trp Phe Arg Gln Ala Pro Gly Lys Glu Arg Glu Gly Val
           35                  40                  45
   Ser Thr Ile Arg Asp Ser Asp Gly Ser Thr Tyr Tyr Ala Asp Ser Val
       50                  55                  60
   Lys Gly Arg Phe Thr Ile Ser Ser Asp Asn Asp Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Val Pro Ala Gly Arg Leu Arg Phe Gly Glu Gln Trp Tyr Pro
               100                 105                 110
   Leu Tyr Glu Tyr Asp Ala Trp Gly Gln Gly Thr Leu Val Thr Val Ser
           115                 120                 125
   Ser
<210> 143
   <211> 129
   <212> PRT
   <213> Artificial
<220>
   <223> Mutated Lama sequence
<400> 143
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Phe Thr Leu Asp Asp Tyr
               20                  25                  30
   Ala Ile Gly Trp Phe Arg Gln Ala Pro Gly Lys Glu Arg Glu Gly Val
           35                  40                  45
   Ser Ala Ile Arg Asp Ser Asp Gly Ser Thr Tyr Tyr Ala Asp Ser Val
       50                  55                  60
   Lys Gly Arg Phe Thr Ile Ser Ser Asp Asn Asp Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Val Pro Ala Gly Arg Leu Arg Phe Gly Glu Gln Trp Tyr Pro
               100                 105                 110
   Leu Tyr Glu Tyr Asp Ala Trp Gly Gln Gly Thr Leu Val Thr Val Ser
           115                 120                 125
   Ser
<210> 144
   <211> 129
   <212> PRT
   <213> Artificial
<220>
   <223> Mutated Lama sequence
<400> 144
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Phe Thr Leu Asp Asp Tyr
               20                  25                  30
   Ala Ile Gly Trp Phe Arg Gln Ala Pro Gly Lys Glu Arg Glu Gly Val
           35                  40                  45
   Ser Ser Ile Arg Asp Asn Asp Gly Ser Thr Tyr Tyr Ala Asp Ser Val
       50                  55                  60
   Lys Gly Arg Phe Thr Ile Ser Ser Asp Asn Asp Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Val Pro Ala Gly Arg Leu Arg Phe Gly Glu Gln Trp Tyr Pro
               100                 105                 110
   Leu Tyr Glu Tyr Asp Ala Trp Gly Gln Gly Thr Leu Val Thr Val Ser
           115                 120                 125
   Ser
<210> 145
   <211> 129
   <212> PRT
   <213> Artificial
<220>
   <223> Mutated Lama sequence
<400> 145
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Leu Asp Asp Tyr
               20                  25                  30
   Ala Ile Gly Trp Phe Arg Gln Ala Pro Gly Lys Glu Arg Glu Gly Val
           35                  40                  45
   Ser Ser Ile Arg Asp Asn Asp Gly Ser Thr Tyr Tyr Ala Asp Ser Val
       50                  55                  60
   Lys Gly Arg Phe Thr Ile Ser Ser Asp Asn Ala Lys Asn Thr Val Tyr
   65                  70                  75                  80
   Leu Gln Met Asn Ser Leu Arg Pro Glu Asp Thr Ala Val Tyr Tyr Cys
                   85                  90                  95
   Ala Ala Val Pro Ala Gly Arg Leu Arg Phe Gly Glu Gln Trp Tyr Pro
               100                 105                 110
   Leu Tyr Glu Tyr Asp Ala Trp Gly Gln Gly Thr Leu Val Thr Val Ser
           115                 120                 125
   Ser
<210> 146
   <211> 129
   <212> PRT
   <213> Artificial
<220>
   <223> Mutated Lama sequence
<400> 146
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Leu Asp Asp Tyr
               20                  25                  30
   Ala Ile Gly Trp Phe Arg Gln Ala Pro Gly Lys Glu Arg Glu Gly Val
   35 40 45
   Ser Ser Ile Arg Asp Asn Asp Gly Ser Thr Tyr Tyr Ala Asp Ser Val
   50 55 60
   Lys Gly Arg Phe Thr Ile Ser Ser Asp Asn Ser Lys Asn Thr Val Tyr
   65 70 75 80
   Leu Gln Met Asn Ser Leu Arg Pro Glu Asp Thr Ala Val Tyr Tyr Cys
   85 90 95
   Ala Ala Val Pro Ala Gly Arg Leu Arg Phe Gly Glu Gln Trp Tyr Pro
   100 105 110
   Leu Tyr Glu Tyr Asp Ala Trp Gly Gln Gly Thr Leu Val Thr Val Ser
   115 120 125
   Ser
<210> 147
   <211> 129
   <212> PRT
   <213> Artificial
<220>
   <223> Mutated Lama sequence
<400> 147
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Leu Asp Asp Tyr
               20                  25                  30
   Ala Ile Gly Trp Phe Arg Gln Ala Pro Gly Lys Glu Arg Glu Gly Val
           35                  40                  45
   Ser Ser Ile Arg Asp Asn Asp Gly Ser Thr Tyr Tyr Ala Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Arg Pro Glu Asp Thr Ala Val Tyr Tyr Cys
                   85                  90                  95
   Ala Ala Val Pro Ala Gly Arg Leu Arg Phe Gly Glu Gln Trp Tyr Pro
               100                 105                 110
   Leu Tyr Glu Tyr Asp Ala Trp Gly Gln Gly Thr Leu Val Thr Val Ser
           115                 120                 125
   Ser
<210> 148
   <211> 129
   <212> PRT
   <213> Artificial
<220>
   <223> Mutated Lama sequence
<400> 148
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Phe Thr Leu Asp Asp Tyr
               20                  25                  30
   Ala Ile Gly Trp Phe Arg Gln Ala Pro Gly Lys Glu Arg Glu Gly Val
           35                  40                  45
   Ser Ser Ile Arg Asp Ser Asp Gly Ser Thr Tyr Tyr Ala Asp Ser Val
       50                  55                  60
   Lys Gly Arg Phe Thr Ile Ser Ser Asp Asn Asp Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Val Pro Ala Gly Arg Leu Arg Phe Gly Glu Gln Trp Tyr Pro
               100                 105                 110
   Leu Tyr Glu Tyr Asp Ala Trp Gly Gln Gly Thr Leu Val Thr Val Ser
           115                 120                 125
   Ser
<210> 149
   <211> 129
   <212> PRT
   <213> Artificial
<220>
   <223> Mutated Lama sequence
<400> 149
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Leu Asp Asp Tyr
               20                  25                  30
   Ala Ile Gly Trp Phe Arg Gln Ala Pro Gly Lys Glu Arg Glu Gly Val
           35                  40                  45
   Ser Ser Ile Arg Asp Asn Asp Gly Ser Thr Tyr Tyr Ala Asp Ser Val
       50                  55                  60
   Lys Gly Arg Phe Thr Ile Ser Ser Asp Asn Asp Lys Asn Thr Val Tyr
   65                  70                  75                  80
   Leu Gln Met Asn Ser Leu Arg Pro Glu Asp Thr Ala Val Tyr Tyr Cys
                   85                  90                  95
   Ala Ala Val Pro Ala Gly Arg Leu Arg Phe Gly Glu Gln Trp Tyr Pro
               100                 105                 110
   Leu Tyr Glu Tyr Asp Ala Trp Gly Gln Gly Thr Leu Val Thr Val Ser
           115                 120                 125
   Ser
<210> 150
   <211> 129
   <212> PRT
   <213> Artificial
<220>
   <223> Mutated Lama sequence
<400> 150
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Leu Asp Asp Tyr
               20                  25                  30
   Ala Ile Gly Trp Phe Arg Gln Ala Pro Gly Lys Glu Arg Glu Gly Val
           35                  40                  45
   Ser Ser Ile Arg Asp Asn Asp Gly Ser Thr Tyr Tyr Ala Asp Ser Val
       50                  55                  60
   Lys Gly Arg Phe Thr Ile Ser Arg Asp Asn Asp Lys Asn Thr Val Tyr
   65 70 75 80
   Leu Gln Met Asn Ser Leu Arg Pro Glu Asp Thr Ala Val Tyr Tyr Cys
   85 90 95
   Ala Ala Val Pro Ala Gly Arg Leu Arg Phe Gly Glu Gln Trp Tyr Pro
   100 105 110
   Leu Tyr Glu Tyr Asp Ala Trp Gly Gln Gly Thr Leu Val Thr Val Ser
   115 120 125
   Ser
<210> 151
   <211> 129
   <212> PRT
   <213> Artificial
<220>
   <223> Mutated Lama sequence
<400> 151
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Leu Asp Asp Tyr
   20 25 30
   Ala Ile Gly Trp Phe Arg Gln Ala Pro Gly Lys Glu Arg Glu Gly Val
   35 40 45
   Ser Ser Ile Arg Asp Asn Asp Gly Ser Thr Tyr Tyr Ala Asp Ser Val
   50 55 60
   Lys Gly Arg Phe Thr Ile Ser Arg Asp Asn Ser Lys Asn Thr Val Tyr
   65 70 75 80
   Leu Gln Met Asn Ser Leu Arg Pro Glu Asp Thr Ala Val Tyr Tyr Cys
   85 90 95
   Ala Ala Val Pro Ala Gly Arg Leu Arg Phe Gly Glu Gln Trp Tyr Pro
   100 105 110
   Leu Tyr Glu Tyr Asp Ala Trp Gly Gln Gly Thr Leu Val Thr Val Ser
   115 120 125
   Ser
<210> 152
   <211> 129
   <212> PRT
   <213> Artificial
<220>
   <223> Mutated Lama sequence
<400> 152
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Phe Thr Leu Asp Asp Tyr
               20                  25                  30
   Ala Ile Gly Trp Phe Arg Gln Ala Pro Gly Lys Glu Arg Glu Gly Val
           35                  40                  45
   Ser Ser Ile Arg Asp Asn Gly Gly Ser Thr Tyr Tyr Ala Asp Ser Val
       50                  55                  60
   Lys Gly Arg Phe Thr Ile Ser Ser Asp Asn Asp Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Val Pro Ala Gly Arg Leu Arg Phe Gly Glu Gln Trp Tyr Pro
               100                 105                 110
   Leu Tyr Glu Tyr Asp Ala Trp Gly Gln Gly Thr Leu Val Thr Val Ser
           115                 120                 125
   Ser
<210> 153
   <211> 129
   <212> PRT
   <213> Artificial
<220>
   <223> Mutated Lama sequence
<400> 153
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Phe Thr Leu Asp Asp Tyr
   20 25 30
   Ala Ile Gly Trp Phe Arg Gln Ala Pro Gly Lys Glu Arg Glu Gly Val
   35 40 45
   Ser Ala Ile Arg Asp Asn Asp Gly Ser Thr Tyr Tyr Ala Asp Ser Val
   50 55 60
   Lys Gly Arg Phe Thr Ile Ser Ser Asp Asn Asp Lys Asn Thr Val Tyr
   65 70 75 80
   Leu Gln Met Asn Ser Leu Lys Pro Glu Asp Thr Ala Val Tyr Tyr Cys
   85 90 95
   Ala Ala Val Pro Ala Gly Arg Leu Arg Phe Gly Glu Gln Trp Tyr Pro
   100 105 110
   Leu Tyr Glu Tyr Asp Ala Trp Gly Gln Gly Thr Leu Val Thr Val Ser
   115 120 125
   Ser
<210> 154
   <211> 129
   <212> PRT
   <213> Artificial
<220>
   <223> Mutated Lama sequence
<400> 154
   Glu Val Gln Leu Val Glu Ser Gly Gly Gly Leu Val Gln Ala Gly Gly
   1 5 10 15
   Ser Leu Arg Leu Ser Cys Ala Ala Ser Gly Phe Thr Leu Asp Asp Tyr
   20 25 30
   Ala Ile Gly Trp Phe Arg Gln Ala Pro Gly Lys Glu Arg Glu Gly Val
   35 40 45
   Ser Ser Ile Arg Asp Ile Asp Gly Ser Thr Tyr Tyr Ala Asp Ser Val
   50 55 60
   Lys Gly Arg Phe Thr Ile Ser Ser Asp Asn Asp Lys Asn Thr Val Tyr
   65 70 75 80
   Leu Gln Met Asn Ser Leu Lys Pro Glu Asp Thr Ala Val Tyr Tyr Cys
   85 90 95
   Ala Ala Val Pro Ala Gly Arg Leu Arg Phe Gly Glu Gln Trp Tyr Pro
   100 105 110
   Leu Tyr Glu Tyr Asp Ala Trp Gly Gln Gly Thr Leu Val Thr Val Ser
   115 120 125
   Ser
<210> 155
   <211> 129
   <212> PRT
   <213> Artificial
<220>
   <223> Mutated Lama sequence
<400> 155
   Glu Val Gln Leu Val Glu Ser Gly Gly Gly Leu Val Gln Ala Gly Gly
   1 5 10 15
   Ser Leu Arg Leu Ser Cys Ala Val Ser Gly Phe Thr Leu Asp Asp Tyr
   20 25 30
   Ala Ile Gly Trp Phe Arg Gln Ala Pro Gly Lys Glu Arg Glu Gly Val
   35 40 45
   Ser Ser Ile Arg Asp Asn Asp Gly Ser Thr Tyr Tyr Ala Asp Ser Val
   50 55 60
   Lys Gly Arg Phe Thr Ile Ser Ser Asp Asn Asp Lys Asn Thr Val Tyr
   65 70 75 80
   Leu Gln Met Asn Ser Leu Lys Pro Glu Asp Thr Ala Val Tyr Tyr Cys
   85 90 95
   Ala Ala Val Pro Ala Gly Arg Leu Arg Phe Gly Glu Gln Trp Tyr Pro
   100 105 110
   Leu Tyr Glu Tyr Asp Ala Trp Gly Gln Gly Thr Leu Val Thr Val Ser
   115 120 125
   Ser
<210> 156
   <211> 129
   <212> PRT
   <213> Artificial
<220>
   <223> Mutated Lama sequence
<400> 156
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Phe Thr Leu Asp Asp Tyr
               20                  25                  30
   Ala Ile Gly Trp Phe Arg Gln Ala Pro Gly Lys Glu Arg Glu Gly Val
           35                  40                  45
   Ser Ala Ile Arg Asp Ser Asp Gly Ser Thr Tyr Tyr Ala Asp Ser Val
       50                  55                  60
   Lys Gly Arg Phe Thr Ile Ser Ser Asp Asn Asp Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Val Pro Ala Gly Arg Leu Arg Phe Gly Glu Gln Trp Tyr Pro
               100                 105                 110
   Leu Tyr Glu Tyr Asp Ala Trp Gly Gln Gly Thr Leu Val Thr Val Ser
           115                 120                 125
   Ser
<210> 157
   <211> 129
   <212> PRT
   <213> Artificial
<220>
   <223> Mutated Lama sequence
<400> 157
   Glu Val Gln Leu Val Glu Ser Gly Gly Gly Leu Val Gln Ala Gly Gly
   1               5                   10                  15
   Ser Leu Arg Leu Ser Cys Ala Ala Ser Gly Phe Thr Leu Asp Asp Tyr
               20                  25                  30
   Ala Ile Gly Trp Phe Arg Gln Ala Pro Gly Lys Glu Arg Glu Gly Val
   35 40 45
   Ser Ala Ile Arg Asp Ser Gly Gly Ser Thr Tyr Tyr Ala Asp Ser Val
   50 55 60
   Lys Gly Arg Phe Thr Ile Ser Ser Asp Asn Asp Lys Asn Thr Val Tyr
   65 70 75 80
   Leu Gln Met Asn Ser Leu Lys Pro Glu Asp Thr Ala Val Tyr Tyr Cys
   85 90 95
   Ala Ala Val Pro Ala Gly Arg Leu Arg Phe Gly Glu Gln Trp Tyr Pro
   100 105 110
   Leu Tyr Glu Tyr Asp Ala Trp Gly Gln Gly Thr Leu Val Thr Val Ser
   115 120 125
   Ser
<210> 158
   <211> 129
   <212> PRT
   <213> Artificial
<220>
   <223> Mutated Lama sequence
<400> 158
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Leu Asp Asp Tyr
   20 25 30
   Ala Ile Gly Trp Phe Arg Gln Ala Pro Gly Lys Glu Arg Glu Gly Val
   35 40 45
   Ser Ala Ile Arg Asp Asn Gly Gly Ser Thr Tyr Tyr Ala Asp Ser Val
   50 55 60
   Lys Gly Arg Phe Thr Ile Ser Ser Asp Asn Ser Lys Asn Thr Val Tyr
   65 70 75 80
   Leu Gln Met Asn Ser Leu Arg Pro Glu Asp Thr Ala Val Tyr Tyr Cys
   85 90 95
   Ala Ala Val Pro Ala Gly Arg Leu Arg Phe Gly Glu Gln Trp Tyr Pro
   100 105 110
   Leu Tyr Glu Tyr Asp Ala Trp Gly Gln Gly Thr Leu Val Thr Val Ser
   115 120 125
   Ser
<210> 159
   <211> 129
   <212> PRT
   <213> Artificial
<220>
   <223> Mutated Lama sequence
<400> 159
   Asp Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Leu Asp Asp Tyr
   20 25 30
   Ala Ile Gly Trp Phe Arg Gln Ala Pro Gly Lys Glu Arg Glu Gly Val
   35 40 45
   Ser Ala Ile Arg Asp Asn Gly Gly Ser Thr Tyr Tyr Ala Asp Ser Val
   50 55 60
   Lys Gly Arg Phe Thr Ile Ser Ser Asp Asn Ser Lys Asn Thr Val Tyr
   65 70 75 80
   Leu Gln Met Asn Ser Leu Arg Pro Glu Asp Thr Ala Val Tyr Tyr Cys
   85 90 95
   Ala Ala Val Pro Ala Gly Arg Leu Arg Phe Gly Glu Gln Trp Tyr Pro
   100 105 110
   Leu Tyr Glu Tyr Asp Ala Trp Gly Gln Gly Thr Leu Val Thr Val Ser
   115 120 125
   Ser
<210> 160
   <211> 129
   <212> PRT
   <213> Artificial
<220>
   <223> Mutated Lama sequence
<400> 160
   Glu Val Gln Leu Val Glu Ser Gly Gly Gly Leu Val Gln Pro Gly Gly
   1               5                   10                  15
   Ser Leu Arg Leu Ser Cys Ala Val Ser Gly Ile Thr Leu Asp Asp Tyr
               20                  25                  30
   Ala Ile Gly Trp Phe Arg Gln Ala Pro Gly Lys Glu Arg Glu Gly Val
           35                  40                  45
   Ser Ser Ile Arg Asp Asn Gly Gly Ser Thr Tyr Tyr Ala Asp Ser Val
       50                  55                  60
   Lys Gly Arg Phe Thr Ile Ser Ser Asp Asn Ser Lys Asn Thr Val Tyr
   65                  70                  75                  80
   Leu Gln Met Asn Ser Leu Arg Pro Glu Asp Thr Ala Val Tyr Tyr Cys
                   85                  90                  95
   Ala Ala Val Pro Ala Gly Arg Leu Arg Tyr Gly Glu Gln Trp Tyr Pro
               100                 105                 110
   Ile Tyr Glu Tyr Asp Ala Trp Gly Gln Gly Thr Leu Val Thr Val Ser
           115                 120                 125
   Ser
<210> 161
   <211> 129
   <212> PRT
   <213> Artificial
<220>
   <223> Mutated Lama sequence
<400> 161
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Ile Thr Leu Asp Asp Tyr
               20                  25                  30
   Ala Ile Gly Trp Phe Arg Gln Ala Pro Gly Lys Glu Arg Glu Gly Val
           35                  40                  45
   Ser Ala Ile Arg Asp Asn Gly Gly Ser Thr Tyr Tyr Ala Asp Ser Val
       50                  55                  60
   Lys Gly Arg Phe Thr Ile Ser Ser Asp Asn Ser Lys Asn Thr Val Tyr
   65                  70                  75                  80
   Leu Gln Met Asn Ser Leu Arg Pro Glu Asp Thr Ala Val Tyr Tyr Cys
                   85                  90                  95
   Ala Ala Val Pro Ala Gly Arg Leu Arg Tyr Gly Glu Gln Trp Tyr Pro
               100                 105                 110
   Ile Tyr Glu Tyr Asp Ala Trp Gly Gln Gly Thr Leu Val Thr Val Ser
           115                 120                 125
   Ser
<210> 162
   <211> 129
   <212> PRT
   <213> Artificial
<220>
   <223> Mutated Lama sequence
<400> 162
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Ile Thr Leu Asp Asp Tyr
               20                  25                  30
   Ala Ile Gly Trp Phe Arg Gln Ala Pro Gly Lys Glu Arg Glu Gly Val
           35                  40                  45
   Ser Ala Ile Arg Glu Ser Gly Gly Ser Thr Tyr Tyr Ala Asp Ser Val
       50                  55                  60
   Lys Gly Arg Phe Thr Ile Ser Ser Asp Asn Ser Lys Asn Thr Val Tyr
   65                  70                  75                  80
   Leu Gln Met Asn Ser Leu Arg Pro Glu Asp Thr Ala Val Tyr Tyr Cys
                   85                  90                  95
   Ala Ala Val Pro Ala Gly Arg Leu Arg Tyr Gly Glu Gln Trp Tyr Pro
               100                 105                 110
   Ile Tyr Glu Tyr Asp Ala Trp Gly Gln Gly Thr Leu Val Thr Val Ser
                 115                 120                 125
   Ser
<210> 163
   <211> 129
   <212> PRT
   <213> Artificial
<220>
   <223> Mutated Lama sequence
<400> 163
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Ile Thr Leu Asp Asp Tyr
               20                  25                  30
   Ala Ile Gly Trp Phe Arg Gln Ala Pro Gly Lys Glu Arg Glu Gly Val
           35                  40                  45
   Ser Ala Ile Arg Ser Ser Gly Gly Ser Thr Tyr Tyr Ala Asp Ser Val
       50                  55                  60
   Lys Gly Arg Phe Thr Ile Ser Ser Asp Asn Ser Lys Asn Thr Val Tyr
   65                  70                  75                  80
   Leu Gln Met Asn Ser Leu Arg Pro Glu Asp Thr Ala Val Tyr Tyr Cys
                   85                  90                  95
   Ala Ala Val Pro Ala Gly Arg Leu Arg Tyr Gly Glu Gln Trp Tyr Pro
               100                 105                 110
   Ile Tyr Glu Tyr Asp Ala Trp Gly Gln Gly Thr Leu Val Thr Val Ser
           115                 120                 125
   Ser
<210> 164
   <211> 129
   <212> PRT
   <213> Artificial
<220>
   <223> Mutated Lama sequence
<400> 164
   Glu Val Gln Leu Val Glu Ser Gly Gly Gly Leu Val Gln Pro Gly Gly
   1 5 10 15
   Ser Leu Arg Leu Ser Cys Ala Val Ser Gly Ile Thr Leu Asp Asp Tyr
   20 25 30
   Ala Ile Gly Trp Phe Arg Gln Ala Pro Gly Lys Glu Arg Glu Gly Val
   35 40 45
   Ser Ala Ile Arg Asp Asn Gly Gly Ser Thr Tyr Tyr Ala Asp Ser Val
   50 55 60
   Lys Gly Arg Phe Thr Ile Ser Ser Asp Asn Ser Lys Asn Thr Val Tyr
   65 70 75 80
   Leu Gln Met Asn Ser Leu Arg Pro Glu Asp Thr Ala Val Tyr Tyr Cys
   85 90 95
   Ala Ala Val Pro Ala Gly Arg Leu Arg Tyr Gly Glu Gln Trp Tyr Pro
   100 105 110
   Ile Tyr Glu Tyr Asp Ala Trp Gly Gln Gly Thr Leu Val Thr Val Ser
   115 120 125
   Ser
<210> 165
   <211> 129
   <212> PRT
   <213> Artificial
<220>
   <223> Mutated Lama sequence
<400> 165
   Glu Val Gln Leu Val Glu Ser Gly Gly Gly Leu Val Gln Pro Gly Gly
   1 5 10 15
   Ser Leu Arg Leu Ser Cys Ala Val Ser Gly Ile Thr Leu Asp Asp Tyr
   20 25 30
   Ala Ile Gly Trp Phe Arg Gln Ala Pro Gly Lys Glu Arg Glu Gly Val
   35 40 45
   Ser Ala Ile Arg Glu Ser Gly Gly Ser Thr Tyr Tyr Ala Asp Ser Val
   50 55 60
   Lys Gly Arg Phe Thr Ile Ser Ser Asp Asn Ser Lys Asn Thr Val Tyr
   65 70 75 80
   Leu Gln Met Asn Ser Leu Arg Pro Glu Asp Thr Ala Val Tyr Tyr Cys
   85 90 95
   Ala Ala Val Pro Ala Gly Arg Leu Arg Tyr Gly Glu Gln Trp Tyr Pro
   100 105 110
   Ile Tyr Glu Tyr Asp Ala Trp Gly Gln Gly Thr Leu Val Thr Val Ser
   115 120 125
   Ser
<210> 166
   <211> 129
   <212> PRT
   <213> Artificial
<220>
   <223> Mutated Lama sequence
<400> 166
   Glu Val Gln Leu Val Glu Ser Gly Gly Gly Leu Val Gln Pro Gly Gly
   1               5                   10                  15
   Ser Leu Arg Leu Ser Cys Ala Val Ser Gly Ile Thr Leu Asp Asp Tyr
               20                  25                  30
   Ala Ile Gly Trp Phe Arg Gln Ala Pro Gly Lys Glu Arg Glu Gly Val
           35                  40                  45
   Ser Ala Ile Arg Ser Ser Gly Gly Ser Thr Tyr Tyr Ala Asp Ser Val
       50                  55                  60
   Lys Gly Arg Phe Thr Ile Ser Ser Asp Asn Ser Lys Asn Thr Val Tyr
   65                  70                  75                  80
   Leu Gln Met Asn Ser Leu Arg Pro Glu Asp Thr Ala Val Tyr Tyr Cys
                   85                  90                  95
   Ala Ala Val Pro Ala Gly Arg Leu Arg Tyr Gly Glu Gln Trp Tyr Pro
               100                 105                 110
   Ile Tyr Glu Tyr Asp Ala Trp Gly Gln Gly Thr Leu Val Thr Val Ser
           115                 120                 125
   Ser
<210> 167
   <211> 129
   <212> **PRT**
   <213> Artificial
<220>
   <223> Mutated Lama sequence
<400> 167
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Leu Asp Asp Tyr
   20 25 30
   Ala Ile Gly Trp Phe Arg Gln Ala Pro Gly Lys Glu Arg Glu Gly Val
   35 40 45
   Ser Ala Ile Arg Ser Ser Gly Gly Ser Thr Tyr Tyr Ala Asp Ser Val
   50 55 60
   Lys Gly Arg Phe Thr Ile Ser Ser Asp Asn Ser Lys Asn Thr Val Tyr
   65 70 75 80
   Leu Gln Met Asn Ser Leu Arg Pro Glu Asp Thr Ala Val Tyr Tyr Cys
   85 90 95
   Ala Ala Val Pro Ala Gly Arg Leu Arg Phe Gly Glu Gln Trp Tyr Pro
   100 105 110
   Leu Tyr Glu Tyr Asp Ala Trp Gly Gln Gly Thr Leu Val Thr Val Ser
   115 120 125
   Ser
<210> 168
   <211> 5
   <212> PRT
   <213> Artificial
<220>
   <223> Mutated Lama sequence
<400> 168
   Asp Tyr Ala Ile Gly
   1 5
<210> 169
   <211> 5
   <212> PRT
   <213> Artificial
<220>
   <223> Mutated Lama sequence
<400> 169
   Asp Tyr Ala Leu Gly
   1 5
<210> 170
   <211> 5
   <212> PRT
   <213> Artificial
<220>
   <223> Mutated Lama sequence
<400> 170
   Tyr Tyr Ala Ile Gly
   1               5
<210> 171
   <211> 17
   <212> PRT
   <213> Artificial
<220>
   <223> Mutated Lama sequence
<400> 171
   Ala Ile Arg Ser Ser Gly Gly Ser Thr Tyr Tyr Ala Asp Ser Val Lys
   1               5                   10                  15
   Gly
<210> 172
   <211> 17
   <212> PRT
   <213> Artificial
<220>
   <223> Mutated Lama sequence
<400> 172
   Cys Ile Arg Cys Ser Gly Gly Ser Thr Tyr Tyr Ala Asp Ser Val Lys
   1               5                   10                  15
   Gly
<210> 173
   <211> 17
   <212> PRT
   <213> Artificial
<220>
   <223> Mutated Lama sequence
<400> 173
   Cys Ile Ser Ser Ser Gly Gly Ile Thr Tyr Tyr Ala Asp Ser Val Lys
   1 5 10 15
   Gly
<210> 174
   <211> 20
   <212> PRT
   <213> Artificial
<220>
   <223> Mutated Lama sequence
<400> 174
   Val Pro Ala Gly Arg Leu Arg Phe Gly Glu Gln Trp Tyr Pro Leu Tyr
   1 5 10 15
   Glu Tyr Asp Ala
   20
<210> 175
   <211> 20
   <212> PRT
   <213> Artificial
<220>
   <223> Mutated Lama sequence
<400> 175
   Val Pro Ala Gly Arg Leu Arg Tyr Gly Glu Gln Trp Tyr Pro Ile Tyr
   1 5 10 15
   Glu Tyr Asp Ala
   20
<210> 176
   <211> 15
   <212> PRT
   <213> Artificial
<220>
   <223> Mutated Lama sequence
<400> 176
   Ser Ile Val Pro Arg Ser Lys Leu Glu Pro Tyr Glu Tyr Asp Ala
   1 5 10 15
<210> 177
   <211> 17
   <212> PRT
   <213> Artificial
<220>
   <223> Mutated Lama sequence
<400> 177
   Asp Ser Gly Gly Tyr Ile Asp Tyr Asp Cys Ser Gly Leu Gly Tyr Asp
   1               5                   10                  15
   Tyr
<210> 178
   <211> 387
   <212> DNA
   <213> Artificial
<220>
   <223> Mutated Lama sequence
<400> 178
   gaggtacagc tggtcgagtc aggtggcgga ttagtgcagc ctgggggttc tctgcgctta     60
   tcttgtgccg catcaggctt cacactggat gactacgcca tcggctggtt ccggcaagcg    120
   cctggaaaag aacgcgaagg tgtttcagca atccgttcaa gcggtggttc aacatattac    180
   gccgactctg ttaaaggacg cttcaccatt agctccgaca atagtaaaaa tacagtctac    240
   ttacaaatga acagtttacg cccagaagat actgcggtat actattgcgc tgccgtgcct    300
   gctggtcgct tacgctttgg cgagcaatgg tatcctctgt acgagtacga cgcctgggga    360
   cagggtacgc tggtaacggt ttcaagc                                        387
<210> 179
   <211> 387
   <212> DNA
   <213> Artificial
<220>
   <223> Mutated Lama sequence
<400> 179
   gaagtgcaac tggttgagtc aggtggcgga ctggtgcaac cgggtggttc actgcgcctg     60
   agttgcgcag ttagcggtat taccctggat gattatgcaa ttggttggtt tcgccaagcc    120
   ccaggcaaag agcgtgaagg cgttagcgca attcgtagca gcggtggtag cacctattat    180
   gccgattcag ttaaaggccg ttttacgatc agcagcgata acagtaaaaa cacggtttat    240
   ctgcaaatga actcattacg tccagaggac actgcagttt actattgcgc agcagttccg    300
   gcaggtcgtc tgcgttatgg tgaacagtgg tatccgattt atgaatatga tgcatggggt    360
   caaggtacac tggttacagt gagtagc                                        387
<210> 180
   <211> 372
   <212> DNA
   <213> Artificial
<220>
   <223> Mutated Lama sequence
<400> 180
   gaagtgcagt tagtcgaaag tggcggaggc ctggtacaac ctggtggcag tctgcgctta      60
   tcttgtgccg cttcaggttt tacattcgac gactacgccc tggggtggtt ccggcaagcg     120
   cctggaaaag aacgtgaggg cgtttcatgc attcgttgtt caggtggttc aacctattat     180
   gccgatagtg taaaaggtcg gttcaccatt agtagcgaca atagcaagaa tacagtctat     240
   ctgcaaatga actctttacg tcctgaagat actgcggtgt actactgcgc tgcatcaatc     300
   gttcctcgtt caaaacttga accttacgag tacgacgcct ggggtcaggg tacgttagta     360
   acggtgtcaa gc                                                        372
<210> 181
   <211> 378
   <212> DNA
   <213> Artificial
<220>
   <223> Mutated Lama sequence
<400> 181
   gaagtgcaac tggttgagtc aggtggcggt ttagtgcaac cgggtggttc actgcgcctg      60
   agttgcgcag ccagcggttt tgcactggat tattatgcaa ttggttggtt tcgccaagcc     120
   ccaggcaaag agcgtgaagg cgttagctgt attagcagca gcggtggtat tacctattat     180
   gccgattcag ttaaaggccg ttttacgatc tctcgtgata atagtaaaaa cacggtttac     240
   ctgcagatga actcattaag accagaggac actgcagttt actattgtgc aaccgatagc     300
   ggtggctata ttgattatga ttgtagcggt ctgggctacg attattgggg acaaggtacg     360
   ctggtgacag ttagcagc                                                  378
<210> 182
   <211> 3375
   <212> DNA
   <213> Homo sapiens
<400> 182
   atggactctt tagccagctt agttctctgt ggagtcagct tgctcctttc tggaactgtg      60
   gaaggtgcca tggacttgat cttgatcaat tccctacctc ttgtatctga tgctgaaaca 120
   tctctcacct gcattgcctc tgggtggcgc ccccatgagc ccatcaccat aggaagggac 180
   tttgaagcct taatgaacca gcaccaggat ccgctggaag ttactcaaga tgtgaccaga 240
   gaatgggcta aaaaagttgt ttggaagaga gaaaaggcta gtaagatcaa tggtgcttat 300
   ttctgtgaag ggcgagttcg aggagaggca atcaggatac gaaccatgaa gatgcgtcaa 360
   caagcttcct tcctaccagc tactttaact atgactgtgg acaagggaga taacgtgaac 420
   atatctttca aaaaggtatt gattaaagaa gaagatgcag tgatttacaa aaatggttcc 480
   ttcatccatt cagtgccccg gcatgaagta cctgatattc tagaagtaca cctgcctcat 540
   gctcagcccc aggatgctgg agtgtactcg gccaggtata taggaggaaa cctcttcacc 600
   tcggccttca ccaggctgat agtccggaga tgtgaagccc agaagtgggg acctgaatgc 660
   aaccatctct gtactgcttg tatgaacaat ggtgtctgcc atgaagatac tggagaatgc 720
   atttgccctc ctgggtttat gggaaggacg tgtgagaagg cttgtgaact gcacacgttt 780
   ggcagaactt gtaaagaaag gtgcagtgga caagagggat gcaagtctta tgtgttctgt 840
   ctccctgacc cctatgggtg ttcctgtgcc acaggctgga agggtctgca gtgcaatgaa 900
   gcatgccacc ctggttttta cgggccagat tgtaagctta ggtgcagctg caacaatggg 960
   gagatgtgtg atcgcttcca aggatgtctc tgctctccag gatggcaggg gctccagtgt 1020
   gagagagaag gcataccgag gatgacccca aagatagtgg atttgccaga tcatatagaa 1080
   gtaaacagtg gtaaatttaa tcccatttgc aaagcttctg gctggccgct acctactaat 1140
   gaagaaatga ccctggtgaa gccggatggg acagtgctcc atccaaaaga ctttaaccat 1200
   acggatcatt tctcagtagc catattcacc atccaccgga tcctcccccc tgactcagga 1260
   gtttgggtct gcagtgtgaa cacagtggct gggatggtgg aaaagccctt caacatttct 1320
   gttaaagttc ttccaaagcc cctgaatgcc ccaaacgtga ttgacactgg acataacttt 1380
   gctgtcatca acatcagctc tgagccttac tttggggatg gaccaatcaa atccaagaag 1440
   cttctataca aacccgttaa tcactatgag gcttggcaac atattcaagt gacaaatgag 1500
   attgttacac tcaactattt ggaacctcgg acagaatatg aactctgtgt gcaactggtc 1560
   cgtcgtggag agggtgggga agggcatcct ggacctgtga gacgcttcac aacagcttct 1620
   atcggactcc ctcctccaag aggtctaaat ctcctgccta aaagtcagac cactctaaat 1680
   ttgacctggc aaccaatatt tccaagctcg gaagatgact tttatgttga agtggagaga 1740
   aggtctgtgc aaaaaagtga tcagcagaat attaaagttc caggcaactt gacttcggtg 1800
   ctacttaaca acttacatcc cagggagcag tacgtggtcc gagctagagt caacaccaag 1860
   gcccaggggg aatggagtga agatctcact gcttggaccc ttagtgacat tcttcctcct 1920
   caaccagaaa acatcaagat ttccaacatt acacactcct cggctgtgat ttcttggaca 1980
   atattggatg gctattctat ttcttctatt actatccgtt acaaggttca aggcaagaat 2040
   gaagaccagc acgttgatgt gaagataaag aatgccacca tcattcagta tcagctcaag 2100
   ggcctagagc ctgaaacagc ataccaggtg gacatttttg cagagaacaa catagggtca 2160
   agcaacccag ccttttctca tgaactggtg accctcccag aatctcaagc accagcggac 2220
   ctcggagggg ggaagatgct gcttatagcc atccttggct ctgctggaat gacctgcctg 2280
   actgtgctgt tggcctttct gatcatattg caattgaaga gggcaaatgt gcaaaggaga 2340
   atggcccaag ccttccaaaa cgtgagggaa gaaccagctg tgcagttcaa ctcagggact 2400
   ctggccctaa acaggaaggt caaaaacaac ccagatccta caatttatcc agtgcttgac 2460
   tggaatgaca tcaaatttca agatgtgatt ggggagggca attttggcca agttcttaag 2520
   gcgcgcatca agaaggatgg gttacggatg gatgctgcca tcaaaagaat gaaagaatat 2580
   gcctccaaag atgatcacag ggactttgca ggagaactgg aagttctttg taaacttgga 2640
   caccatccaa acatcatcaa tctcttagga gcatgtgaac atcgaggcta cttgtacctg 2700
   gccattgagt acgcgcccca tggaaacctt ctggacttcc ttcgcaagag ccgtgtgctg 2760
   gagacggacc cagcatttgc cattgccaat agcaccgcgt ccacactgtc ctcccagcag 2820
   ctccttcact tcgctgccga cgtggcccgg ggcatggact acttgagcca aaaacagttt 2880
   atccacaggg atctggctgc cagaaacatt ttagttggtg aaaactatgt ggcaaaaata 2940
   gcagattttg gattgtcccg aggtcaagag gtgtacgtga aaaagacaat gggaaggctc 3000
   ccagtgcgct ggatggccat cgagtcactg aattacagtg tgtacacaac caacagtgat 3060
   gtatggtcct atggtgtgtt actatgggag attgttagct taggaggcac accctactgc 3120
   gggatgactt gtgcagaact ctacgagaag ctgccccagg gctacagact ggagaagccc 3180
   ctgaactgtg atgatgaggt gtatgatcta atgagacaat gctggcggga gaagccttat 3240
   gagaggccat catttgccca gatattggtg tccttaaaca gaatgttaga ggagcgaaag 3300
   acctacgtga ataccacgct ttatgagaag tttacttatg caggaattga ctgttctgct 3360
   gaagaagcgg cctag 3375
<210> 183
   <211> 4654
   <212> DNA
   <213> Mouse
<400> 183
   gagcaggagc cggagcagga gcagaagata agccttggat gaagggcaag atggataggg 60
   ctcgctctgc cccaagccct gctgatacca agtgccttta agatacagcc tttcccatcc 120
   taatctgcaa aggaaacagg aaaaaggaac ttaaccctcc ctgtgctcag acagaaatga 180
   gactgttacc gcctgcttct gtggtgtttc tccttgccgc caacttgtaa acaagagcga 240
   gtggaccatg cgagcgggaa gtcgcaaagt tgtgagttgt tgaaagcttc ccagggactc 300
   atgctcatct gtggacgctg gatggggaga tctggggaag tatggactct ttagccggct 360
   tagttctctg tggagtcagc ttgctccttt atggagtagt agaaggcgcc atggacctga 420
   tcttgatcaa ttccctacct cttgtgtctg atgccgaaac atccctcacc tgcattgcct 480
   ctgggtggca cccccatgag cccatcacca taggaaggga ctttgaagcc ttaatgaacc 540
   agcaccaaga tccactggag gttactcaag atgtgaccag agaatgggcg aaaaaagttg 600
   tttggaagag agaaaaggcc agtaagatta atggtgctta tttctgtgaa ggtcgagttc 660
   gaggacaggc tataaggata cggaccatga agatgcgtca acaagcatcc ttcctacctg 720
   ctactttaac tatgaccgtg gacaggggag ataatgtgaa catatctttc aaaaaggtgt 780
   taattaaaga agaagatgca gtgatttaca aaaatggctc cttcatccac tcagtgcccc 840
   ggcatgaagt acctgatatt ttagaagttc acttgccgca tgctcagccc caggatgctg 900
   gtgtgtactc ggccaggtac ataggaggaa acctgttcac ctcagccttc accaggctga 960
   ttgttcggag atgtgaagct cagaagtggg ggcccgactg tagccgtcct tgtactactt 1020
   gcaagaacaa tggagtctgc catgaagata ccggggaatg catttgccct cctgggttta 1080
   tggggagaac atgtgagaaa gcttgtgagc cgcacacatt tggcaggacc tgtaaagaaa 1140
   ggtgtagtgg accagaagga tgcaagtctt atgtgttctg tctcccagac ccttacgggt 1200
   gttcctgtgc cacaggctgg agggggttgc agtgcaatga agcatgccca tctggttact 1260
   acggaccaga ctgtaagctc aggtgccact gtaccaatga agagatatgt gatcggttcc 1320
   aaggatgcct ctgctctcaa ggatggcaag ggctgcagtg tgagaaagaa ggcaggccaa 1380
   ggatgactcc acagatagag gatttgccag atcacattga agtaaacagt ggaaaattta 1440
   accccatctg caaagcctct gggtggccac tacctactag tgaagaaatg accctagtga 1500
   agccagatgg gacagtgctc caaccaaatg acttcaacta tacagatcgt ttctcagtgg 1560
   ccatattcac tgtcaaccga gtcttacctc ctgactcagg agtctgggtc tgcagtgtga 1620
   acacagtggc tgggatggtg gaaaagcctt tcaacatttc cgtcaaagtt cttccagagc 1680
   ccctgcacgc cccaaatgtg attgacactg gacataactt tgctatcatc aatatcagct 1740
   ctgagcctta ctttggggat ggacccatca aatccaagaa gcttttctat aaacctgtca 1800
   atcaggcctg gaaatacatt gaagtgacga atgagatttt cactctcaac tacttggagc 1860
   cgcggactga ctacgagctg tgtgtgcagc tggcccgtcc tggagagggt ggagaagggc 1920
   atcctgggcc tgtgagacga tttacaacag cgtctatcgg actccctcct ccaagaggtc 1980
   tcagtctcct gccaaaaagc cagacagctc taaatttgac ttggcaaccg atatttacaa 2040
   actcagaaga tgaattttat gtggaagtcg agaggcgatc cctgcaaaca acaagtgatc 2100
   agcagaacat caaagtgcct gggaacctga cctcggtgct actgagcaac ttagtcccca 2160
   gggagcagta cacagtccga gctagagtca acaccaaggc gcagggggag tggagtgaag 2220
   aactcagggc ctggaccctt agtgacattc tccctcctca accagaaaac atcaagatct 2280
   ccaacatcac tgactccaca gctatggttt cttggacaat agtggatggc tattcgattt 2340
   cttccatcat catccggtat aaggttcagg gcaaaaatga agaccagcac attgatgtga 2400
   agatcaagaa tgctaccgtt actcagtacc agctcaaggg cctagagcca gagactacat 2460
   accatgtgga tatttttgct gagaacaaca taggatcaag caacccagcc ttttctcatg 2520
   aactgaggac gcttccacat tccccagcct ctgcagacct cggaggggga aagatgctac 2580
   tcatagccat ccttgggtcg gctggaatga cttgcatcac cgtgctgttg gcgtttctga 2640
   ttatgttgca actgaagaga gcaaatgtcc aaaggagaat ggctcaggca ttccagaacg 2700
   tgagagaaga accagctgtg cagtttaact caggaactct ggcccttaac aggaaggcca 2760
   aaaacaatcc ggatcccaca atttatcctg tgcttgactg gaatgacatc aagtttcaag 2820
   acgtgatcgg agagggcaac tttggccagg ttctgaaggc acgcatcaag aaggatgggt 2880
   tacggatgga tgccgccatc aagaggatga aagagtatgc ctccaaagat gatcacaggg 2940
   acttcgcagg agaactggag gttctttgta aacttggaca ccatccaaac atcattaatc 3000
   tcttgggagc atgtgaacac cgaggctatt tgtacctagc tattgagtat gccccgcatg 3060
   gaaacctcct ggacttcctg cgtaagagca gagtgctaga gacagaccct gcttttgcca 3120
   tcgccaacag tacagcttcc acactgtcct cccaacagct tcttcatttt gctgcagatg 3180
   tggcccgggg gatggactac ttgagccaga aacagtttat ccacagggac ctggctgcca 3240
   gaaacatttt agttggtgaa aactacatag ccaaaatagc agattttgga ttgtcacgag 3300
   gtcaagaagt gtatgtgaaa aagacaatgg gaaggctccc agtgcgttgg atggcaatcg 3360
   aatcactgaa ctatagtgtc tatacaacca acagtgatgt ctggtcctat ggtgtattgc 3420
   tctgggagat tgttagctta ggaggcaccc cctactgcgg catgacgtgc gcggagctct 3480
   atgagaagct accccagggc tacaggctgg agaagcccct gaactgtgat gatgaggtgt 3540
   atgatctaat gagacagtgc tggagggaga agccttatga gagaccatca tttgcccaga 3600
   tattggtgtc cttaaacagg atgctggaag aacggaagac atacgtgaac accacactgt 3660
   atgagaagtt tacctatgca ggaattgact gctctgcgga agaagcagcc tagagcagaa 3720
   ctcttcatgt acaacggcca tttctcctca ctggcgcgag agcgccttga cacctgtacc 3780
   aagcaagcca cccactgcca agagatgtga tatataagtg tatatattgt gctgtgtttg 3840
   ggaccctcct catacagctc gtgcggatct gcagtgtgtt ctgactctaa tgtgactgta 3900
   tatactgctc ggagtaagaa tgtgctaaga tcagaatgcc tgttcgtggt ttcatataat 3960
   atatttttct aaaagcatag attgcacagg aaggtatgag tacaaatact gtaatgcata   4020
   acttgttatt gtcctagatg tgtttgatat ttttccttta caactgaatg ctataaaagt   4080
   gttttgctgt gtacacataa gatactgttc gttaaaataa gcattccctt gacagcacag   4140
   gaagaaaagc gagggaaatg tatggattat attaaatgtg ggttactaca caagaggccg   4200
   aacattccaa gtagcagaag agagggtctc tcaactctgc tcctcacctg cagaagccag   4260
   tttgtttggc catgtgacaa ttgtcctgtg tttttatagc acccaaatca ttctaaaata   4320
   tgaacatcta aaaactttgc taggagacta agaacctttg gagagataga tataagtacg   4380
   gtcaaaaaac aaaactgtgg gacttacatt tattttctat agtaatctgt tgtacatttt   4440
   aagaagtaaa actaggaatt taggagtgat gtgtgacatt tctgacatgg agttaccatc   4500
   cccacatgta tcacatactg tcatattccc acatgtatca cacatgtatt gtaaaatttt   4560
   gtagttttga tcacttgtga atttactgtt gatgtggtag ccacctgctg caatggttcc   4620
   tcttgtaggt gaataaatgt cttgtctacc caca                               4654
<210> 184
   <211> 3378
   <212> DNA
   <213> Artificial
<220>
   <223> Mutated Cyno sequence
<400> 184
   atggacagcc tggccagcct ggtgctgtgc ggagtgtccc tgctgctgag cggcacagtg 60
   gaaggcgcca tggacctgat cctgatcaac tccctgcccc tggtgtccga cgccgagaca 120
   agcctgacct gtatcgctag cggctggcac ccccacgagc ctatcaccat cggccgggac 180
   ttcgaggccc tgatgaacca gcaccaggac cccctggaag tgacccagga cgtgacccgc 240
   gagtgggcca agaaagtcgt gtggaagaga gagaaggcca gcaagatcaa cggcgcctac 300
   ttctgcgagg gcagagtgcg gggcgaggcc atcagaatcc ggacaatgaa gatgcggcag 360
   caggccagct tcctgcccgc caccctgacc atgaccgtgg acaagggcga caacgtgaac 420
   atcagcttca agaaggtgct gatcaaagaa gaggacgccg tcatctacaa gaacggcagc 480
   ttcatccaca gcgtgccccg gcacgaggtg cccgacatcc tggaagtgca tctgccccac 540
   gcccagcctc aggatgccgg cgtgtacagc gcccggtaca tcggcggcaa cctgttcacc 600
   agcgccttca cccggctgat cgtgcggaga tgcgaggccc agaaatgggg ccctgagtgt 660
   aaccggctgt gcaccgtgtg cgtgaacaac ggcgtgtgcc acgaggacac cggcgagtgc 720
   atctgtcctc ccggcttcat gggccggacc tgcgagaagg cctgcgagag acacaccttc 780
   ggcagaacct gcaaagagcg gtgtagcggc caggacggct gcaagagcta cgtgttctgc 840
   ctgcccgacc cctacggctg cagctgtgcc acaggctgga agggcctgca gtgcaacgag 900
   gcctgccacc acggcttcta cggccccgac tgcaagctgc ggtgcagctg cagcaacggc 960
   gagacatgcg accggttcca gggatgcctg tgcagccctg gcagacaggg actccagtgc 1020
   gagcgcgagg gcatccccag aatgaccccc aagatcgtgg acctgcccga tcacatcgaa 1080
   gtgaacagcg gcaagttcaa ccccatctgc aaggcctccg gctggcccct gcccaccaac 1140
   gaagagatga ccctggtcaa gcccgacggc accgtgctgc accccaagga cttcaaccac 1200
   accgaccact tcagcgtggc catcttcacc atccaccgga tcctgccccc cgactctggc 1260
   gtgtgggtct gcagcgccaa taccgtggcc ggcatggtgg aaaagccctt caacatcagc 1320
   gtgaaggtgc tgcccaagcc cctgaacgcc cccaacgtga tcgacaccgg ccacaacttc 1380
   gccgtgatca acatctccag cgagccctac ttcggcgacg gccccatcaa gagcaagaag 1440
   ctgctgtaca agcccgtgaa ccactacgag gcctggcggc acatccaggt cacaaacgag 1500
   atcgtgaccc tgaaccacct ggaaccccgg accgagtacg agctgtgcgt gcagctcgtg 1560
   cggaggggcg aaggcggcga aggacatcct ggccccgtgc gcagattcac caccgcctct 1620
   atcggcctgc ccccacccag aggcctgaac ctgctgccta agagccagac caccctgaac 1680
   ctgacctggc agcccatctt ccccagcagc gaggacgact tctacgtgga agtggaacgg 1740
   cggagcgtgc agaagtccga ccagcagaac atcaaggtgc ccggcaacct gaccagcgtg 1800
   ctgctgaaca acctgcaccc ccgcgagcag tacgtcgtgc gggccagagt gaacaccaag 1860
   gcccagggcg agtggtccga ggatctgacc gcctggaccc tgagcgacat cctgcctccc 1920
   cagcccgaga atatcaaaat cagcaacatc acccacagca gcgccgtgat ctcttggacc 1980
   atcctggacg gctacagcat cagctccatc accatccggt acaaggtgca gggcaagaac 2040
   gaggaccagc acatcgacgt gaagatcaag aacgccacca tcacccagta ccagctgaag 2100
   ggcctggaac ccgagacagc ctaccaggtg gacatcttcg ccgagaacaa tatcggcagc 2160
   agcaaccccg ccttcagcca cgagctggtc accctgcccg agtctgaggc ccctgctgat 2220
   ctgggcggag gcaagatgct gctgatcgcc atcctgggca gcgccggcat gacctgtctg 2280
   acagtgctgc tggccttcct gatcatcctg cagctgaagc gggccaacgt gcagcggaga 2340
   atggcccagg ccttccagaa cgtgcgcgag gaacccgccg tgcagttcaa cagcggcacc 2400
   ctggccctga accggaaagt gaagaacaac cccgacccca ccatctaccc cgtgctggac 2460
   tggaacgaca tcaagttcca ggatgtgatc ggcgagggca acttcggcca ggtgctgaag 2520
   gcccggatca agaaagacgg cctgcggatg gacgccgcca tcaagcggat gaaggaatac 2580
   gccagcaagg acgaccacag agacttcgcc ggggagctgg aagtgctgtg caagctgggc 2640
   caccacccca acatcatcaa cctgctgggc gcctgcgagc accggggcta tctgtacctg 2700
   gccatcgagt acgcccccca cggcaatctg ctggatttcc tgcggaagtc ccgggtgctg 2760
   gaaaccgacc ctgccttcgc cattgccaac agcaccgcca gcaccctgag cagccagcag   2820
   ctcctgcact tcgccgccga tgtggccaga ggcatggact acctgagcca gaagcagttc   2880
   atccaccggg acctggccgc cagaaacatc ctcgtgggcg agaactacgt ggccaagatc   2940
   gccgacttcg gcctgagcag aggccaggaa gtgtacgtca agaaaaccat gggccggctg   3000
   cccgtgcggt ggatggccat tgagagcctg aactacagcg tgtacaccac caacagcgac   3060
   gtgtggtcct acggggtgct gctgtgggag atcgtgtctc tgggcggcac cccctactgc   3120
   ggcatgacat gcgccgagct gtacgagaag ctgccccagg gctaccggct ggaaaagcct   3180
   ctgaactgcg acgacgaggt gttcgacctg atgcggcagt gctggcgcga gaagccctac   3240
   gagcggccta gcttcgccca gatcctggtg tccctgaaca gaatgctgga agaacgcaag   3300
   acctacgtga acaccaccct gtatgagaag ttcacctacg ccggcatcga ctgcagcgcc   3360
   gaagaggccg cttgatga                                                 3378
<210> 185
   <211> 1491
   <212> DNA
   <213> Mouse
<400> 185
   atgtggcaga tcattttcct aacttttggc tgggatcttg tcttggcctc agcctacagt      60
   aactttagga agagcgtgga cagcacaggc agaaggcagt accaggtcca gaacggaccc     120
   tgcagctaca cgttcctgct gccggagacc gacagctgcc gatcttcctc cagcccctac     180
   atgtccaatg ccgtgcagag ggatgcaccc ctcgactacg acgactcagt gcaaaggctg     240
   caggtgctgg agaacattct agagaacaac acacagtggc tgatgaagct ggagaattac     300
   attcaggaca acatgaagaa ggagatggtg gagatccaac agaatgtggt gcagaaccag     360
   acagctgtga tgatagagat tggaaccagc ttgctgaacc agacagcagc acaaactcgg     420
   aaactgactg atgtggaagc ccaagtacta aaccagacga caagactcga gctgcagctt     480
   ctccaacatt ctatttctac caacaaattg gaaaagcaga ttttggatca gaccagtgaa     540
   ataaacaagc tacaaaataa gaacagcttc ctagaacaga aagttctgga catggagggc     600
   aagcacagcg agcagctaca gtccatgaag gagcagaagg acgagctcca ggtgctggtg     660
   tccaagcaga gctctgtcat tgacgagctg gagaagaagc tggtgacagc cacggtcaac     720
   aactcgctcc ttcagaagca gcagcatgac ctaatggaga ccgtcaacag cttgctgacc     780
   atgatgtcat cacccaactc caagagctcg gttgctatcc gtaaagaaga gcaaaccacc     840
   ttcagagact gtgcggaaat cttcaagtca ggactcacca ccagtggcat ctacacactg     900
   accttcccca actccacaga ggagatcaag gcctactgtg acatggacgt gggtggagga     960
   gggtggacag tcatccaaca ccgagaagat ggcagtgtgg acttccagag gacgtggaaa    1020
   gaatacaaag agggcttcgg gagccctctg ggagagtact ggctgggcaa tgagtttgtc    1080
   tcccagctga ccggtcagca ccgctacgtg cttaagatcc agctgaagga ctgggaaggc    1140
   aacgaggcgc attcgctgta tgatcacttc tacctcgctg gtgaagagtc caactacagg    1200
   attcacctta caggactcac ggggaccgcg ggcaaaataa gtagcatcag ccaaccagga    1260
   agtgatttta gcacaaagga ttcggacaat gacaaatgca tctgcaagtg ttcccagatg    1320
   ctctcaggag gctggtggtt tgacgcatgt ggtccttcca acttgaatgg acagtactac    1380
   ccacaaaaac agaatacaaa taagtttaac ggtatcaagt ggtactactg gaaggggtcc    1440
   ggctactcgc tcaaggccac aaccatgatg atccggccag cagatttcta a              1491
<210> 186
   <211> 1488
   <212> DNA
   <213> Cyno
<400> 186
   atgtggcaga ttgttttctt taccctgagc tgtgatcttg tcttggccgc agcctataac      60
   aactttcgga agagcatgga cagcatagga aagaagcagt atcaggtcca gcatgggtcc     120
   tgcagctaca ctttcctcct gccagagatg gacaactgcc gctcttcctc cagcccctac     180
   gtgtccaatg ctgtgcagag ggacgcgccg ctagaatatg atgactcggt gcagaggctg     240
   caagtgctgg agaacatcat ggaaaacaac actcagtggc taatgaagct tgagaattat     300
   atccaggaca acatgaaaaa agaaatggta gagatacagc agaacgcagt acagaaccag     360
   acggctgtga tgatagaagt agtgacgaac ctgttgaacc aaacggcgga gcaaacgcgg     420
   aagttaactg atgtggaagc ccaagtatta aaccagacca caagacttga acttcagctc     480
   ttggaacact ccctctcgac aaacaaattg gaaaaacaga ttttggacca gaccagtgaa     540
   ataaacaaat tgcaagataa gaacagtttc ctagaaaaga aggtgctagc tatggaagac     600
   aagcacatca tccaactaca gtcaatcaaa gaagagaaag atcagctgca ggtgttagta     660
   tccaagcaaa attccatcat tgaagaacta gaaaaaaaaa tagtgactgc cacggtgaat     720
   aattcagttc ttcagaagca gcaacatgat ctcatggaga cagttaataa cttactgact     780
   atgatgtcca catcaaactc taaggacccc actgttgcta aggaagaaca aatcagcttc     840
   agagactgtg ctgaagtgtt caaatcagga cacaccacga atggcgtcta cacgttaaca     900
   ctccccaact ctacagaaga ggtcaaggcc tactgtgaca tggaagctgg tggaggcggg     960
   tggacaatta ttcagcgacg tgaggacggc agcgttgact ttcagaggac ttggaaagaa    1020
   tataaagtgg gatttggtaa cccttcagga gaatattggc tgggaaatga gtttgtttca    1080
   caactgacta atcagcaacg ctatgtgctt aaaatacacc ttaaagactg ggaagggaac    1140
   gaggcttact cattgtatga acatttctat ctctcaagtg aagaactcaa ttataggatt    1200
   caccttaaag gacttacagg gacagccggc aaaataagca gcatcagtca accaggaaat    1260
   gattttagca caaaggatgc agacaacgac aaatgtattt gcaaatgttc acaaatgcta    1320
   acaggaggct ggtggtttga tgcatgtggt ccttccaact tgaacggaat gtactatcca    1380
   caaaggcaga acacaaataa gttcaacggc attaagtggt actactggaa aggctcaggc    1440
   tactcgctca agggcacaac catgatgatc cgaccggcag atttctaa                 1488
<210> 187
   <211> 495
   <212> PRT
   <213> Cyno
<400> 187
   Met Trp Gln Ile Val Phe Phe Thr Leu Ser Cys Asp Leu Val Leu Ala
   1               5                   10                  15
   Ala Ala Tyr Asn Asn Phe Arg Lys Ser Met Asp Ser Ile Gly Lys Lys
               20                  25                  30
   Gln Tyr Gln Val Gln His Gly Ser Cys Ser Tyr Thr Phe Leu Leu Pro
           35                  40                  45
   Glu Met Asp Asn Cys Arg Ser Ser Ser Ser Pro Tyr Val Ser Asn Ala
       50                  55                  60
   Val Gln Arg Asp Ala Pro Leu Glu Tyr Asp Asp Ser Val Gln Arg Leu
   65                  70                  75                  80
   Gln Val Leu Glu Asn Ile Met Glu Asn Asn Thr Gln Trp Leu Met Lys
                   85                  90                  95
   Leu Glu Asn Tyr Ile Gln Asp Asn Met Lys Lys Glu Met Val Glu Ile
               100                 105                 110
   Gln Gln Asn Ala Val Gln Asn Gln Thr Ala Val Met Ile Glu Val Val
           115                 120                 125
   Thr Asn Leu Leu Asn Gln Thr Ala Glu Gln Thr Arg Lys Leu Thr Asp
       130                 135                 140
   Val Glu Ala Gln Val Leu Asn Gln Thr Thr Arg Leu Glu Leu Gln Leu
   145                 150                 155                 160
   Leu Glu His Ser Leu Ser Thr Asn Lys Leu Glu Lys Gln Ile Leu Asp
                   165                 170                 175
   Gln Thr Ser Glu Ile Asn Lys Leu Gln Asp Lys Asn Ser Phe Leu Glu
   180 185 190
   Lys Lys Val Leu Ala Met Glu Asp Lys His Ile Ile Gln Leu Gln Ser
   195 200 205
   Ile Lys Glu Glu Lys Asp Gln Leu Gln Val Leu Val Ser Lys Gln Asn
   210 215 220
   Ser Ile Ile Glu Glu Leu Glu Lys Lys Ile Val Thr Ala Thr Val Asn
   225 230 235 240
   Asn Ser Val Leu Gln Lys Gln Gln His Asp Leu Met Glu Thr Val Asn
   245 250 255
   Asn Leu Leu Thr Met Met Ser Thr Ser Asn Ser Lys Asp Pro Thr Val
   260 265 270
   Ala Lys Glu Glu Gln Ile Ser Phe Arg Asp Cys Ala Glu Val Phe Lys
   275 280 285
   Ser Gly His Thr Thr Asn Gly Val Tyr Thr Leu Thr Leu Pro Asn Ser
   290 295 300
   Thr Glu Glu Val Lys Ala Tyr Cys Asp Met Glu Ala Gly Gly Gly Gly
   305 310 315 320
   Trp Thr Ile Ile Gln Arg Arg Glu Asp Gly Ser Val Asp Phe Gln Arg
   325 330 335
   Thr Trp Lys Glu Tyr Lys Val Gly Phe Gly Asn Pro Ser Gly Glu Tyr
   340 345 350
   Trp Leu Gly Asn Glu Phe Val Ser Gln Leu Thr Asn Gln Gln Arg Tyr
   355 360 365
   Val Leu Lys Ile His Leu Lys Asp Trp Glu Gly Asn Glu Ala Tyr Ser
   370 375 380
   Leu Tyr Glu His Phe Tyr Leu Ser Ser Glu Glu Leu Asn Tyr Arg Ile
   385 390 395 400
   His Leu Lys Gly Leu Thr Gly Thr Ala Gly Lys Ile Ser Ser Ile Ser
   405 410 415
   Gln Pro Gly Asn Asp Phe Ser Thr Lys Asp Ala Asp Asn Asp Lys Cys
   420 425 430
   Ile Cys Lys Cys Ser Gln Met Leu Thr Gly Gly Trp Trp Phe Asp Ala
   435 440 445
   Cys Gly Pro Ser Asn Leu Asn Gly Met Tyr Tyr Pro Gln Arg Gln Asn
   450 455 460
   Thr Asn Lys Phe Asn Gly Ile Lys Trp Tyr Tyr Trp Lys Gly Ser Gly
   465 470 475 480
   Tyr Ser Leu Lys Gly Thr Thr Met Met Ile Arg Pro Ala Asp Phe
   485 490 495
<210> 188
   <211> 496
   <212> PRT
   <213> Mouse
<400> 188
   Met Trp Gln Ile Ile Phe Leu Thr Phe Gly Trp Asp Leu Val Leu Ala
   1 5 10 15
   Ser Ala Tyr Ser Asn Phe Arg Lys Ser Val Asp Ser Thr Gly Arg Arg
   20 25 30
   Gln Tyr Gln Val Gln Asn Gly Pro Cys Ser Tyr Thr Phe Leu Leu Pro
   35 40 45
   Glu Thr Asp Ser Cys Arg Ser Ser Ser Ser Pro Tyr Met Ser Asn Ala
   50 55 60
   Val Gln Arg Asp Ala Pro Leu Asp Tyr Asp Asp Ser Val Gln Arg Leu
   65 70 75 80
   Gln Val Leu Glu Asn Ile Leu Glu Asn Asn Thr Gln Trp Leu Met Lys
   85 90 95
   Leu Glu Asn Tyr Ile Gln Asp Asn Met Lys Lys Glu Met Val Glu Ile
   100 105 110
   Gln Gln Asn Val Val Gln Asn Gln Thr Ala Val Met Ile Glu Ile Gly
   115 120 125
   Thr Ser Leu Leu Asn Gln Thr Ala Ala Gln Thr Arg Lys Leu Thr Asp
   130 135 140
   Val Glu Ala Gln Val Leu Asn Gln Thr Thr Arg Leu Glu Leu Gln Leu
   145 150 155 160
   Leu Gln His Ser Ile Ser Thr Asn Lys Leu Glu Lys Gln Ile Leu Asp
                   165                 170                 175
   Gln Thr Ser Glu Ile Asn Lys Leu Gln Asn Lys Asn Ser Phe Leu Glu
               180                 185                 190
   Gln Lys Val Leu Asp Met Glu Gly Lys His Ser Glu Gln Leu Gln Ser
           195                 200                 205
   Met Lys Glu Gln Lys Asp Glu Leu Gln Val Leu Val Ser Lys Gln Ser
       210                 215                 220
   Ser Val Ile Asp Glu Leu Glu Lys Lys Leu Val Thr Ala Thr Val Asn
   225                 230                 235                 240
   Asn Ser Leu Leu Gln Lys Gln Gln His Asp Leu Met Glu Thr Val Asn
                   245                 250                 255
   Ser Leu Leu Thr Met Met Ser Ser Pro Asn Ser Lys Ser Ser Val Ala
               260                 265                 270
   Ile Arg Lys Glu Glu Gln Thr Thr Phe Arg Asp Cys Ala Glu Ile Phe
           275                 280                 285
   Lys Ser Gly Leu Thr Thr Ser Gly Ile Tyr Thr Leu Thr Phe Pro Asn
       290                 295                 300
   Ser Thr Glu Glu Ile Lys Ala Tyr Cys Asp Met Asp Val Gly Gly Gly
   305                 310                 315                 320
   Gly Trp Thr Val Ile Gln His Arg Glu Asp Gly Ser Val Asp Phe Gln
                   325                 330                 335
   Arg Thr Trp Lys Glu Tyr Lys Glu Gly Phe Gly Ser Pro Leu Gly Glu
               340                 345                 350
   Tyr Trp Leu Gly Asn Glu Phe Val Ser Gln Leu Thr Gly Gln His Arg
           355                 360                 365
   Tyr Val Leu Lys Ile Gln Leu Lys Asp Trp Glu Gly Asn Glu Ala His
       370                 375                 380
   Ser Leu Tyr Asp His Phe Tyr Leu Ala Gly Glu Glu Ser Asn Tyr Arg
   385                 390                 395                 400
   Ile His Leu Thr Gly Leu Thr Gly Thr Ala Gly Lys Ile Ser Ser Ile
                   405                 410                 415
   Ser Gln Pro Gly Ser Asp Phe Ser Thr Lys Asp Ser Asp Asn Asp Lys
   420 425 430
   Cys Ile Cys Lys Cys Ser Gln Met Leu Ser Gly Gly Trp Trp Phe Asp
   435 440 445
   Ala Cys Gly Pro Ser Asn Leu Asn Gly Gln Tyr Tyr Pro Gln Lys Gln
   450 455 460
   Asn Thr Asn Lys Phe Asn Gly Ile Lys Trp Tyr Tyr Trp Lys Gly Ser
   465 470 475 480
   Gly Tyr Ser Leu Lys Ala Thr Thr Met Met Ile Arg Pro Ala Asp Phe
   485 490 495
<210> 189
   <211> 253
   <212> PRT
   <213> Mouse
<400> 189
   Met Glu Thr Asp Thr Leu Leu Leu Trp Val Leu Leu Leu Trp Val Pro
   1 5 10 15
   Gly Ser Thr Gly Asp Asp Tyr Lys Asp Asp Asp Asp Lys Gly Ile Leu
   20 25 30
   Glu Glu Gln Thr Thr Phe Arg Asp Cys Ala Glu Ile Phe Lys Ser Gly
   35 40 45
   Leu Thr Thr Ser Gly Ile Tyr Thr Leu Thr Phe Pro Asn Ser Thr Glu
   50 55 60
   Glu Ile Lys Ala Tyr Cys Asp Met Asp Val Gly Gly Gly Gly Trp Thr
   65 70 75 80
   Val Ile Gln His Arg Glu Asp Gly Ser Val Asp Phe Gln Arg Thr Trp
   85 90 95
   Lys Glu Tyr Lys Glu Gly Phe Gly Ser Pro Leu Gly Glu Tyr Trp Leu
   100 105 110
   Gly Asn Glu Phe Val Ser Gln Leu Thr Gly Gln His Arg Tyr Val Leu
   115 120 125
   Lys Ile Gln Leu Lys Asp Trp Glu Gly Asn Glu Ala His Ser Leu Tyr
   130 135 140
   Asp His Phe Tyr Leu Ala Gly Glu Glu Ser Asn Tyr Arg Ile His Leu
   145 150 155 160
   Thr Gly Leu Thr Gly Thr Ala Gly Lys Ile Ser Ser Ile Ser Gln Pro
   165 170 175
   Gly Ser Asp Phe Ser Thr Lys Asp Ser Asp Asn Asp Lys Cys Ile Cys
   180 185 190
   Lys Cys Ser Gln Met Leu Ser Gly Gly Trp Trp Phe Asp Ala Cys Gly
   195 200 205
   Pro Ser Asn Leu Asn Gly Gln Tyr Tyr Pro Gln Lys Gln Asn Thr Asn
   210 215 220
   Lys Phe Asn Gly Ile Lys Trp Tyr Tyr Trp Lys Gly Ser Gly Tyr Ser
   225 230 235 240
   Leu Lys Ala Thr Thr Met Met Ile Arg Pro Ala Asp Phe
   245 250
<210> 190
   <211> 253
   <212> PRT
   <213> Cyno
<400> 190
   Met Glu Thr Asp Thr Leu Leu Leu Trp Val Leu Leu Leu Trp Val Pro
   1 5 10 15
   Gly Ser Thr Gly Asp Asp Tyr Lys Asp Asp Asp Asp Lys Gly Ile Leu
   20 25 30
   Glu Glu Gln Ile Ser Phe Arg Asp Cys Ala Glu Val Phe Lys Ser Gly
   35 40 45
   His Thr Thr Asn Gly Val Tyr Thr Leu Thr Leu Pro Asn Ser Thr Glu
   50 55 60
   Glu Val Lys Ala Tyr Cys Asp Met Glu Ala Gly Gly Gly Gly Trp Thr
   65 70 75 80
   Ile Ile Gln Arg Arg Glu Asp Gly Ser Val Asp Phe Gln Arg Thr Trp
   85 90 95
   Lys Glu Tyr Lys Val Gly Phe Gly Asn Pro Ser Gly Glu Tyr Trp Leu
   100 105 110
   Gly Asn Glu Phe Val Ser Gln Leu Thr Asn Gln Gln Arg Tyr Val Leu
           115                 120                 125
   Lys Ile His Leu Lys Asp Trp Glu Gly Asn Glu Ala Tyr Ser Leu Tyr
       130                 135                 140
   Glu His Phe Tyr Leu Ser Ser Glu Glu Leu Asn Tyr Arg Ile His Leu
   145                 150                 155                 160
   Lys Gly Leu Thr Gly Thr Ala Gly Lys Ile Ser Ser Ile Ser Gln Pro
                   165                 170                 175
   Gly Asn Asp Phe Ser Thr Lys Asp Ala Asp Asn Asp Lys Cys Ile Cys
               180                 185                 190
   Lys Cys Ser Gln Met Leu Thr Gly Gly Trp Trp Phe Asp Ala Cys Gly
           195                 200                 205
   Pro Ser Asn Leu Asn Gly Met Tyr Tyr Pro Gln Arg Gln Asn Thr Asn
       210                 215                 220
   Lys Phe Asn Gly Ile Lys Trp Tyr Tyr Trp Lys Gly Ser Gly Tyr Ser
   225                 230                 235                 240
   Leu Lys Gly Thr Thr Met Met Ile Arg Pro Ala Asp Phe
                   245                 250